# EUROPEAN PATENT APPLICATION

(11) **EP 4 082 614 A1**
(43) Date of publication of application: **02.11.2022**
(21) Application number: 20905600.1
(22) Date of filing: 25.12.2020
(51) Int. Cl.: A61P 13/12, A61P 25/00, A61P 43/00, C07D 413/12, C07D 413/14, C07D 491/048, C07D 491/107, C07D 263/48, C07D 487/04, C07D 487/08, C07D 498/08, C07D 498/10, A61K 31/421, A61K 31/422, A61K 31/454, A61K 31/496, A61K 31/519, A61K 31/5377, A61K 31/5386, A61K 31/541, A61K 31/553

(54) **ACYLSULFAMIDE COMPOUND AND PHARMACEUTICAL USE THEREFOR**

(30) Priority: 27.12.2019 JP 2019238920
(71) Applicant: Japan Tobacco Inc., Tokyo 105-6927 (JP)
(72) Inventor: OHBA, Yusuke, Takatsuki-shi, Osaka 569-1125 (JP); FURUKAWA, Takayuki, Takatsuki-shi, Osaka 569-1125 (JP); ADACHI, Kaoru, Takatsuki-shi, Osaka 569-1125 (JP); NISHIMARU, Tatsuya, Takatsuki-shi, Osaka 569-1125 (JP); SAKURAI, Kentaro, Takatsuki-shi, Osaka 569-1125 (JP); MASUO, Ritsuki, Takatsuki-shi, Osaka 569-1125 (JP); INAMI, Tasuku, Takatsuki-shi, Osaka 569-1125 (JP)
(74) Representative: Vossius & Partner Patentanwälte Rechtsanwälte mbB
(86) International application number: PCT/JP2020/048775
(87) International publication number: WO 2021/132577

(57) **Abstract**

An acylsulfamide compound, or a pharmaceutically acceptable salt thereof, having NLRP3 inflammasome inhibitory activity, a pharmaceutical composition comprising the same, and their medical use, etc., are provided.

A compound of Formula [Ia]: 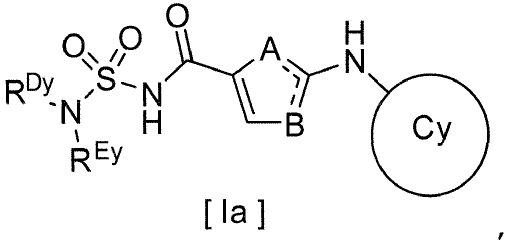 or a pharmaceutically acceptable salt thereof, wherein
a partial structure: 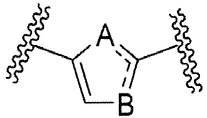 is: 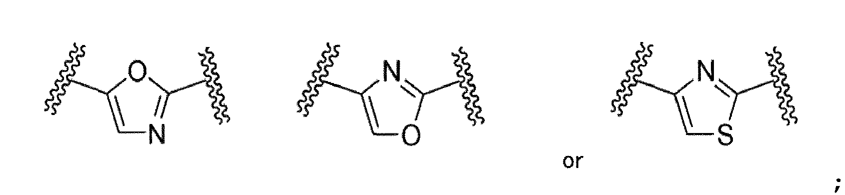 Ring Cy is an optionally substituted heteroaryl or phenyl;
R^{Dy} and R^{Ey} are each independently hydrogen, optionally substituted C₁₋₆ alkyl, optionally substituted C₁₋₄ haloalkyl, optionally substituted C₃₋₆ cycloalkyl, optionally substituted heterocycloalkyl, or optionally substituted heteroaryl, or alternatively, R^{Dy} and R^{Ey} may combine together with the nitrogen atom to which they attach to form heterocycloalkyl, etc.

## Description

### TECHNICAL FIELD

The present invention relates to an acylsulfamide compound, or a pharmaceutically acceptable salt thereof, having NLRP3 inflammasome inhibitory activity, a pharmaceutical composition comprising the same, and medical use thereof, etc.

### BACKGROUND ART

NLRP3 (NOD-, LRR-, and pyrin domain-containing protein 3) is a pattern recognition receptor that belongs to an NLR (NOD-like receptors) family, and is also expressed in non-immune cells such as glomerular epithelial cells and tubular epithelial cells as well as phagocytes such as macrophage and microglia.

NLRP3 recognizes DAMPs (Danger Associated Molecular Patterns) which are a molecular pattern specific to cellular damage factors, such as ATP, HMGB1, S100, urate crystals, and silica, and PAMPs (Pathogen Associated Molecular Patterns) which are a molecular pattern specific to pathogenic microorganisms, such as viruses, bacteria, and fungi, and binds to these molecules to be activated.

Activated NLRP3 associates with an adaptor protein, ASC (Apoptosis-associated speck-like protein containing a caspase recruitment domain), and a cysteine protease, caspase 1, by protein-protein interaction to form an NLRP3 inflammasome, which is a cellular protein complex. The formation of an NLRP3 inflammasome converts caspase 1 in the complex into its activated form, and the activated caspase 1 converts proIL1β, which is a precursor of a proinflammatory cytokine, IL-1β, into an activated form of IL-1β, while it also converts proIL-18, which is a precursor of IL-18, into an activated form of IL-18. The activated IL-1β secreted outside the cell induces proinflammatory cytokine-chemokine production by surrounding cells, and activates immune cells such as T cells, which causes inflammatory reactions.

In multiple sclerosis patients, the increase of the amount of DAMPs was observed in the brain and cerebral spinal fluid (Non Patent Literature 1), and the increase of the expression level of caspase 1 in involved sites and the increase of the amount of IL-1β in cerebral spinal fluid were also observed (Non Patent Literature 2). It has been reported that activated microglia was present in involved sites during the chronic progressive phase of this disease (Non Patent Literature 3), and the activated microglia stimulated by DAMPs produced proinflammatory cytokine such as IL-1β, which induced nerve inflammation and nerve disorder (Non Patent Literature 4). Thus, an NLRP3 inflammasome is considered to get involved in the expression of disease states of multiple sclerosis.

MOG₃₅₋₅₅EAE model mice prepared by sensitization of Myelin Oligodendrocyte Glycoprotein (MOG) expressed impairment of motor function as seen in multiple sclerosis. The onset of the impairment of motor function was inhibited in NLRP3-knockout mice in the MOG₃₅₋₅₅EAE model (Non Patent Literature 5). Demyelination of central nerve as seen in multiple sclerosis was expressed in cuprizone-model mice prepared by administration of a copper-chelate compound, cuprizone, to mice, while the progress of demyelination was delayed in NLRP3-knockout mice in the cuprizone model (Non Patent Literature 6). Administration of an NLPR3 inflammasome inhibitor, JC-171, after the onset inhibited the impairment of motor function in the MOG₃₅₋₅₅EAE model (Non Patent Literature 7). Thus, an NLRP3 inflammasome inhibitor is considered to become a drug for treating multiple sclerosis.

The increase of the expression of NLRP3 inflammasome-related genes has been reported in the kidney of patients suffering from chronic kidney disease (Non Patent Literatures 8, 9). Further, the inhibitory activity of proteinuria and tubulointerstitial fibrosis by NLRP3-knockout has been reported in a non-clinical chronic kidney disease model, i.e., a 5/6 kidney-enucleated model (Non Patent Literature 10). Accordingly, an NLRP3 inflammasome inhibitor is considered to become a drug for treating chronic kidney disease.

The increase of the expression of NLRP3 inflammasome-related genes has been reported in the intestine of patients suffering from inflammatory bowel disease (for example, ulcerative colitis and Crohn's disease) (Non Patent Literature 11). It has been reported that IL-1β produced by the activation of NLRP 3 was increased in the intestinal mucosa of IBD patients, and that the increased IL-1β secretion from the colonic region was positively correlated with the deterioration of the disease state (Non Patent Literature 11). It has also been reported that the dysfunction of CARD8, which negatively regulates inflammasome activity, increases susceptibility to Crohn's disease, and that the activation of NLRP3 inflammasome enhances IL-1β production from monocytes (Non Patent Literature 12). The suppression of intestinal pathology by NLRP3 deficiency has been reported in TNBS-induced colitis model, a colitis model (Non Patent Literature 13). Accordingly, an NLRP3 inflammasome inhibitor is considered to become a drug for treating inflammatory bowel disease.

The increase of the expression of NLRP3 inflammasome-related genes has been reported in the arteriosclerotic region of coronary arteries of patients suffering from myocardial infarction (Non Patent Literature 14). In addition, the suppressed lesion formation by NLRP3-knockout has been reported in low-density lipoprotein receptor (LDL) receptor-deficient mice fed high-fat diet, an arteriosclerosis model (Non Patent Literature 15). Accordingly, an NLRP3 inflammasome inhibitor is considered to become a drug for treating arteriosclerosis.

Cryopyrin-associated periodic syndrome (CAPS), a generic name of autoinflammatory diseases caused by activating mutation of NLRP3 gene, is classified into 3 disease types as follows: a mild disease type of familial cold autoinflammatory syndrome (FCAS),
a moderate disease type of Muckle-Wells syndrome (MWS), a severe disease type of chronic infantile neurologic cutaneous and articular syndrome (CINCA) / Neonatal onset multisystem inflammatory disease (NOMID) (Non Patent Literature 16). More than 200 mutations in NLRP3 gene have been reported in CAPS (Non Patent Literature 17). These NLRP3 gene mutations cause the formation and activation of NLRP3 inflammasome even in the absence of an activation signal. Mice expressing CAPS-related NLRP3 mutations exhibit systemic lethal inflammation dependent on IL-1β and IL-18 which are NLRP3 inflammasome and a downstream signal transduction molecule (Non Patent Literature 18). In a mouse strain expressing CAPS-related NLRP3 mutations, CY-09, an NLRP3 inflammasome inhibitor, suppressed systemic lethal inflammation and improved the survival (Non Patent Literature 19). Accordingly, an NLRP3 inflammasome inhibitor is considered to become a drug for treating CAPS.

The increase of the expression of NLRP3 inflammasome-related genes has been reported in liver tissues of patients suffering from nonalcoholic steato-hepatitis (NASH) (Non Patent Literature 20). In addition, the suppressed hepatic fibrogenesis by NLRP3-knockout has been reported in a choline deficient amino acid defined diet fed model, an NASH model (Non Patent Literature 20). Accordingly, an NLRP3 inflammasome inhibitor is considered to become a drug for treating NASH.

In gout and gouty arthritis, urate crystals deposited in the joint and periarticular tissues induce inflammation (Non Patent Literature 21). Urate crystals activate macrophage NLRP3 to produce IL-1β and IL-18 (Non Patent Literature 22). OLT1177, an NLRP3 inflammasome inhibitor, suppressed arthritis in an intra-articular urate-injected arthritis model (Non Patent Literature 23). Accordingly, an NLRP3 inflammasome inhibitor is considered to become a drug for treating gout and gouty arthritis.

The increase of the expression of NLRP3 inflammasome-related genes has been reported in joint synovium, peripheral-blood mononuclear cells of patients suffering from rheumatoid arthritis (Non Patent Literature 24). In addition, the increase of the expression of NLRP3 inflammasome-related genes in synovium has been reported in collagen-induced arthritis, a model of rheumatoid arthritis (Non Patent Literature 25). Accordingly, an NLRP3 inflammasome inhibitor is considered to become a drug for treating rheumatoid arthritis.

It has been reported that trinitrochlorobenzene, which induces contact dermatitis, increased IL-1β production from human skin keratinocytes via NLRP3 activation, and that NLRP3 knockout inhibits development of dermatitis in a trinitrochlorobenzene-induced dermatitis model, a model of contact dermatitis (Non Patent Literature 26). Accordingly, an NLRP3 inflammasome inhibitor is considered to become a drug for treating contact dermatitis.

The increase of the expression of NLRP3 inflammasome-related genes has been reported in the tear fluid and ocular surface of patients suffering from dry eye (Non Patent Literatures 27 and 28). In addition, it has been reported that increased expression of NLRP3 inflammasome-related genes and increased IL-1β production were observed when hypertonic stress was applied to cultured human corneal epithelial cells to induce a dry eye condition, and that IL-1β production was suppressed by knockdown of NLRP3 gene (Non Patent Literature 28). Accordingly, an NLRP3 inflammasome inhibitor is considered to become a drug for treating dry eye.

The increase of the expression of ASC domain of NLRP3 inflammasome has been reported in macrophages and neutrophils infiltrated into myocardial tissue of patients suffering from acute myocardial infarction (Non Patent Literature 29). In addition, it has been reported that the increased expression of NLRP3 inflammasome-related genes were observed in the infarct site in an ischemia-reperfusion model, a model of myocardial infarction, and that knockdown of NLRP3 gene decreased the infarct area and suppressed the reduction of myocardial contractility (Non Patent Literature 30). Accordingly, an NLRP3 inflammasome inhibitor is considered to become a drug for treating ischemic heart disease such as acute myocardial infarction.

It has been reported that the expression of IL-1β and IL-18 was increased in sera and glomeruli of patients with systemic lupus erythematosus (SLE) (Non Patent Literature 31, 32), and that the expression of NLRP3 gene and the production of IL-1β were increased in the macrophages (Non Patent Literature 33). In Nlrp3-R258W mice, which have an activating mutation of NLRP3 gene, lupus nephritis-like symptoms caused by pristane administration were exacerbated (Non Patent Literature 34). Accordingly, an NLRP3 inflammasome inhibitor is considered to become a drug for treating SLE.

### CITATION LIST

### NON PATENT LITERATURES

[Non Patent Literature 1] Andersson, A et al., Pivotal advance: HMGB1 expression in active lesions of human and experimental multiple sclerosis. J Leukoc Biol., 2008, Vol 84 (5), p.1248-55
[Non Patent Literature 2] Voet, S et al., A20 critically controls microglia activation and inhibits inflammasome-dependent neuroinflammation. Nat Commun., 2018, Vol 9(1), p2036.
[Non Patent Literature 3] Politis, M et al., Increased PK11195 PET binding in the cortex of patients with MS correlates with disability. Neurology, 2012, Vol 79(6), p523-30.
[Non Patent Literature 4] Hernandez-Pedro, N et al., PAMP-DAMPs interactions mediates development and progression of multiple sclerosis. Front Biosci (Schol Ed), 2016, Vol 8, p13-28.
[Non Patent Literature 5] Denis, G et al., NLRP3 Plays a Critical Role in the Development of Experimental Autoimmune Encephalomyelitis by Mediating Th1 and Th17 Responses. J Immunol., 2010, Vol 185 (2) p974-981
[Non Patent Literature 6] Jha, S et al., The inflammasome sensor, NLRP3, regulates CNS inflammation and demyelination via caspase-1 and interleukin-18. J Neurosci., 2010 Vol 30(47), p15811-20
[Non Patent Literature 7] Guo, C et al., Development and Characterization of a Hydroxyl-Sulfonamide Analogue, 5-Chloro-N-[2-(4-hydroxysulfamoyl-phenyl)-ethyl]-2-methoxy-benzamide, as a Novel NLRP3 Inflammasome Inhibitor for Potential Treatment of Multiple Sclerosis. ACS Chem Neurosci. , 2017, Vol 8(10), p2194-2201
[Non Patent Literature 8] Akosua Vilaysane et al., The NLRP3 Inflammasome Promotes Renal Inflammation and Contributes to CKD. J Am Soc Nephrol. 2010 Oct; 21(10): 1732-1744.
[Non Patent Literature 9] Shahzad K et al., Nlrp3-inflammasome activation in non-myeloid-derived cells aggravates diabetic nephropathy. Kidney Int. 2015 Jan;87(1):74-84.
[Non Patent Literature 10] Gong W et al., NLRP3 deletion protects against renal fibrosis and attenuates mitochondrial abnormality in mouse with 5/6 nephrectomy. Am J Physiol Renal Physiol. 2016 May 15;310(10):F1081-8
[Non Patent Literature 11] Ranson N et al., NLRP3-dependent and -independent processing Interleiukin-1β in active Ulcerative colitis. Int J mol Sci 2018: 20pii:E57.
[Non Patent Literature 12] Mao L et al., Loss-of-function CARD8 mutation causes NLRP3 inflammasome activation and Crohn's disease. J Clin Invest 2018: vol 128:1793-1806.
[Non Patent Literature 13] Bauer c. et al., Protective and aggravating effects of NLRP3 inlammasome activation in IBD models : influence of genetic and environmental factors. Dig.Dis 2012 vol 30 suppl 1 82-90.
[Non Patent Literature 14] Paramel V G et al., NLRP3 Inflammasome Expression and Activation in Human Atherosclerosis. J Am Heart Assoc. 2016 May 20;5(5):e003031. doi: 10.1161/JAHA.115.003031. PMID: 27207962; PMCID: PMC4889178.
[Non Patent Literature 15] Duewell P et al., NLRP3 inflammasomes are required for atherogenesis and activated by cholesterol crystals. Nature. 2010 Apr 29;464(7293) :1357-61. doi: 10.1038/nature08938. Erratum in: Nature. 2010 Jul 29;466(7306):652. PMID: 20428172; PMCID: PMC2946640.
[Non Patent Literature 16] Broderick L et al., The inflammasomes and autoinflammatory syndromes. Annu Rev Pathol. 2015;10:395-424. doi: 10.1146/annurev-pathol-012414-040431. Epub 2014 Nov 19. PMID: 25423351.
[Non Patent Literature 17] Sarrauste M C et al., INFEVERS: the Registry for FMF and hereditary inflammatory disorders mutations. Nucleic Acids Res. 2003 Jan 1;31(1):282-5. doi: 10.1093/nar/gkg031. PMID: 12520003; PMCID: PMC165478.
[Non Patent Literature 18] Brydges SD et al., Divergence of IL-1, IL-18, and cell death in NLRP3 inflammasomopathies. J Clin Invest. 2013 Nov;123(11):4695-705. doi: 10.1172/JCI71543. PMID: 24084736; PMCID: PMC3809806.
[Non Patent Literature 19] Jiang H et al., Identification of a selective and direct NLRP3 inhibitor to treat inflammatory disorders. J Exp Med. 2017 Nov 6;214(11):3219-3238. doi: 10.1084/jem.20171419. Epub 2017 Oct 11. PMID: 29021150; PMCID: PMC5679172.
[Non Patent Literature 20] Wree A et al., NLRP3 inflammasome activation is required for fibrosis development in NAFLD. J Mol Med (Berl). 2014 Oct;92(10):1069-82. doi: 10.1007/s00109-014-1170-1. Epub 2014 May 28. PMID: 24861026; PMCID: PMC4349416.
[Non Patent Literature 21] So AK et al., Inflammation in gout: mechanisms and therapeutic targets. Nat Rev Rheumatol. 2017 Nov;13(11):639-647. doi: 10.1038/nrrheum.2017.155. Epub 2017 Sep 28. PMID: 28959043.
[Non Patent Literature 22] Martinon F et al., Gout-associated uric acid crystals activate the NALP3 inflammasome. Nature. 2006 Mar 9;440(7081):237-41. doi: 10.1038/nature04516. Epub 2006 Jan 11. PMID: 16407889.
[Non Patent Literature 23] Marchetti C et al., NLRP3 inflammasome inhibitor OLT1177 suppresses joint inflammation in murine models of acute arthritis. Arthritis Res Ther. 2018 Aug 3;20(1):169. doi: 10.1186/s13075-018-1664-2. PMID: 30075804; PMCID: PMC6091035.
[Non Patent Literature 24] Mathews RJ et al., Evidence of NLRP3-inflammasome activation in rheumatoid arthritis (RA); genetic variants within the NLRP3-inflammasome complex in relation to susceptibility to RA and response to anti-TNF treatment. Ann Rheum Dis. 2014 Jun;73(6):1202-10. doi: 10.1136/annrheumdis-2013-203276. Epub 2013 May 17. PMID: 23687262.
[Non Patent Literature 25] Zhang Y et al., NLRP3 Inflammasome Plays an Important Role in the Pathogenesis of Collagen-Induced Arthritis. Mediators Inflamm. 2016;2016:9656270. doi: 10.1155/2016/9656270. Epub 2016 Mar 2. PMID: 27034595; PMCID: PMC4807043.
[Non Patent Literature 26] Watanabe H et al., Activation of the IL-1beta-processing inflammasome is involved in contact hypersensitivity. J Invest Dermatol. 2007 Aug;127(8):1956-63. doi: 10.1038/sj.jid.5700819. Epub 2007 Apr 12. PMID: 17429439.
[Non Patent Literature 27] Niu L et al., Upregulation of NLRP3 Inflammasome in the Tears and Ocular Surface of Dry Eye Patients. PLoS One. 2015 May 11;10(5):e0126277. doi: 10.1371/journal.pone.0126277. PMID: 25962072; PMCID: PMC4427105.
[Non Patent Literature 28] Zheng Q et al., Reactive oxygen species activated NLRP3 inflammasomes initiate inflammation in hyperosmolarity stressed human corneal epithelial cells and environment-induced dry eye patients. Exp Eye Res. 2015 May;134:133-40. doi: 10.1016/j.exer.2015.02.013. Epub 2015 Feb 18. PMID: 25701684.
[Non Patent Literature 29] Kawaguchi M et al., Inflammasome activation of cardiac fibroblasts is essential for myocardial ischemia/reperfusion injury. Circulation. 2011 Feb 15;123(6):594-604. doi: 10.1161/CIRCULATIONAHA.110.982777. Epub 2011 Jan 31. PMID: 21282498.
[Non Patent Literature 30] Sandanger O et al., The NLRP3 inflammasome is up-regulated in cardiac fibroblasts and mediates myocardial ischaemia-reperfusion injury. Cardiovasc Res. 2013 Jul 1;99(1):164-74. doi: 10.1093/cvr/cvt091. Epub 2013 Apr 10. PMID: 23580606.
[Non Patent Literature 31] Dellalibera-Joviliano R et al., Kinins and cytokines in plasma and cerebrospinal fluid of patients with neuropsychiatric lupus. J Rheumatol. 2003 Mar;30(3):485-92. PMID: 12610806.
[Non Patent Literature 32] Tucci M et al., Glomerular accumulation of plasmacytoid dendritic cells in active lupus nephritis: role of interleukin-18. Arthritis Rheum. 2008 Jan;58(1):251-62. doi: 10.1002/art.23186. PMID: 18163476.
[Non Patent Literature 33] Yang CA et al., Sex-dependent differential activation of NLRP3 and AIM2 inflammasomes in SLE macrophages. Rheumatology (Oxford). 2015 Feb;54(2) :324-31. doi: 10.1093/rheumatology/keu318. Epub 2014 Aug 25. PMID: 25161312.
[Non Patent Literature 34] Lu A et al., Hyperactivation of the NLRP3 Inflammasome in Myeloid Cells Leads to Severe Organ Damage in Experimental Lupus. J Immunol. 2017 Feb 1;198(3):1119-1129. doi: 10.4049/jimmunol.1600659. Epub 2016 Dec 30. PMID: 28039299.

### SUMMARY OF INVENTION

The present invention provides an acylsulfamide compound, or a pharmaceutically acceptable salt thereof, having NLRP3 inflammasome inhibitory activity, a pharmaceutical composition comprising the same, and medical use thereof, etc. Specifically, the present invention includes the embodiments illustrated as follows.

### DESCRIPTION OF EMBODIMENTS

### Item 1

A compound of Formula [I]: 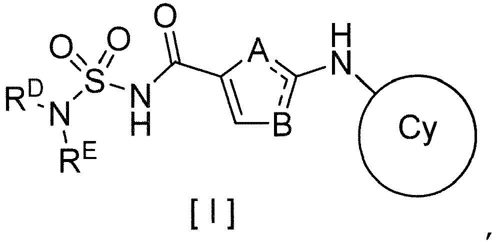 or a pharmaceutically acceptable salt thereof, wherein a partial structure: 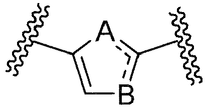 is: 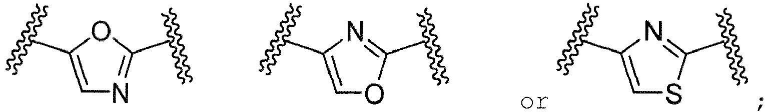 Ring Cy is 5- to 6-membered heteroaryl comprising 1 to 3 nitrogen atoms, or phenyl, wherein the heteroaryl or the phenyl is substituted with R^{F} at one of the atoms of α-position to the NH group directly attached to the partial structure, and may be optionally substituted with 1 to 4 R^{G}s;
R^{D} and R^{E} are each independently
   (1) hydrogen,
   (2) C₁₋₆ alkyl, wherein the alkyl may be optionally substituted with 1 to 3 R^{d1}s,
   (3) C₁₋₄ haloalkyl,
   (4) C₃₋₆ cycloalkyl, wherein the cycloalkyl may be optionally substituted with the same or different 1 to 3 halogen atoms,
   (5) 4- to 7-membered heterocycloalkyl comprising 1 to 3 heteroatoms independently selected from the group consisting of nitrogen and oxygen atoms, wherein the heterocycloalkyl may be optionally substituted with 1 to 3 R^{d2}s, or
   (6) 5- to 6-membered heteroaryl comprising 1 to 3 heteroatoms independently selected from the group consisting of nitrogen and oxygen atoms, wherein the heteroaryl may be optionally substituted with C₁₋₆ alkyl or C₁₋₄ haloalkyl, or alternatively,
R^{D} and R^{E} may combine together with the nitrogen atom to which they attach to form:
   (a) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 to 3 R^{s1}s and may be fused with a 5- to 6-membered heteroaromatic ring comprising 1 to 3 nitrogen atoms wherein the heteroaromatic ring may be optionally substituted with 1 or 2 R^{d3}s,
   (b) 7- to 11-membered spiro heterocycloalkyl comprising 1 to 3 heteroatoms independently selected from the group consisting of nitrogen and oxygen atoms, wherein the spiro heterocycloalkyl may be optionally substituted with 1 to 3 R^{d4}s and may be fused with a benzene ring,
   (c) 6- to 10-membered fused heterocycloalkyl comprising 1 or 2 heteroatoms independently selected from the group consisting of nitrogen and oxygen atoms, wherein the fused heterocycloalkyl may be optionally substituted with 1 to 3 R^{d5}s, or
   (d) 5- to 9-membered bridged heterocycloalkyl comprising 1 or 2 heteroatoms independently selected from the group consisting of nitrogen and oxygen atoms, wherein the bridged heterocycloalkyl may be optionally substituted with 1 to 3 R^{d6}s;
R^{F} is
   (1) halogen,
   (2) C₁₋₄ alkyl,
   (3) C₁₋₄ haloalkyl,
   (4) C₁₋₆ alkoxy, wherein the alkoxy may be optionally substituted with the same or different 1 to 3 halogen atoms, or
   (5) C₃₋₅ cycloalkyl,
R^{G} is each independently a substituent selected from the group consisting of:
   (1) halogen,
   (2) C₁₋₆ alkyl, wherein the alkyl may be optionally substituted with:
      (a) C₁₋₄ alkoxy,
      (b) cyano,
      (c) phenyl, or
      (d) 5- to 6-membered heteroaryl comprising 1 or 2 heteroatoms independently selected from the group consisting of nitrogen, oxygen, and sulfur atoms,
   (3) C₁₋₄ haloalkyl,
   (4) C₁₋₆ alkoxy, wherein the alkoxy may be optionally substituted with the same or different 1 to 3 halogen atoms,
   (5) cyano,
   (6) CONR^{G1}R^{G2}, wherein R^{G1} and R^{G2} are each independently
      (a) hydrogen,
      (b) C₁₋₆ alkyl, or
      (c) C₁₋₄ haloalkyl,
   (7) trialkylsilyl,
   (8) pentafluorosulfanyl,
   (9) C₃₋₇ cycloalkyl, wherein the cycloalkyl may be optionally substituted with 1 to 3 R^{s2}s,
   (10) C₅₋₁₃ spiro cycloalkyl, wherein the spiro cycloalkyl may be optionally substituted with 1 to 3 R^{s3}s,
   (11) 4- to 7-membered heterocycloalkyl comprising 1 or 2 heteroatoms independently selected from the group consisting of nitrogen and oxygen atoms, wherein the heterocycloalkyl may be optionally substituted with 1 to 3 R^{s4}s,
   (12) 7- to 11-membered spiro heterocycloalkyl comprising 1 or 2 heteroatoms independently selected from the group consisting of nitrogen and oxygen atoms, wherein the spiro heterocycloalkyl may be optionally substituted with 1 to 3 R^{s5}s,
   (13) phenyl, wherein the phenyl may be optionally substituted with 1 to 3 R^{s6}s, and
   (14) 5- to 6-membered heteroaryl comprising 1 to 3 heteroatoms independently selected from the group consisting of nitrogen, oxygen, and sulfur atoms, wherein the heteroaryl may be optionally substituted with 1 to 3 R^{s7}s, or alternatively,
in the case where R^{F} and R^{G} or two R^{G}s are substituted on neighboring atoms, then the R^{F} and R^{G} and/or the two R^{G}s may combine together with the atoms to which they attach to form:
   (a) a C₅₋₆ cycloalkene ring, wherein the cycloalkene ring may be optionally substituted with 1 to 3 R^{c1}s,
   (b) a 5- to 7-membered heterocycloalkene ring comprising 1 or 2 heteroatoms independently selected from the group consisting of nitrogen and oxygen atoms, wherein the heterocycloalkene ring may be optionally subsituted with 1 to 3 R^{c2}s,
   (c) a benzene ring, wherein the benzene ring may be optionally substituted with 1 to 3 R^{c3}s, or
   (d) a 5- to 7-membered heteroaromatic ring comprising 1 or 2 heteroatoms independently selected from the group consisting of nitrogen and oxygen atoms, wherein the heteroaromatic ring may be optionally substituted with 1 to 3 R^{c4}s,
      so that Ring Cy may form a bi- or tri-cyclic fused ring group;
R^{c1}, R^{c2}, R^{c3}, and R^{c4} are each independently a substituent selected from the group consisting of:
   (1) halogen,
   (2) C₁₋₆ alkyl,
   (3) C₁₋₄ haloalkyl, and
   (4) oxo;
R^{d1} is each independently a substituent selected from the group consisting of:
   (1) halogen,
   (2) hydroxy,
   (3) C₁₋₆ alkoxy, wherein the alkoxy may be optionally substituted with the same or different 1 to 3 halogen atoms,
   (4) cyano,
   (5) COOR^{e1}, wherein R^{e1} is hydroxy or C₁₋₆ alkyl,
   (6) CONR^{e2}R^{e3}, wherein R^{e2} and R^{e3} are independently
      (a) hydrogen,
      (b) C₁₋₆ alkyl, or
      (c) C₁₋₄ haloalkyl, or alternatively, R^{e2} and R^{e3} may combine together with the nitrogen atom to which they attach to form 5-to 6-membered heterocycloalkyl comprising 1 or 2 heteroatoms independently selected from the group consisting of nitrogen and oxygen atoms, wherein the heterocycloalkyl may be optionally substituted with the same or different 1 or 2 halogen atoms,
   (7) COR^{e4}, wherein R^{e4} is C₁₋₆ alkyl,
   (8) SO₂R^{e5}, wherein R^{e5} is C₁₋₆ alkyl,
   (9) NR^{e6}R^{e7}, wherein R^{e6} is
      (a) hydrogen,
      (b) C₁₋₆ alkyl, or
      (c) C₁₋₄ haloalkyl, and
         R^{e7} is
         (a) hydrogen,
         (b) C₁₋₆ alkyl,
         (c) C₁₋₄ haloalkyl,
         (d) COR^{e8}, wherein R^{e8} is C₁₋₆ alkyl, and the alkyl may be optionally substituted with 1 to 3 substituents independently selected from the group consisting of:
            (i) halogen,
            (ii) hydroxy,
            (iii)C₁₋₆ alkoxy, and
            (iv) cyano, or
         (e) SO₂R^{e9}, wherein R^{e9} is C₁₋₆ alkyl or C₁₋₄ haloalkyl,
   (10) SO₂NR^{f1}R^{f2}, wherein R^{f1} and R^{f2} are independently
      (a) hydrogen,
      (b) C₁₋₆ alkyl, or
      (c) C₁₋₄ haloalkyl,
   (11) C₃₋₆ cycloalkyl, wherein the cycloalkyl may be optionally substituted with hydroxy or C₁₋₆ alkoxy,
   (12) 4- to 6-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of halogen and oxo,
   (13) phenyl, and
   (14) 5- to 6-membered heteroaryl comprising 1 or 2 heteroatoms independently selected from the group consisting of nitrogen, oxygen, and sulfur atoms, wherein the heteroaryl may be optionally substituted with C₁₋₆ alkyl or C₁₋₄ haloalkyl;
R^{d2} is each independently a substituent selected from the group consisting of:
   (1) C₁₋₆ alkyl,
   (2) C₁₋₄ haloalkyl,
   (3) oxo, and
   (4) COR^{g1}, wherein R^{g1} is C₁₋₆ alkyl, and the alkyl may be optionally substituted with:
      (a) hydroxy,
      (b) C₁₋₆ alkoxy, and
      (c) cyano;
R^{d3} is each independently a substituent selected from the group consisting of:
   (1) C₁₋₆ alkyl, and
   (2) COOR^{g2}, wherein R^{g2} is hydroxy or C₁₋₆ alkyl;
R^{d4}, R^{d5}, and R^{d6} are each independently a substituent selected from the group consisting of:
   (1) halogen,
   (2) hydroxy,
   (3) oxo,
   (4) cyano,
   (5) C₁₋₆ alkyl,
   (6) C₁₋₄ haloalkyl,
   (7) C₁₋₆ alkoxy, wherein the alkoxy may be optionally substituted with the same or different 1 to 3 halogen atoms,
   (8) NR^{g3}R^{g4}, wherein R^{g3} and R^{g4} are independently
      (a) hydrogen,
      (b) C₁₋₆ alkyl, or
      (c) C₁₋₄ haloalkyl, and
   (9) 4- to 7-membered heterocycloalkyl comprising 1 or 2 heteroatoms independently selected from the group consisting of nitrogen and oxygen atoms, wherein the heterocycloalkyl may be optionally substituted with 1 or 2 substituents independently selected from the group consisting of:
      (a) halogen,
      (b) hydroxy,
      (c) C₁₋₆ alkyl,
      (d) C₁₋₆ alkoxy, wherein the alkoxy may be optionally substituted with the same or different 1 to 3 halogen atoms, and
      (e) oxo;
R^{s1}, R^{s2}, R^{s3}, R^{s4}, R^{s5}, R^{s6}, and R^{s7} are each independently a substituent selected from the group consisting of:
   (1) halogen,
   (2) hydroxy,
   (3) C₁₋₆ alkyl, wherein the alkyl may be optionally substituted with 1 to 3 R¹¹s,
   (4) C₁₋₆ alkoxy, wherein the alkoxy may be optionally substituted with the same or different 1 to 3 halogen atoms,
   (5) cyano,
   (6) OR^{t1}, wherein R^{t1} is C₃₋₆ cycloalkyl,
   (7) COR¹²,
   (8) SO₂R¹³,
   (9) NR^{t2}R^{t3}, wherein R^{t2} is
      (a) hydrogen,
      (b) C₁₋₆ alkyl, or
      (c) C₁₋₄ haloalkyl, and
      R^{t3} is
      (a) hydrogen,
      (b) C₁₋₆ alkyl,
      (c) C₁₋₄ haloalkyl,
      (d) COR¹⁴, or
      (e) SO₂R¹⁵,
   (10) CONR^{t4}R^{t5}, wherein R^{t4} and R^{t5} are independently
      (a) hydrogen,
      (b) C₁₋₆ alkyl, or
      (c) C₁₋₄ haloalkyl, or alternatively, R^{t4} and R^{t5} may combine together with the nitrogen atom to which they attach to form 4-to 6-membered heterocycloalkyl comprising 1 or 2 heteroatoms independently selected from the group consisting of nitrogen and oxygen atoms, wherein the heterocycloalkyl may be optionally substituted with the same or different 1 or 2 halogen or hydroxy,
   (11) SO₂NR^{t6}R^{t7}, wherein R^{t6} and R^{t7} are independently
      (a) hydrogen,
      (b) C₁₋₆ alkyl, or
      (c) C₁₋₄ haloalkyl,
   (12) COOR^{t8}, wherein R^{t8} is hydroxy or C₁₋₆ alkyl,
   (13) oxo,
   (14) C₃₋₆ cycloalkyl,
   (15) phenyl,
   (16) 4- to 7-membered heterocycloalkyl comprising 1 or 2 heteroatoms independently selected from the group consisting of nitrogen and oxygen atoms, wherein the heterocycloalkyl may be optionally substituted with 1 to 3 R¹⁶s, and
   (17) 5- to 6-membered heteroaryl comprising 1 to 3 heteroatoms independently selected from the group consisting of nitrogen, oxygen, and sulfur atoms, wherein the heteroaryl may be optionally substituted with 1 to 3 R¹⁷s;
R¹¹ is each independently a substituent selected from the group consisting of:
   (1) halogen,
   (2) hydroxy,
   (3) C₁₋₆ alkoxy, wherein the alkoxy may be optionally substituted with the same or different 1 to 3 halogen atoms,
   (4) cyano,
   (5) NR²¹R²², wherein R²¹ is
      (a) hydrogen,
      (b) C₁₋₆ alkyl, or
      (c) C₁₋₄ haloalkyl, and
      R²² is
      (a) hydrogen,
      (b) C₁₋₆ alkyl,
      (c) C₁₋₄ haloalkyl,
      (d) COR^{1a}, or
      (e) SO₂R^{1b},
   (6) COR²³, wherein R²³ is C₁₋₆ alkyl or C₁₋₄ haloalkyl,
   (7) SO₂R²⁴, wherein R²⁴ is C₁₋₆ alkyl or C₁₋₄ haloalkyl,
   (8) COOR²⁵, wherein R²⁵ is hydroxy or C₁₋₆ alkyl,
   (9) CONR²⁶R²⁷, wherein R²⁶ and R²⁷ are independently
      (a) hydrogen,
      (b) C₁₋₆ alkyl, or
      (c) C₁₋₄ haloalkyl, or alternatively, R²⁶ and R²⁷ may combine together with the nitrogen atom to which they attach to form 5-to 6-membered heterocycloalkyl comprising 1 or 2 heteroatoms independently selected from the group consisting of nitrogen and oxygen atoms, wherein the heterocycloalkyl may be optionally substituted with the same or different 1 or 2 halogen atoms,
   (10) SO₂NR²⁸R²⁹, wherein R²⁸ and R²⁹ are independently
      (a) hydrogen,
      (b) C₁₋₆ alkyl, or
      (c) C₁₋₄ haloalkyl, or alternatively, R²⁸ and R²⁹ may combine together with the nitrogen atom to which they attach to form 4-to 7-membered heterocycloalkyl comprising 1 or 2 heteroatoms independently selected from the group consisting of nitrogen and oxygen atoms, wherein the heterocycloalkyl may be optionally substituted with the same or different 1 or 2 halogen atoms,
   (11) C₃₋₆ cycloalkyl,
   (12) 4- to 6-membered heterocycloalkyl comprising 1 or 2 heteroatoms independently selected from the group consisting of nitrogen and oxygen atoms, wherein the heterocycloalkyl may be optionally substituted with 1 to 3 R^{1c}s,
   (13) phenyl, and
   (14) 5- to 6-membered heteroaryl comprising 1 or 2 heteroatoms independently selected from the group consisting of nitrogen and oxygen atoms;
R¹⁷ is each independently a substituent selected from the group consisting of:
   (1) halogen,
   (2) C₁₋₆ alkyl,
   (3) C₁₋₄ haloalkyl,
   (4) C₁₋₆ alkoxy,
   (5) cyano,
   (6) C₃₋₆ cycloalkyl, and
   (7) 4- to 6-membered heterocycloalkyl comprising an oxygen atom;
R¹², R¹³, R¹⁴, R¹⁵, R^{1a}, and R^{1b} are each independently a substituent selected from the group consisting of:
   (1) C₁₋₆ alkyl,
   (2) C₁₋₄ haloalkyl,
   (3) C₃₋₆ cycloalkyl,
   (4) 4- to 6-membered heterocycloalkyl comprising an oxygen atom,
   (5) phenyl, and
   (6) 5- to 6-membered heteroaryl comprising 1 or 2 heteroatoms independently selected from the group consisting of nitrogen and oxygen atoms;
R¹⁶ and R^{1c} are each independently a substituent selected from the group consisting of:
   (1) halogen,
   (2) hydroxy,
   (3) C₁₋₆ alkyl,
   (4) C₁₋₄ haloalkyl,
   (5) C₁₋₆ alkoxy, wherein the alkoxy may be optionally substituted with the same or different 1 to 3 halogen atoms, and
   (6) oxo.

### Item 2

The compound according to Item 1, or a pharmaceutically acceptable salt thereof, wherein Ring Cy is: 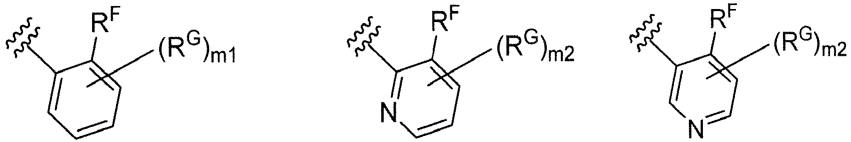 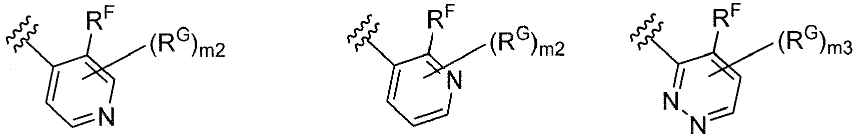 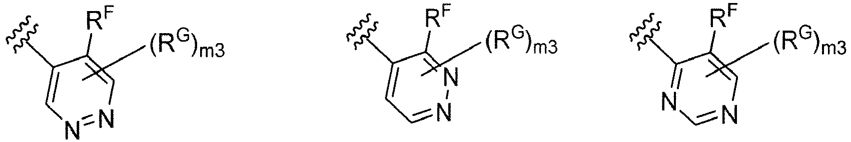 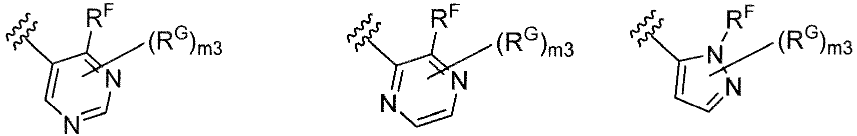 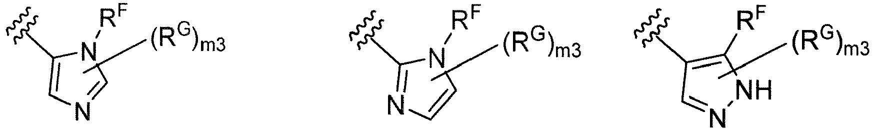 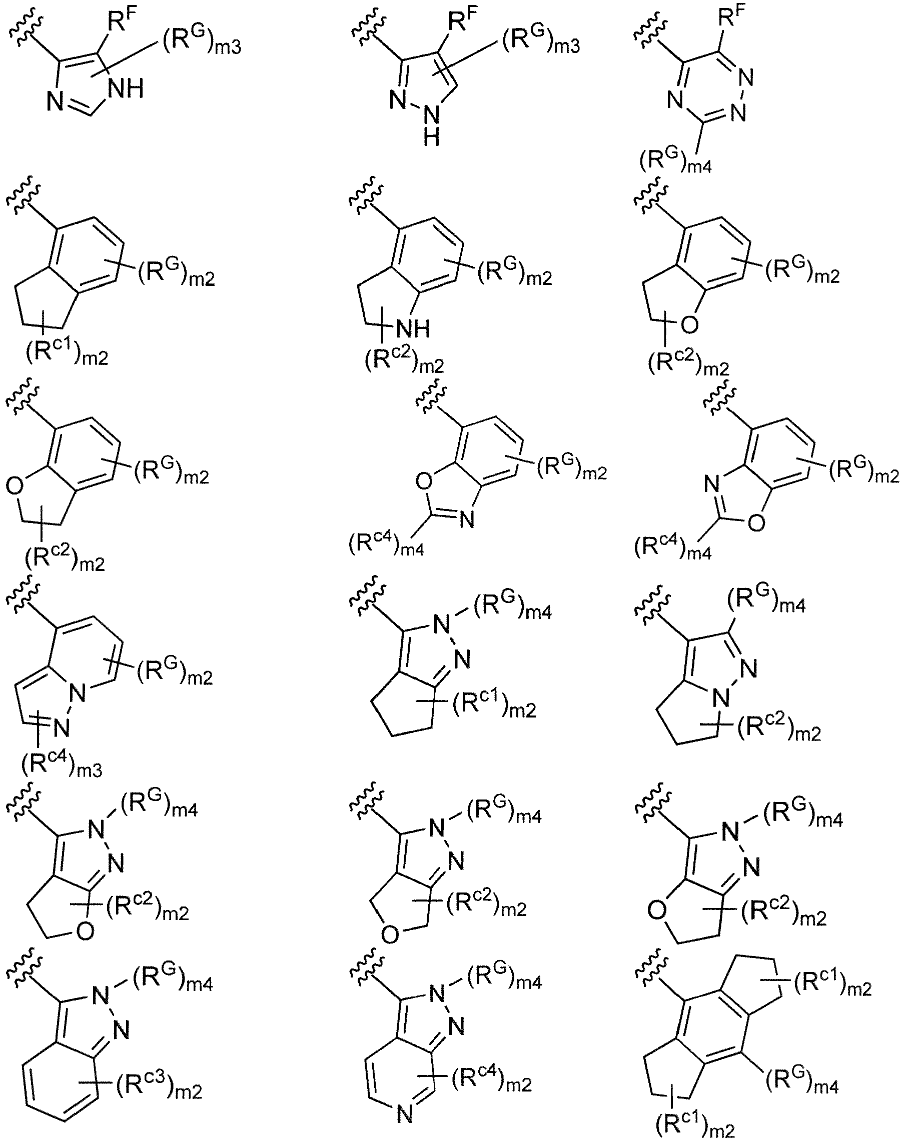 or 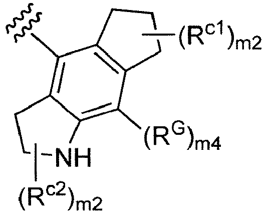 wherein m1 is an integer from 0 to 4,
m2 is each independently an integer from 0 to 3,
m3 is an integer from 0 to 2,
m4 is an integer of 0 or 1, and
R^{F}, R^{G}, R^{c1}, R^{c2}, R^{c3}, and R^{c4} are those as defined in Item 1.

### Item 3

The compound according to Item 1 or 2, or a pharmaceutically acceptable salt thereof, wherein Ring Cy is: 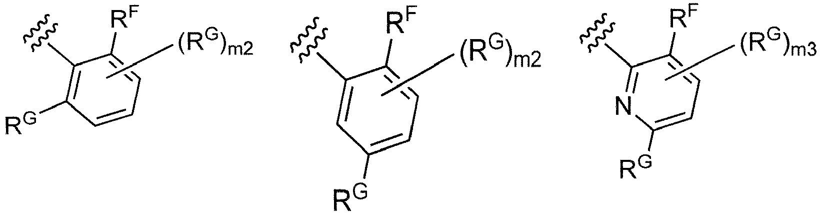 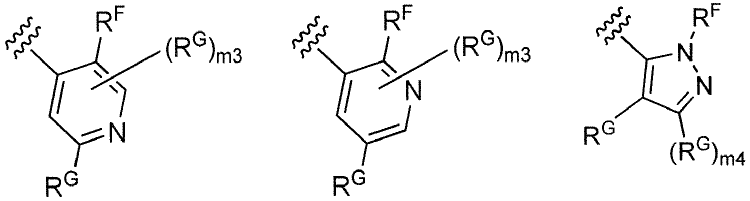 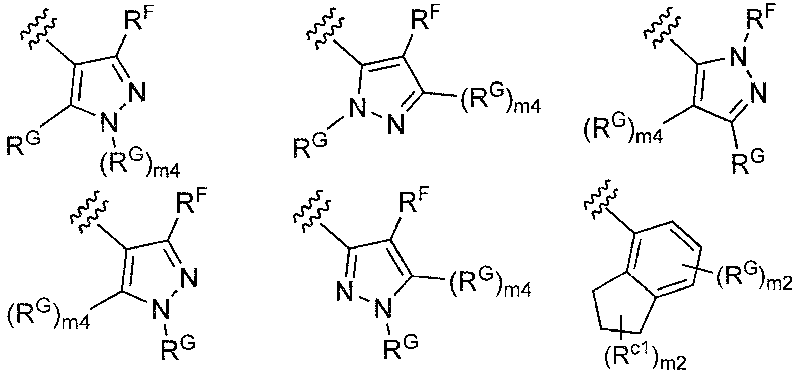 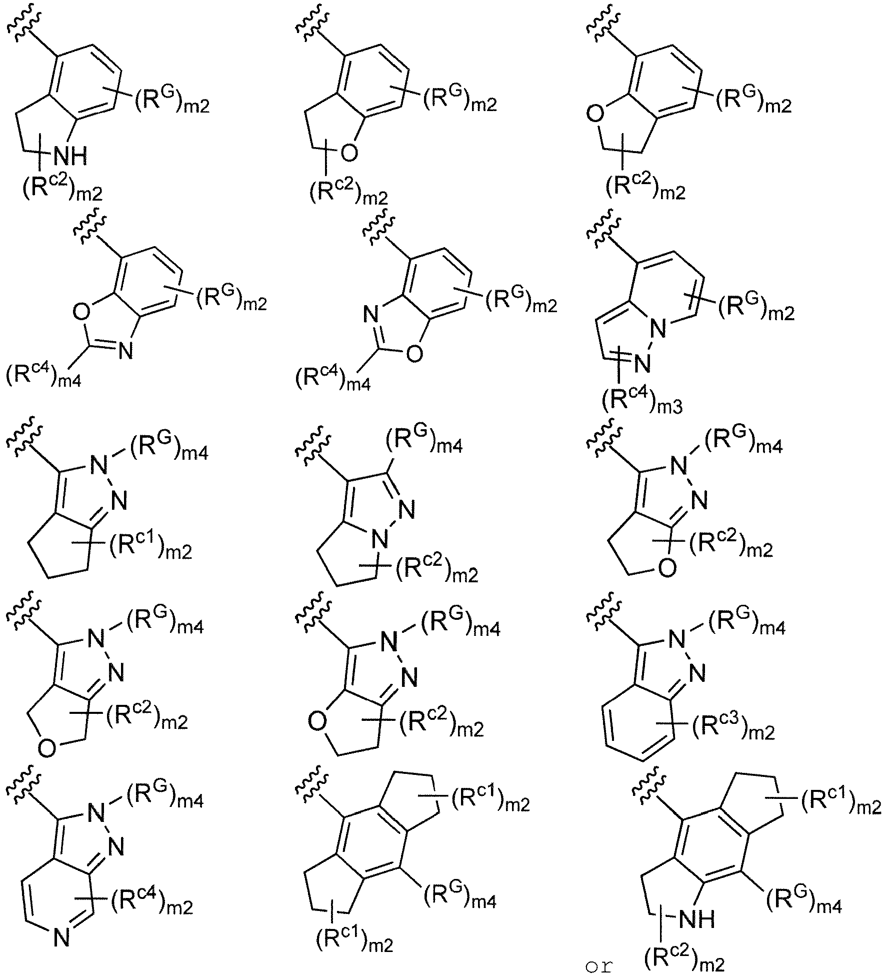 wherein m2 is each independently an integer from 0 to 3,
m3 is an integer from 0 to 2,
m4 is 0 or 1, and
R^{F}, R^{G}, R^{c1}, R^{c2}, R^{c3}, and R^{c4} are those as defined in Item 1.

### Item 4

The compound according to any one of Items 1 to 3, or a pharmaceutically acceptable salt thereof, wherein a partial structure: 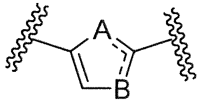 is a group of either the following formula:

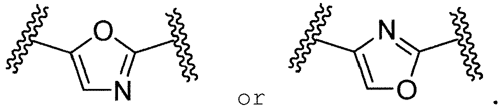

### Item 5

The compound according to any one of Items 1 to 3, or a pharmaceutically acceptable salt thereof, wherein a partial structure: 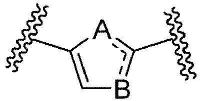 is a group of the following formula:

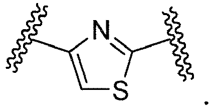

### Item 6

The compound according to any one of Items 1 to 5, or a pharmaceutically acceptable salt thereof, wherein R^{D} and R^{E} are each independently
(1) hydrogen,
(2) C₁₋₆ alkyl, wherein the alkyl may be optionally substituted with 1 to 3 R^{d1}s,
(3) C₁₋₄ haloalkyl,
(4) C₃₋₆ cycloalkyl, wherein the cycloalkyl may be optionally substituted with the same or different 1 to 3 halogen atoms,
(5) 4- to 7-membered heterocycloalkyl comprising 1 to 3 heteroatoms independently selected from the group consisting of nitrogen and oxygen atoms, wherein the heterocycloalkyl may be optionally substituted with 1 to 3 R^{d2}s, or
(6) 5- to 6-membered heteroaryl comprising 1 to 3 heteroatoms independently selected from the group consisting of nitrogen and oxygen atoms, wherein the heteroaryl may be optionally substituted with C₁₋₆ alkyl or C₁₋₄ haloalkyl.

### Item 7

The compound according to any one of Items 1 to 5, or a pharmaceutically acceptable salt thereof, wherein R^{D} and R^{E} combine together with the nitrogen atom to which they attach to form:
(1) 4- to 7-membered heterocycloalkyl comprising 1 or 2 heteroatoms independently selected from the group consisting of nitrogen, oxygen, and sulfur atoms, wherein the heterocycloalkyl may be optionally substituted with 1 to 3 R^{s1}s, and may be optionally fused with a 5- to 6-membered heteroaromatic ring comprising 1 to 3 nitrogen atoms, wherein the heteroaromatic ring may be optionally substituted with 1 or 2 R^{d3}s,
(2) 7- to 11-membered spiro heterocycloalkyl comprising 1 to 3 heteroatoms independently selected from the group consisting of nitrogen and oxygen atoms, wherein the spiro heterocycloalkyl may be optionally substituted with 1 to 3 R^{d4}s, and may be optionally fused with a benzene ring,
(3) 6- to 10-membered fused heterocycloalkyl comprising 1 or 2 heteroatoms independently selected from the group consisting of nitrogen and oxygen atoms, wherein the fused heterocycloalkyl may be optionally substituted with 1 to 3 R^{d5}s, or
(4) 5- to 9-membered bridged heterocycloalkyl comprising 1 or 2 heteroatoms independently selected from the group consisting of nitrogen and oxygen atoms, wherein the bridged heterocycloalkyl may be optionally substituted with 1 to 3 R^{d6}s.

### Item 8

A pharmaceutical composition comprising a compound according to any one of Items 1 to 7, or a pharmaceutically acceptable salt thereof, and a pharmaceutically acceptable carrier.

### Item 9

An NLRP3 inflammasome inhibitor comprising a compound according to any one of Items 1 to 7, or a pharmaceutically acceptable salt thereof.

### Item 10

A medicament for treating or preventing a disease selected from the group consisting of multiple sclerosis and chronic kidney disease, comprising a compound according to any one of Items 1 to 7, or a pharmaceutically acceptable salt thereof.

### Item 11

A method for inhibiting NLRP3 inflammasome, comprising administering a therapeutically effective amount of a compound according to any one of Items 1 to 7, or a pharmaceutically acceptable salt thereof, to a mammal.

### Item 12

A method for treating or preventing a disease selected from the group consisting of multiple sclerosis and chronic kidney disease, comprising administering a therapeutically effective amount of a compound according to any one of Items 1 to 7, or a pharmaceutically acceptable salt thereof, to a mammal.

### Item 13

Use of a compound according to any one of Items 1 to 7, or a pharmaceutically acceptable salt thereof, in the manufacture of an NLRP3 inflammasome inhibitor.

### Item 14

Use of a compound according to any one of Items 1 to 7, or a pharmaceutically acceptable salt thereof, in the manufacture of a medicament for treating or preventing a disease selected from the group consisting of multiple sclerosis and chronic kidney disease.

### Item 15

A compound according to any one of Items 1 to 7, or a pharmaceutically acceptable salt thereof, for use in inhibiting NLRP3 inflammasome.

### Item 16

A compound according to any one of Items 1 to 7, or a pharmaceutically acceptable salt thereof, for use in treating or preventing a disease selected from the group consisting of multiple sclerosis and chronic kidney disease.

### Item 17

A compound of Formula [Ia]: 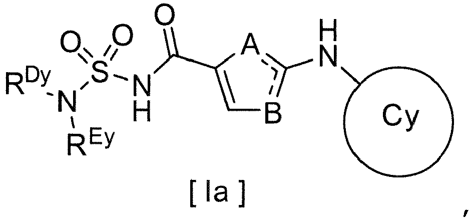 or a pharmaceutically acceptable salt thereof, wherein
a partial structure: 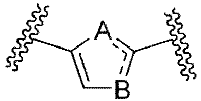 is: 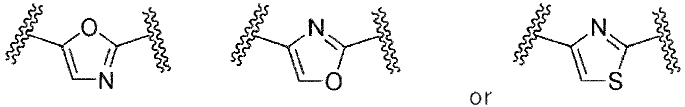
Ring Cy is 5- to 6-membered heteroaryl comprising 1 to 3 nitrogen atoms, or phenyl, wherein the heteroaryl or the phenyl is substituted with R^{F} at one of the atoms of α-position to a Cy ring-constituting atom attached to the NH group directly attached to the partial structure, and may be optionally substituted with the same or different 1 to 4 R^{G}s;
R^{D y} and R^{E y} are each independently
   (1) hydrogen,
   (2) C₁₋₆ alkyl, wherein the alkyl may be optionally substituted with the same or different 1 to 3 R^{d1}s,
   (3) C₁₋₄ haloalkyl,
   (4) C₃₋₆ cycloalkyl, wherein the cycloalkyl may be optionally substituted with the same or different 1 to 3 halogen atoms,
   (5) 4- to 7-membered heterocycloalkyl comprising 1 to 3 heteroatoms independently selected from the group consisting of nitrogen and oxygen atoms, wherein the heterocycloalkyl may be optionally substituted with the same or different 1 to 3 R^{d2}s, or
   (6) 5- to 6-membered heteroaryl comprising 1 to 3 heteroatoms independently selected from the group consisting of nitrogen and oxygen atoms, wherein the heteroaryl may be optionally substituted with C₁₋₆ alkyl or C₁₋₄ haloalkyl, or alternatively, R^{D y} and R^{E y} may combine together with the nitrogen atom to which they attach to form
      (a) 4- to 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s 1 y} s and / or may be fused with a 5- to 6-membered heteroaromatic ring comprising 1 to 3 nitrogen atoms wherein the heteroaromatic ring may be optionally substituted with the same or different 1 or 2 R^{d3}s,
      (b) 7- to 11-membered spiro heterocycloalkyl comprising 1 to 3 heteroatoms independently selected from the group consisting of nitrogen and oxygen atoms, wherein the spiro heterocycloalkyl may be optionally substituted with the same or different 1 to 3 R^{d4}s and/or may be optionally fused with a benzene ring,
      (c) 6- to 10-membered fused heterocycloalkyl comprising 1 or 2 heteroatoms independently selected from the group consisting of nitrogen and oxygen atoms, wherein the fused heterocycloalkyl may be optionally substituted with the same or different 1 to 3 R^{d5}s, or
      (d) 5- to 9-membered bridged heterocycloalkyl comprising 1 or 2 heteroatoms independently selected from the group consisting of nitrogen and oxygen atoms, wherein the bridged heterocycloalkyl may be optionally substituted with the same or different 1 to 3 R^{d6}s;
R^{F} is
   (1) halogen,
   (2) C₁₋₄ alkyl,
   (3) C₁₋₄ haloalkyl,
   (4) C₁₋₆ alkoxy, wherein the alkoxy may be optionally substituted with the same or different 1 to 3 halogen atoms, or
   (5) C₃₋₅ cycloalkyl,
R^{G} is each independently a substituent selected from the group consisting of:
   (1) halogen,
   (2) C₁₋₆ alkyl, wherein the alkyl may be optionally substituted with:
      (a) C₁₋₄ alkoxy,
      (b) cyano,
      (c) phenyl, or
      (d) 5- to 6-membered heteroaryl comprising 1 or 2 heteroatoms independently selected from the group consisting of nitrogen, oxygen, and sulfur atoms,
   (3) C₁₋₄ haloalkyl,
   (4) C₁₋₆ alkoxy, wherein the alkoxy may be optionally substituted with the same or different 1 to 3 halogen atoms,
   (5) cyano,
   (6) CONR^{G1}R^{G2}, wherein R^{G1} and R^{G2} are each independently
      (a) hydrogen,
      (b) C₁₋₆ alkyl, or
      (c) C₁₋₄ haloalkyl,
   (7) trialkylsilyl,
   (8) pentafluorosulfanyl,
   (9) C₃₋₇ cycloalkyl, wherein the cycloalkyl may be optionally substituted with the same or different 1 to 3 R^{s2}s,
   (10) C₅₋₁₃ spiro cycloalkyl, wherein the spiro cycloalkyl may be optionally substituted with the same or different 1 to 3 R^{s3}s,
   (11) 4- to 7-membered heterocycloalkyl comprising 1 or 2 heteroatoms independently selected from the group consisting of nitrogen and oxygen atoms, wherein the heterocycloalkyl may be optionally substituted with the same or different 1 to 3 R^{s4}s,
   (12) 7- to 11-membered spiro heterocycloalkyl comprising 1 or 2 heteroatoms independently selected from the group consisting of nitrogen and oxygen atoms, wherein the spiro heterocycloalkyl may be optionally substituted with the same or different 1 to 3 R^{s5}s,
   (13) phenyl, wherein the phenyl may be optionally substituted with the same or different 1 to 3 R^{s6}s, and
   (14) 5- to 6-membered heteroaryl comprising 1 to 3 heteroatoms independently selected from the group consisting of nitrogen, oxygen, and sulfur atoms, wherein the heteroaryl may be optionally substituted with the same or different 1 to 3 R^{s7}s, or alternatively,
in the case where R^{F} and R^{G} or two R^{G}s are substituted on neighboring atoms, then the R^{F} and R^{G} and/or the two R^{G}s may combine together with the atoms to which they attach to form:
   (a) a C₅₋₆ cycloalkene ring, wherein the cycloalkene ring may be optionally substituted with the same or different 1 to 3 R^{c1}s,
   (b) a 5- to 7-membered heterocycloalkene ring comprising 1 or 2 heteroatoms independently selected from the group consisting of nitrogen and oxygen atoms, wherein the heterocycloalkene ring may be optionally subsituted with the same or different 1 to 3 R^{c2}s,
   (c) a benzene ring, wherein the benzene ring may be optionally substituted with the same or different 1 to 3 R^{c3}s, or
   (d) a 5- to 7-membered heteroaromatic ring comprising 1 or 2 heteroatoms independently selected from the group consisting of nitrogen and oxygen atoms, wherein the heteroaromatic ring may be optionally substituted with the same or different 1 to 3 R^{c4}s, so that Ring Cy may form a bi- or tri-cyclic fused ring group;
R^{c1}, R^{c2}, R^{c3}, and R^{c4} are each independently a substituent selected from the group consisting of:
   (1) halogen,
   (2) C₁₋₆ alkyl,
   (3) C₁₋₄ haloalkyl, and
   (4) oxo;
R^{d1} is each independently a substituent selected from the group consisting of:
   (1) halogen,
   (2) hydroxy,
   (3) C₁₋₆ alkoxy, wherein the alkoxy may be optionally substituted with the same or different 1 to 3 halogen atoms,
   (4) cyano,
   (5) COOR^{e1}, wherein R^{e1} is hydrogen or C₁₋₆ alkyl,
   (6) CONR^{e2}R^{e3}, wherein R^{e2} and R^{e3} are independently
      (a) hydrogen,
      (b) C₁₋₆ alkyl, or
      (c) C₁₋₄ haloalkyl, or alternatively, R^{e2} and R^{e3} may combine together with the nitrogen atom to which they attach to form 5-to 6-membered heterocycloalkyl comprising 1 or 2 heteroatoms independently selected from the group consisting of nitrogen and oxygen atoms, wherein the heterocycloalkyl may be optionally substituted with the same or different 1 or 2 halogen atoms,
   (7) COR^{e4}, wherein R^{e4} is C₁₋₆ alkyl,
   (8) SO₂R^{e5}, wherein R^{e5} is C₁₋₆ alkyl,
   (9) NR^{e6}R^{e7}, wherein R^{e6} is
      (a) hydrogen,
      (b) C₁₋₆ alkyl, or
      (c) C₁₋₄ haloalkyl, and
R^{e7} is
   (a) hydrogen,
   (b) C₁₋₆ alkyl,
   (c) C₁₋₄ haloalkyl,
   (d) COR^{e8}, wherein R^{e8} is C₁₋₆ alkyl, and the alkyl may be optionally substituted with 1 to 3 substituents independently selected from the group consisting of:
      (i) halogen,
      (ii) hydroxy,
      (iii) C₁₋₆ alkoxy, and
      (iv) cyano, or
   (e) SO₂R^{e9}, wherein R^{e9} is C₁₋₆ alkyl or C₁₋₄ haloalkyl,
      (10) SO₂NR^{f1}R^{f2}, wherein R^{f1} and R^{f2} are independently
         (a) hydrogen,
         (b) C₁₋₆ alkyl, or
         (c) C₁₋₄ haloalkyl,
      (11) C₃₋₆ cycloalkyl, wherein the cycloalkyl may be optionally substituted with hydroxy or C₁₋₆ alkoxy,
      (12) 4- to 6-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of halogen and oxo,
      (13) phenyl, and
      (14) 5- to 6-membered heteroaryl comprising 1 or 2 heteroatoms independently selected from the group consisting of nitrogen, oxygen, and sulfur atoms, wherein the heteroaryl may be optionally substituted with C₁₋₆ alkyl or C₁₋₄ haloalkyl;
R^{d2} is each independently a substituent selected from the group consisting of:
   (1) C₁₋₆ alkyl,
   (2) C₁₋₄ haloalkyl,
   (3) oxo, and
   (4) COR^{g1}, wherein R^{g1} is C₁₋₆ alkyl, and the alkyl may be optionally substituted with:
      (a) hydroxy,
      (b) C₁₋₆ alkoxy, and
      (c) cyano;
R^{d3} is each independently a substituent selected from the group consisting of:
   (1) C₁₋₆ alkyl, and
   (2) COOR^{g2}, wherein R^{g2} is hydrogen or C₁₋₆ alkyl;
R^{d4}, R^{d5}, and R^{d6} are each independently a substituent selected from the group consisting of:
   (1) halogen,
   (2) hydroxy,
   (3) oxo,
   (4) cyano,
   (5) C₁₋₆ alkyl,
   (6) C₁₋₄ haloalkyl,
   (7) C₁₋₆ alkoxy, wherein the alkoxy may be optionally substituted with the same or different 1 to 3 halogen atoms,
   (8) NR^{g3}R^{g4}, wherein R^{g3} and R^{g4} are independently
      (a) hydrogen,
      (b) C₁₋₆ alkyl, or
      (c) C₁₋₄ haloalkyl, and
   (9) 4- to 7-membered heterocycloalkyl comprising 1 or 2 heteroatoms independently selected from the group consisting of nitrogen and oxygen atoms, wherein the heterocycloalkyl may be optionally substituted with 1 or 2 substituents independently selected from the group consisting of:
      (a) halogen,
      (b) hydroxy,
      (c) C₁₋₆ alkyl,
      (d) C₁₋₆ alkoxy, wherein the alkoxy may be optionally substituted with the same or different 1 to 3 halogen atoms, and
      (e) oxo;
R^{s1y}, R^{s2}, R^{s3}, R^{s4}, R^{s5}, R^{s6}, and R^{s7} are each independently a substituent selected from the group consisting of:
   (1) halogen,
   (2) hydroxy,
   (3) C₁₋₆ alkyl, wherein the alkyl may be optionally substituted with the same or different 1 to 3 R^{11y}s,
   (4) C₁₋₆ alkoxy, wherein the alkoxy may be optionally substituted with the same or different 1 to 3 halogen atoms,
   (5) cyano,
   (6) OR^{t1}, wherein R^{t1} is C₃₋₆ cycloalkyl,
   (7) COR¹²,
   (8) SO₂R¹³,
   (9) NR^{t2}R^{t3}, wherein R^{t2} is
      (a) hydrogen,
      (b) C₁₋₆ alkyl, or
      (c) C₁₋₄ haloalkyl, and
      R^{t3} is
      (a) hydrogen,
      (b) C₁₋₆ alkyl,
      (c) C₁₋₄ haloalkyl,
      (d) COR¹⁴, or
      (e) SO₂R¹⁵,
   (10) CONR^{t4}R^{t5}, wherein R^{t4} and R^{t5} are independently
      (a) hydrogen,
      (b) C₁₋₆ alkyl, or
      (c) C₁₋₄ haloalkyl, or alternatively, R^{t4} and R^{t5} may combine together with the nitrogen atom to which they attach to form 4-to 6-membered heterocycloalkyl comprising 1 or 2 heteroatoms independently selected from the group consisting of nitrogen and oxygen atoms, wherein the heterocycloalkyl may be optionally substituted with the same or different 1 or 2 halogen or hydroxy,
   (11) SO₂NR^{t6}R^{t7}, wherein R^{t6} and R^{t7} are independently
      (a) hydrogen,
      (b) C₁₋₆ alkyl, or
      (c) C₁₋₄ haloalkyl,
   (12) COOR^{t8}, wherein R^{t8} is hydrogen or C₁₋₆ alkyl,
   (13) oxo,
   (14) C₃₋₆ cycloalkyl,
   (15) phenyl,
   (16) 4- to 7-membered heterocycloalkyl comprising 1 or 2 heteroatoms independently selected from the group consisting of nitrogen and oxygen atoms, wherein the heterocycloalkyl may be optionally substituted with the same or different 1 to 3 R¹⁶s, and
   (17) 5- to 6-membered heteroaryl comprising 1 to 3 heteroatoms independently selected from the group consisting of nitrogen, oxygen, and sulfur atoms, wherein the heteroaryl may be optionally substituted with the same or different 1 to 3 R¹⁷s;
R^{11y} is each independently a substituent selected from the group consisting of:
   (1) halogen,
   (2) hydroxy,
   (3) C₁₋₆ alkoxy, wherein the alkoxy may be optionally substituted with 1 to 3 substituents independently selected from the group consisting of:
      (a) halogen,
      (b) hydroxy,
      (c) C₁₋₆ alkoxy, and
      (d) C₃₋₆ cycloalkyl, wherein the cycloalkyl may be optionally substituted with the same or different 1 to 3 R^{1dy}s,
   (4) cyano,
   (5) NR²¹R²², wherein R²¹ is
      (a) hydrogen,
      (b) C₁₋₆ alkyl, or
      (c) C₁₋₄ haloalkyl, andR²² is
         (a) hydrogen,
         (b) C₁₋₆ alkyl,
         (c) C₁₋₄ haloalkyl,
         (d) COR^{1a}, or
         (e) SO₂R^{1b},
   (6) COR²³, wherein R²³ is C₁₋₆ alkyl or C₁₋₄ haloalkyl,
   (7) SO₂R^{24y}, wherein R^{24y} is C₁₋₆ alkyl, wherein the alkyl may be optionally substituted with 1 to 3 substituents independently selected from the group consisting of:
      (a) halogen, and
      (b) C₃₋₆ cycloalkyl,
   (8) COOR²⁵, wherein R²⁵ is hydrogen or C₁₋₆ alkyl,
   (9) CONR²⁶R²⁷, wherein R²⁶ and R²⁷ are independently
      (a) hydrogen,
      (b) C₁₋₆ alkyl, or
      (c) C₁₋₄ haloalkyl, or alternatively, R²⁶ and R²⁷ may combine together with the nitrogen atom to which they attach to form 5-to 6-membered heterocycloalkyl comprising 1 or 2 heteroatoms independently selected from the group consisting of nitrogen and oxygen atoms, wherein the heterocycloalkyl may be optionally substituted with the same or different 1 or 2 halogen atoms,
   (10) SO₂NR²³R²⁹, wherein R²⁸ and R²⁹ are independently
      (a) hydrogen,
      (b) C₁₋₆ alkyl, or
      (c) C₁₋₄ haloalkyl, or alternatively, R²⁸ and R²⁹ may combine together with the nitrogen atom to which they attach to form 4-to 7-membered heterocycloalkyl comprising 1 or 2 heteroatoms independently selected from the group consisting of nitrogen and oxygen atoms, wherein the heterocycloalkyl may be optionally substituted with the same or different 1 or 2 halogen atoms,
   (11) C₃₋₆ cycloalkyl,
   (12) 4- to 6-membered heterocycloalkyl comprising 1 or 2 heteroatoms independently selected from the group consisting of nitrogen and oxygen atoms, wherein the heterocycloalkyl may be optionally substituted with the same or different 1 to 3 R^{1c}s,
   (13) phenyl,
   (14) 5- to 6-membered heteroaryl comprising 1 or 2 heteroatoms independently selected from the group consisting of nitrogen and oxygen atoms, wherein the heteroaryl may be optionally substituted with the same or different 1 to 3 R^{1ey}s, and
   (15) OR^{30y}, wherein R^{30y} is C₃₋₆ cycloalkyl which may be optionally substituted with the same or different 1 to 3 R^{1fy}s;
R¹⁷ and R^{1ey} are each independently a substituent selected from the group consisting of:
   (1) halogen,
   (2) C₁₋₆ alkyl,
   (3) C₁₋₄ haloalkyl,
   (4) C₁₋₆ alkoxy,
   (5) cyano,
   (6) C₃₋₆ cycloalkyl, and
   (7) 4- to 6-membered heterocycloalkyl comprising an oxygen atom;
R¹², R¹³, R¹⁴, R¹⁵, R^{1a}, and R^{1b} are each independently a substituent selected from the group consisting of:
   (1) C₁₋₆ alkyl,
   (2) C₁₋₄ haloalkyl,
   (3) C₃₋₆ cycloalkyl,
   (4) 4- to 6-membered heterocycloalkyl comprising an oxygen atom,
   (5) phenyl, and
   (6) 5- to 6-membered heteroaryl comprising 1 or 2 heteroatoms independently selected from the group consisting of nitrogen and oxygen atoms;
R¹⁶, R^{1c}, R^{1dy}, and R^{1fy} are each independently a substituent selected from the group consisting of:
   (1) halogen,
   (2) hydroxy,
   (3) C₁₋₆ alkyl,
   (4) C₁₋₄ haloalkyl,
   (5) C₁₋₆ alkoxy, wherein the alkoxy may be optionally substituted with the same or different 1 to 3 halogen atoms, and
   (6) oxo.

### Item 18

The compound according to Item 17, or a pharmaceutically acceptable salt thereof, wherein
R^{Dy} and R^{Ey} are each independently
   (1) hydrogen,
   (2) C₁₋₆ alkyl, wherein the alkyl may be optionally substituted with the same or different 1 to 3 R^{d1}s,
   (3) C₃₋₆ cycloalkyl,
   (4) 4- to 7-membered heterocycloalkyl comprising 1 to 3 heteroatoms independently selected from the group consisting of nitrogen and oxygen atoms, or
   (5) 5- to 6-membered heteroaryl comprising 1 to 3 heteroatoms independently selected from the group consisting of nitrogen and oxygen atoms, wherein the heteroaryl may be optionally substituted with C₁₋₄ alkyl,
   or alternatively,
R^{Dy} and R^{Ey} may combine together with the nitrogen atom to which they attach to form
   (a) 4- to 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s1y}s and / or may be fused with a 5- to 6-membered heteroaromatic ring comprising 1 to 3 nitrogen atoms wherein the heteroaromatic ring may be optionally substituted with the same or different 1 or 2 R^{d3}s,
   (b) 7- to 11-membered spiro heterocycloalkyl comprising 1 to 3 heteroatoms independently selected from the group consisting of nitrogen and oxygen atoms, wherein the spiro heterocycloalkyl may be optionally substituted with the same or different 1 to 3 R^{d4}s,
   (c) 6- to 10-membered fused heterocycloalkyl comprising 1 or 2 heteroatoms independently selected from the group consisting of nitrogen and oxygen atoms, or
   (d) 5- to 9-membered bridged heterocycloalkyl comprising 1 or 2 heteroatoms independently selected from the group consisting of nitrogen and oxygen atoms,
R^{F} is
   (1) halogen,
   (2) C₁₋₄ alkyl,
   (3) C₁₋₄ haloalkyl,
   (4) C₁₋₄ alkoxy, wherein the alkoxy may be optionally substituted with the same or different 1 to 3 halogen atoms, or
   (5) C₃₋₅ cycloalkyl,
R^{G} is each independently a substituent selected from the group consisting of:
   (1) halogen,
   (2) C₁₋₆ alkyl, wherein the alkyl may be optionally substituted with:
      (a) C₁₋₄ alkoxy, or
      (b) cyano,
   (3) C₁₋₄ haloalkyl,
   (4) C₁₋₄ alkoxy, wherein the alkoxy may be optionally substituted with the same or different 1 to 3 halogen atoms,
   (5) cyano,
   (6) pentafluorosulfanyl, and
   (7) C₃₋₇ cycloalkyl, wherein the cycloalkyl may be optionally substituted with the same or different 1 or 2 R^{s2}s, or alternatively,
in the case where R^{F} and R^{G} or two R^{G}s are substituted on neighboring atoms, then the R^{F} and R^{G} and/or the two R^{G}s may combine together with the atoms to which they attach to form:
   (a) a C₅₋₆ cycloalkene ring,
   (b) a 5- to 7-membered heterocycloalkene ring comprising 1 or 2 heteroatoms independently selected from the group consisting of nitrogen and oxygen atoms, wherein the heterocycloalkene ring may be optionally subsituted with the same or different 1 or 2 R^{c2}s, or
   (c) a 5- to 7-membered heteroaromatic ring comprising 1 or 2 heteroatoms independently selected from the group consisting of nitrogen and oxygen atoms,
   so that Ring Cy may form a bi- or tri-cyclic fused ring group;,
R^{c2} is each independently C₁₋₄ alkyl,
R^{d1} is each independently a substituent selected from the group consisting of:
   (1) halogen,
   (2) C₁₋₄ alkoxy,
   (3) cyano,
   (4) CONR^{e2}R^{e3}, wherein R^{e2} and R^{e3} are independently
      (a) hydrogen, or
      (b) C₁₋₄ alkyl,
   (5) SO₂R^{e5}, wherein R^{e5} is C₁₋₄ alkyl,
   (6) NR^{e6}R^{e7}, wherein R^{e6} is
      (a) hydrogen, or
      (b) C₁₋₄ alkyl, and
         R^{e7} is
         (a) hydrogen,
         (b) C₁₋₄ alkyl, or
         (c) COR^{e8}, wherein R^{e8} is C₁₋₄ alkyl,
   (7) C₃₋₆ cycloalkyl,
   (8) phenyl, and
   (9) 5- to 6-membered heteroaryl comprising 1 or 2 heteroatoms independently selected from the group consisting of nitrogen, oxygen, and sulfur atoms, wherein the heteroaryl may be optionally substituted with C1-4 alkyl,
R^{d3} is each independently a substituent selected from the group consisting of:
   (1) C₁₋₄ alkyl, and
   (2) COOR^{g2}, wherein R^{g2} is hydrogen or C₁₋₄ alkyl;
R^{d4} is each independently a substituent selected from the group consisting of:
   (1) oxo, and
   (2) C₁₋₄ alkyl,
R^{s1y} and R^{s2} are each independently a substituent selected from the group consisting of:
   (1) halogen,
   (2) hydroxy,
   (3) C₁₋₆ alkyl, wherein the alkyl may be optionally substituted with the same or different 1 to 3 R^{13-y}s,
   (4) C₁₋₆ alkoxy,
   (5) cyano,
   (6) COR¹²,
   (7) SO₂R¹³,
   (8) NR^{t2}R^{t3}, wherein R^{t2} is
      (a) hydrogen, or
      (b) C₁₋₆ alkyl, and
         R^{t3} is
         (a) hydrogen,
         (b) C₁₋₆ alkyl,
         (c) COR¹⁴, or
         (d) SO₂R¹⁵,
   (9) CONR^{t4}R^{t5}, wherein R^{t4} and R^{t5} are independently
      (a) hydrogen, or
      (b) C₁₋₆ alkyl, or alternatively, R^{t4} and R^{t5} may combine together with the nitrogen atom to which they attach to form 4-to 6-membered heterocycloalkyl comprising 1 or 2 heteroatoms independently selected from the group consisting of nitrogen and oxygen atoms,
   (10) SO₂NR^{t6}R^{t7}, wherein R^{t6} and R^{t7} are independently
      (a) hydrogen, or
      (b) C₁₋₆ alkyl,
   (11) COOR^{t8}, wherein R^{t8} is hydrogen or C₁₋₄ alkyl,
   (12) oxo,
   (13) phenyl, and
   (14) 5- to 6-membered heteroaryl comprising 1 to 3 heteroatoms independently selected from the group consisting of nitrogen, oxygen, and sulfur atoms, wherein the heteroaryl may be optionally substituted with the same or different 1 to 3 R¹⁷s;
R^{11y} is each independently a substituent selected from the group consisting of:
   (1) halogen,
   (2) hydroxy,
   (3) C₁₋₆ alkoxy, wherein the alkoxy may be optionally substituted with 1 to 3 substituents independently selected from the group consisting of:
      (a) halogen,
      (b) hydroxy,
      (c) C₁₋₄ alkoxy, and
      (d) C₃₋₆ cycloalkyl, wherein the cycloalkyl may be optionally substituted with the same or different 1 to 3 R^{1dy}s,
   (4) cyano,
   (5) NR²¹R²², wherein R²¹ and R²² are each independently
      (a) hydrogen, or
      (b) C₁₋₄ alkyl,
   (6) SO₂R^{24y}, wherein R^{24y} is C₁₋₆ alkyl, wherein the alkyl may be optionally substituted with 1 to 3 substituents independently selected from the group consisting of:
      (a) halogen, and
      (b) C₃₋₆ cycloalkyl,
   (7) 4- to 6-membered heterocycloalkyl comprising 1 or 2 heteroatoms independently selected from the group consisting of nitrogen and oxygen atoms, wherein the heterocycloalkyl may be optionally substituted with the same or different 1 to 3 R^{1c}s, and
   (8) 5- to 6-membered heteroaryl comprising 1 or 2 heteroatoms independently selected from the group consisting of nitrogen and oxygen atoms, wherein the heteroaryl may be optionally substituted with the same or different 1 to 3 R^{1ey}s;
R¹⁷ and R^{1ey} are each independently a substituent selected from the group consisting of:
   (1) C₁₋₆ alkyl,
   (2) C₁₋₄ haloalkyl, and
   (3) C₁₋₆ alkoxy;
R₁₂, R¹³, R¹⁴, and R¹⁵ are each independently a substituent selected from the group consisting of:
   (1) C₁₋₆ alkyl,
   (2) C₁₋₄ haloalkyl,
   (3) C₃₋₆ cycloalkyl, and
   (4) 4- to 6-membered heterocycloalkyl comprising an oxygen atom;
R^{1dy} is each independently a substituent selected from the group consisting of:
   (1) halogen,
   (2) hydroxy, and
   (3) C₁₋₄ alkyl.

### Item 19

The compound according to Item 17 or 18, or a pharmaceutically acceptable salt thereof, wherein
R^{F} is
   (1) methyl, ethyl, isopropyl, or tert-butyl,
   (2) C₁₋₄ haloalkyl,
   (3) C₁₋₄ alkoxy, wherein the alkoxy may be optionally substituted with the same or different 1 to 3 halogen atoms, or
   (4) C₃₋₅ cycloalkyl,
R^{G} is each independently a substituent selected from the group consisting of:
   (1) halogen,
   (2) C₁₋₆ alkyl, wherein the alkyl may be optionally substituted with:
      (a) C₁₋₄ alkoxy, or
      (b) cyano,
   (3) C₁₋₄ haloalkyl,
   (4) C₁₋₄ alkoxy, wherein the alkoxy may be optionally substituted with the same or different 1 to 3 halogen atoms,
   (5) cyano,
   (6) pentafluorosulfanyl, and
   (7) C₃₋₇ cycloalkyl, wherein the cycloalkyl may be optionally substituted with the same or different 1 or 2 R^{s2}s, or alternatively,
in the case where R^{F} and R^{G} or two R^{G}s are substituted on neighboring atoms, then the R^{F} and R^{G} and/or the two R^{G}s may combine together with the atoms to which they attach to form:
   (a) a C₅₋₆ cycloalkene ring,
   (b) a 5- to 7-membered heterocycloalkene ring comprising 1 or 2 heteroatoms independently selected from the group consisting of nitrogen and oxygen atoms, wherein the heterocycloalkene ring may be optionally subsituted with the same or different 1 or 2 R^{c2}s, or
   (c) a 5- to 7-membered heteroaromatic ring comprising 1 or 2 heteroatoms independently selected from the group consisting of nitrogen and oxygen atoms,
   so that Ring Cy may form a bi- or tri-cyclic fused ring group;,
R^{s2} is each independently a substituent selected from the group consisting of:
   (1) C₁₋₄ alkyl, and
   (2) cyano;
R^{11y} is each independently a substituent selected from the group consisting of:
   (1) halogen,
   (2) hydroxy,
   (3) C₁₋₆ alkoxy, wherein the alkoxy may be optionally substituted with 1 to 3 substituents independently selected from the group consisting of:
      (a) halogen,
      (b) hydroxy,
      (c) C₁₋₄ alkoxy, and
      (d) C₃₋₆ cycloalkyl, wherein the cycloalkyl may be optionally substituted with the same or different 1 to 3 R^{1dy}s,
   (4) cyano,
   (5) NR²¹R²², wherein R²¹ and R²² are each independently
      (a) hydrogen, or
      (b) C₁₋₄ alkyl,
   (6) SO₂R^{24y}, wherein R^{24y} is C₁₋₆ alkyl, wherein the alkyl may be optionally substituted with 1 to 3 substituents independently selected from the group consisting of:
      (a) halogen, and
      (b) C₃₋₆ cycloalkyl, and
   (7) 5- to 6-membered heteroaryl comprising 1 or 2 heteroatoms independently selected from the group consisting of nitrogen and oxygen atoms, wherein the heteroaryl may be optionally substituted with the same or different 1 to 3 R^{1ey}s_{.}

### Item 20

The compound according to any one of Items 17 to 19, or a pharmaceutically acceptable salt thereof, wherein
a partial structure: 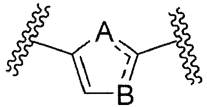 is a group of the following formula:

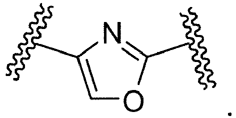

### Item 21

The compound according to any one of Items 17 to 20, or a pharmaceutically acceptable salt thereof, wherein
R^{Dy} and R^{Ey} combine together with the nitrogen atom to which they attach to form
(1) 4- to 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s1y}s and / or may be fused with a 5- to 6-membered heteroaromatic ring comprising 1 to 3 nitrogen atoms wherein the heteroaromatic ring may be optionally substituted with the same or different 1 or 2 R^{d3}s,
(2) 7- to 11-membered spiro heterocycloalkyl comprising 1 to 3 heteroatoms independently selected from the group consisting of nitrogen and oxygen atoms, wherein the spiro heterocycloalkyl may be optionally substituted with the same or different 1 to 3 R^{d4}s,
(3) 6- to 10-membered fused heterocycloalkyl comprising 1 or 2 heteroatoms independently selected from the group consisting of nitrogen and oxygen atoms, or
(4) 5- to 9-membered bridged heterocycloalkyl comprising 1 or 2 heteroatoms independently selected from the group consisting of nitrogen and oxygen atoms.

### Item 22

The compound according to any one of Items 17 to 21, or a pharmaceutically acceptable salt thereof, wherein
R^{Dy} and R^{Ey} combine together with the nitrogen atom to which they attach to form 4- to 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s1y}s and / or may be fused with a 5- to 6-membered heteroaromatic ring comprising 1 to 3 nitrogen atoms wherein the heteroaromatic ring may be optionally substituted with the same or different 1 or 2 R^{d3}s.

### Item 23

The compound according to any one of Items 17 to 22, having a structure of the following formula [II]: 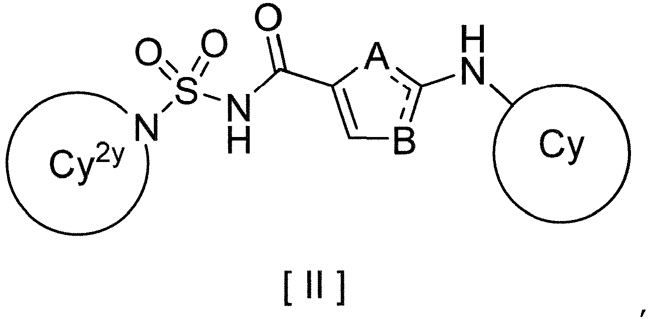 or a pharmaceutically acceptable salt thereof,
wherein
Ring Cy^{2y} is 4- to 7-membered heterocycloalkyl comprising 1 or 2 heteroatoms independently selected from the group consisting of nitrogen, oxygen, and sulfur atoms, wherein the heterocycloalkyl comprises at least one nitrogen atom and may be optionally substituted with the same or different 1 to 3 R^{s1y}s,
R^{s1y}, Ring Cy, and the partial structure comprising A and B are those as defined in Item 17.

### Item 24

The compound according to any one of Items 17 to 23, having a structure of the following formula [III]: 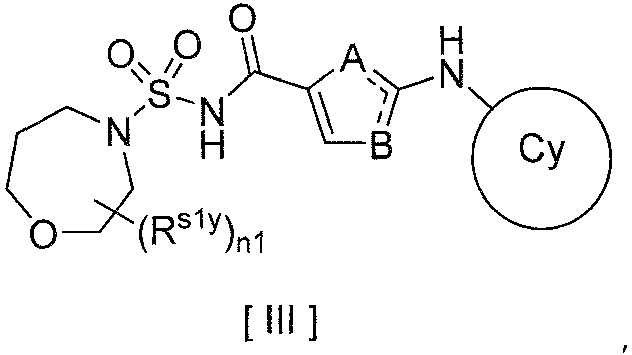 or a pharmaceutically acceptable salt thereof,
wherein
n1 is an integer from 0 to 3,
R^{s1y}, Ring Cy, and the partial structure comprising A and B are those as defined in Item 17.

### Item 25

The compound according to any one of Items 17 to 24, having a structure of the following formula [IV]: 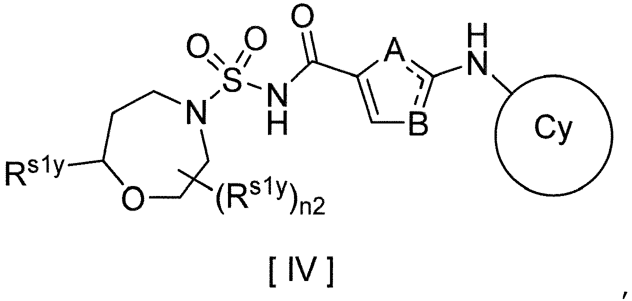 or a pharmaceutically acceptable salt thereof,
wherein
n2 is an integer from 0 to 2,
R^{s1y}, Ring Cy, and the partial structure comprising A and B are those as defined in Item 17.

### Item 26

The compound according to any one of Items 17 to 25, or a pharmaceutically acceptable salt thereof, wherein
Ring Cy is the following formula: 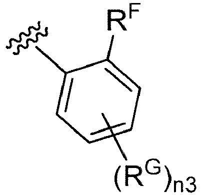 wherein
n3 is an integer from 0 to 4,
R^{F} and R^{G} are those as defined in Item 17.

### Item 27

The compound according to any one of Items 17 to 26, or a pharmaceutically acceptable salt thereof, wherein
Ring Cy is the following formula: 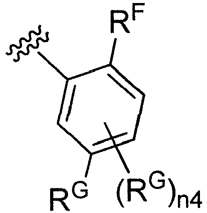 wherein
n4 is an integer from 0 to 3,
R^{F} and R^{G} are those as defined in Item 17.

### Item 28

The compound according to any one of Items 17 to 27, or a pharmaceutically acceptable salt thereof, wherein
Ring Cy is the following formula: 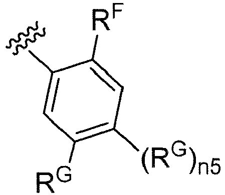 wherein
n5 is an integer of 0 or 1,
R^{F} and R^{G} are those as defined in Item 17.

### Item 29

The compound according to Item 17 selected from: 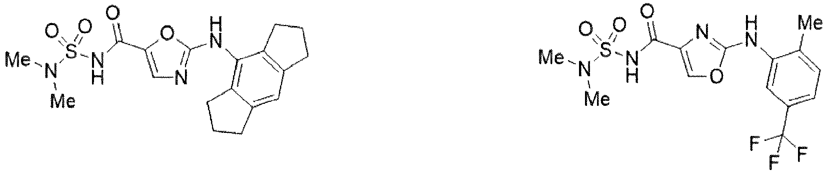 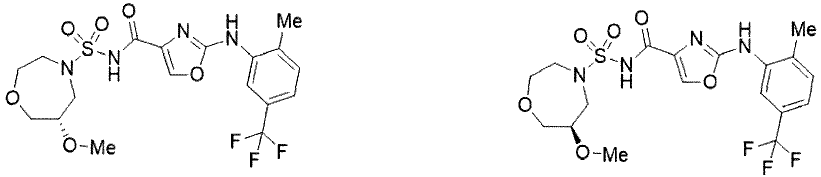 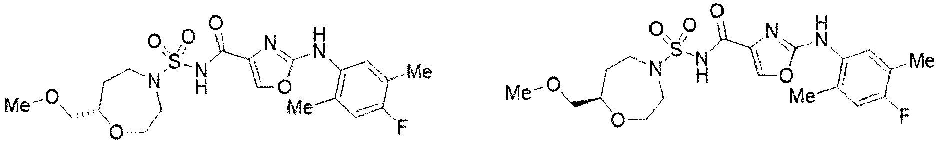 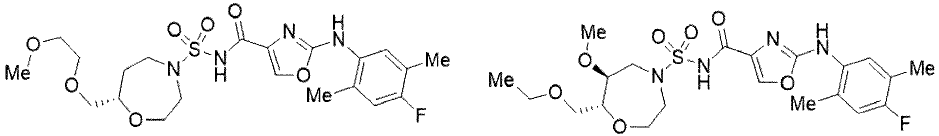 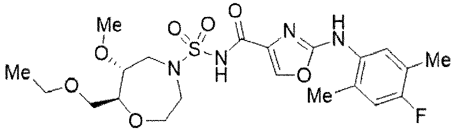 or , or a pharmaceutically acceptable salt thereof.

### Item 30

A pharmaceutical composition comprising a compound according to any one of Items 17 to 29, or a pharmaceutically acceptable salt thereof, and a pharmaceutically acceptable carrier.

### Item 31

An NLRP3 inflammasome inhibitor comprising a compound according to any one of Items 17 to 29, or a pharmaceutically acceptable salt thereof.

### Item 32

A medicament for treating or preventing a disease selected from the group consisting of multiple sclerosis, chronic kidney disease and inflammatory bowel disease, comprising a compound according to any one of Items 17 to 29, or a pharmaceutically acceptable salt thereof.

### Item 33

The medicament according to Item 32, wherein the inflammatory bowel disease is ulcerative colitis or Crohn's disease.

### Item 34

A method for inhibiting NLRP3 inflammasome, comprising administering a therapeutically effective amount of a compound according to any one of Items 17 to 29, or a pharmaceutically acceptable salt thereof, to a mammal.

### Item 35

A method for treating or preventing a disease selected from the group consisting of multiple sclerosis, chronic kidney disease and inflammatory bowel disease, comprising administering a therapeutically effective amount of a compound according to any one of Items 17 to 29, or a pharmaceutically acceptable salt thereof, to a mammal.

### Item 36

The method according to Item 35, wherein the inflammatory bowel disease is ulcerative colitis or Crohn's disease.

### Item 37

Use of a compound according to any one of Items 17 to 29, or a pharmaceutically acceptable salt thereof, in the manufacture of an NLRP3 inflammasome inhibitor.

### Item 38

Use of a compound according to any one of Items 17 to 29, or a pharmaceutically acceptable salt thereof, in the manufacture of a medicament for treating or preventing a disease selected from the group consisting of multiple sclerosis, chronic kidney disease and inflammatory bowel disease.

### Item 39

The use according to Item 38, wherein the inflammatory bowel disease is ulcerative colitis or Crohn's disease.

### Item 40

A compound according to any one of Items 17 to 29, or a pharmaceutically acceptable salt thereof, for use in inhibiting NLRP3 inflammasome.

### Item 41

A compound according to any one of Items 17 to 29, or a pharmaceutically acceptable salt thereof, for use in treating or preventing a disease selected from the group consisting of multiple sclerosis, chronic kidney disease and inflammatory bowel disease.

### Item 42

The compound according to Item 41, or a salt thereof, wherein the inflammatory bowel disease is ulcerative colitis or Crohn's disease.

A double wavy line as follows: 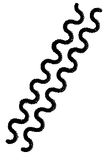 in the partial structure herein refers to a binding site of the structure.

The expression "Me" in a compound and a partial structure herein refers to "CH₃".

The term "halogen" includes, for example, fluorine, chlorine, bromine, and iodine.

The term "C₁₋₄ alkyl" refers to a straight- or branched-chain saturated hydrocarbon group having 1 to 4 carbon atoms. "C₁₋₄ alkyl" includes methyl, ethyl, n-propyl, isopropyl, n-butyl, sec-butyl, isobutyl, and tert-butyl. A preferable C₁₋₄ alkyl includes methyl, ethyl, n-propyl, isopropyl, n-butyl, isobutyl, sec-butyl, and tert-butyl.

The term "C₁₋₆ alkyl" refers to a straight- or branched-chain saturated hydrocarbon group having 1 to 6 carbon atoms. "C₁₋₆ alkyl" includes, for example, methyl, ethyl, n-propyl, isopropyl, n-butyl, isobutyl, sec-butyl, tert-butyl, n-pentyl, isopentyl, neopentyl, 2-methylpropyl, 1,1-dimethylpropyl, 1-ethylpropyl, n-hexyl, isohexyl, 1,1-dimethylbutyl, 2,2-dimethylbutyl, 3,3-dimethylbutyl, and 2-ethylbutyl. A preferable C₁₋₆ alkyl includes methyl, ethyl, n-propyl, isopropyl, n-butyl, isobutyl, sec-butyl, tert-butyl, neopentyl, and 1-ethylpropyl.

The term "C₁₋₄ haloalkyl" refers to the above-defined "C₁₋₄ alkyl" that is substituted with 1 to 7 halogen atoms independently selected from the group of the above-defined "halogen". "C₁₋₄ haloalkyl" includes, for example, monofluoromethyl, difluoromethyl, trifluoromethyl, 2-fluoroethyl, 2-chloroethyl, 2-bromoethyl, 1,1-difluoroethyl, 1-fluoro-1-methylethyl, 2,2,2-trifluoro-1-methylethyl, 2,2,2-trifluoroethyl, pentafluoroethyl, 3-fluoropropyl, 3-chloropropyl, 1,1-difluoropropyl, 3,3,3-trifluoropropyl, and 4,4,4-trifluorobutyl. A preferable C₁₋₄ haloalkyl includes monofluoromethyl, difluoromethyl, trifluoromethyl, 1,1-difluoroethyl, 1-fluoro-1-methylethyl, 2,2,2-trifluoro-1-methylethyl, 2,2,2-trifluoroethyl, 3-fluoropropyl, 3,3,3-trifluoropropyl and pentafluoroethyl. A more preferable C₁₋₄ haloalkyl includes difluoromethyl, trifluoromethyl, 1,1-difluoroethyl, 2,2,2-trifluoro-1-methylethyl, 2,2,2-trifluoroethyl, 3-fluoropropyl, and 3,3,3-trifluoropropyl.

The term "C₁₋₄ alkoxy" refers to a group wherein the above-defined "C₁₋₄ alkyl" binds to an oxygen atom. "C₁₋₄ alkoxy" includes methoxy, ethoxy, n-propoxy, isopropoxy, n-butoxy, sec-butoxy, isobutoxy, and tert-butoxy. A preferable C₁₋₄ alkoxy includes methoxy, ethoxy, n-propoxy, isopropoxy, and tert-butoxy. A more preferable C₁₋₄ alkoxy includes methoxy, ethoxy, n-propoxy, and isobutoxy.

The term "C₁₋₆ alkoxy" refers to a group wherein the above-defined "C₁₋₆ alkyl" binds to an oxygen atom. "C₁₋₆ alkoxy" includes, for example, methoxy, ethoxy, n-propoxy, isopropoxy, n-butoxy, sec-butoxy, isobutoxy, tert-butoxy, pentyloxy, isopentyloxy, 2-methylbutoxy, 1,1-dimethylpropoxy, neopentyloxy, 3,3-dimethylbutoxy, 1-ethylpropoxy, and hexyloxy. A preferable C₁₋₆ alkoxy includes methoxy, ethoxy, n-propoxy, isopropoxy, tert-butoxy, neopentyloxy, 1,1-dimethylpropoxy, and 3,3-dimethylbutoxy. A more preferable C₁₋₆ alkoxy includes methoxy, ethoxy, n-propoxy, and isobutoxy.

The term "trialkylsilyl" refers to a silyl group to which three alkyl groups are attached, and includes tri-C₁₋₆ alkylsilyl. The term "trialkylsilyl" includes, for example, trimethylsilyl (TMS), triethylsilyl (TES), tert-butyldimethylsilyl (TBS), and triisopropylsilyl (TIPS).

The term "C₃₋₅ cycloalkyl" refers to a monocyclic saturated hydrocarbon group having 3 to 5 carbon atoms. "C₃₋₅ cycloalkyl" includes cyclopropyl, cyclobutyl, and cyclopentyl.

The term "C₃₋₆ cycloalkyl" refers to a monocyclic saturated hydrocarbon group having 3 to 6 carbon atoms. "C₃₋₆ cycloalkyl" includes cyclopropyl, cyclobutyl, cyclopentyl, and cyclohexyl.

The term "C₃₋₇ cycloalkyl" refers to a monocyclic saturated hydrocarbon group having 3 to 7 carbon atoms. "C₃₋₇ cycloalkyl" includes cyclopropyl, cyclobutyl, cyclopentyl, cyclohexyl, and cycloheptyl.

The term "5- to 6-membered heteroaryl" refers to a 5- to 6-membered aromatic heterocyclyl group comprising 1 to 3 heteroatoms independently selected from the group consisting of nitrogen, oxygen, and sulfur atoms, besides carbon atoms, as a ring-constituting atom. Such an aromatic heterocyclyl group may bind to another group at any carbon atom or nitrogen atom on its ring, if chemically applicable. The term "5- to 6-membered heteroaryl" includes, for example, pyrrolyl, furanyl, thiophenyl, imidazolyl, pyrazolyl, triazolyl, oxazolyl, isoxazolyl, oxadiazolyl, thiazolyl, isothiazolyl, pyridinyl, pyrazinyl, pyrimidinyl, pyridazinyl, and triazinyl.

The "5- to 6-membered heteroaryl comprising 1 to 3 nitrogen atoms" includes, for example, pyrrolyl, imidazolyl, pyrazolyl, triazolyl, pyridinyl, pyrazinyl, pyrimidinyl, pyridazinyl, and triazinyl. Preferably, pyrazolyl, pyridinyl, pyrazinyl, pyrimidinyl, pyridazinyl, and triazinyl are included.

The "5- to 6-membered heteroaryl comprising 1 or 2 nitrogen atoms" includes, for example, pyrrolyl, imidazolyl, pyrazolyl, pyridinyl, pyrazinyl, pyrimidinyl, and pyridazinyl. Preferably, pyrazolyl, pyridinyl, pyrazinyl, pyrimidinyl, and pyridazinyl are included.

The "5- to 6-membered heteroaryl comprising 1 to 3 heteroatoms independently selected from the group consisting of nitrogen and oxygen atoms" includes, for example, pyrrolyl, furanyl, imidazolyl, pyrazolyl, triazolyl, oxazolyl, isoxazolyl, oxadiazolyl, pyridinyl, pyrazinyl, pyrimidinyl, pyridazinyl, and triazinyl. Preferably, pyrazolyl and pyridinyl are included.

The "5- to 6-membered heteroaryl comprising 1 or 2 heteroatoms independently selected from the group consisting of nitrogen, oxygen, and sulfur atoms" includes, for example, pyrrolyl, furanyl, thiophenyl, imidazolyl, pyrazolyl, oxazolyl, isoxazolyl, thiazolyl, isothiazolyl, pyridinyl, pyrazinyl, pyrimidinyl, and pyridazinyl. Preferably, imidazolyl, pyridinyl, and pyrazolyl are included. More preferably, pyridinyl, and pyrazolyl are included.

The "5- to 6-membered heteroaryl comprising 1 to 3 heteroatoms independently selected from the group consisting of nitrogen, oxygen, and sulfur atoms" includes, for example, pyrrolyl, furanyl, thiophenyl, imidazolyl, pyrazolyl, triazolyl, oxazolyl, isoxazolyl, oxadiazolyl, thiazolyl, isothiazolyl, pyridinyl, pyrazinyl, pyrimidinyl, pyridazinyl, and triazinyl. Preferably, pyrazolyl, imidazolyl, thiazolyl, isothiazolyl, oxazolyl, isoxazolyl, triazolyl, oxadiazolyl, pyridinyl, pyrazinyl, pyrimidinyl, and triazinyl are included. More preferably, pyrazolyl, oxadiazolyl, pyrazinyl, and pyrimidinyl are included.

The "5- to 6-membered heteroaryl comprising 1 or 2 heteroatoms independently selected from the group consisting of nitrogen and oxygen atoms" includes, for example, pyrrolyl, furanyl, imidazolyl, pyrazolyl, oxazolyl, isoxazolyl, pyridinyl, pyrazinyl, pyrimidinyl, and pyridazinyl. Preferably, pyridinyl and pyridazinyl are included.

The "5-membered heteroaryl comprising two nitrogen atoms" includes, for example, imidazolyl and pyrazolyl.

The term "4- to 7-membered heterocycloalkyl" refers to a 4-to 7-membered monocyclic saturated heterocyclyl group comprising 1 to 3 heteroatoms independently selected from the group consisting of nitrogen, oxygen, and sulfur atoms, besides carbon atoms, as a ring-constituting atom. The heterocycloalkyl group may bind to another group at any carbon atom, nitrogen atom, or sulfur atom on its ring, if chemically applicable. The term "4-to 7-membered heterocycloalkyl" includes, for example, azetidinyl, oxetanyl, thietanyl, diazetidinyl, dioxetanyl, dithietanyl, pyrrolidinyl, tetrahydrofuranyl, tetrahydrothiophenyl, imidazolidinyl, pyrazolidinyl, oxazolidinyl, isoxazolidinyl, thiazolidinyl, isothiazolidinyl, dioxolanyl, dithiolanyl, piperidinyl, tetrahydropyranyl, 1,3-diazacyclohexanyl, piperazinyl, morpholinyl, thiomorpholinyl, dioxanyl, hexahydrotriazinyl, azepanyl, oxepanyl, diazepanyl, oxazepanyl, and thiazepanyl .

The "4- to 7-membered heterocycloalkyl comprising 1 to 3 heteroatoms independently selected from the group consisting of nitrogen and oxygen atoms" includes, for example, azetidinyl, oxetanyl, diazetidinyl, dioxetanyl, pyrrolidinyl, tetrahydrofuranyl, imidazolidinyl, pyrazolidinyl, oxazolidinyl, isoxazolidinyl, dioxolanyl, piperidinyl, tetrahydropyranyl, 1,3-diazacyclohexanyl, piperazinyl, morpholinyl, dioxanyl, hexahydrotriazinyl, azepanyl, oxepanyl, diazepanyl, and oxazepanyl. Preferably, azetidinyl, pyrrolidinyl, tetrahydrofuranyl, and tetrahydropyranyl are included. More preferably, tetrahydrofuranyl, and tetrahydropyranyl are included.

The "4- to 7-membered heterocycloalkyl comprising 1 or 2 heteroatoms independently selected from the group consisting of nitrogen, oxygen, and sulfur atoms" includes, for example, azetidinyl, oxetanyl, thietanyl, diazetidinyl, dioxetanyl, dithietanyl, pyrrolidinyl, tetrahydrofuranyl, tetrahydrothiophenyl, imidazolidinyl, pyrazolidinyl, oxazolidinyl, isoxazolidinyl, thiazolidinyl, isothiazolidinyl, dioxolanyl, dithiolanyl, piperidinyl, tetrahydropyranyl, 1,3-diazacyclohexanyl, piperazinyl, morpholinyl, thiomorpholinyl, dioxanyl, azepanyl, oxepanyl, diazepanyl, oxazepanyl, and thiazepanyl. Preferably, azetidinyl, pyrrolidinyl, piperidinyl, piperazinyl, morpholinyl, thiomorpholinyl, 1,3-diazacyclohexanyl, azepanyl, oxazepanyl, diazepanyl, and thiazepanyl are included. Among the above, the "7-membered heterocycloalkyl comprising 1 or 2 heteroatoms independently selected from the group consisting of nitrogen, oxygen, and sulfur atoms" includes, for example, azepanyl, oxepanyl, diazepanyl, oxazepanyl, and thiazepanyl.

The "4- to 7-membered heterocycloalkyl comprising 1 or 2 heteroatoms independently selected from the group consisting of nitrogen and oxygen atoms" includes, for example, azetidinyl, oxetanyl, diazetidinyl, dioxetanyl, pyrrolidinyl, tetrahydrofuranyl, imidazolidinyl, pyrazolidinyl, oxazolidinyl, isoxazolidinyl, dioxolanyl, piperidinyl, tetrahydropyranyl, 1,3-diazacyclohexanyl, piperazinyl, morpholinyl, dioxanyl, azepanyl, oxepanyl, diazepanyl, and oxazepanyl. Preferably, azetidinyl, pyrrolidinyl, piperidinyl, piperazinyl, morpholinyl, and azepanyl are included.

The "4- to 6-membered heterocycloalkyl comprising 1 or 2 heteroatoms independently selected from the group consisting of nitrogen and oxygen atoms" includes, for example, azetidinyl, oxetanyl, diazetidinyl, dioxetanyl, pyrrolidinyl, tetrahydrofuranyl, imidazolidinyl, pyrazolidinyl, oxazolidinyl, isoxazolidinyl, dioxolanyl, piperidinyl, tetrahydropyranyl, 1,3-diazacyclohexanyl, piperazinyl, morpholinyl, and dioxanyl. Preferably, azetidinyl, pyrrolidinyl, piperidinyl, and morpholinyl are included. More preferably, pyrrolidinyl is included.

The "5- to 6-membered heterocycloalkyl comprising 1 or 2 heteroatoms independently selected from the group consisting of nitrogen and oxygen atoms" includes, for example, pyrrolidinyl, tetrahydrofuranyl, imidazolidinyl, pyrazolidinyl, oxazolidinyl, isoxazolidinyl, dioxolanyl, piperidinyl, tetrahydropyranyl, 1,3-diazacyclohexanyl, piperazinyl, morpholinyl, and dioxanyl. Preferably, piperidinyl and morpholinyl are included.

The "4- to 6-membered heterocycloalkyl comprising 1 or 2 heteroatoms independently selected from the group consisting of nitrogen, oxygen, and sulfur atoms" includes, for example, azetidinyl, oxetanyl, thietanyl, diazetidinyl, dioxetanyl, dithietanyl, pyrrolidinyl, tetrahydrofuranyl, tetrahydrothiophenyl, imidazolidinyl, pyrazolidinyl, oxazolidinyl, isoxazolidinyl, thiazolidinyl, isothiazolidinyl, dioxolanyl, dithiolanyl, piperidinyl, tetrahydropyranyl, 1,3-diazacyclohexanyl, piperazinyl, morpholinyl, thiomorpholinyl, and dioxanyl. Preferably, oxetanyl, piperidinyl, and morpholinyl are included.

The "4- to 6-membered heterocycloalkyl comprising an oxygen atom" includes, for example, oxetanyl, dioxetanyl, tetrahydrofuranyl, dioxolanyl, tetrahydropyranyl, and dioxanyl. Preferably, oxetanyl, tetrahydrofuranyl, tetrahydropyranyl, and dioxanyl are included. More preferably, tetrahydropyranyl is included.

The "5 or 6-membered heterocycloalkyl comprising an oxygen atom" includes, for example, tetrahydrofuranyl, dioxolanyl, tetrahydropyranyl, and dioxanyl. Preferably, tetrahydrofuranyl, tetrahydropyranyl, and dioxanyl are included. More preferably, tetrahydropyranyl is included.

The term "C₅₋₆ cycloalkene ring" refers to a monocyclic unsaturated hydrocarbon ring having 5 to 6 carbon atoms and comprising at least one double bond. "C₅₋₆ cycloalkene ring" includes cyclopentene, cyclopentadiene, cyclohexene, and cyclohexadiene. A preferable C₅₋₆ cycloalkene ring includes cyclopentene.

The term "5- to 7-membered heterocycloalkene ring" refers to a 5- to 7-membered monocyclic heterocycle comprising 1 or 2 heteroatoms independently selected from the group consisting of nitrogen and oxygen atoms, besides carbon atoms, as a ring-constituting atom, and comprising at least one double bond. "5-to 7-membered heterocycloalkene ring" includes, for example, dihydrofuran, dihydropyrrole, pyrane, dihydropyran, oxazine, and tetrahydroazepine. Preferably, dihydrofuran, dihydropyrrole, and dihydropyran are included. More preferably, dihydropyran is included.

The term "5- to 7-membered heteroaromatic ring" refers to a 5- to 7-membered aromatic heterocycle comprising 1 to 3 heteroatoms independently selected from the group consisting of nitrogen and oxygen atoms, besides carbon atoms, as a ring-constituting atom. "5- to 7-membered heteroaromatic ring" includes, for example, pyrrole, furan, imidazole, pyrazole, triazole, oxazole, isoxazole, pyridine, pyrazine, pyrimidine, pyridazine, triazine, azepine, diazepine, and oxepine.

The "5- to 7-membered heteroaromatic ring comprising 1 or 2 heteroatoms independently selected from the group consisting of nitrogen and oxygen atoms" includes, for example, pyrrole, furan, imidazole, pyrazole, oxazole, isoxazole, pyridine, pyrazine, pyrimidine, pyridazine, azepine, diazepine, and oxepine. Preferably, oxazole is included.

The term "5- to 6-membered heteroaromatic ring" refers to a 5- to 6-membered heteroaromatic ring among the above-defined "5-to 7-membered heteroaromatic ring", and includes a 5- to 6-membered heteroaromatic ring comprising 1 to 3 nitrogen atoms. The "5- to 6-membered heteroaromatic ring comprising 1 to 3 nitrogen atoms" includes, for example, pyrazole, triazole, and pyridine. Preferably, pyrazole, and triazole are included.

The term "bi- or tri-cyclic fused ring group" refers to a bi- or tri-cyclic fused ring group wherein the above-defined "5-to 6-membered heteroaryl comprising 1 to 3 nitrogen atoms" or phenyl is fused with the above-defined "C₅₋₆ cycloalkene ring", "5- to 7-membered heterocycloalkene ring", a benzene ring, or "5-to 7-membered heteroaromatic ring". The "bi- or tri-cyclic fused ring group" includes, for example, the following ring groups:

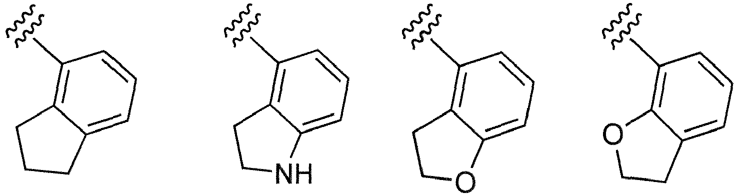

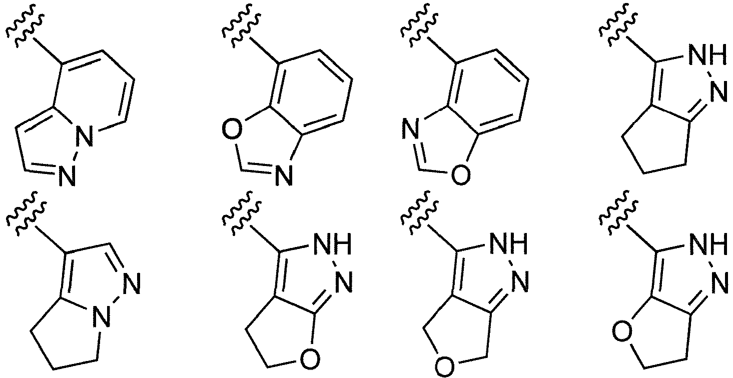

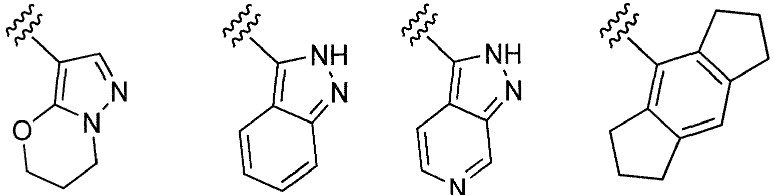

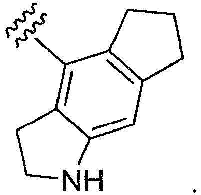

Preferably, the following ring groups: 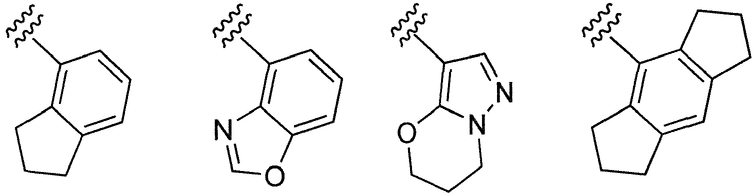 are included. When Ring Cy is a bicyclic fused ring group, it is meant that, for example, in the following ring group: 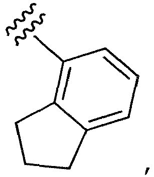 R^{F} and R^{G} combine together with the atoms to which they attach to form a ring, so that Ring Cy forms a bicyclic fused ring group.

The term "C₅₋₁₃ spiro cycloalkyl" refers to a saturated hydrocarbon ring group having 5 to 13 carbon atoms. "C₅₋₁₃ spiro cycloalkyl" includes, for example, the following groups:

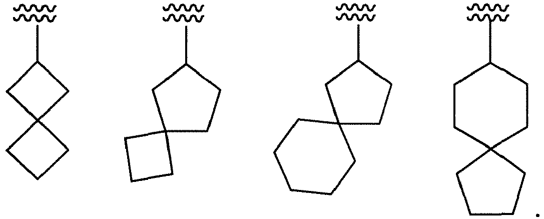

The term "7- to 11-membered spiro heterocycloalkyl" refers to a 7- to 11-membered saturated heterocyclyl group comprising 1 to 3 heteroatoms independently selected from the group consisting of nitrogen and oxygen atoms, besides carbon atoms, as a ring-constituting atom. "7- to 11-membered spiro heterocycloalkyl" includes, for example, the following groups:

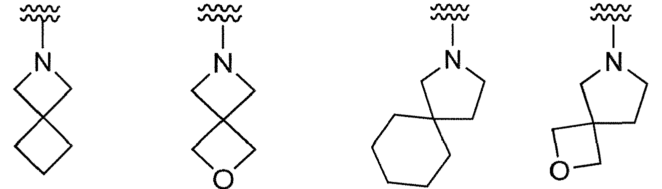

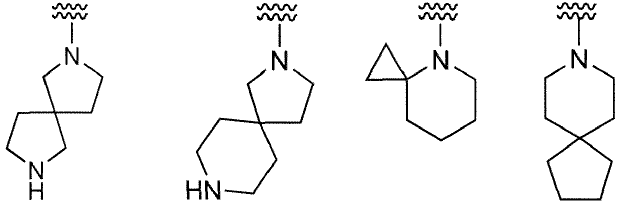

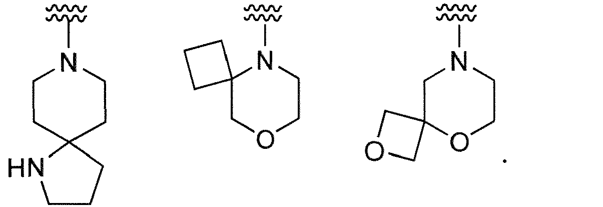

Preferably, the following groups: 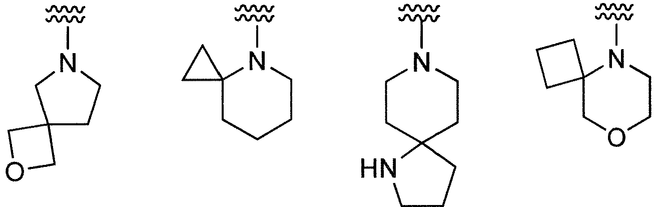 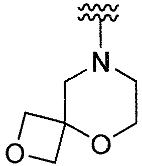 are included.

The "7- to 11-membered spiro heterocycloalkyl comprising 1 or 2 heteroatoms independently selected from the group consisting of nitrogen and oxygen atoms" includes, for example, the following groups:

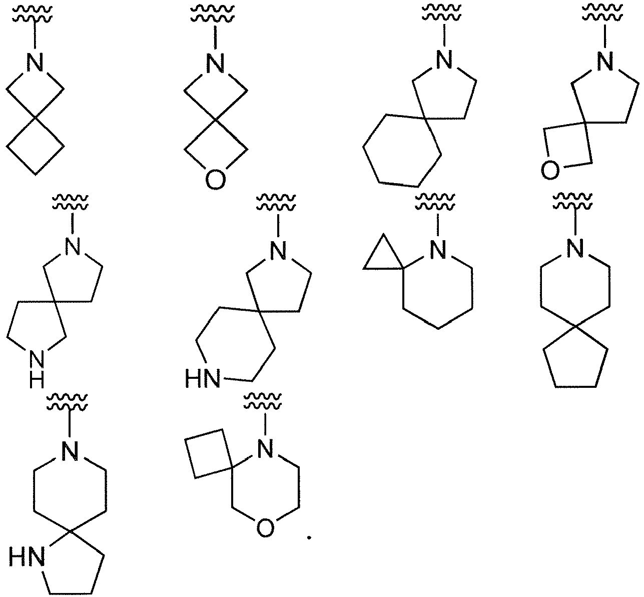

The "6- to 10-membered fused heterocycloalkyl" refers to a 6- to 10-membered fused heterocyclyl group comprising 1 or 2 heteroatoms independently selected from the group consisting of nitrogen and oxygen atoms, besides carbon atoms, as a ring-constituting atom. The "6- to 10-membered fused heterocycloalkyl" includes, for example, the following groups:

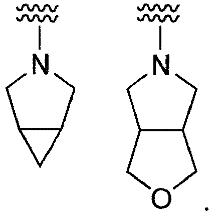

Preferably, the following group: 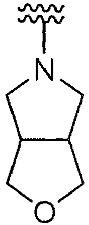 is included.

The term "5- to 9-membered bridged heterocycloalkyl" refers to a 5- to 9-membered saturated bridged heterocyclyl group comprising 1 or 2 heteroatoms independently selected from the group consisting of nitrogen and oxygen atoms, besides carbon atoms, as a ring-constituting atom. "5- to 9-membered bridged heterocycloalkyl" includes, for example, the following groups:

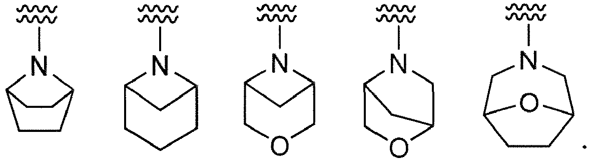

Preferably, the following groups: 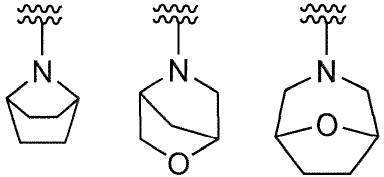 are included.

The phrase wherein α may be "optionally substituted with" β means that α is unsubstituted, or any of replaceable hydrogen atoms of α is replaced with β. For example, "C₁₋₆ alkyl optionally substituted with hydroxy" means that C₁₋₆ alkyl is unsubstituted, or any of hydrogen atoms of C₁₋₆ alkyl is replaced with hydroxy.

Embodiments of each substituent of a compound of Formula [I] and a compound of Formula [Ia], also referred to as "Compound [I] and Compound [Ia]" herein respectively, are illustrated as below. Each substituent of Compound [I] and Compound [Ia] is, however, not limited to these embodiments, and Compound [I] and Compound [Ia] also includes any combination of two or more of these embodiments in each substituent.

Herein, a partial structure: 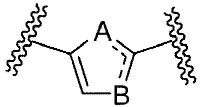 is preferably a group of the following formula:
or

More preferably, a partial structure: 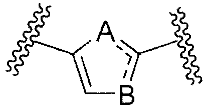 is a group of the following formula:

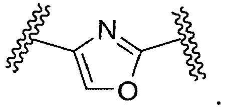

In another embodiment, a partial structure: 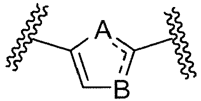 is preferably a group of the following formula:

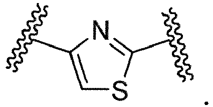

Preferably, R^{D} and R^{E} are each independently
(1) hydrogen,
(2) C₁₋₆ alkyl, wherein the alkyl may be optionally substituted with the same or different 1 to 3 R^{d1}s,
(3) C₁₋₄ haloalkyl,
(4) C₃₋₆ cycloalkyl, wherein the cycloalkyl may be optionally substituted with the same or different 1 to 3 halogen atoms,
(5) 4- to 7-membered heterocycloalkyl comprising 1 to 3 heteroatoms independently selected from the group consisting of nitrogen and oxygen atoms, wherein the heterocycloalkyl may be optionally substituted with the same or different 1 to 3 R^{d2}s, or
(6) 5- to 6-membered heteroaryl comprising 1 to 3 heteroatoms independently selected from the group consisting of nitrogen and oxygen atoms, wherein the heteroaryl may be optionally substituted with C₁₋₆ alkyl or C₁₋₄ haloalkyl.

In another embodiment, R^{D} and R^{E} preferably combine together with the nitrogen atom to which they attach to form:
(1) 4- to 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s1}s, and / or may be fused with a 5- to 6-membered heteroaromatic ring comprising 1 to 3 nitrogen atoms, wherein the heteroaromatic ring may be optionally substituted with 1 or 2 R^{d3}s,
(2) 7- to 11-membered spiro heterocycloalkyl comprising 1 to 3 heteroatoms independently selected from the group consisting of nitrogen and oxygen atoms, wherein the spiro heterocycloalkyl may be optionally substituted with the same or different 1 to 3 R^{d4}s, and / or may be fused with a benzene ring,
(3) 6- to 10-membered fused heterocycloalkyl comprising 1 or 2 heteroatoms independently selected from the group consisting of nitrogen and oxygen atoms, wherein the fused heterocycloalkyl may be optionally substituted with the same or different 1 to 3 R^{d5}s, or
(4) 5- to 9-membered bridged heterocycloalkyl comprising 1 or 2 heteroatoms independently selected from the group consisting of nitrogen and oxygen atoms, wherein the bridged heterocycloalkyl may be optionally substituted the same or different with 1 to 3 R^{d6}s.

Preferably, R^{Dy} and R^{Ey} are each independently,
(1) hydrogen,
(2) C₁₋₆ alkyl, wherein the alkyl may be optionally substituted with the same or different 1 to 3 R^{d1}s,
(3) C₃₋₆ cycloalkyl,
(4) 4- to 7-membered heterocycloalkyl comprising 1 to 3 heteroatoms independently selected from the group consisting of nitrogen and oxygen atoms, or
(5) 5- to 6-membered heteroaryl comprising 1 to 3 heteroatoms independently selected from the group consisting of nitrogen and oxygen atoms, wherein the heteroaryl may be optionally substituted with C₁₋₄ alkyl.

More preferably, R^{Dy} and R^{Ey} are each independently,
(1) hydrogen,
(2) C₁₋₄ alkyl, wherein the alkyl may be optionally substituted with the same or different 1 or 2 R^{d1}s,
(3) C₃₋₅ cycloalkyl selected from the group consisting of cyclopropyl, cyclobutyl and cyclopentyl,
(4) 5 or 6-membered heterocycloalkyl comprising at least one oxygen atom, or
(5) 5-membered heteroaryl comprising two nitrogen atoms, wherein the heteroaryl may be optionally substituted with methyl.

In another embodiment, R^{Dy} and R^{Ey} preferably combine together with the nitrogen atom to which they attach to form
(1) 4- to 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s1y}s and / or may be fused with a 5- to 6-membered heteroaromatic ring comprising 1 to 3 nitrogen atoms wherein the heteroaromatic ring may be optionally substituted with the same or different 1 or 2 R^{d3}s,
(2) 7- to 11-membered spiro heterocycloalkyl comprising 1 to 3 heteroatoms independently selected from the group consisting of nitrogen and oxygen atoms, wherein the spiro heterocycloalkyl may be optionally substituted with the same or different 1 to 3 R^{d4}s and/or may be optionally fused with a benzene ring,
(3) 6- to 10-membered fused heterocycloalkyl comprising 1 or 2 heteroatoms independently selected from the group consisting of nitrogen and oxygen atoms, wherein the fused heterocycloalkyl may be optionally substituted with the same or different 1 to 3 R^{d5}s, or
(4) 5- to 9-membered bridged heterocycloalkyl comprising 1 or 2 heteroatoms independently selected from the group consisting of nitrogen and oxygen atoms, wherein the bridged heterocycloalkyl may be optionally substituted with the same or different 1 to 3 R^{d6}s.

In another embodiment, R^{Dy} and R^{Ey} more preferably combine together with the nitrogen atom to which they attach to form
(1) 4- to 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s1y}s and / or may be fused with a 5- to 6-membered heteroaromatic ring comprising 1 to 3 nitrogen atoms wherein the heteroaromatic ring may be optionally substituted with the same or different 1 or 2 R^{d3}s,
(2) 7- to 11-membered spiro heterocycloalkyl comprising 1 to 3 heteroatoms independently selected from the group consisting of nitrogen and oxygen atoms, wherein the spiro heterocycloalkyl may be optionally substituted with the same or different 1 to 3 R^{d4}s,
(3) 6- to 10-membered fused heterocycloalkyl comprising 1 or 2 heteroatoms independently selected from the group consisting of nitrogen and oxygen atoms, or
(4) 5- to 9-membered bridged heterocycloalkyl comprising 1 or 2 heteroatoms independently selected from the group consisting of nitrogen and oxygen atoms.

In another embodiment, R^{Dy} and R^{Ey} more preferably combine together with the nitrogen atom to which they attach to form 4-to 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s1y}s and / or may be fused with a 5- to 6-membered heteroaromatic ring comprising 1 to 3 nitrogen atoms wherein the heteroaromatic ring may be optionally substituted with the same or different 1 or 2 R^{d3}s.

In another embodiment, R^{Dy} and R^{Ey} more preferably combine together with the nitrogen atom to which they attach to form 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s1y}s.

In another embodiment, R^{Dy} and R^{Ey} preferably combine together with the nitrogen atom to which they attach to form a partial structure: 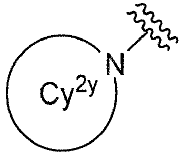 wherein Ring Cy^{2y} is as defined above.

In another embodiment, R^{Dy} and R^{Ey}, more preferably, combine together with the nitrogen atom to which they attach to form a group of the following formula: 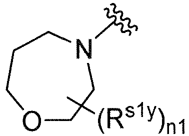 wherein R^{S1y} and n1 are as defined above.

In another embodiment, R^{Dy} and R^{Ey} more preferably combine together with the nitrogen atom to which they attach to form a group of the following formula: 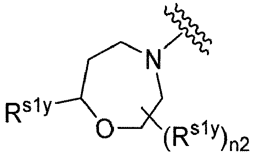 wherein R^{S1y} and n2 are as defined above.

In another embodiment, R^{Dy} and R^{Ey} more preferably combine together with the nitrogen atom to which they attach to form a group of the following formula: 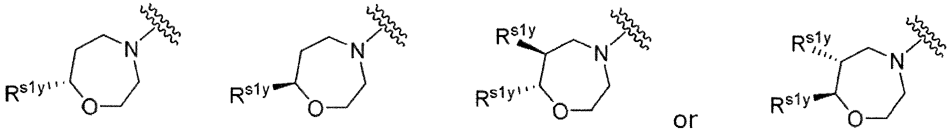 wherein R^{S1y} is as defined above.

In a specific embodiment, R^{Dy} and R^{Ey}, for example, combine together with the nitrogen atom to which they attach to form a group of the following formula:

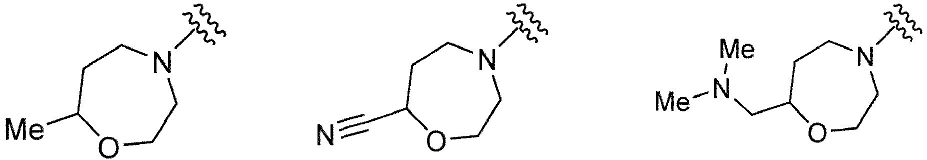

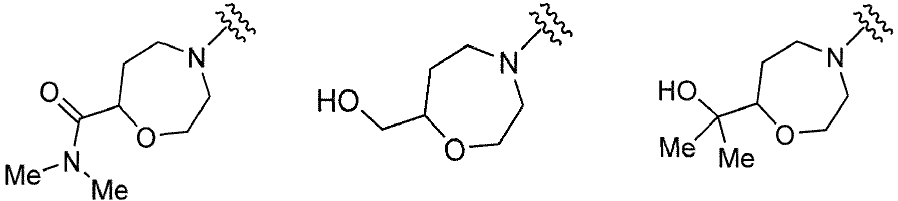

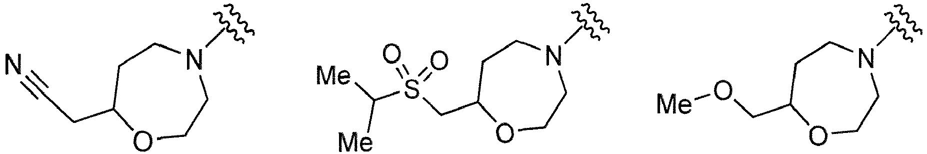

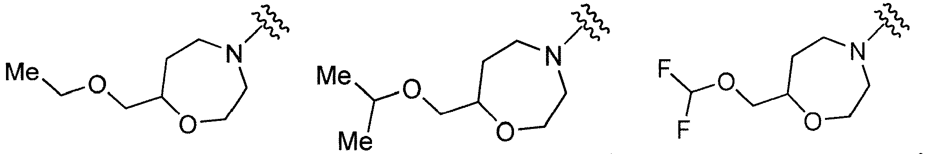

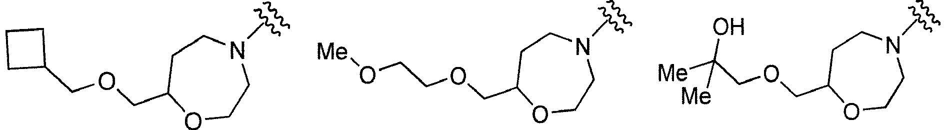

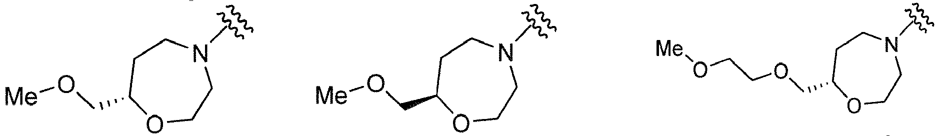

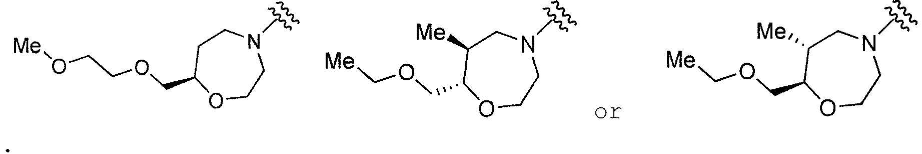

A preferable embodiment of Ring Cy is a group of the following formula: 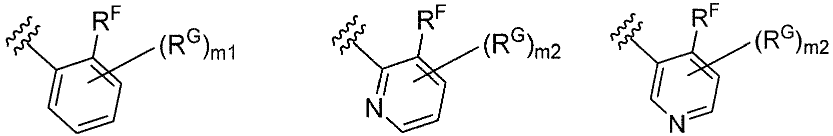 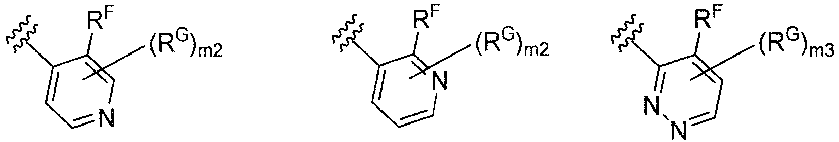 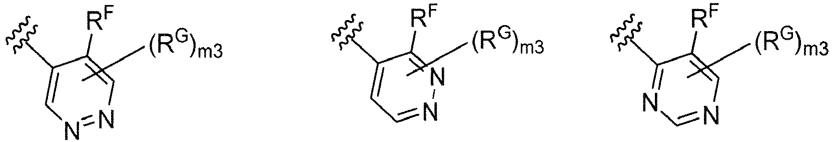 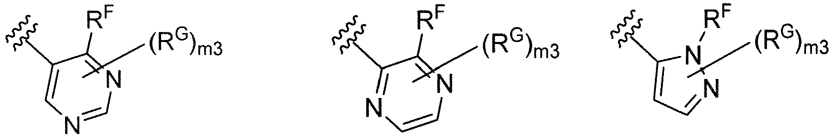 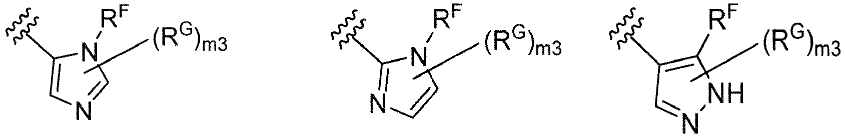 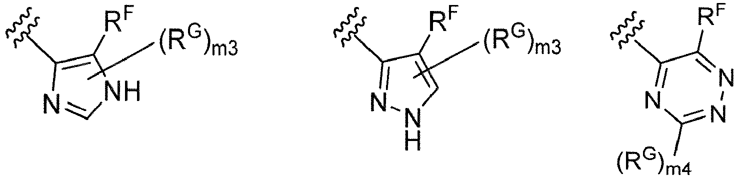 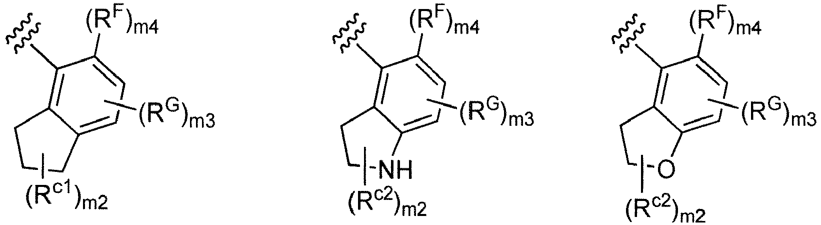 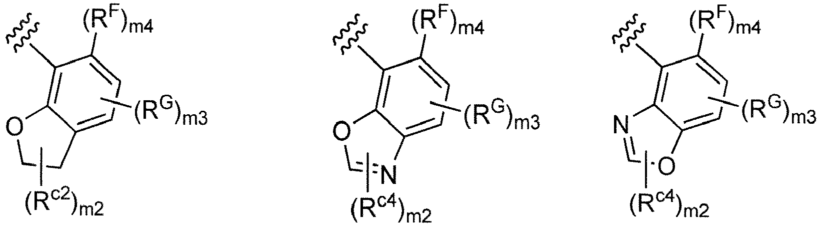 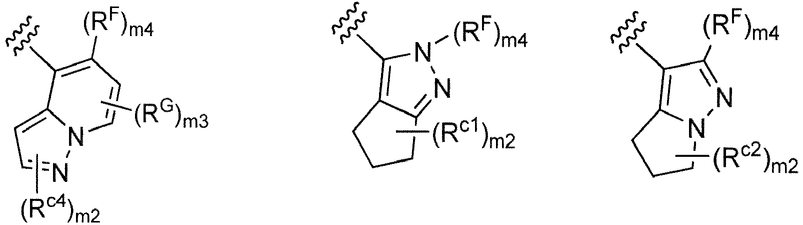 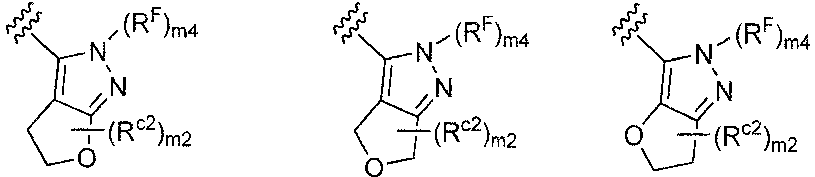 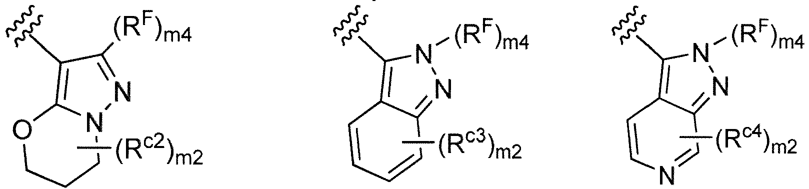 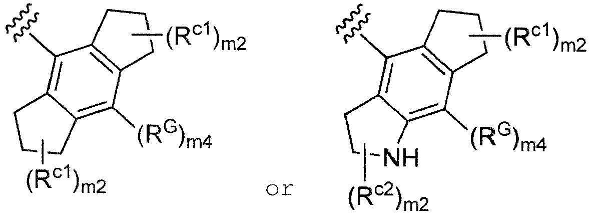 wherein m1, m2, m3, m4, R^{F}, R^{G}, R^{c1}, R^{c2}, R^{c3}, and R^{c4} are as defined above, and a more preferable one is a group of the following formula: 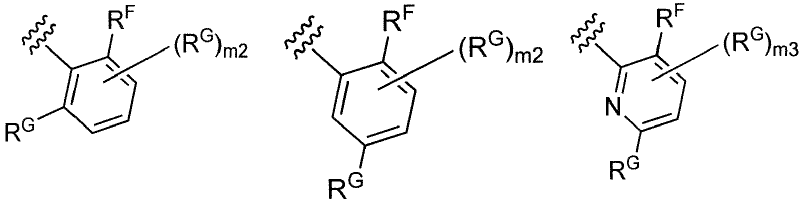 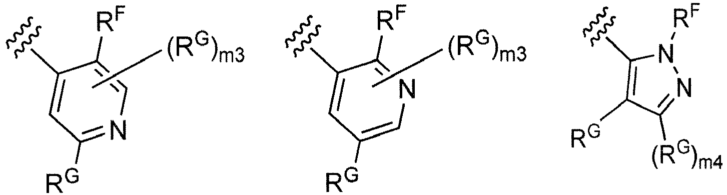 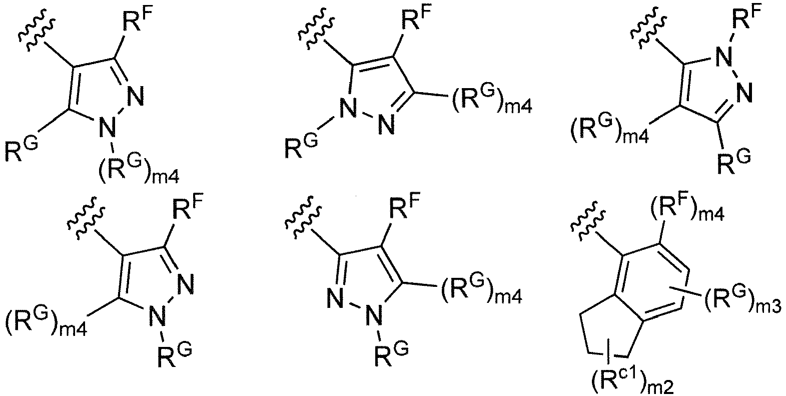 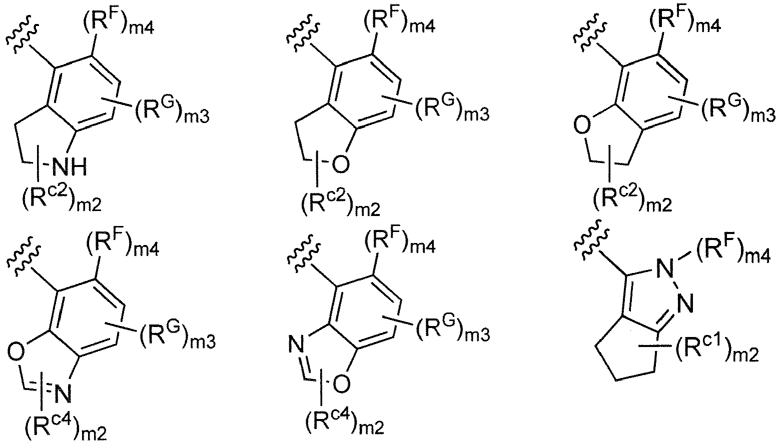 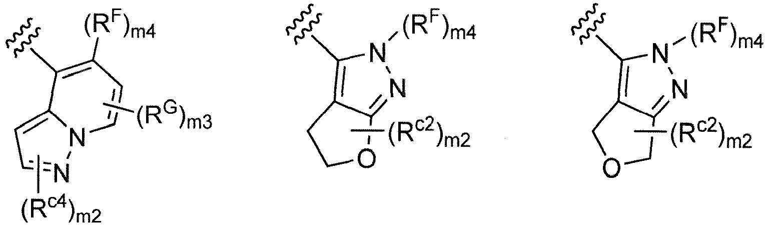 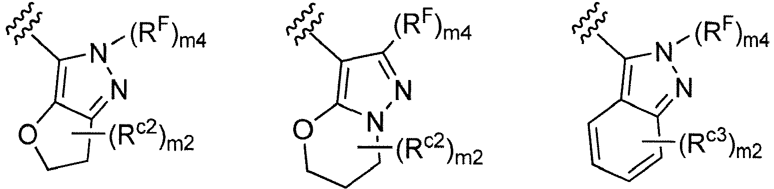 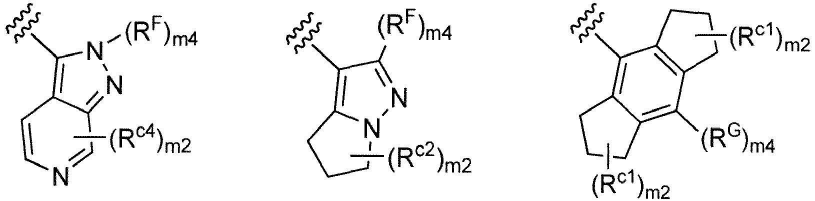 or 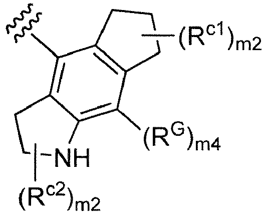 wherein m2, m3, m4, R^{F}, R^{G}, R^{c1}, R^{c2}, R^{c3}, and R^{c4} are as defined above.

In another preferable embodiment, Ring Cy is phenyl, wherein the phenyl is substituted with R^{F} at one of the atoms of α-position to a Cy ring-constituting atom attached to the NH group directly attached to the partial structure, and may be optionally substituted with the same or different 1 to 4 R^{G}s.

Ring Cy is more preferably a group of the following formula: 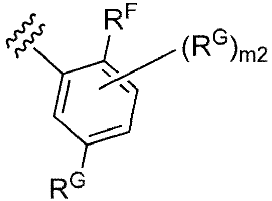 wherein R^{F}, R^{G} and m2 are as defined above.

Ring Cy is more preferably a group of the following formula: 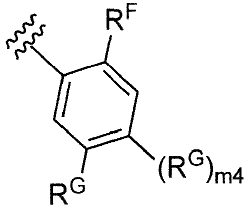 wherein R^{F} and R^{G} are as defined above.

Ring Cy is more preferably a group of the following formula: 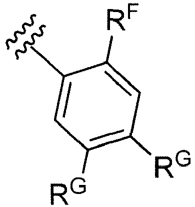 wherein R^{F} and R^{G} are as defined above.

In a specific embodiment, Ring Cy is, for example, a group of the following formula:

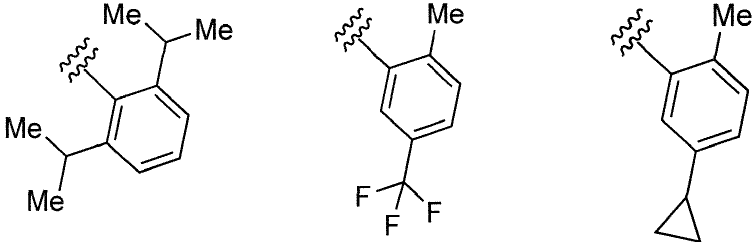

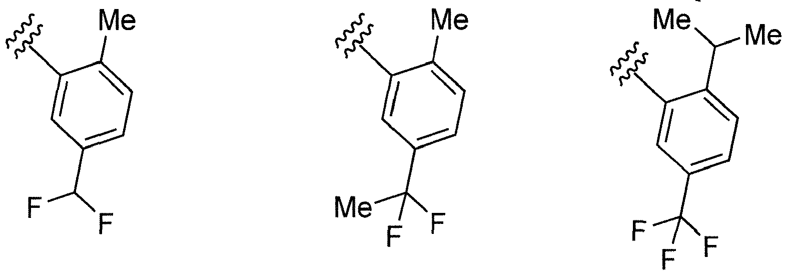

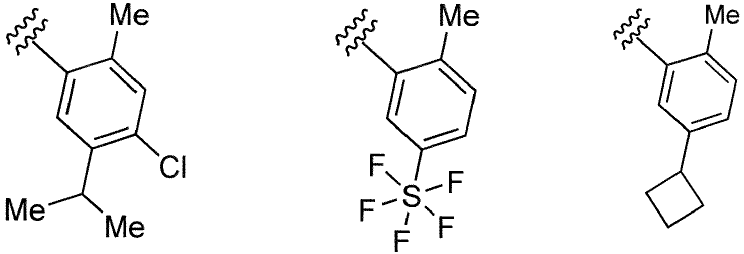

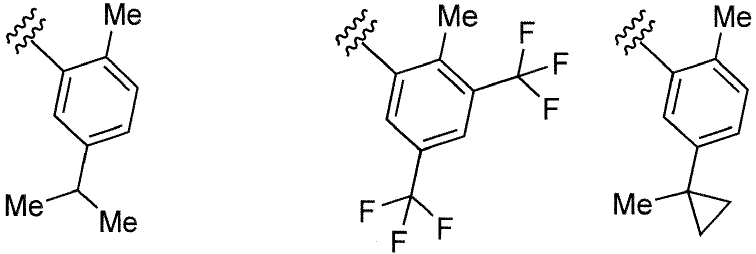

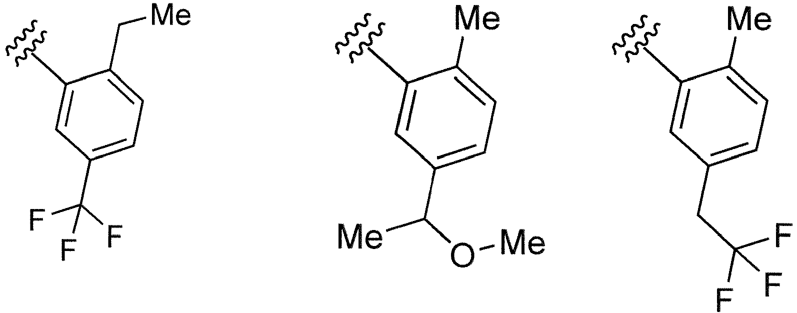

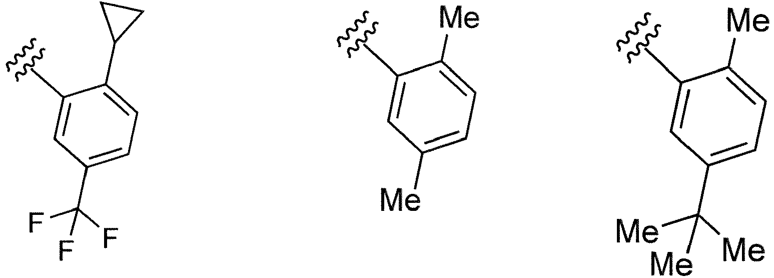

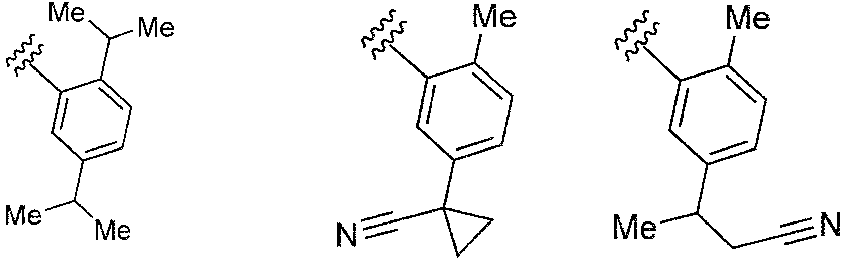

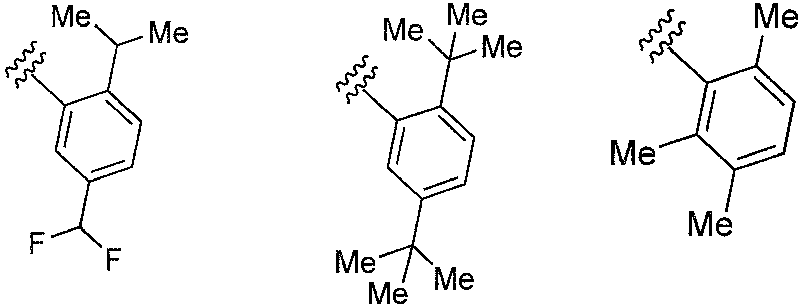

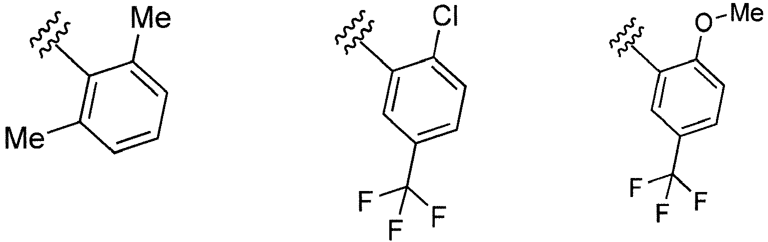

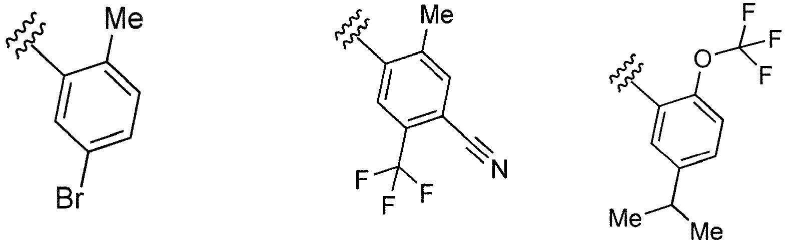

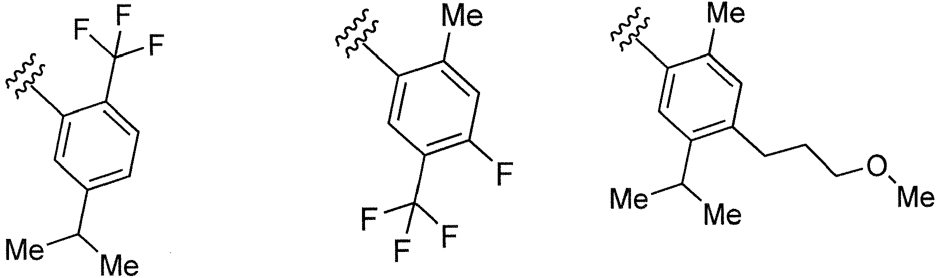

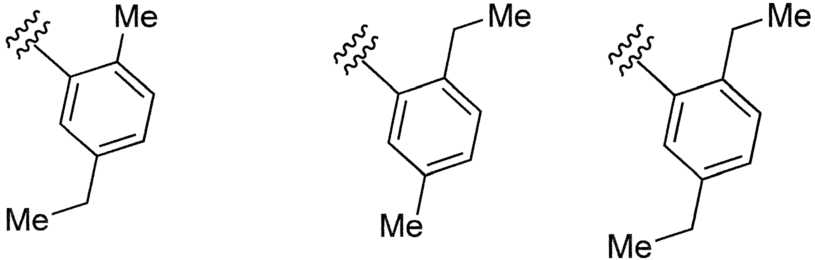

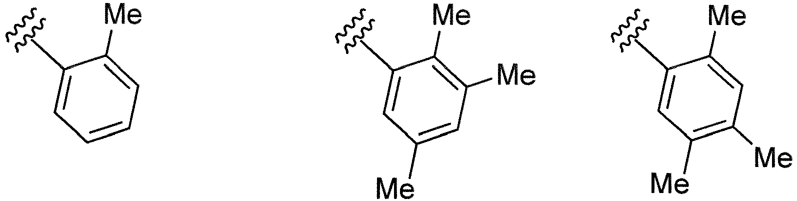

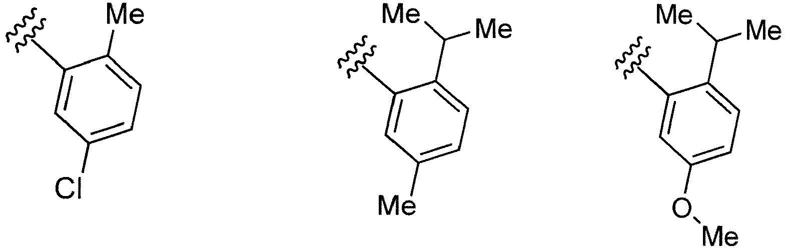

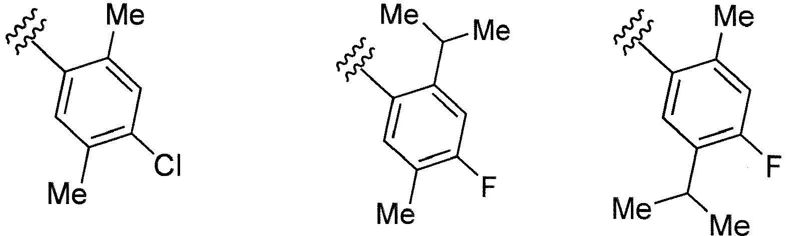

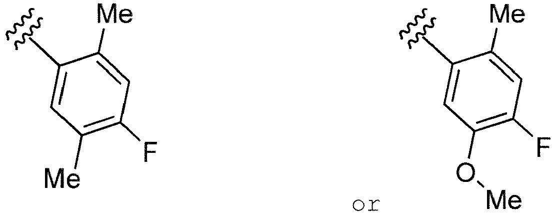

In another embodiment, Ring Cy is preferably a group of the following formula: 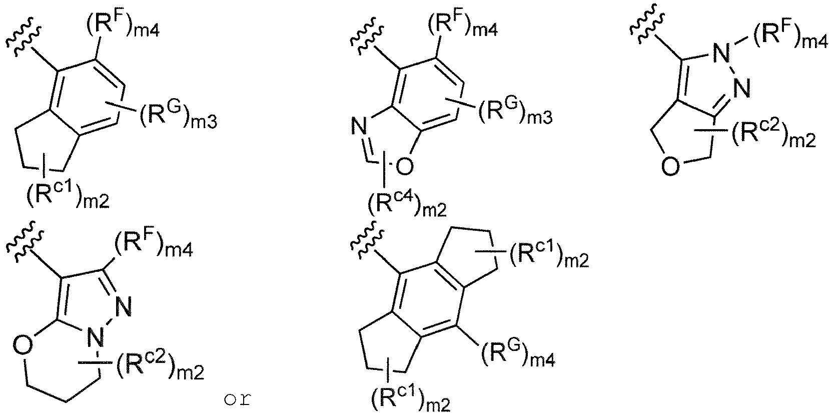 wherein R^{F}, R^{G}, R^{c1}, R^{c2}, R^{c4}, m2, m3 and m4 are as defined above.

Ring Cy is more preferably a group of the following formula: 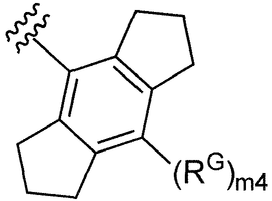 wherein R^{G} and m4 are as defined above.

R^{F} is preferably,
   (1) halogen,
   (2) C₁₋₄ alkyl,
   (3) C₁₋₄ haloalkyl,
   (4) C₁₋₄ alkoxy, wherein the alkoxy may be optionally substituted with the same or different 1 to 3 halogen atoms, or
   (5) C₃₋₅ cycloalkyl,
R^{G} is, preferably, each independently a substituent selected from the group consisting of:
   (1) halogen,
   (2) C₁₋₆ alkyl, wherein the alkyl may be optionally substituted with:
      (a) C₁₋₄ alkoxy, or
      (b) cyano,
   (3) C₁₋₄ haloalkyl,
   (4) C₁₋₄ alkoxy, wherein the alkoxy may be optionally substituted with the same or different 1 to 3 halogen atoms,
   (5) cyano,
   (6) pentafluorosulfanyl, and
   (7) C₃₋₇ cycloalkyl, wherein the cycloalkyl may be optionally substituted with the same or different 1 or 2 R^{s2}s, or alternatively,
in the case where R^{F} and R^{G} or two R^{G}s are substituted on neighboring atoms, then the R^{F} and R^{G} and/or the two R^{G}s may combine together with the atoms to which they attach to form:
   (a) a C₅₋₆ cycloalkene ring,
   (b) a 5- to 7-membered heterocycloalkene ring comprising 1 or 2 heteroatoms independently selected from the group consisting of nitrogen and oxygen atoms, wherein the heterocycloalkene ring may be optionally subsituted with the same or different 1 to 2 R^{c2}s, or
   (c) a 5- to 7-membered heteroaromatic ring comprising 1 or 2 heteroatoms independently selected from the group consisting of nitrogen and oxygen atoms,
   so that Ring Cy may form a bi- or tri-cyclic fused ring group.

R^{F} is more preferably,
   (1) methyl, ethyl, isopropyl, or tert-butyl,
   (2) C₁₋₄ haloalkyl,
   (3) C₁₋₄ alkoxy, wherein the alkoxy may be optionally substituted with the same or different 1 to 3 halogen atoms, or
   (4) C₃₋₅ cycloalkyl,
R^{G} is, more preferably, each independently a substituent selected from the group consisting of:
   (1) halogen,
   (2) C₁₋₆ alkyl, wherein the alkyl may be optionally substituted with:
      (a) C₁₋₄ alkoxy, or
      (b) cyano,
   (3) C₁₋₄ haloalkyl,
   (4) C₁₋₄ alkoxy, wherein the alkoxy may be optionally substituted with the same or different 1 to 3 halogen atoms,
   (5) cyano,
   (6) pentafluorosulfanyl, and
   (7) C₃₋₇ cycloalkyl, wherein the cycloalkyl may be optionally substituted with the same or different 1 or 2 R^{s2}s, or alternatively,
in the case where R^{F} and R^{G} or two R^{G}s are substituted on neighboring atoms, then the R^{F} and R^{G} and/or the two R^{G}s may combine together with the atoms to which they attach to form:
   (a) a C₅₋₆ cycloalkene ring,
   (b) a 5- to 7-membered heterocycloalkene ring comprising 1 or 2 heteroatoms independently selected from the group consisting of nitrogen and oxygen atoms, wherein the heterocycloalkene ring may be optionally subsituted with the same or different 1 or 2 R^{c2}s, or
   (c) a 5- to 7-membered heteroaromatic ring comprising 1 or 2 heteroatoms independently selected from the group consisting of nitrogen and oxygen atoms,
   so that Ring Cy may form a bi- or tri-cyclic fused ring group.
R^{F} is more preferably,
   (1) methyl, ethyl, isopropyl, or tert-butyl,
   (2) C₁₋₄ haloalkyl,
   (3) C₁₋₄ alkoxy, wherein the alkoxy may be optionally substituted with the same or different 1 to 3 halogen atoms, or
   (4) C₃₋₅ cycloalkyl,
R^{G} is, more preferably, each independently a substituent selected from the group consisting of:
   (1) halogen,
   (2) C₁₋₆ alkyl, wherein the alkyl may be optionally substituted with:
      (a) C₁₋₄ alkoxy, or
      (b) cyano,
   (3) C₁₋₄ haloalkyl,
   (4) C₁₋₄ alkoxy, wherein the alkoxy may be optionally substituted with the same or different 1 to 3 halogen atoms,
   (5) cyano,
   (6) pentafluorosulfanyl, and
   (7) C₃₋₇ cycloalkyl, wherein the cycloalkyl may be optionally substituted with the same or different 1 or 2 R^{s2}s.
R^{F} is more preferably,
   (1) methyl, ethyl, isopropyl, or tert-butyl,
   (2) trifluoromethyl,
   (3) methoxy, wherein the methoxy may be optionally substituted with three fluorine atoms, or
   (4) cyclopropyl or cyclobutyl,
R^{G} is, more preferably, each independently a substituent selected from the group consisting of:
   (1) halogen selected from the group consisting of fluorine, chlorine and bromine,
   (2) C₁₋₆ alkyl, wherein the alkyl may be optionally substituted with methoxy or cyano,
   (3) C₁₋₄ haloalkyl,
   (4) methoxy,
   (5) cyano,
   (6) pentafluorosulfanyl, and
   (7) C₃₋₅ cycloalkyl selected from the group consisting of cyclopropyl, cyclobutyl and cyclopentyl, wherein the cycloalkyl may be optionally substituted with methyl or cyano.

Each R^{c2} is, preferably, each independently C₁₋₄ alkyl.

Each R^{c2} is, more preferably, methyl.

Each R^{d1} is, preferably, each independently a substituent selected from the group consisting of:
(1) halogen,
(2) C₁₋₄ alkoxy,
(3) cyano,
(4) CONR^{e2}R^{e3}, wherein R^{e2} and R^{e3} are independently
   (a) hydrogen, or
   (b) C₁₋₄ alkyl,
(5) SO₂R^{e5}, wherein R^{e5} is C₁₋₄ alkyl
(6) NR^{e6}R^{e7}, wherein R^{e6} is
   (a) hydrogen, or
   (b) C₁₋₄ alkyl, and
      R^{e7} is
      (a) hydrogen,
      (b) C₁₋₄ alkyl, or
      (c) COR^{e8}, wherein R^{e8} is C₁₋₄ alkyl,
(7) C₃₋₆ cycloalkyl,
(8) phenyl, and
(9) 5- to 6-membered heteroaryl comprising 1 or 2 heteroatoms independently selected from the group consisting of nitrogen, oxygen, and sulfur atoms, wherein the heteroaryl may be optionally substituted with C₁₋₄ alkyl.

Each R^{d1} is, more preferably, each independently a substituent selected from the group consisting of:
(1) chlorine,
(2) methoxy,
(3) cyano,
(4) CONR^{e2}R^{e3}, wherein R^{e2} and R^{e3} are methyl,
(5) SO₂R^{e5}, wherein R^{e5} is methyl,
(6) NR^{e6}R^{e7}, wherein R^{e6} is hydrogen or methyl, and
   R^{e7} is
   (a) methyl, or
   (b) COR^{e8}, wherein R^{e8} is methyl,
(7) cyclopentyl or cyclohexyl,
(8) phenyl, and
(9) 5- to 6-membered heteroaryl comprising 1 or 2 nitrogen atoms, wherein the heteroaryl may be optionally substituted with methyl.

Each R^{d2} is, preferably, each independently C₁₋₄alkyl.

Each R^{d2} is, more preferably, each independently methyl.

Each R^{d3} is, preferably, each independently,
(1) C₁₋₄alkyl, or
(2) COOR^{g2}, wherein R^{g2} is hydrogen or C₁₋₄ alkyl.

Each R^{d3} is, more preferably, each independently,
(1) methyl, or
(2) COOR^{g2}, wherein R^{g2} is ethyl.

Each R^{d4} is preferably, each independently a substituent selected from the group consisting of:
(1) oxo, and
(2) C₁₋₄ alkyl.

Each R^{d4} is, more preferably, each independently a substituent selected from the group consisting of:
(1) oxo, and
(2) methyl.

Each R^{s1y} is, preferably, each independently a substituent selected from the group consisting of:
(1) halogen,
(2) hydroxy,
(3) C₁₋₆ alkyl, wherein the alkyl may be optionally substituted with the same or different 1 to 3 R^{11y}s,
(4) C₁₋₆ alkoxy,
(5) cyano,
(6) COR¹²,
(7) SO₂R¹³,
(8) NR^{t2}R^{t3}, wherein R^{t2} is
   (a) hydrogen, or
   (b) C₁₋₆ alkyl, and
      R^{t3} is
      (a) hydrogen,
      (b) C₁₋₆ alkyl,
      (c) COR¹⁴, or
      (d) SO₂R¹⁵,
(9) CONR^{t4}R^{t5}, wherein R^{t4} and R^{t5} are independently
   (a) hydrogen, or
   (b) C₁₋₆ alkyl, or alternatively, Rt4 and R^{t5} may combine together with the nitrogen atom to which they attach to form 4-to 6-membered heterocycloalkyl comprising 1 or 2 heteroatoms independently selected from the group consisting of nitrogen and oxygen atoms,
(10) SO₂NR^{t6}R^{t7}, wherein R^{t6} and R^{t7} are independently
   (a) hydrogen, or
   (b) C₁₋₆ alky,
(11) COOR^{t8}, wherein R^{t8} is hydrogen or C₁₋₄ alkyl,
(12) oxo,
(13) phenyl, and
(14) 5- to 6-membered heteroaryl comprising 1 to 3 heteroatoms independently selected from the group consisting of nitrogen, oxygen, and sulfur atoms, wherein the heteroaryl may be optionally substituted with the same or different 1 to 3 R¹⁷s.

Each R^{s1y} is, more preferably, each independently a substituent selected from the group consisting of:
(1) fluorine,
(2) hydroxy,
(3) C₁₋₄ alkyl, wherein the alkyl may be optionally substituted with the same or different 1 to 3 R^{11y}s,
(4) C₁₋₄ alkoxy,
(5) cyano,
(6) COR¹²,
(7) SO₂R¹³,
(8) NR^{t2}R^{t3}, wherein R^{t2} is methyl or ethyl,
   R^{t3} is
   (a) methyl or ethyl,
   (b) COR¹⁴, or
   (c) SO₂R¹⁵,
(9) CONR^{t4}R^{t5}, wherein R^{t4} and R^{t5} are methyl, or alternatively, R^{t4} and R^{t5} may combine together with the nitrogen atom to which they attach to form pyrrolidinyl,
(10) SO₂NR^{t6}R^{t7}, wherein R^{t6} and R^{t7} are independently methyl or ethyl,
(11) COOR^{t8}, wherein R^{t8} is methyl,
(12) oxo,
(13) phenyl, and
(14) 5- to 6-membered heteroaryl comprising 1 to 3 heteroatoms independently selected from the group consisting of nitrogen and oxygen atoms, wherein the heteroaryl may be optionally substituted with the same or different 1 or 2 R¹⁷s.

Each R^{S2} is, preferably, each independently a substituent selected from the group consisting of:
(1) C₁₋₄ alkyl, and
(2) cyano.

Each R^{S2} is, more preferably, each independently a substituent selected from the group consisting of:
(1) methyl, and
(2) cyano.

Each R^{11y} is , preferably, each independently a substituent selected from the group consisting of:
(1) halogen,
(2) hydroxy,
(3) C₁₋₆ alkoxy, wherein the alkoxy may be optionally substituted with 1 to 3 substituents independently selected from the group consisting of:
   (a) halogen,
   (b) hydroxy,
   (c) C₁₋₄ alkoxy, and
   (d) C₃₋₆ cycloalkyl, wherein the cycloalkyl may be optionally substituted with the same or different 1 to 3 R^{1dy}s,
(4) cyano,
(5) NR²¹R²², wherein R²¹ is
   (a) hydrogen, or
   (b) C₁₋₄ alkyl,
      R²² is
      (a) hydrogen, or
      (b) C₁₋₄ alkyl,
(6) SO₂R^{24y}, wherein R^{24y} is C₁₋₆ alkyl, wherein the alkyl may be optionally substituted with 1 to 3 substituents independently selected from the group consisting of:
   (a) halogen, and
   (b) C₃₋₆ cycloalkyl, and
(7) 4- to 6-membered heterocycloalkyl comprising 1 or 2 heteroatoms independently selected from the group consisting of nitrogen and oxygen atoms,
(8) 5- to 6-membered heteroaryl comprising 1 or 2 heteroatoms independently selected from the group consisting of nitrogen and oxygen atoms, wherein the heteroaryl may be optionally substituted with the same or different 1 to 3 R^{1ey}s.

Each R^{11y} is, more preferably, each independently a substituent selected from the group consisting of:
(1) halogen,
(2) hydroxy,
(3) C₁₋₆ alkoxy, wherein the alkoxy may be optionally substituted with 1 to 3 substituents independently selected from the group consisting of:
   (a) halogen,
   (b) hydroxy,
   (c) C₁₋₄ alkoxy, and
   (d) C₃₋₆ cycloalkyl, wherein the cycloalkyl may be optionally substituted with the same or different 1 to 3 R^{1dy}s,
(4) cyano,
(5) NR²¹R²², wherein R²¹ and R²² are each independently
   (a) hydrogen, or
   (b) C₁₋₄ alkyl,
(6) SO₂R^{24y}, wherein R^{24y} is C₁₋₆ alkyl, wherein the alkyl may be optionally substituted with 1 to 3 substituents independently selected from the group consisting of:
   (a) halogen, and
   (b) C₃₋₆ cycloalkyl, and
(7) 5- to 6-membered heteroaryl comprising 1 or 2 heteroatoms independently selected from the group consisting of nitrogen and oxygen atoms, wherein the heteroaryl may be optionally substituted with the same or different 1 to 3 R^{1ey}s.

Each R^{11y} is, more preferably, each independently a substituent selected from the group consisting of:
(1) fluorine,
(2) hydroxy,
(3) C₁₋₄ alkoxy, wherein the alkoxy may be optionally substituted with 1 or 2 substituents independently selected from the group consisting of:
   (a) fluorine,
   (b) hydroxy,
   (c) methoxy, and
   (d) cyclobutyl, wherein the cyclobutyl may be optionally substituted with the same or different 1 or 2 R^{1dy}s,
(4) cyano,
(5) NR²¹R²², wherein R²¹ and R²² are methyl,
(6) SO₂R^{24y}, wherein R^{24y} is C₁₋₄ alkyl, wherein the alkyl may be optionally substituted with 1 to 3 substituents independently selected from the group consisting of:
   (a) fluorine, and
   (b) cyclopropyl, and
(7) 5- to 6-membered heteroaryl comprising 1 or 2 heteroatoms independently selected from the group consisting of nitrogen and oxygen atoms, wherein the heteroaryl may be optionally substituted with the same or different 1 to 3 R^{1ey}s.

R¹⁷ and R^{1ey} are, preferably, each independently a substituent selected from the group consisting of:
(1) C₁₋₆ alkyl,
(2) C₁₋₄ haloalkyl, and
(3) C₁₋₆ alkoxy.

R¹⁷ and R^{1ey} are, more preferably, each independently a substituent selected from the group consisting of:
(1) C₁₋₄ alkyl,
(2) C₁₋₄ haloalkyl, and
(3) C₁₋₄ alkoxy.

R¹², R¹³, R¹⁴, and R¹⁵ are, preferably, each independently a substituent selected from the group consisting of:
(1) C₁₋₆ alkyl,
(2) C₁₋₄ haloalkyl,
(3) C₃₋₆ cycloalkyl, and
(4) 4- to 6-membered heterocycloalkyl comprising an oxygen atom.

R¹², R¹³, R¹⁴ and R¹⁵ are, more preferably, each independently:
(1) C₁₋₆ alkyl,
(2) C₁₋₄ haloalkyl,
(3) cyclopropyl, and
(4) tetrahydrofuranyl.

Each R^{1dy} is, preferably, each independently a substituent selected from the group consisting of:
(1) halogen,
(2) hydroxy, and
(3) C₁₋₄ alkyl.

Each R^{1dy} is , more preferably, each independently a substituent selected from the group consisting of:
(1) fluorine,
(2) hydroxy, and
(3) methyl.

One preferable embodiment of a compound of Formula [I] is Compound [I] wherein a partial structure: 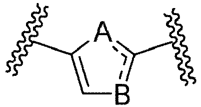 is a group of the following formula: 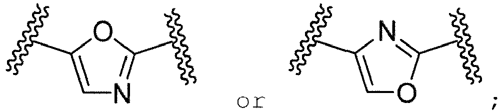
R^{D} and R^{E} are each independently
   (1) hydrogen,
   (2) C₁₋₆ alkyl, wherein the alkyl may be optionally substituted with the same or different 1 to 3 R^{d1}s,
   (3) C₁₋₄ haloalkyl,
   (4) C₃₋₆ cycloalkyl, wherein the cycloalkyl may be optionally substituted with the same or different 1 to 3 halogen atoms,
   (5) 4- to 7-membered heterocycloalkyl comprising 1 to 3 heteroatoms independently selected from the group consisting of nitrogen and oxygen atoms, wherein the heterocycloalkyl may be optionally substituted with the same or different 1 to 3 R^{d2}s, or
   (6) 5- to 6-membered heteroaryl comprising 1 to 3 heteroatoms independently selected from the group consisting of nitrogen and oxygen atoms, wherein the heteroaryl may be optionally substituted with C₁₋₆ alkyl or C₁₋₄ haloalkyl; and
Ring Cy is: 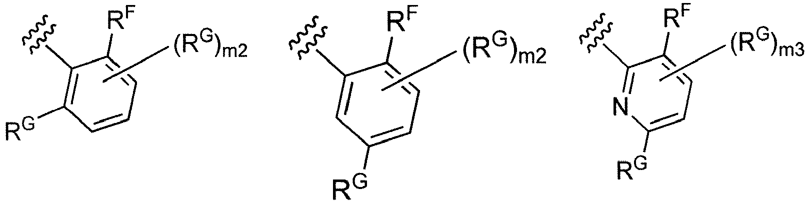 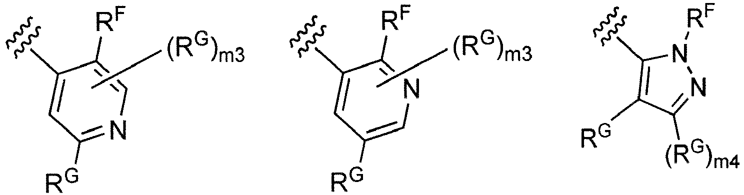 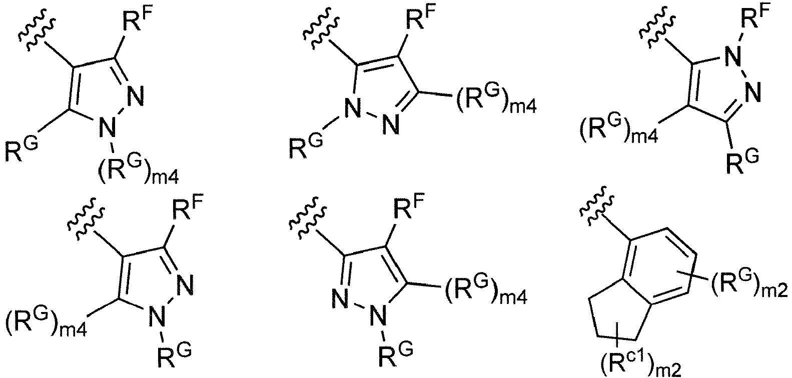 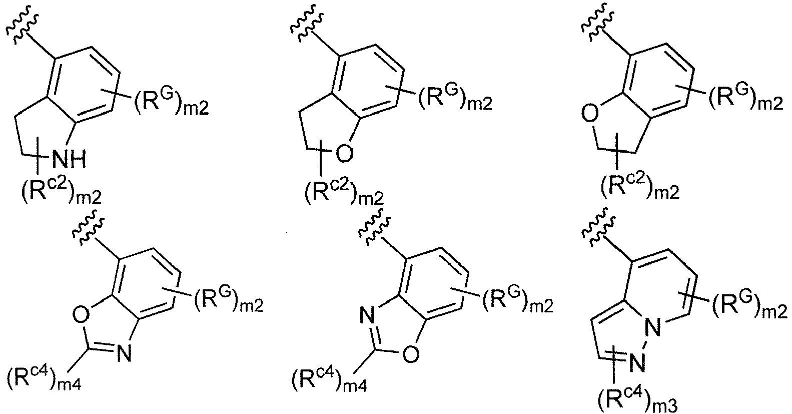 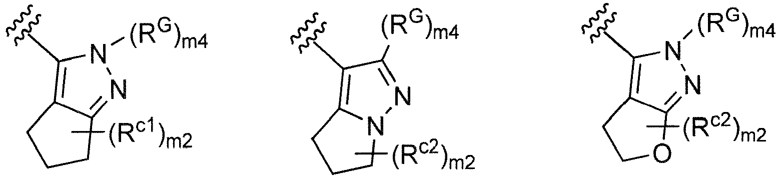 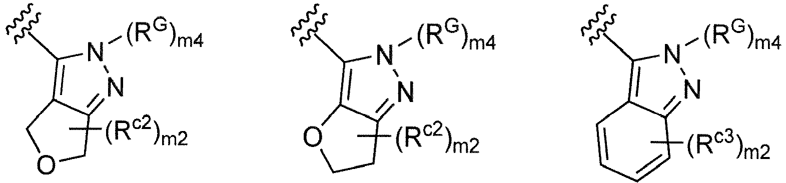 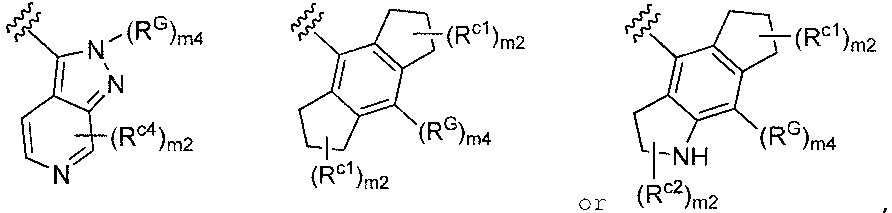 wherein m2, m3, m4, R^{F}, R^{G}, R^{c1}, R^{c2}, R^{c3}, and R^{c4} are as defined above.

Another embodiment of Compound [I] is Compound [I] wherein a partial structure: 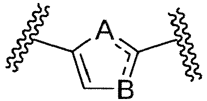 is a group of the following formula: 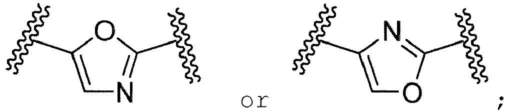
R^{D} and R^{E} combine together with the nitrogen atom to which they attach to form:
   (1) 4- to 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s1}s, and / or may be fused with a 5- to 6-membered heteroaromatic ring comprising 1 to 3 nitrogen atoms, wherein the heteroaromatic ring may be optionally substituted with the same or different 1 or 2 R^{d3}s,
   (2) 7- to 11-membered spiro heterocycloalkyl comprising 1 to 3 heteroatoms independently selected from the group consisting of nitrogen and oxygen atoms, wherein the spiro heterocycloalkyl may be optionally substituted with the same or different 1 to 3 R^{d4}s, and / or may be fused with a benzene ring,
   (3) 6- to 10-membered fused heterocycloalkyl comprising 1 or 2 heteroatoms independently selected from the group consisting of nitrogen and oxygen atoms, wherein the fused heterocycloalkyl may be optionally substituted with the same or different 1 to 3 R^{d5}s, or
   (4) 5- to 9-membered bridged heterocycloalkyl comprising 1 or 2 heteroatoms independently selected from the group consisting of nitrogen and oxygen atoms, wherein the bridged heterocycloalkyl may be optionally substituted with the same or different 1 to 3 R^{d6}s; and
Ring Cy is: 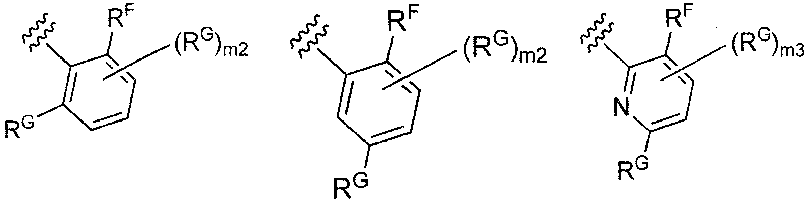 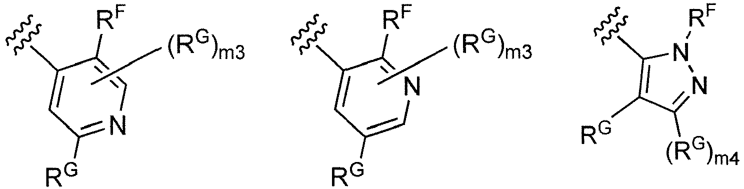 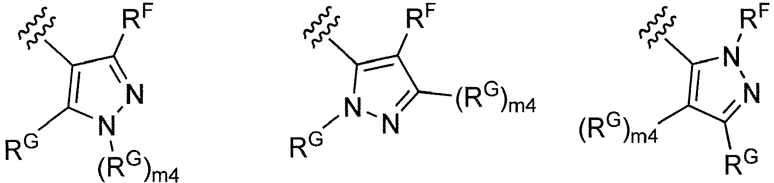 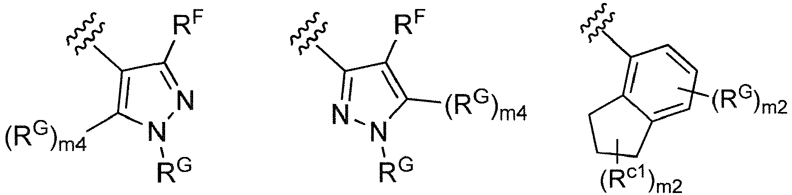 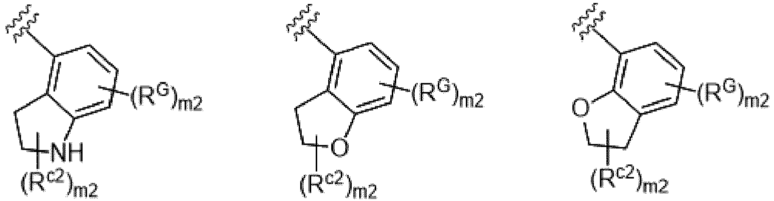 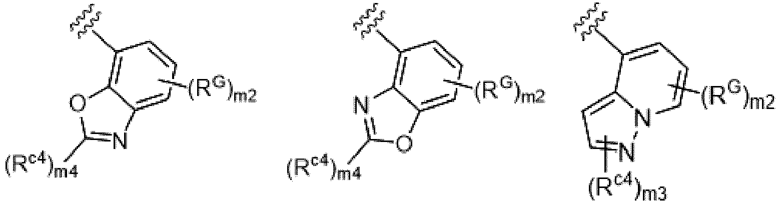 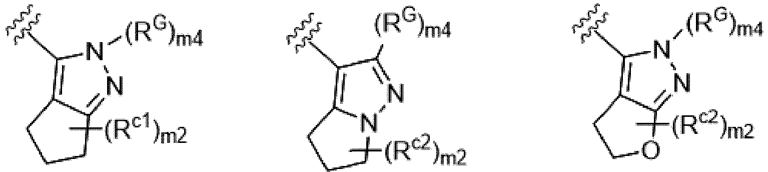 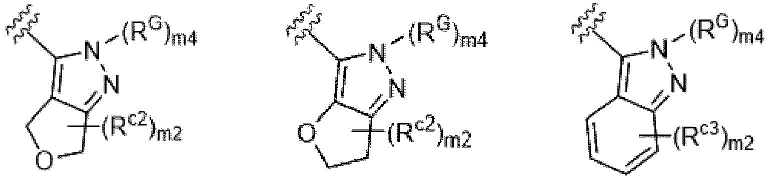 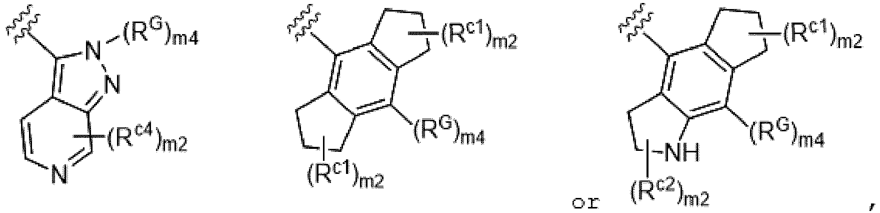 wherein m2, m3, m4, R^{F}, R^{G}, R^{c1}, R^{c2}, R^{c3}, and R^{c4} are as defined above.

In one preferable embodiment, a compound of Formula [Ia] wherein a partial structure: 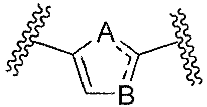 is 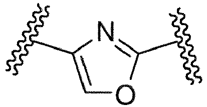 is a compound of Formula [IIb]: 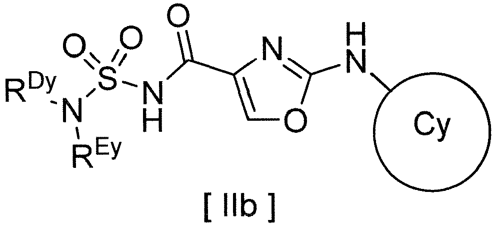 wherein R^{Dy}, R^{Ey}, Ring Cy are as defined above.

Another preferable embodiment of a compound of Formula [Ia] is a compound of Formula [IIIb]: 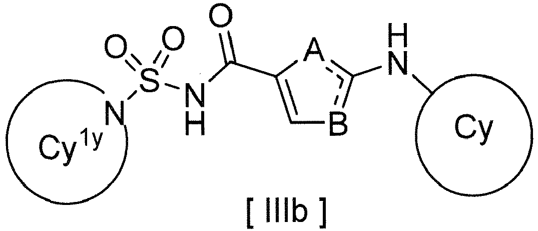 wherein
Ring Cy^{1y} is
   (1) 4- to 7-membered heterocycloalkyl comprising 1 or 2 heteroatoms independently selected from the group consisting of nitrogen, oxygen, and sulfur atoms, wherein the heterocycloalkyl comprises at least one nitrogen atom, and may be optionally substituted with the same or different 1 to 3 R^{s1y}, and / or may be fused with a 5- to 6-membered heteroaromatic ring comprising 1 to 3 nitrogen atoms wherein the heteroaromatic ring may be optionally substituted with the same or different 1 or 2 R^{d3}s,
   (2) 7- to 11-membered spiro heterocycloalkyl comprising 1 to 3 heteroatoms independently selected from the group consisting of nitrogen and oxygen atoms, wherein the spiro heterocycloalkyl may be optionally substituted with the same or different 1 to 3 R^{d4}s,
   (3) 6- to 10-membered fused heterocycloalkyl comprising 1 or 2 heteroatoms independently selected from the group consisting of nitrogen and oxygen atoms, or
   (4) 5- to 9-membered bridged heterocycloalkyl comprising 1 or 2 heteroatoms independently selected from the group consisting of nitrogen and oxygen atoms,
Each R^{d3}, R^{d4}, R^{s1y}, Ring Cy, and the partial structure comprising A and B are as defined above.

One other preferable embodiment of a compound of Formula [Ia] is a compound of Formula [II]: 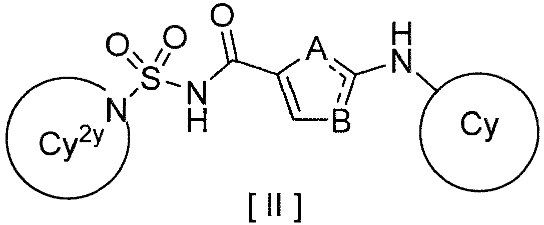 wherein Ring Cy^{2y}, Ring Cy, and the partial structure comprising A and B are as defined above.

One other preferable embodiment of a compound of Formula [Ia] is a compound of Formula [III]: 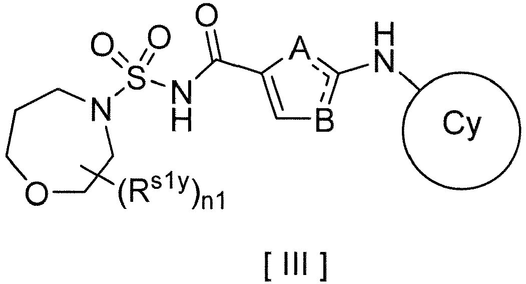 wherein n1, R^{s1y}, Ring Cy, and the partial structure comprising A and B are as defined above.
one other preferable embodiment of a compound of Formula [Ia] is a compound of Formula [IV]: 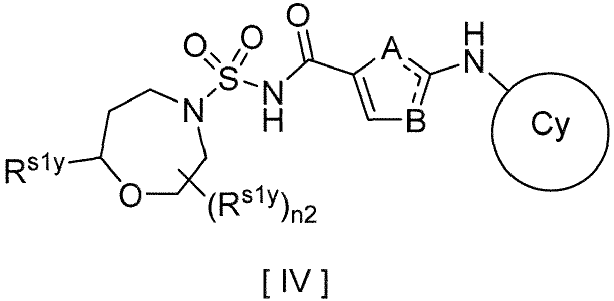 wherein n2, R^{s1y}, Ring Cy, and the partial structure comprising A and B are as defined above.

One other preferable embodiment of a compound of Formula [Ia] is a compound of Formula [IV] wherein

Each R^{S1y} is each independently a substituent selected from the group consisting of:
(1) fluorine,
(2) hydroxy,
(3) C₁₋₄ alkyl, wherein the alkyl may be optionally substituted with the same or different 1 to 3 R^{11y}s,
(4) C₁₋₄ alkoxy,
(5) cyano,
(6) COR¹²,
(7) SO₂ R¹³,
(8) NR^{t2}R^{t3}, wherein R^{t2} is methyl or ethyl, and
   R^{t3} is
   (a) methyl or ethyl,
   (b) COR¹⁴, or
   (c) SO₂R¹⁵,
(9) CONR^{t4}R^{t5}, wherein R^{t4} and R^{t5} are methyl or alternatively, R^{t4} and R^{t5} may combine together with the nitrogen atom to which they attach to form pyrrolidinyl,
(10) SO₂NR^{t6}R^{t7}, wherein R^{t6} and R^{t7} are independently methyl or ethyl,
(11) COOR^{t8}, wherein R^{t8} is methyl,
(12) oxo,
(13) phenyl, and
(14) 5- to 6-membered heteroaryl comprising 1 to 3 heteroatoms independently selected from the group consisting of nitrogen and oxygen atoms, wherein the heteroaryl may be optionally substituted with the same or different 1 or 2 R¹⁷s.

One other preferable embodiment of a compound of Formula [Ia] is a compound of Formula [IVb], Formula [Vb], Formula [VIb] or Formula [VIIb]: 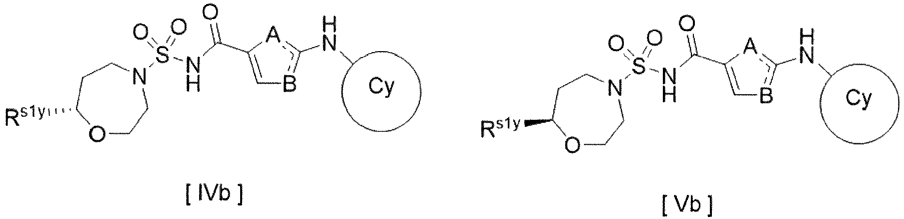 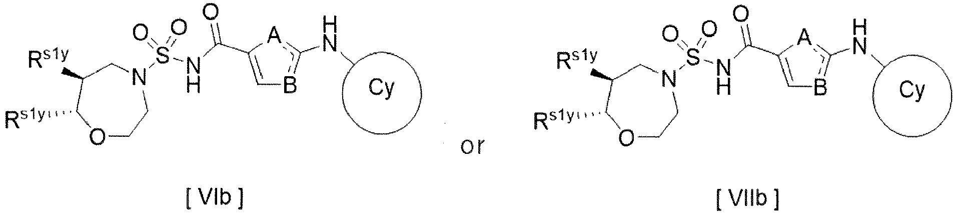 wherein
R^{S1y} is each independently a substituent selected from the group consisting of:,
   (1) fluorine,
   (2) hydroxy,
   (3) C₁₋₄ alkyl, wherein the alkyl may be optionally substituted with the same or different 1 to 3 R^{11y}s,
   (4) C₁₋₄ alkoxy,
   (5) cyano,
   (6) COR¹²,
   (7) SO₂R¹³,
   (8) NR^{t2}R^{t3}, wherein R^{t2} is methyl or ethyl, and
      R^{t3} is
      (a) methyl or ethyl,
      (b) COR¹⁴, or
      (c) SO₂R¹⁵,
   (9) CONR^{t4}R^{t5}, wherein R^{t4} and R^{t5} are methyl or alternatively, R^{t4} and R^{t5} may combine together with the nitrogen atom to which they attach to form pyrrolidinyl,
   (10) SO₂NR^{t6}R^{t7}, wherein R^{t6} and R^{t7} are independently methyl or ethyl,
   (11) COOR^{t8}, wherein R^{t8} is methyl,
   (12) oxo,
   (13) phenyl, and
   (14) 5- to 6-membered heteroaryl comprising 1 to 3 heteroatoms independently selected from the group consisting of nitrogen and oxygen atoms, wherein the heteroaryl may be optionally substituted with the same or different 1 or 2 R¹⁷s,
each of the other symbols has the same meaning as defined above.

One other preferable embodiment of a compound of Formula [Ia] is a compound of Formula [VIIIb]: 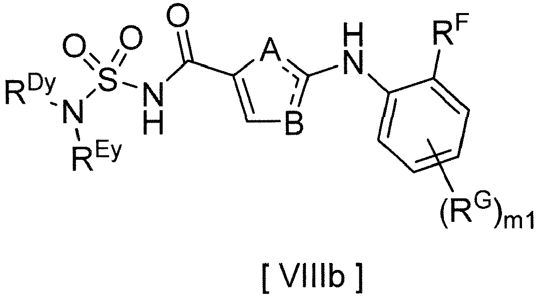 wherein
R^{Dy}, R^{Ey}, R^{F}, R^{G}, m1, and the partial structure comprising A and B are as defined above.

One other preferable embodiment of a compound of Formula [Ia] is a compound of Formula [IXb]: 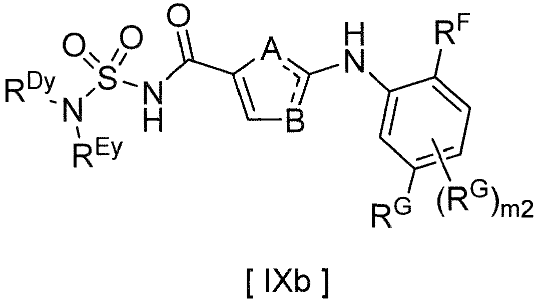 wherein
R^{Dy}, R^{Ey}, R^{F}, R^{G}, m2 and the partial structure comprising A and B are as defined above.

One other preferable embodiment of a compound of Formula [Ia] is a compound of Formula [Xb]: 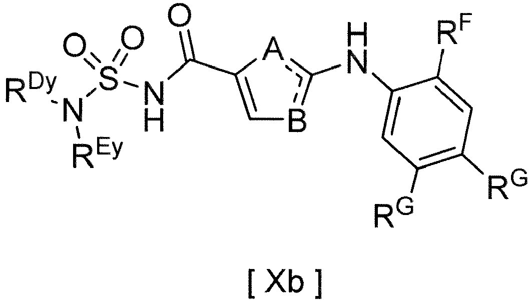 wherein R^{Dy}, R^{Ey}, R^{F}, R^{G} and the partial structure comprising A and B are as defined above.

One other preferable embodiment of a compound of Formula [Ia] is a compound of Formula [Xb], wherein
R^{F} is
   (1) methyl, ethyl, isopropyl, or tert-butyl,
   (2) trifluoromethyl,
   (3) methoxy, wherein the methoxy may be optionally substituted with three fluorine atoms, or
   (4) cyclopropyl or cyclobutyl,
R^{G} is each independently a substituent selected from the group consisting of:
   (1) fluorine, chlorine or bromine,
   (2) C₁₋₆ alkyl, wherein the alkyl may be optionally substituted with methoxy or cyano,
   (3) C₁₋₄ haloalkyl,
   (4) methoxy,
   (5) cyano,
   (6) pentafluorosulfanyl, and
   (7) C₃₋₅ cycloalkyl selected from the group consisting of cyclopropyl, cyclobutyl and cyclopentyl, wherein the cycloalkyl may be optionally substituted with methyl or cyano.

One other preferable embodiment of a compound of Formula [Ia] is a compound of Formula [XIb]: 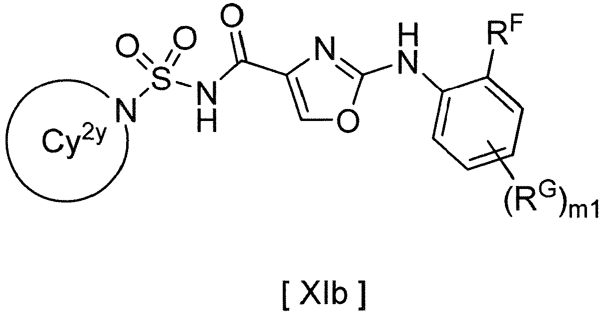 wherein Ring Cy^{2y}, R^{F}, R^{G} and m1 are as defined above.

One other preferable embodiment of a compound of Formula [Ia] is a compound of Formula [XIIb]: 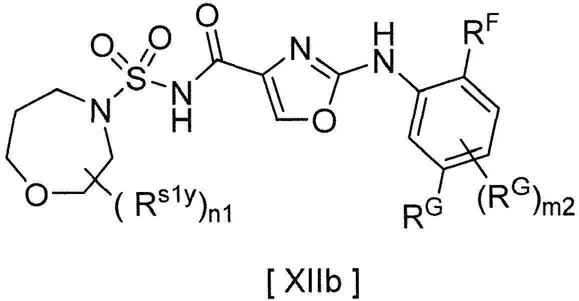 wherein R^{s1y}, R^{F}, R^{G}, n1, and m2 are as defined above.

One other preferable embodiment of a compound of Formula [Ia] is a compound of Formula [XIIIb]: 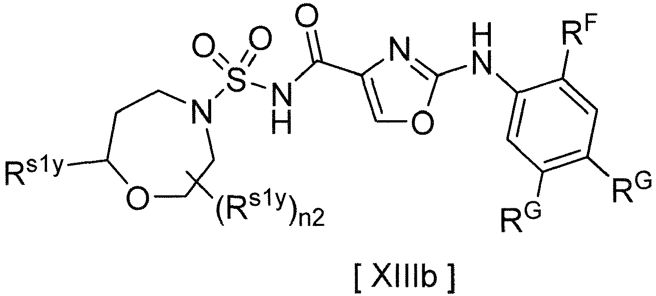 wherein R^{s1y}, R^{F}, R^{G}, and n2 are as defined above.

One other preferable embodiment of a compound of Formula [Ia] is a compound of Formula [XIVb], Formula [XVb], Formula [XVIb], or Formula [XVIIb]: 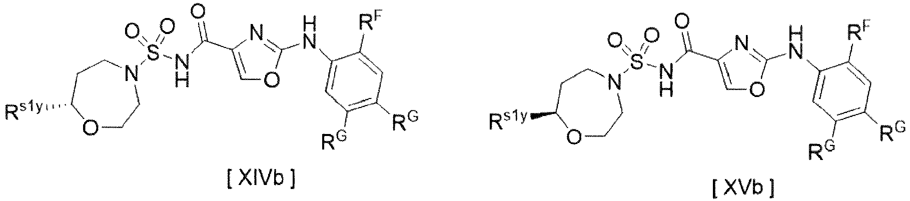 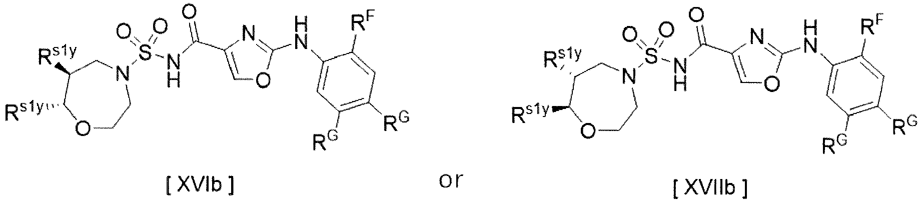 wherein
R^{S1y} is each independently a substituent selected from the group consisting of:
   (1) fluorine,
   (2) hydroxy,
   (3) C₁₋₄ alkyl, wherein the alkyl may be optionally substituted with the same or different 1 to 3 R^{11y}s,
   (4) C₁₋₄ alkoxy,
   (5) cyano,
   (6) COR¹²,
   (7) SO₂R¹³,
   (8) NR^{t2}R^{t3}, wherein R^{t2} is methyl or ethyl,
      R^{t3} is
      (a) methyl or ethyl,
      (b) COR¹⁴, or
      (c) SO₂R¹⁵,
   (9) CONR^{t4}R^{t5}, wherein R^{t4} and R^{t5} are methyl, or alternatively, R^{t4} and R^{t5} may combine together with the nitrogen atom to which they attach to form pyrrolidinyl,
   (10) SO₂NR^{t6}R^{t7}, wherein R^{t6} and R^{t7} are independently methyl or ethyl,
   (11) COOR^{t8}, wherein R^{t8} is methyl,
   (12) oxo,
   (13) phenyl, and
   (14) 5- to 6-membered heteroaryl comprising 1 to 3 heteroatoms independently selected from the group consisting of nitrogen and oxygen atoms, wherein the heteroaryl may be optionally substituted with the same or different 1 or 2 R¹⁷s,
R^{F} is
(1) methyl, ethyl, isopropyl, or tert-butyl,
(2) trifluoromethyl,
(3) methoxy, wherein the methoxy may be optionally substituted with three fluorine atoms, or
(4) cyclopropyl or cyclobutyl,
R^{G} is each independently a substituent selected from the group consisting of:
   (1) fluorine, chlorine or bromine,
   (2) C₁₋₆ alkyl, wherein the alkyl may be optionally substituted with methoxy or cyano,
   (3) C₁₋₄ haloalkyl,
   (4) methoxy,
   (5) cyano,
   (6) pentafluorosulfanyl, and
   (7) C₃₋₅ cycloalkyl selected from the group consisting of cyclopropyl, cyclobutyl and cyclopentyl, wherein the cycloalkyl may be optionally substituted with methyl or cyano,
each of the other symbols has the same meaning as defined above.

The term "pharmaceutically acceptable salt" used herein may be any salts known in the art that are not associated with excessive toxicity. Such a pharmaceutically acceptable salt includes, specifically, salts with inorganic acids, salts with organic acids, salts with inorganic bases, and salts with organic bases. Various forms of pharmaceutically acceptable salts are well known in the art, and are described in, for example, the following references:
(a) Berge et al., J. Pharm. Sci., 66, p1-19 (1977),
(b) Stahl et al., "Handbook of Pharmaceutical Salt: Properties, Selection, and Use" (Wiley-VCH, Weinheim, Germany, 2002),
(c) Paulekuhn et al., J. Med. Chem., 50, p6665-6672 (2007).

A compound of Formula [I] or Formula [Ia] may be reacted with an inorganic acid, organic acid, inorganic base, or organic base according to methods known per se to give a corresponding pharmaceutically acceptable salt thereof.

Such a salt with inorganic acid includes salts with hydrofluoric acid, hydrochloric acid, hydrobromic acid, hydroiodic acid, nitric acid, phosphoric acid, and sulfuric acid. Such a salt preferably includes salts with hydrochloric acid, nitric acid, sulfuric acid, phosphoric acid, and hydrobromic acid.

Such a salt with organic acid includes salts with acetic acid, adipic acid, alginic acid, 4-aminosalicylic acid, anhydromethylenecitric acid, benzoic acid, benzenesulfonic acid, calcium edetate, camphor acid, camphor-10-sulfonic acid, carbonic acid, citric acid, edetic acid, ethane-1,2-disulfonic acid, dodecylsulfuric acid, ethanesulfonic acid, fumaric acid, glucoheptonic acid, gluconic acid, glucuronic acid, glucoheptonic acid, glycollylarsanilic acid, hexylresorcinol acid, hydroxynaphthoic acid, 2-hydroxy-1-ethanesulfonic acid, lactic acid, lactobionic acid, malic acid, maleic acid, mandelic acid, methanesulfonic acid, methylsulfuric acid, methylnitric acid, methylenebis(salicylic acid), galactaric acid, naphthalene-2-sulfonic acid, 2-naphthoic acid, 1,5-naphthalenedisulfonic acid, oleic acid, oxalic acid, pamoic acid, pantothenic acid, pectic acid, picric acid, propionic acid, polygalacturonic acid, salicylic acid, stearic acid, succinic acid, tannic acid, tartaric acid, teoclic acid, thiocyanic acid, trifluoroacetic acid, p-toluenesulfonic acid, undecanoic acid, aspartic acid, and glutamic acid. Such a salt preferably includes salts with oxalic acid, maleic acid, citric acid, fumaric acid, lactic acid, malic acid, succinic acid, tartaric acid, acetic acid, trifluoroacetic acid, benzoic acid, glucuronic acid, oleic acid, pamoic acid, methanesulfonic acid, benzenesulfonic acid, p-toluenesulfonic acid, and 2-hydroxy-1-ethanesulfonic acid.

Such a salt with inorganic base includes salts with lithium, sodium, potassium, magnesium, calcium, barium, aluminum, zinc, bismuth, and ammonium. Such a salt preferably includes salts with sodium, potassium, calcium, magnesium, and zinc.

Such a salt with organic base includes salts with arecoline, betaine, choline, clemizole, ethylenediamine, N-methylglucamine, N-benzylphenethylamine, tris(hydroxymethyl)methylamine, arginine, and lysine. Such a salt preferably includes salts with tris(hydroxymethyl)methylamine, N-methylglucamine, and lysine.

A compound of Formula [I] or Formula [Ia], or a pharmaceutically acceptable salt thereof, may exist in its solvate form. The term "solvate" means a compound where a solvent molecule is coordinated with, for example, a compound of Formula [I] or Formula [Ia], or a pharmaceutically acceptable salt thereof. The solvate may be any pharmaceutically acceptable solvates; and includes, for example, a hydrate, an acetic acid solvate, an ethanolate, and a dimethyl sulfoxide solvate of a compound of Formula [I] or Formula [Ia], or a pharmaceutically acceptable salt thereof. Such a solvate specifically includes a hemihydrate, monohydrate, dihydrate, acetic acid monosolvate, and monoethanolate of a compound of Formula [I] or Formula [Ia]; and a monohydrate of sodium salt of a compound of Formula [I] or Formula [Ia] and a 2/3 ethanolate of dihydrochloride salt thereof. These solvates may be obtained according to any of known methods.

A compound of Formula [I] or Formula [Ia] may be labelled with an isotope such as ²H (D), ³H, ¹⁴C, and ³⁵S.

A compound of Formula [I] or Formula [Ia], or a pharmaceutically acceptable salt thereof, is preferably a compound of Formula [I] or Formula [Ia], or a pharmaceutically acceptable salt thereof, that is substantively purified, and more preferably a compound of Formula [I] or Formula [Ia], or a pharmaceutically acceptable salt thereof, that has a purity of 80% or more.

The expression "inhibiting NLRP3 inflammasome" means that the function of NLRP3 inflammasome is inhibited so as to disappear or reduce its activity; and, for example, it means that the function of NLRP3 inflammasome is inhibited on the basis of the condition of Test example 1 as described below. Preferably, it means inhibiting human NLRP3 inflammasome. The inhibition of the function of NLRP3 inflammasome, or the disappearance or reduction of its activity is prefeably carried out in human clinical indication.

A compound of Formula [I] or Formula [Ia], or a pharmaceutically acceptable salt thereof, may be useful as an NLRP3 inflammasome inhibitor, and may be useful for the treatment or prevention of a disease selected from the group consisting of multiple sclerosis, chronic kidney disease, inflammatory bowel disease (for example, ulcerative colitis, Crohn's disease), arteriosclerosis, Cryopyrin-associated periodic syndrome(CAPS), nonalcoholic steato-hepatitis(NASH), gout, gouty arthritis, rheumatoid arthritis, contact dermatitis, dry eye, ischemic heart disease and systemic lupus erythematosus(SLE).

The term "therapeutically effective amount" used herein may be changed depending on subjects to be administered, administration routes, target diseases, conditions, the severity of diseases, and any combination thereof. In the oral administration to a human (body weight: 60 kg), the lower limit of a therapeutically effective amount includes, for example, about 0.01 mg, about 0.1 mg, about 0.5 mg, about 1 mg, about 10 mg, about 20 mg, and about 50 mg, per day, and the upper limit of a therapeutically effective amount includes, for example, about 1 mg, about 5 mg, about 10 mg, about 20 mg, about 50 mg, about 100 mg, about 200 mg, about 500 mg, and about 1000 mg, per day.

The frequency of administration of an NLRP3 inflammasome inhibitor herein includes once, twice, thrice, and more per day.

The term "treatment" used herein includes the amelioration of conditions, prevention of aggravation, maintenance of remission, prevention of exacerbation, and prevention of relapse.

The term "prevention" used herein includes delaying the onset of conditions.

A pharmaceutical composition herein may be prepared by, for example, blending a therapeutically effective amount of an active ingredient (e.g., a compound of Formula [I] or Formula [Ia], or a pharmaceutically acceptable salt thereof) with at least one pharmaceutically acceptable carrier, etc., according to known methods in the drug formulation field. The content of the active ingredient in the pharmaceutical composition varies depending on a factor such as dosage forms and dosage amounts, and ranges, for example, from 0.1 to 100% by weight of the total amount of the composition.

A dosage form of a pharmaceutical composition herein includes oral preparations such as tablets, capsules, granules, powders, lozenges, syrups, emulsions, and suspensions; and parenteral preparations such as external preparations, suppositories, injections, eye drops, nasal preparations, and pulmonary preparations.

A pharmaceutically acceptable carrier used herein includes various organic or inorganic carrier substances which are conventionally used for a component of a formulation. Such substances include, for example, excipients, disintegrants, binders, fluidizers, and lubricants for solid preparations; solvents, solubilization agents, suspending agents, tonicity agents, buffering agents, and soothing agents for liquid preparations; and bases, emulsifying agents, wetting agents, stabilizers, stabilizing agents, dispersing agents, plasticizing agents, pH adjusters, absorption promoters, gelators, antiseptic agents, bulking agents, solubilizers, solubilization agents, and suspending agents for semisolid preparations. Additives such as preserving agents, antioxidant agents, coloring agents, and sweetening agents may be further added, if needed.

Such excipients include, for example, lactose, white soft sugar, D-mannitol, D-sorbitol, corn starch, dextrin, microcrystalline cellulose, crystalline cellulose, carmellose, carmellose calcium, sodium carboxymethylstarch, low-substituted hydroxypropylcellulose, and gum arabic.

Such disintegrants include, for example, carmellose, carmellose calcium, carmellose sodium, sodium carboxymethylstarch, croscarmellose sodium, crospovidone, low-substituted hydroxypropylcellulose, hydroxypropylmethyl cellulose, and crystalline cellulose.

Such binders include, for example, hydroxypropylcellulose, hydroxypropylmethyl cellulose, povidone, crystalline cellulose, white soft sugar, dextrin, starch, gelatin, carmellose sodium, and gum arabic.

Such fluidizers include, for example, light anhydrous silicic acid and magnesium stearate.

Such lubricants include, for example, magnesium stearate, calcium stearate, and talc.

Such solvents include, for example, purified water, ethanol, propylene glycol, macrogol, sesame oil, corn oil, and olive oil.

Such solubilization agents include, for example, propylene glycol, D-mannitol, benzyl benzoate, ethanol, triethanolamine, sodium carbonate, and sodium citrate.

Such suspending agents include, for example, benzalkonium chloride, carmellose, hydroxypropylcellulose, propylene glycol, povidone, methylcellulose, and glyceryl monostearate.

Such tonicity agents include, for example, glucose, D-sorbitol, sodium chloride, and D-mannitol.

Such buffering agents include, for example, sodium hydrogen phosphate, sodium acetate, sodium carbonate, and sodium citrate.

Such soothing agents include, for example, benzyl alcohol.

Such bases include, for example, water, oils from animals or vegetables such as olive oil, corn oil, arachis oil, sesame oil, and castor oil, lower alcohols such as ethanol, propanol, propylene glycol, 1,3-butylene glycol, and phenol, higher fatty acids and esters thereof, waxes, higher alcohol, polyhydric alcohol, hydrocarbons such as white petrolatum, liquid paraffin, and paraffin, hydrophilic petrolatum, purified lanolin, absorption ointment, hydrous lanolin, hydrophilic ointment, starch, pullulan, gum arabic, tragacanth gum, gelatin, dextran, cellulose derivatives such as methylcellulose, carboxymethyl cellulose, hydroxyethyl cellulose, and hydroxypropyl cellulose, synthetic polymers such as carboxyvinyl polymer, sodium polyacrylate, polyvinylalcohol, and polyvinylpyrrolidone, propylene glycol, macrogol such as Macrogol 200 to 600, and a combination of two or more of them.

Such preserving agents include, for example, ethyl parahydroxybenzoate, chlorobutanol, benzyl alcohol, sodium dehydroacetate, and sorbic acid.

Such anti-oxidant agents include, for example, sodium sulfite and ascorbic acid.

Such coloring agents include, for example, food colors (e.g., Food Red No. 2 or No. 3, Food Yellow No. 4 or No. 5) and β-carotene.

Such sweetening agents include, for example, saccharin sodium, dipotassium glycyrrhizinate, and aspartame.

A pharmaceutical composition herein may be administered orally or parenterally (e.g., topically, rectally, intravenously, intramuscularly, and subcutaneously) to humans as well as mammals other than humans such as mice, rats, hamsters, guinea pigs, rabbits, cats, dogs, pigs, cows, horses, sheeps, and monkeys. The dosage amount varies depending on subjects to be administered, diseases, conditions, dosage forms, and administration routes. For example, a daily dose for oral administration to an adult patient (body weight: 60 kg) is typically within the range of about 0.01 mg to about 1 g of the active ingredient. Such a dosage amount can be administered at one time or in several divided doses.

In one embodiment, an NLRP3 inflammasome inhibitor or a pharmaceutical composition herein may be provided in the form of a kit such as kits for administration, treatment, and/or prevention, a package such as packaged goods, and a set and/or case of medicines which comprises the same and a written matter indicating that the same may or should be used for treatment and/or prevention. Such a kit, package, and set of medicines may comprise one or more containers filled with an NLRP3 inflammasome inhibitor and/or other drugs or medicines (or ingredients). Examples of such a kit, package, and set of medicines include commercial kits, commercial packages, and commercial medicine set for appropriate use in the treatment and/or prevention of intended diseases. The written matter comprised in such a kit, package, and set of medicines includes a cautionary note or package insert in the form designated by the government organization that regulates manufactures, use, or sales of pharmaceutical or biological products which ensures an approval by the government organization on manufactures, use, or sales of products concerning administration to humans. The kit, package, and set of medicines may include packaged products as well as structures configured for appropriate administration steps or those configured so as to be able to achieve more preferable medical treatment and/or prevention including treatment and/or prevention of intended diseases.

### General Method of Preparation

General methods for preparing a compound of Formula [I] and A compound of Formula [Ia], or a pharmaceutically acceptable salt thereof, is illustrated as follows. A method for preparing a compound of Formula [I] and A compound of Formula [Ia], or a pharmaceutically acceptable salt thereof, is however not limited thereto.

Each compound obtained in each step may be isolated and/or purified, if necessary, according to any of known methods such as distillation, recrystallization, and column chromatography, or optionally, a subsequent step can proceed without isolation and/or purification.

Herein, the term "room temperature" refers to a temperature which has not been controlled and includes 1°C to 40°C as one embodiment.

### Preparation method A1: A method for preparing a compound of Formula [I] or a salt thereof

Compound [I], or a salt thereof, may be prepared by, for example, Preparation method A1 as follows.

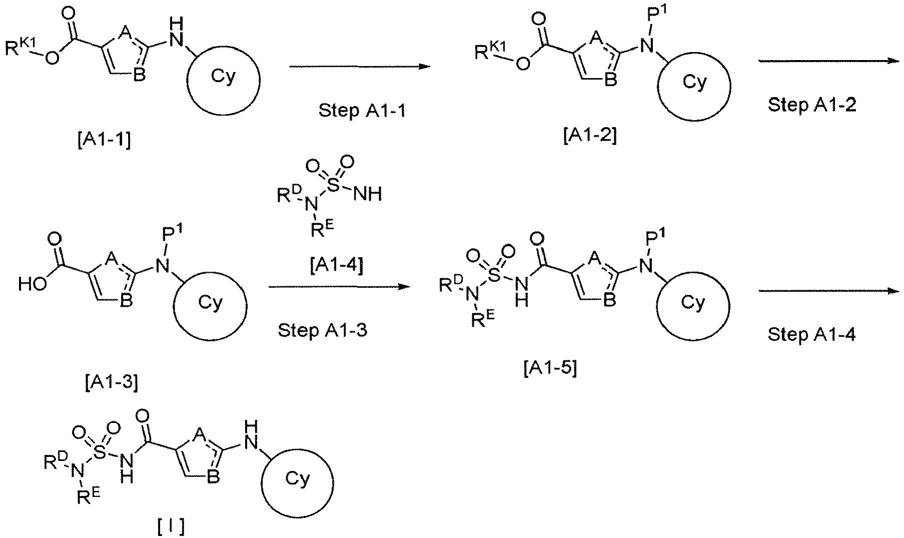

In the scheme, A, B, Ring Cy, R^{D}, and R^{E} are as defined above,
R^{K1} is C₁₋₆ alkyl, e.g., methyl and ethyl, and
P¹ is a protective group of amine, e.g., tert-butoxycarbonyl and benzyloxycarbonyl.

### Step A1-1

Compound [A1-2], or a salt thereof, may be prepared by introducing a protective group P¹ to Compound [A1-1], or a salt thereof. The introduction of the protective group may be carried out under any conditions suitable for P¹.

For example, when P¹ is tert-butoxycarbonyl, Compound [A1-2], or a salt thereof, may be prepared in the reaction of Compound [A1-1], or a salt thereof, with a urethanation agent in a solvent in the presence of a base.

The base used herein includes, for example, 4-dimethylaminopyridine, diisopropylethylamine, and 1,8-diazabicyclo[5.4.0]-7-undecene. A preferable base is 4-dimethylaminopyridine.

The urethanation agent used herein includes, for example, di-tert-butyl dicarbonate, N-tert-butoxycarbonylimidazole, and carbonic acid tert-butyl phthalimido ester. A preferable urethanation agent is di-tert-butyl dicarbonate.

The solvent used herein includes, for example, tetrahydrofuran, acetonitrile, and dichloromethane. A preferable solvent is tetrahydrofuran.

The reaction temperature herein ranges, for example, from 0°C to 150°C, preferably from 50°C to 70°C.

Compound [Al-1], or a salt thereof, may be commercially available, or may be prepared from a commercialized product according to known methods; for example, it may also be prepared by Preparation method B1, B2, B3, or B4 as below.

### Step A1-2

Compound [A1-3], or a salt thereof, may be prepared by hydrolysis of Compound [A1-2], or a salt thereof, in a solvent in the presence of a base.

The base used herein includes, for example, sodium hydroxide, potassium hydroxide, and lithium hydroxide. A preferable base is sodium hydroxide or potassium hydroxide.

The solvent used herein includes, for example, methanol, ethanol, water, and a mixed solvent thereof. A preferable solvent is a mixed solvent of methanol and water.

The reaction temperature herein ranges, for example, from 0°C to 100°C, preferably from 0°C to room temperature.

### Step A1-3

Compound [A1-5], or a salt thereof, may be prepared by condensation of Compound [A1-3], or a salt thereof, and Compound [A1-4], or a salt thereof, in a solvent in the presence of a condensation agent. A base may also be added, if necessary.

The condensation agent used herein includes, for example, 1-ethyl-3-(3-dimethylaminopropyl)carbodiimide hydrochloride, carbonyldiimidazole, and 4-(4,6-dimethoxy-1,3,5-triazin-2-yl)-4-methylmorpholinium chloride. A preferable condensation agent is 1-ethyl-3-(3-dimethylaminopropyl)carbodiimide hydrochloride or carbonyldiimidazole.

The base used herein includes, for example, 1,8-diazabicyclo[5.4.0]-7-undecene, 4-dimethylaminopyridine, and triethylamine. A preferable base is 1,8-diazabicyclo[5.4.0]-7-undecene or 4-dimethylaminopyridine.

The solvent used herein includes, for example, tetrahydrofuran, chloroform, and N,N-dimethylformamide. A preferable solvent is tetrahydrofuran or chloroform.

The reaction temperature herein ranges, for example, from 0°C to 100°C, preferably from 0°C to 70°C.

Compound [Al-4], or a salt thereof, may be commercially available, or may be prepared from a commercialized product according to known methods, for example, Preparation method B5 as below.

### Step A1-4

Compound [I], or a salt thereof, may be prepared by removal of P¹ from Compound [A1-5], or a salt thereof, in the deprotection reaction. The deprotection reaction may be carried out under any conditions suitable for P¹.

For example, when P¹ is tert-butoxycarbonyl, Compound [I], or a salt thereof, may be prepared in the reaction of Compound [A1-5], or a salt thereof, with an acid in a solvent.

The solvent used herein includes, for example, dichloromethane, chloroform, and tetrahydrofuran. A preferable solvent is dichloromethane or tetrahydrofuran.

The acid used herein includes, for example, trifluoroacetic acid, perchloric acid, and hydrochloric acid. A preferable acid is trifluoroacetic acid or perchloric acid.

The reaction temperature herein ranges, for example, from 0°C to 100°C, preferably from 0°C to 60°C.

### Preparation method A2: A method for preparing Compound [Ia], or a salt thereof

Compound [Ia], or a salt thereof, may be prepared by, for example, Preparation method A2 as follows.

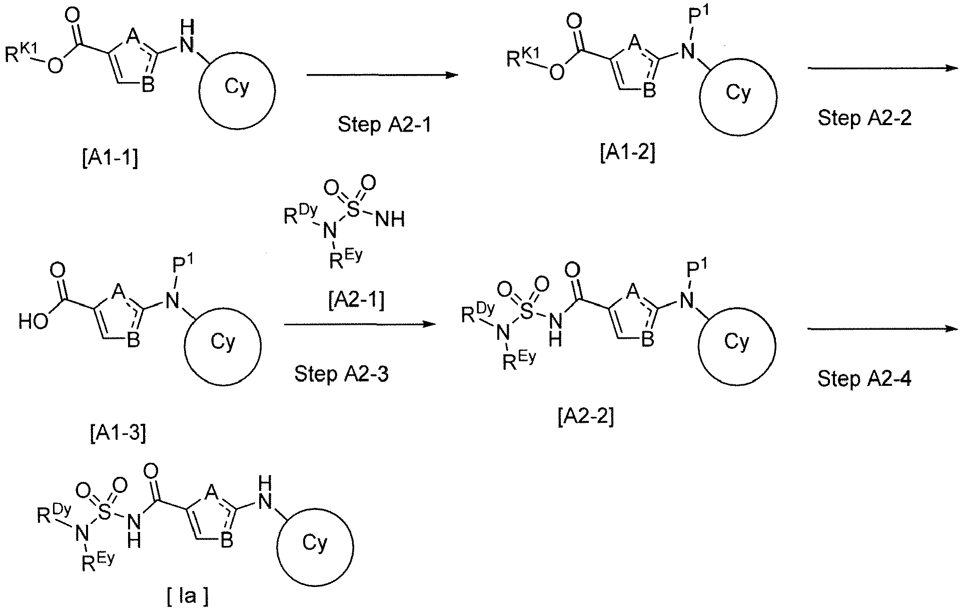

In the scheme, A, B, Ring Cy, R^{Dy}, R^{Ey}, R^{K1}, and P¹ are as defined above.

### Step A2-1

Compound [A1-2], or a salt thereof, may be prepared by introducing a protective group P¹ to Compound [A1-1], or a salt thereof. The introduction of the protective group may be carried out under any conditions suitable for P¹.

For example, when P¹ is tert-butoxycarbonyl, Compound [A1-2], or a salt thereof, may be prepared in the reaction of Compound [A1-1], or a salt thereof, according to Step A1-1.

### Step A2-2

Compound [A1-3], or a salt thereof, may be prepared in the reaction of Compound [Al-2], or a salt thereof, according to Step A1-2.

### Step A2-3

Compound [A2-2], or a salt thereof, may be prepared in the reaction of Compound [A1-3], or a salt thereof, with Compound [A2-1], or a salt thereof, according to Step A1-3.

Compound [A2-1], or a salt thereof, may be commercially available, or may be prepared from a commercialized product according to known methods; for example, it may also be prepared by Preparation method B6 as below.

### Step A2-4

Compound [Ia], or a salt thereof, may be prepared by removal of P¹ from Compound [A2-2], or a salt thereof, in the deprotection reaction. The deprotection reaction may be carried out under any conditions suitable for P¹.

For example, when P¹ is tert-butoxycarbonyl, Compound [Ia], or a salt thereof, may be prepared in the reaction of Compound [A2-2], or a salt thereof, according to Step A1-4.

### Preparation method B1: Preparation method of Compound [A1-1], or a salt thereof

Compound [A1-1], or a salt thereof, used in Preparation methods A1 and A2 may be prepared by, for example, Preparation method B1 as follows.

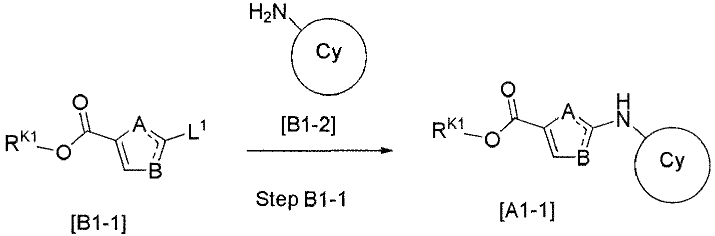

In the scheme, A, B, Ring Cy, and R^{K1} are as defined above, and L¹ is a leaving group (e.g., chlorine, bromine, and trifluoromethanesulfonyloxy).

### Step B1-1

Compound [A1-1], or a salt thereof, may be prepared in the reaction of Compound [B1-1], or a salt thereof, with Compound [B1-2], or a salt thereof, in a solvent.

The solvent used herein includes, for example, isopropanol, tert-butanol, N-methylpyrrolidone, and dimethylsulfoxide. A preferable solvent is isopropanol or N-methylpyrrolidone.

The reaction temperature herein ranges from 0°C to 200°C, preferably from 100°C to 160°C.

Compound [A1-1], or a salt thereof, may also be prepared in the reaction of Compound [B1-1], or a salt thereof, with Compound [B1-2], or a salt thereof, in the presence of a base and a palladium catalyst in a solvent. A ligand may also be added, if necessary.

The solvent used herein includes isopropanol, tert-butanol, and 1,2-dimethoxyethane. A preferable solvent is tert-butanol.

The palladium catalyst used herein includes [(2-di-tert-butylphosphino-3,6-dimethoxy-2',4',6'-triisopropyl-1,1'-biphenyl)-2-(2'-amino-1,1'-biphenyl)]palladium (II) methanesulfonate (tBuBrettPhos Pd G3), palladium (II) acetate, and tris(dibenzylideneacetone)dipalladium (0). A preferable palladium catalyst is tBuBrettPhos Pd G3.

The ligand used herein includes [3,6-dimethoxy-2',4',6'-tris(1-methylethyl)[1,1'-biphenyl]-2-yl]bis(1,1-dimethylethyl)phosphine (tBuBrettPhos), 2-(dicyclohexylphosphino)-3,6-dimethoxy-2',4',6'-triisopropyl-1,1'-biphenyl (BrettPhos), and 2-dicyclohexylphosphino-2',4',6'-triisopropylbiphenyl (XPhos).

The base used herein includes tripotassium phosphate, cesium carbonate, and potassium carbonate. A preferable base is tripotassium phosphate.

The reaction temperature ranges, for example, from 60°C to 150°C, preferably from 80°C to 120°C.

Compound [B1-1], or a salt thereof, may be commercially available, and may also be prepared from a commercialized product according to known methods.

Compound [B1-2], or a salt thereof, may be commercially available, and may also be prepared from a commercialized product according to known methods, for example, Preparation method C1, C2, C3, or C4 as below.

### Preparation method B2: Alternative preparation method of Compound [A1-1], or a salt thereof

Compound [A1-1], or a salt thereof, used in Preparation methods A1 and A2 may also be prepared by, for example, Preparation method B2 as follows.

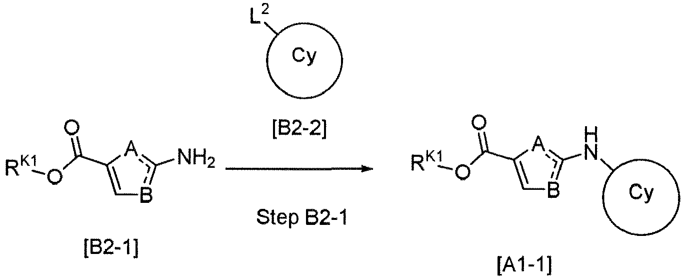

In the scheme, A, B, Ring Cy, and R^{K1} are as defined above, and L² is a leaving group (e.g., chlorine, bromine, and trifluoromethanesulfonyloxy).

### Step B2-1

Compound [A1-1], or a salt thereof, may be prepared in the reaction of Compound [B2-1], or a salt thereof, with Compound [B2-2], or a salt thereof, according to Step B1-1.

Compound [B2-1], or a salt thereof, may be commercially available, or may also be prepared from a commercialized product according to known methods.

Compound [B2-2], or a salt thereof, may be commercially available, or may also be prepared from a commercialized product according to known methods.

### Preparation method B3: Preparation method of Compound [B3-5], or a salt thereof

Compound [B3-5], or a salt thereof, having the following formula: 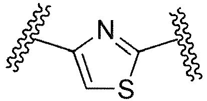 for a partial structure: 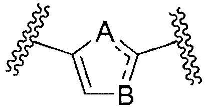 in Compound [A1-1] used in Preparation methods A1 and A2 may be prepared according to, for example, Preparation method B3 as follows.

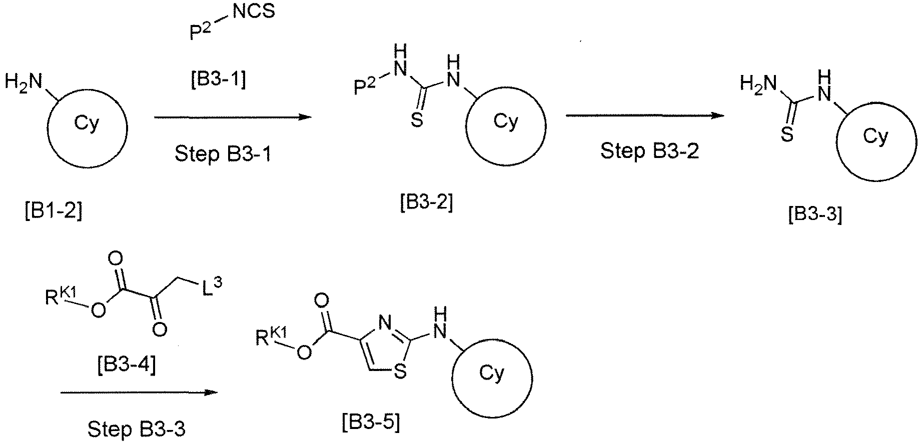

In the scheme, Ring Cy and R^{K1} are as defined above, P² is a protective group for amine (e.g., acetyl and phenylcarbonyl), and L³ is a leaving group (e.g., chlorine and bromine).

### Step B3-1

Compound [B3-2], or a salt thereof, may be prepared in the reaction of Compound [B1-2], or a salt thereof, with Compound [B3-1], or a salt thereof, in a solvent.

The solvent used herein includes, for example, acetone, acetonitrile, and tetrahydrofuran. A preferable solvent is acetone.

The reaction temperature ranges, for example, from 0°C to 100°C, preferably from 40°C to 80°C.

Compound [B1-2], or a salt thereof, may be commercially available, or may also be prepared from a commercialized product according to known methods, for example, Preparation method C1, C2, C3, or C4 as below.

Compound [B3-1], or a salt thereof, may be commercially available, or may also be prepared from a commercialized product according to known methods.

### Step B3-2

Compound [B3-3], or a salt thereof, may be prepared by removal of P² from Compound [B3-2], or a salt thereof, in the deprotection reaction. The deprotection reaction may be carried out under any conditions suitable for P².

For example, when P² is phenylcarbonyl, Compound [B3-3], or a salt thereof, may be prepared in the reaction of Compound [B3-2], or a salt thereof, with a base in a solvent.

The solvent used herein includes, for example, methanol, water, tetrahydrofuran, and a mixed solvent thereof. A preferable solvent is a mixed solvent of methanol and water.

The base used herein includes, for example, sodium hydroxide, potassium hydroxide, and lithium hydroxide. A preferable base is sodium hydroxide.

The reaction temperature herein ranges from 0°C to 120°C, preferably from 50°C to 100°C.

### Step B3-3

Compound [B3-5], or a salt thereof, may be prepared in the reaction of Compound [B3-3], or a salt thereof, with Compound [B3-4], or a salt thereof, in a solvent.

The solvent used herein includes, for example, methanol, ethanol, and tetrahydrofuran. A preferable solvent is methanol or ethanol.

The reaction temperature ranges, for example, from 0°C to 120°C, preferably from 50°C to 100°C.

Compound [B3-4], or a salt thereof, may be commercially available, or may also be prepared from a commercialized product according to known methods.

### Preparation method B4: Preparation method of Compound [B4-5], or a salt thereof

Compound [B4-5], or a salt thereof, having phenyl for Ring Cy in Compound [A1-1], or a salt thereof, used in Preparation methods A1 and A2 may be prepared according to, for example, Preparation method B4 as follows.

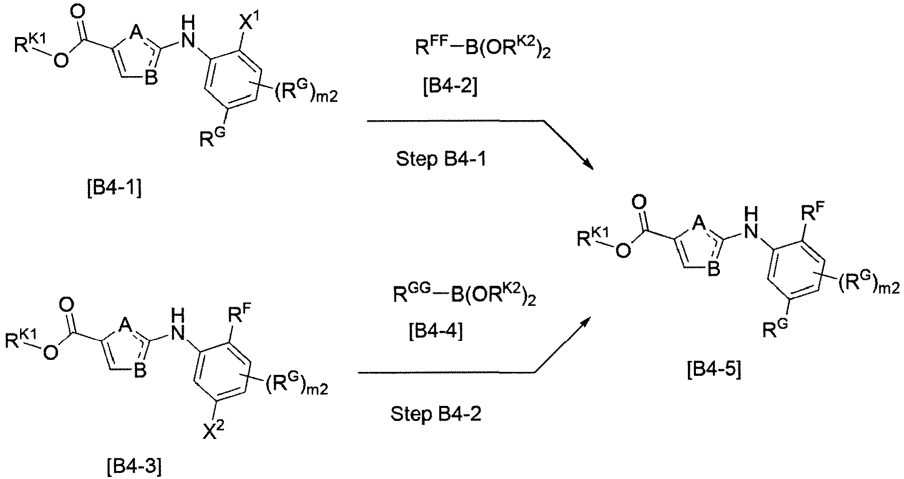

In the scheme, A, B, R^{K1}, R^{F}, R^{G}, and m2 are as defined above,
R^{FF} is as defined in R^{F}, or a substituent group wherein any one or more of bonds of any one of groups listed in the definition of R^{F} are replaced with an unsaturated bond (e.g., isopropenyl and cyclopentenyl),
R^{GG} is each independently as defined in R^{G}, or a substituent group wherein any one or more of bonds of any one of groups listed in the definition of R^{G} are replaced with an unsaturated bond (e.g., isopropenyl and cyclopentenyl),
R^{K2} is each independently C₁₋₆ alkyl, or one R^{K2} may be combined with the other R^{K2} to form a ring, and
X¹ and X² are a leaving group (e.g., bromine and trifluoromethanesulfonyloxy).

### Step B4-1

Compound [B4-5], or a salt thereof, may be prepared by cross coupling reaction of Compound [B4-1], or a salt thereof, with Compound [B4-2], or a salt thereof, in the presence of a base and a palladium catalyst in a solvent. A ligand may also be added, if necessary. When R^{FF} introduced includes an unsaturated bond that does not constitute an aromatic ring, Compound [B4-5], or a salt thereof, may be prepared by catalytic hydrogenation of the compound, or a salt thereof, obtained in the cross coupling reaction in the presence of a catalyst in a solvent.

The solvent used in the cross coupling reaction includes, for example, 1,2-dimethoxyethane, 1,4-dioxane, and toluene. A preferable solvent is 1,2-dimethoxyethane or 1,4-dioxane.

The palladium catalyst used herein includes, for example, [1,1'-bis(diphenylphosphino)ferrocene]dichloropalladium (II), palladium acetate, and tris(dibenzylideneacetone)dipalladium (0). A preferable palladium catalyst is [1,1'-bis(diphenylphosphino)ferrocene]dichloropalladium (II).

The ligand used in the cross coupling reaction includes, for example, triphenylphosphine, 2-dicyclohexylphosphino-2',6'-dimethoxybiphenyl (SPhos), and 2-dicyclohexylphosphino-2',4',6'-triisopropylbiphenyl (XPhos).

The base used in the cross coupling reaction includes, for example, tripotassium phosphate, cesium carbonate, and potassium carbonate. A preferable base is tripotassium phosphate.

The reaction temperature in the cross coupling reaction ranges, for example, from 20°C to 150°C, preferably from 70°C to 120°C.

The solvent used in the catalytic hydrogenation includes, for example, methanol, ethanol, and ethyl acetate. A preferable solvent is ethanol.

The catalyst used in the catalytic hydrogenation includes, for example, palladium carbon, palladium hydroxide, and platinum (IV) oxide. A preferable solvent is palladium carbon.

The reaction temperature in the catalytic hydrogenation ranges, for example, from 0°C to 120°C, preferably from 20°C to 70°C.

Compound [B4-1], or a salt thereof, may be commercially available, or may also be prepared from a commercialized product according to known methods, for example, Preparation method B1, B2, or B3 as described above.

Compound [B4-2], or a salt thereof, may be commercially available, or may also be prepared from a commercialized product according to known methods.

### Step B4-2

Compound [B4-5], or a salt thereof, may be prepared in the reaction of Compound [B4-3], or a salt thereof, with Compound [B4-4], or a salt thereof, according to Step B4-1.

Compound [B4-3], or a salt thereof, may be commercially available, or may also be prepared from a commercialized product according to known methods, for example, Preparation method B1, B2, or B3 as described above.

Compound [B4-4], or a salt thereof, may be commercially available, or may also be prepared from a commercialized product according to known methods.

### Preparation method B5: Preparation method of Compound [A1-4], or a salt thereof

Compound [A1-4], or a salt thereof, used in Preparation method A1 may be prepared according to, for example, Preparation method B5 as follows.

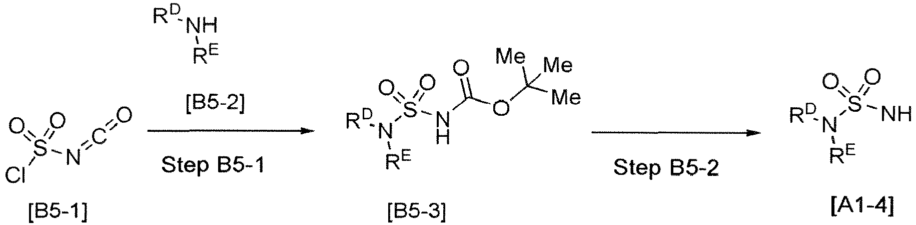

In the scheme, R^{D} and R^{E} are as defined above.

### Step B5-1

Compound [B5-3], or a salt thereof, may be prepared in the reaction of Compound [B5-1], or a salt thereof, with tert-butanol in a solvent, followed by the reaction with Compound [B5-2], or a salt thereof, in the presence of a base.

The solvent used herein includes, for example, dichloromethane, chloroform, and tetrahydrofuran. A preferable solvent is dichloromethane or chloroform.

The base used herein includes, for example, triethylamine, tributylamine, diisopropylethylamine, and 1,8-diazabicyclo[5.4.0]-7-undecene. A preferable base is triethylamine or tributylamine.

The reaction temperature ranges, for example, from -40°C to 100°C, preferably from 0°C to 40°C.

Compound [B5-1], or a salt thereof, may be commercially available, or may also be prepared from a commercialized product according to known methods.

Compound [B5-2], or a salt thereof, may be commercially available, or may also be prepared from a commercialized product according to known methods.

### Step B5-2

Compound [A1-4], or a salt thereof, may be prepared in the reaction of Compound [B5-3], or a salt thereof, with an acid in a solvent.

The solvent used herein includes, for example, ethyl acetate, tetrahydrofuran, and cyclopentylmethyl ether. A preferable solvent is ethyl acetate.

The acid used herein includes, for example, hydrogen chloride, trifluoroacetic acid, and trifluoromethanesulfonic acid. A preferable acid is hydrogen chloride.

The reaction temperature ranges from 0°C to 100°C, preferably from 10°C to 40°C.

### Preparation method B6: A method for preparing Compound [A2-1], or a salt thereof

Compound [A2-1], or a salt thereof, used in Preparation method A2, may be prepared by, for example, Preparation method B6 as follows.

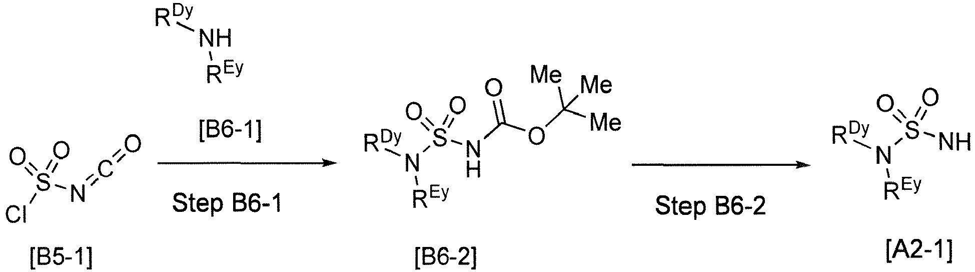

In the scheme, R^{Dy} and R^{Ey} are as defined above.

### Step B6-1

Compound [B6-2], or a salt thereof, may be prepared in the reaction of Compound [B5-1], or a salt thereof with Compound [B6-1], or a salt thereof, according to Step B5-1.

Compound [B6-1], or a salt thereof, may be commercially available, or may be prepared from a commercialized product according to known methods; for example, it may also be prepared by Preparation method C5 or C6 as below.

### Step B6-2

Compound [A2-1], or a salt thereof, may be prepared in the reaction of Compound [B6-2], or a salt thereof, according to Step B5-2.

### Preparation method C1: Preparation method of Compound [C1-3], or a salt thereof

Compound [C1-3], or a salt thereof, having phenyl for Ring Cy in Compound [B1-2], or a salt thereof, used in Preparation methods B1 and B3 may be prepared according to, for example, Preparation method C1 as follows.

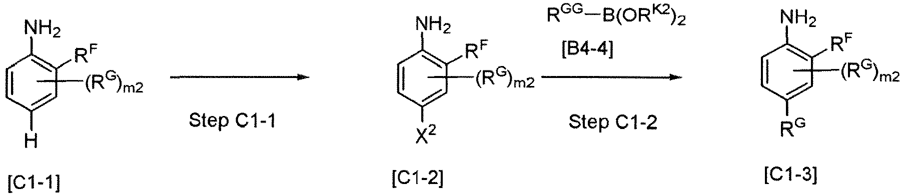

In the scheme, R^{F}, R^{GG}, R^{G}, R^{K2}, m2, and X² are as defined above.

### Step C1-1

Compound [C1-2], or a salt thereof, may be prepared in the reaction of Compound [C1-1], or a salt thereof, with a halogenating agent in a solvent.

The solvent used herein includes, for example, dichloromethane, chloroform, and acetonitrile. A preferable solvent is dichloromethane or acetonitrile.

The halogenating agent used herein includes, for example, N-bromosuccinimide, bromine, and iodine. A preferable halogenating agent is N-bromosuccinimide.

The reaction temperature ranges, for example, from 0°C to 100°C, preferably from 0°C to 60°C.

Compound [C1-1], or a salt thereof, may be commercially available, or may also be prepared from a commercialized product according to known methods.

### Step C1-2

Compound [C1-3], or a salt thereof, may be prepared in the reaction of Compound [C1-2], or a salt thereof, with Compound [B4-4], or a salt thereof, according to Step B4-1.

### Preparation method C2: Preparation method of Compound [C2-4], or a salt thereof

Compound [C2-4], or a salt thereof, having phenyl for Ring Cy in Compound [B1-2], or a salt thereof, used in Preparation methods B1 and B3 may be prepared according to, for example, Preparation method C2 as follows.

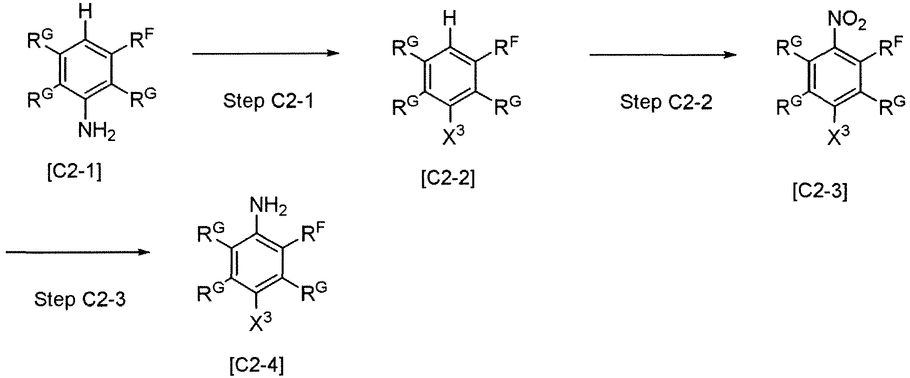

In the scheme, R^{F} and R^{G} are as defined above, and X³ is halogen (e.g., fluorine and chlorine).

### Step C2-1

Compound [C2-2], or a salt thereof, may be prepared in the reaction of Compound [C2-1], or a salt thereof, in the presence of a diazotization agent and a halogenating agent in a solvent.

The solvent used herein includes, for example, tetrahydrofuran, water, 1,2-dimethoxyethane, and a mixed solvent thereof. A preferable solvent is a mixed solvent of tetrahydrofuran and water.

The halogenating agent used herein includes, for example, tetrafluoroboric acid, copper chloride, and copper bromide. A preferable halogenating agent is tetrafluoroboric acid or copper chloride.

The diazotization agent used herein includes, for example, sodium nitrite, tert-butyl nitrite, and n-butyl nitrite. A preferable diazotization agent is sodium nitrite.

The reaction temperature herein ranges, for example, from - 40°C to 60°C, preferably from -20°C to 20°C.

Compound [C2-1], or a salt thereof, may be commercially available, or may also be prepared from a commercialized product according to known methods.

### Step C2-2

Compound [C2-3], or a salt thereof, may be prepared in the reaction of Compound [C2-2], or a salt thereof, in the presence of a nitrating agent in a solvent.

The solvent used herein includes, for example, acetonitrile and sulfolane. A preferable solvent is acetonitrile.

The nitrating agent used herein includes, for example, nitronium tetrafluoroborate, nitronium trifluoromethanesulfonate, and nitric acid. A preferable nitrating agent is nitronium tetrafluoroborate.

The reaction temperature ranges, for example, from -20°C to 40°C, preferably from -10°C to 10°C.

### Step C2-3

Compound [C2-4], or a salt thereof, may be prepared by catalytic hydrogenation of Compound [C2-3], or a salt thereof, in the presence of a catalyst in a solvent.

The solvent used herein includes, for example, methanol, ethanol, and ethyl acetate. A preferable solvent is methanol or ethanol.

The catalyst used herein includes, for example, palladium carbon, palladium hydroxide, and Raney nickel. A preferable catalyst is palladium carbon.

The reaction temperature herein ranges, for example, from 0°C to 100°C, preferably from 10°C to 60°C.

### Preparation method C3: Preparation method of Compound [C3-6], or a salt thereof

Compound [C3-6], or a salt thereof, having phenyl for Cy in Compound [B1-2], or a salt thereof, used in Preparation methods B1 and B3 may be prepared according to, for example, Preparation method C3 as follows.

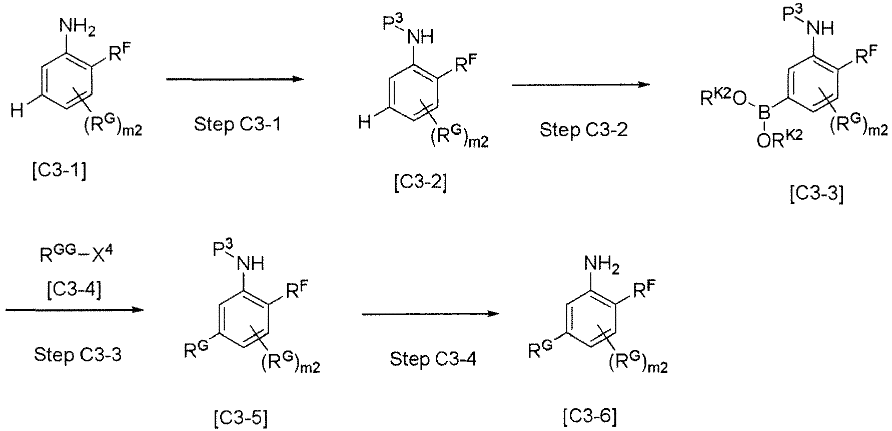

In the scheme, R^{F}, R^{G}, R^{GG}, R^{K2}, and m2 are as defined above,
X⁴ is a leaving group (e.g., bromine and trifluoromethanesulfonyloxy), and
P³ is a protective group for amine (e.g., tert-butylcarbonyl and tert-butoxycarbonyl).

### Step C3-1

Compound [C3-2], or a salt thereof, may be prepared by introduction of a protective group P³ into Compound [C3-1], or a salt thereof. The introduction of the protective group may be carried out under any conditions suitable for P³.

For example, when P³ is tert-butoxycarbonyl, Compound [C3-2], or a salt thereof, may be prepared in the reaction of Compound [C3-1], or a salt thereof, according to Step A1-1 wherein P¹ is tert-butoxycarbonyl.

Compound [C3-1], or a salt thereof, may be commercially available, or may also be prepared from a commercialized product according to known methods.

### Step C3-2

Compound [C3-3], or a salt thereof, may be prepared in the reaction of Compound [C3-2], or a salt thereof, in the presence of a catalyst, a ligand, and a borylation agent in a solvent.

The solvent used herein includes, for example, tetrahydrofuran, 1,2-dimethoxyethane, and 1,4-dioxane. A preferable solvent is tetrahydrofuran.

The catalyst used herein includes, for example, (1,5-cyclooctadiene)(methoxy)iridium (I) dimer, bis(1,5-cyclooctadiene)diiridium (I) dichloride, and bis(1,5-cyclooctadiene)iridium (I) tetrafluoroborate. A preferable catalyst is (1,5-cyclooctadiene)(methoxy)iridium (I) dimer.

The borylation agent used herein includes, for example, bis(pinacolato)diborane, 4,4,5,5,-tetramethyl-1,3,2-dioxaborolane, and tetrahydroxydiboron. A preferable borylation agent is bis(pinacolato)diborane.

The ligand used herein includes, for example, 4,4'-di-tert-butyl-2,2'-bipyridyl, and 4,4'-dimethyl-2,2'-bipyridyl. A preferable ligand is 4,4'-di-tert-butyl-2,2'-bipyridyl.

The reaction temperature ranges, for example, from 0°C to 120°C, preferably from 20°C to 60°C.

### Step C3-3

Compound [C3-5], or a salt thereof, may be prepared in the reaction of Compound [C3-3], or a salt thereof, with Compound [C3-4], or a salt thereof, according to Step B4-1.

Compound [C3-4], or a salt thereof, may be commercially available, or may also be prepared from a commercialized product according to known methods.

### Step C3-4

Compound [C3-6], or a salt thereof, may be prepared by removal of P³ from Compound [C3-5], or a salt thereof, in the deprotection reaction. The deprotection reaction may be carried out under any conditions suitable for P³.

For example, when P³ is tert-butoxycarbonyl, Compound [C3-6], or a salt thereof, may be prepared in the reaction of Compound [C3-5], or a salt thereof, according to Step A1-5 wherein P¹ is tert-butoxycarbonyl.

### Preparation method C4: Preparation method of Compound [C4-3] or Compound [C4-6], or a salt thereof

Compound [C4-3] or Compound [C4-6], or a salt thereof, having pyrazole for Cy in Compound [B1-2], or a salt thereof, used in Preparation methods B1 and B3 may be prepared according to, for example, Preparation method C4 as follows.

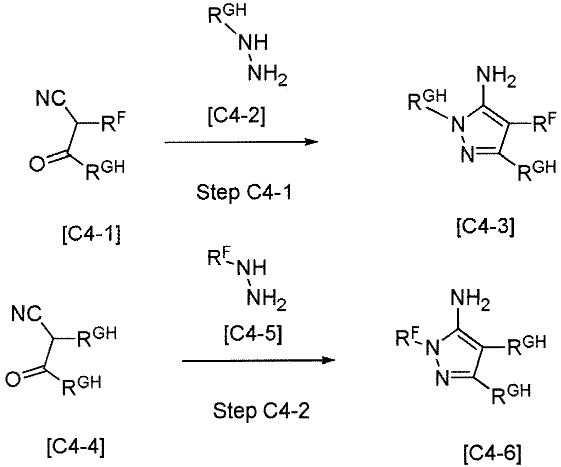

In the scheme, R^{F} is as defined above, and R^{GH} is each independently as defined in R^{G}, or hydrogen.

### Step C4-1

Compound [C4-3], or a salt thereof, may be prepared in the reaction of Compound [C4-1], or a salt thereof, with Compound [C4-2], or a salt thereof, in a solvent.

The solvent used herein includes, for example, methanol, ethanol, and isopropanol. A preferable solvent is ethanol or isopropanol.

The reaction temperature ranges, for example, from 0°C to 120°C, preferably from 60°C to 100°C.

Compound [C4-1], or a salt thereof, may be commercially available, or may also be prepared from a commercialized product according to known methods.

Compound [C4-2], or a salt thereof, may be commercially available, or may also be prepared from a commercialized product according to known methods.

### Step C4-2

Compound [C4-6], or a salt thereof, may be prepared in the reaction of Compound [C4-4], or a salt thereof, with Compound [C4-5], or a salt thereof, according to Step C4-1.

Compound [C4-4], or a salt thereof, may be commercially available, or may also be prepared from a commercialized product according to known methods.

Compound [C4-5], or a salt thereof, may be commercially available, or may also be prepared from a commercialized product according to known methods.

### Preparation method C5: A method for preparing Compound [C5-4], Compound [C5-8], Compound [C5-11], or Compound [C5-16], or a salt thereof

As one exaple of Compound [B6-1], Compound [C5-4], Compound [C5-8], Compound [C5-11] or Compound [C5-16], or a salt thereof may be prepared by Preparation method C5 as follows.

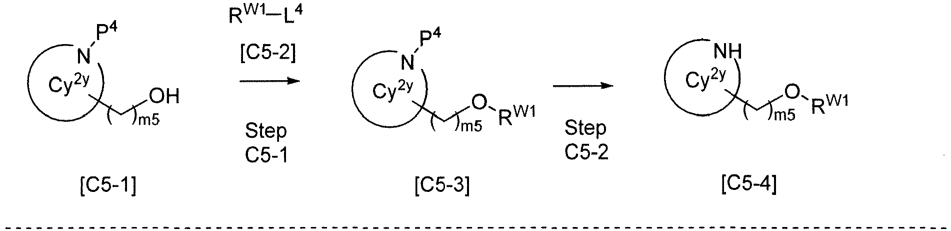

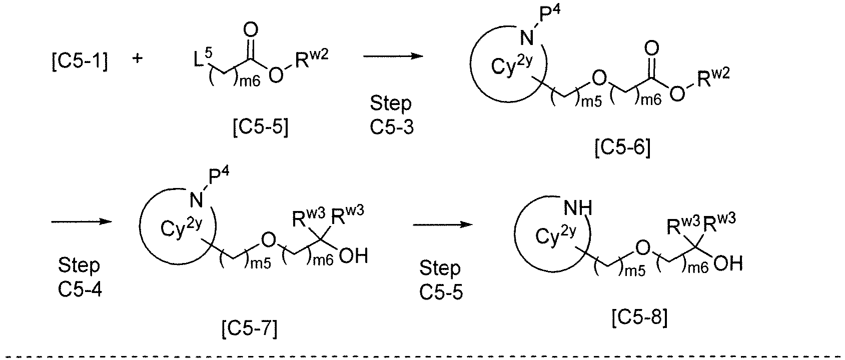

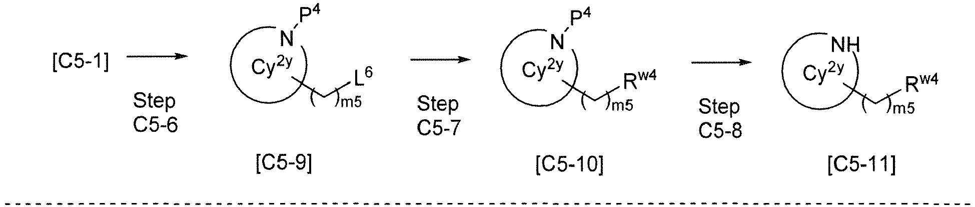

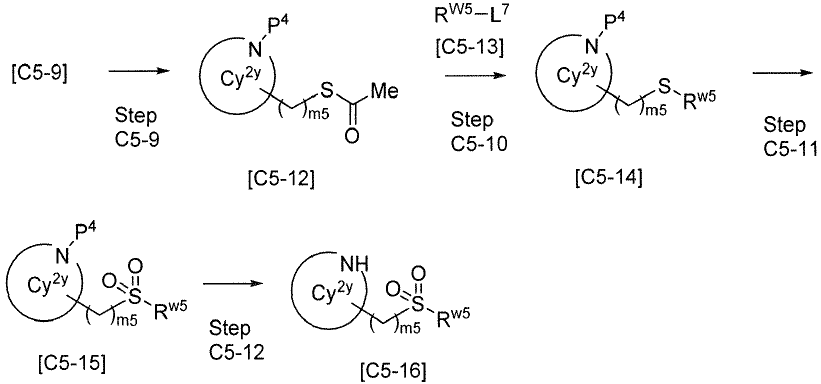

In the scheme, Ring Cy^{2y} is as defined above,
R^{W1} is C₁₋₆ alkyl, wherein the alkyl may be optionally substituted with 1 to 3 substituents independently selected from the group consisting of:
   (1) halogen,
   (2) hydroxy,
   (3) C₁₋₆ alkoxy, and
   (4) C₃₋₆ cycloalkyl, wherein the cycloalkyl may be optionally substituted with the same or different 1 to 3 R^{1dy}s,
R^{W2} is C₁₋₆ alkyl,
R^{W3} is methyl,
R^{W4} is
   (1) C₁₋₆ alkoxy, wherein the alkoxy may be optionally substituted with 1 to 3 substituents independently selected from the group consisting of:
      (a) halogen,
      (b) hydroxy,
      (c) C₁₋₄ alkoxy, and
      (d) C₃₋₆ cycloalkyl, wherein the cycloalkyl may be optionally substituted with the same or different 1 to 3 R^{1dy}s,
   (2) cyano,
   (3) NR^{t2}R^{t3}, wherein R^{t2} is
      (a) hydrogen, or
      (b) C₁₋₆ alkyl, and
         R^{t3} is
         (a) hydrogen,
         (b) C₁₋₆ alkyl,
         (c) COR¹⁴, or
         (d) SO₂R¹⁵,
   (4) 4- to 6-membered heterocycloalkyl comprising 1 or 2 heteroatoms independently selected from the group consisting of nitrogen and oxygen atoms,
   (5) phenyl, or
   (6) 5- to 6-membered heteroaryl comprising 1 to 3 heteroatoms independently selected from the group consisting of nitrogen, oxygen, and sulfur atoms, wherein the heteroaryl may be optionally substituted with the same or different 1 to 3 R¹⁷s,
R^{W5} is
   (1) C₁₋₆ alkyl,
   (2) C₁₋₄ haloalkyl,
   (3) C₃₋₆ cycloalkyl, or
   (4) 4- to 6-membered heterocycloalkyl comprising an oxygen atom,
P⁴ is a protective group (e.g., tert-butoxycarbonyl, benzyloxycarbonyl, benzyl and 1-naphthylmethyl),
L⁴ is a leaving group (e.g., bromine, iodine, methanesulfonyloxy and paratoluenesulfonyloxy),
L⁵ is a leaving group (e.g., bromine, iodine, methanesulfonyloxy and paratoluenesulfonyloxy),
L⁶ is a leaving group (e.g., bromine, iodine, methanesulfonyloxy and paratoluenesulfonyloxy),
L⁷ is a leaving group (e.g., bromine, iodine, methanesulfonyloxy and paratoluenesulfonyloxy),
m5 is an integer from 0 to 6,
m6 is an integer from 1 to 3,
R^{1dy}, R¹⁴, R¹⁵ and R¹⁷ are as defined above.

### Step C5-1

Compound [C5-3], or a salt thereof, may be prepared in the reaction of Compound [C5-1], or a salt thereof with Compound [C5-2], or a salt thereof in a solvent in the presence of a base.

The base used herein includes, for example, sodium hydride, and potassium hexamethyldisilazide. A preferable base is sodium hydride.

The solvent used herein includes, for example, 1,4-dioxane, tetrahydrofuran, N,N-dimethylacetamide, N,N-dimethylformamide, and a mixed solvent thereof. A preferable solvent is a mixed solvent of tetrahydrofuran and N,N-dimethylformamide.

The reaction temperature herein ranges, for example, from 0°C to 70°C, preferably from 0°C to 40°C.

Compound [C5-1], or a salt thereof, may be commercially available, or may be prepared from a commercialized product according to known methods.

Compound [C5-2], or a salt thereof, may be commercially available, or may be prepared from a commercialized product according to known methods.

### Step C5-2

Compound [C5-4], or a salt thereof, may be prepared by removal of P⁴ from Compound [C5-3], or a salt thereof, in the deprotection reaction. The deprotection reaction may be carried out under any conditions suitable for P⁴.

For example, when P⁴ is tert-butoxycarbonyl, Compound [C5-4], or a salt thereof, may be prepared in the reaction of Compound [C5-3], or a salt thereof, according to Step A1-4.

### Step C5-3

Compound [C5-6], or a salt thereof, may be prepared in the reaction of Compound [C5-1], or a salt thereof with Compound [C5-5], or a salt thereof, according to Step C5-1.

Compound [C5-5], or a salt thereof, may be commercially available, or may be prepared from a commercialized product according to known methods.

### Step C5-4

Compound [C5-7], or a salt thereof, may be prepared in the reaction of Compound [C5-6], or a salt thereof with an alkylating agent in a solvent.

The alkylating agent used herein includes, for example, methylmagnesium chloride, methylmagnesium bromide, and methyllithium. A preferable alkylating agent is methylmagnesium chloride.

The solvent used herein includes, for example, tetrahydrofuran and N,N-dimethylformamide. A preferable solvent is tetrahydrofuran.

The reaction temperature herein ranges, for example, from - 78°C to 30°C, preferablyfrom -78°C to 0°C.

### Step C5-5

Compound [C5-8], or a salt thereof, may be prepared by removal of P⁴ from Compound [C5-7], or a salt thereof, in the deprotection reaction. The deprotection reaction may be carried out under any conditions suitable for P⁴.

For example, when P⁴ is tert-butoxycarbonyl, Compound [C5-8], or a salt thereof, may be prepared in the reaction of Compound [C5-7], or a salt thereof, according to Step A1-4.

### Step C5-6

Compound [C5-9], or a salt thereof, may be prepared by a conversion of the hydroxy of Compound [C5-1], or a salt thereof to L⁶. The conversion may be carried out under any conditions suitable for L⁶. For example, when L⁶ is methanesulfonyloxy, Compound [C5-9], or a salt thereof, may be prepared by a methanesulfonylation of Compound [C5-1], or a salt thereof in a solvent in the presence of a base.

The methanesulfonylation agent used herein includes, for example, methanesulfonic anhydride, methanesulfonyl chloride. A preferable methanesulfonylation agent is methanesulfonic anhydride.

The solvent used herein includes, for example, tetrahydrofuran, and N,N-dimethylformamide. A preferable solvent is tetrahydrofuran.

The reaction temperature herein ranges, for example, from 0°C to 80°C, preferably from 0°C to 40°C.

### Step C5-7

Compound [C5-10], or a salt thereof, may be prepared by a conversion of Compound [C5-9], or a salt thereof to R^{W4}. The conversion may be carried out under any conditions suitable for R^{W4}. For example, when R^{W4} is cyano, Compound [C5-10], or a salt thereof, may be prepared in the reaction of Compound [C5-9], or a salt thereof with a cyanating agent in a solvent in the presence of a catalyst.

The cyanating agent used herein includes, for example, trimethylsilyl cyanide and sodium cyanide. A preferable cyanating agent is trimethylsilyl cyanide.

The catalyst used herein includes, for example, tetrabutylammonium fluoride, potassium carbonate and cesium fluoride. A preferable catalyst is tetrabutylammonium fluoride.

The solvent used herein includes, for example, tetrahydrofuran, and N,N-dimethylformamide. A preferable solvent is tetrahydrofuran.

The reaction temperature herein ranges, for example, from 50°C to 120°C, preferably from 80°C to 100°C.

### Step C5-8

Compound [C5-11], or a salt thereof, may be prepared by removal of P⁴ from Compound [C5-10], or a salt thereof, in the deprotection reaction. The deprotection reaction may be carried out under any conditions suitable for P⁴.

For example, when P⁴ is tert-butoxycarbonyl, Compound [C5-11], or a salt thereof, may be prepared in the reaction of Compound [C5-10], or a salt thereof, according to Step A1-4.

### Step C5-9

Compound [C5-12], or a salt thereof, may be prepared in the reaction of Compound [C5-9], or a salt thereof with potassium thioacetate in a solvent.

The solvent used herein includes, for example, tetrahydrofuran, and N,N-dimethylformamide. A preferable solvent is N,N-dimethylformamide.

The reaction temperature herein ranges, for example, from 0°C to 100°C, preferably6from 0°C to 80°C.

### Step C5-10

Compound [C5-14], or a salt thereof, may be prepared in the reaction of Compound [C5-12], or a salt thereof with Compound [C5-13], or a salt thereof in a solvent in the presence of a base.

The base used herein includes, for example, sodium hydroxide, potassium hydroxide, lithium hydroxide. A preferable base is lithium hydroxide.

The solvent used herein includes, for example, methanol and ethanol. A preferable solvent is ethanol.

The reaction temperature herein ranges, for example, from 0°C to 50°C, preferably from 0°C to 30°C.

### Step C5-11

Compound [C5-15], or a salt thereof, may be prepared in the reaction of Compound [C5-14], or a salt thereof in a solvent in the presence of an oxidizing agent.

The oxidizing agent used herein includes, for example, meta-chloroperbenzoic acid, tert-butyl hydroperoxide and hydrogen peroxide. A preferable an oxidizing agent is meta-chloroperbenzoic acid.

The solvent used herein includes, for example, chloroform, dichloromethane. A preferable solvent is chloroform.

The reaction temperature herein ranges, for example, from 0°C to 50°C, preferably from 0°C to 40°C.

### Step C5-12

Compound [C5-16], or a salt thereof, may be prepared by removal of P⁴ from Compound [C5-15], or a salt thereof, in the deprotection reaction. The deprotection reaction may be carried out under any conditions suitable for P⁴.

For example, when P⁴ is tert-butoxycarbonyl, Compound [C5-16], or a salt thereof, may be prepared in the reaction of Compound [C5-15], or a salt thereof, according to Step A1-4.

### Preparation method C6: A method for preparing Compound [C6-5], Compound [C6-8], Compound [C6-15], or Compound [C6-20], or a salt thereof

As one exaple of Compound [B6-1], Compound [C6-5], Compound [C6-8], Compound [C6-15], or Compound [C6-20], or a salt thereof, may be prepared by Preparation method C6 as follows. 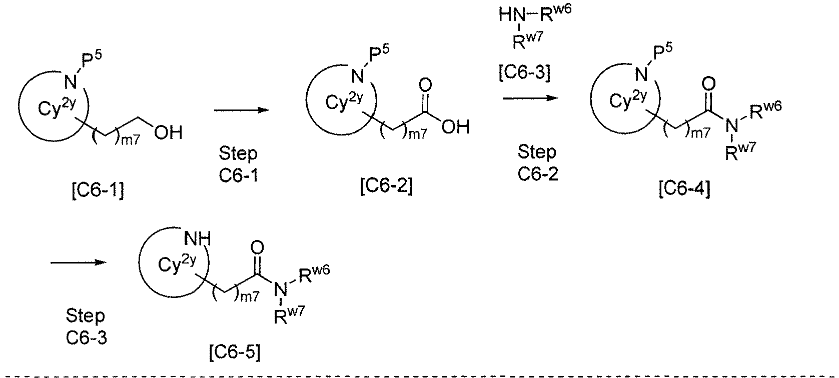 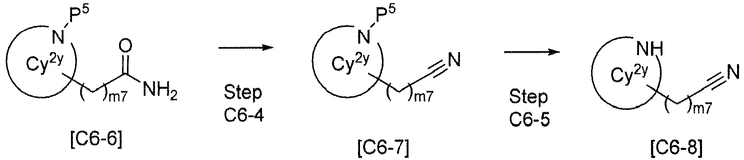 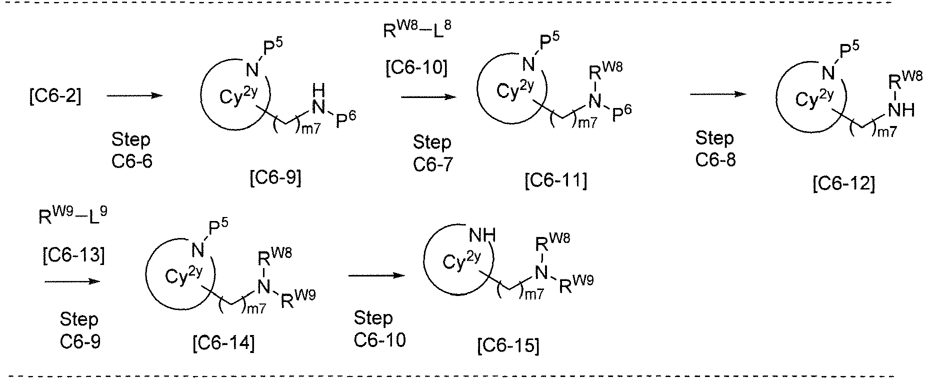 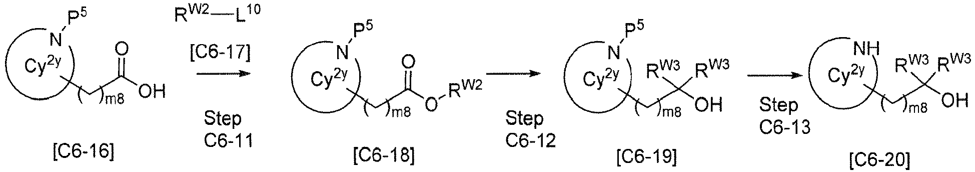 wherein
Ring Cy^{2y}, R^{W2} and R^{W3} are as defined above,
R^{W6} and R^{W7} are each independently
   (1) hydrogen,
   (2) C₁₋₆ alkyl, or
   (3) C₁₋₄ haloalkyl, or alternatively,
R^{W6} and R^{W7} may combine together with the nitrogen atom to which they attach to form 4- to 6-membered heterocycloalkyl comprising 1 or 2 heteroatoms independently selected from the group consisting of nitrogen and oxygen atoms, wherein the heterocycloalkyl may be optionally substituted with the same or different 1 or 2 halogen or hydroxy,
R^{W8} is ,
   (1) hydrogen,
   (2) C₁₋₆ alkyl, or
   (3) C₁₋₄ haloalkyl,
R^{W9} is
   (1) hydrogen,
   (2) C₁₋₆ alkyl,
   (3) C₁₋₄ haloalkyl,
   (4) COR¹⁴, or
   (5) SO₂R¹⁵,
P⁵ is a protective group (e.g., tert-butoxycarbonyl, benzyloxycarbonyl, benzyl and 1-naphthylmethyl),
P⁶ is a protective group (e.g., tert-butoxycarbonyl and benzyloxycarbonyl),
L⁸ is a leaving group (e.g., for example, bromine, iodine, methanesulfonyloxy and paratoluenesulfonyloxy),
L⁹ is a leaving group (e.g., for example, bromine, iodine, methanesulfonyloxy and paratoluenesulfonyloxy),
L¹⁰ is a leaving group (e.g., bromine, iodine, methanesulfonyloxy and paratoluenesulfonyloxy),
m7 is an integer from 0 to 5,
m8 is an integer from 0 to 3,
R¹⁴ and R¹⁵ are as defined above.

### Step C6-1

Compound [C6-2], or a salt thereof, may be prepared in the reaction of Compound [C6-1], or a salt thereof in a solvent in the presence of an oxidizing agent.

The oxidizing agent used herein includes, 2-azaadamantane-N-oxyl and 2,2,6,6-tetramethylpiperidine 1-oxyl. A preferable an oxidizing agent is 2,2,6,6-tetramethylpiperidine 1-oxyl.

The solvent used herein includes, for example, acetone, dichloromethane. A preferable solvent is acetone.

The reaction temperature herein ranges, for example, from 0°C to 40°C, preferably from 0°C to 30°C.

Compound [C6-1], or a salt thereof, may be commercially available, or may be prepared from a commercialized product according to known methods.

### Step C6-2

Compound [C6-4], or a salt thereof, may be prepared in the reaction of Compound [C6-2], or a salt thereof with Compound [C6-3], or a salt thereof, according to Step A1-3.

Compound [C6-3], or a salt thereof, may be commercially available, or may be prepared from a commercialized product according to known methods.

### Step C6-3

Compound [C6-5], or a salt thereof, may be prepared by removal of P⁵ from Compound [C6-4], or a salt thereof, in the deprotection reaction. The deprotection reaction may be carried out under any conditions suitable for P⁵.

For example, when P⁵ is benzyloxycarbonyl, Compound [C6-5], or a salt thereof, may be prepared in the reaction of Compound [C6-4], or a salt thereof, according to Step C2-3.

### Step C6-4

Compound [C6-7], or a salt thereof, may be prepared in the reaction of Compound [C6-6], or a salt thereof in a solvent in the presence of a dehydrating agent and a base.

The dehydrating agent used herein includes, trifluoroacetic anhydride, phosphorus oxychloride and thionyl chloride. A preferable dehydrating agent is trifluoroacetic anhydride.

The base used herein includes, for example, triethylamine and N,N-diisopropylethylamine. A preferable base is triethylamine.

The solvent used herein includes, for example, chloroform, dichloromethane. A preferable solvent is dichloromethane.

The reaction temperature herein ranges, for example, from 0°C to 40°C, preferably from 0°C to 30°C.

Compound [C6-6], or a salt thereof may be commercially available, or may be prepared from a commercialized product according to known methods; for example, it may also be prepared by Steps C6-1 and C6-2 described above.

### Step C6-5

Compound [C6-8], or a salt thereof, may be prepared by removal of P⁵ from Compound [C6-7], or a salt thereof, in the deprotection reaction. The deprotection reaction may be carried out under any conditions suitable for P⁵.

For example, when P⁵ is tert-butoxycarbonyl, Compound [C6-8], or a salt thereof, may be prepared in the reaction of Compound [C6-7], or a salt thereof, according to Step A1-4.

### Step C6-6

Compound [C6-9], or a salt thereof, may be prepared in the Curtius rearrangement reaction of Compound [C6-2], or a salt thereof in a solvent in the presence of a an azidating agent, a base and a nucleophilic agent.

The azidating agent used herein includes, diphenylphosphoryl azide and sodium azide. A preferable an azidating agent 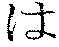 ,diphenylphosphoryl azide.

The base used herein includes, for example, triethylamine and N,N-diisopropylethylamine. A preferable base is triethylamine.

The nucleophilic agent used herein includes, benzyl alcohol, tert-butanol. A preferable a nucleophilic agent is benzyl alcohol.

The solvent used herein includes, for example, toluene and benzene. A preferable solvent is toluene.

The reaction temperature herein ranges, for example, from 80°C to 120°C, preferablyfrom 100°C to 120°C.

### Step C6-7

Compound [C6-11], or a salt thereof, may be prepared in the reaction of Compound [C6-9], or a salt thereof with Compound [C6-10], or a salt thereof, according to Step C5-1. When R^{W8} is hydrogen, the next step can be conducted without this step.

Compound [C6-10], or a salt thereof, may be commercially available, or may be prepared from a commercialized product according to known methods.

### Step C6-8

Compound [C6-12], or a salt thereof, may be prepared by removal of P⁶ from Compound [C6-11], or a salt thereof, in the deprotection reaction. The deprotection reaction may be carried out under any conditions suitable for P⁶.

For example, when P⁶ is benzyloxycarbonyl, Compound [C6-12], or a salt thereof, may be prepared in the reaction of Compound [C6-11], or a salt thereof, according to Step C2-3.

### Step C6-9

Compound [C6-14], or a salt thereof, may be prepared in the reaction of Compound [C6-12], or a salt thereof with Compound [C6-13], or a salt thereof in a solvent in the presence of a base. When R^{W9} is hydrogen, the next step can be conducted without this step.

The base used herein includes, for example, triethylamine, N,N-diisopropylethylamine, pyridine, potassium carbonate and cesium carbonate. A preferable base is triethylamine.

The solvent used herein includes, for example, dichloromethane, tetrahydrofuran and acetonitrile. A preferable solvent is dichloromethane.

The reaction temperature herein ranges, for example, from 0°C to 60°C, preferably from 0°C to 40°C.

Compound [C6-13], or a salt thereof, may be commercially available, or may be prepared from a commercialized product according to known methods.

### Step C6-10

Compound [C6-15], or a salt thereof, may be prepared by removal of P⁵ from Compound [C6-14], or a salt thereof, in the deprotection reaction. The deprotection reaction may be carried out under any conditions suitable for P⁵.

For example, when P⁵ is tert-butoxycarbonyl, Compound [C6-15], or a salt thereof, may be prepared in the reaction of Compound [C6-14], or a salt thereof, according to Step A1-4.

### Step C6-11

Compound [C6-18], or a salt thereof, may be prepared in the reaction of Compound [C6-16], or a salt thereof with Compound [C6-17], or a salt thereof, according to Step C6-9.

Compound [C6-16], or a salt thereof, may be commercially available, or may be prepared from a commercialized product according to known methods; for example, it may also be prepared by Step C6-1 described above.

Compound [C6-17], or a salt thereof, may be commercially available, or may be prepared from a commercialized product according to known methods.

### Step C6-12

Compound [C6-19], or a salt thereof, may be prepared in the reaction of Compound [C6-18], or a salt thereof, according to Step C5-4.

### Step C6-13

Compound [C6-20], or a salt thereof, may be prepared by removal of P⁵ from Compound [C6-19], or a salt thereof, in the deprotection reaction. The deprotection reaction may be carried out under any conditions suitable for P⁵.

For example, wnen P⁵ is benzyloxycarbonyl, Compound [C6-20], or a salt thereof, may be prepared in the reaction of Compound [C6-19], or a salt thereof, according to Step C2-3.

### EXAMPLES

Preparation methods of Compound [I] or Compound [Ia], or a pharmaceutically acceptable salt thereof, are described specifically in the following Preparation examples. However, preparation methods of Compound [I] or Compound [Ia], or a pharmaceutically acceptable salt thereof, are not intended to be limited thereto.

NMR was determined at 400MHz.

### [Preparation example 1]: Synthesis of N-(N,N-dimethylsulfamoyl)-2-((1,2,3,5,6,7-hexahydro-s-indacen-4-yl)amino)oxazole-5-carboxamide (Example 1)

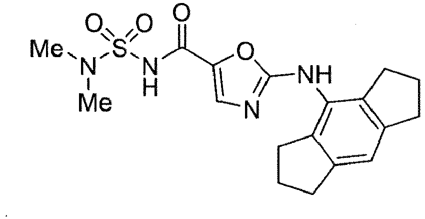

### (1) Ethyl 2-((1,2,3,5,6,7-hexahydro-s-indacen-4-yl)amino)oxazole-5-carboxylate

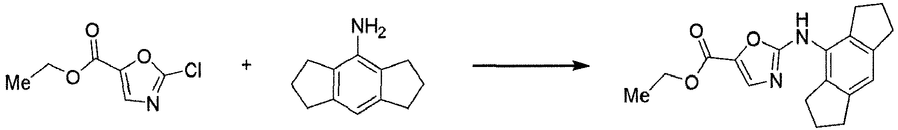

To a solution of ethyl 2-chlorooxazole-5-carboxylate (4.6 g) in isopropanol (46 mL) was added 1,2,3,5,6,7-hexahydro-s-indacen-4-amine (4.6 g), and the mixture was stirred at 120°C for 15 minutes with a microwave reactor (Product No. 356007, Biotage). To the reaction mixture were added aqueous solution of saturated sodium hydrogen carbonate and water, and then the mixture was extracted with ethyl acetate. The resulted organic layer was washed with saturated brine, dried over anhydrous magnesium sulfate, and then solvent was removed under reduced pressure. To the residue was added a mixed solution of hexane/ethyl acetate (v/v = 3/1), and the mixture was stirred, and then the resulted solid was filtered to give the title compound (3.2 g).

¹H-NMR (CDCl₃) δ: 7.52 (1H, s), 7.01 (1H, s), 6.57 (1H, s), 4.32 (2H, q, J = 7.1 Hz), 2.89 (4H, t, J = 7.4 Hz), 2.78 (4H, t, J = 7.4 Hz), 2.10-2.03 (4H, m), 1.34 (3H, t, J = 7.1 Hz).

### (2) Ethyl 2-((tert-butoxycarbonyl)(1,2,3,5,6,7-hexahydro-s-indacen-4-yl)amino)oxazole-5-carboxylate

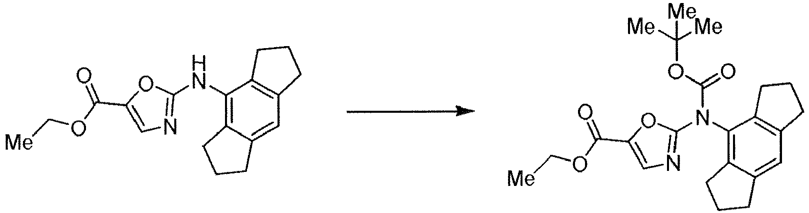

To a solution of ethyl 2-((1,2,3,5,6,7-hexahydro-s-indacen-4-yl)amino)oxazole-5-carboxylate (240 mg) obtained in (1) in tetrahydrofuran (4.8 mL) were added di-tert-butyl dicarbonate (200 mg) and 4-dimethylaminopyridine (110 mg), and the mixture was stirred at 50°C for 1 hour. Then, solvent was removed under reduced pressure. The residue was purified by column chromatography (hexane/ethyl acetate) to give the title compound (350 mg).

¹H-NMR (CDCl₃) δ: 7.58 (1H, s), 7.07 (1H, s), 4.34 (2H, q, J = 7.1 Hz), 2.90-2.83 (4H, m), 2.78-2.70 (2H, m), 2.65-2.57 (2H, m), 2.07-2.01 (4H, m), 1.49 (9H, s), 1.35 (3H, t, J = 7.2 Hz).

### (3) 2-((tert-Butoxycarbonyl)(1,2,3,5,6,7-hexahydro-s-indacen-4-yl)amino)oxazole-5-carboxylic acid

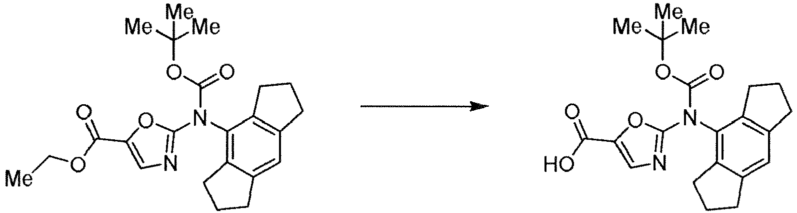

To a solution of ethyl 2-((tert-butoxycarbonyl)(1,2,3,5,6,7-hexahydro-s-indacen-4-yl)amino)oxazole-5-carboxylate (350 mg) obtained in (2) in ethanol (3.2 mL) was added a 2 M aqueous solution of sodium hydroxide (0.76 mL), and the mixture was stirred at 60°C for 1 hour. The reaction mixture was allowed to cool to room temperature, and then thereto were added 2 M hydrochloric acid (0.76 mL) and water. Then, the mixture was extracted with ethyl acetate. The resulted organic layer was washed with saturated brine, dried over magnesium sulfate, and then solvent was removed under reduced pressure to give the title compound (280 mg).

¹H-NMR (CDCl₃) δ: 7.69 (1H, s), 7.07 (1H, s), 2.89 (4H, t, J = 7.3 Hz), 2.74-2.71 (2H, m), 2.65-2.57 (2H, m), 2.08-2.02 (4H, m), 1.48 (9H, s).

### (4) tert-Butyl (5-((N,N-dimethylsulfamoyl)carbamoyl)oxazol-2-yl) (1,2,3,5,6,7-hexahydro-s-indacen-4-yl)carbamate

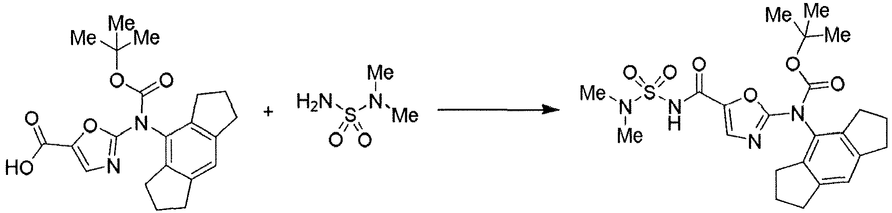

To a solution of 2-((tert-butoxycarbonyl)(1,2,3,5,6,7-hexahydro-s-indacen-4-yl)amino)oxazole-5-carboxylic acid (100 mg) obtained in (3) in deuterated chloroform (2 mL) were added N,N-dimethylsulfamide (42 mg), 1-ethyl-3-(3-dimethylaminopropyl)carbodiimide hydrochloride (75 mg), triethylamine (53 mg), and 4-dimethylaminopyridine (32 mg), and the mixture was stirred at room temperature for 3 days. To the reaction mixture was added acetic acid (74 µL), and then the reaction mixture was purified by column chromatography (hexane/ethyl acetate/acetic acid) to give the title compound (95 mg) .

¹H-NMR (CDCl₃) δ: 8.70 (1H, br s), 7.67 (1H, s), 7.10 (1H, s), 3.02 (6H, s), 2.90 (4H, t, J = 7.3 Hz), 2.75-2.68 (2H, m), 2.63-2.58 (2H, m), 2.08-2.04 (4H, m), 1.47 (9H, s).

### (5) N-(N,N-Dimethylsulfamoyl)-2-((1,2,3,5,6,7-hexahydro-s-indacen-4-yl)amino)oxazole-5-carboxamide

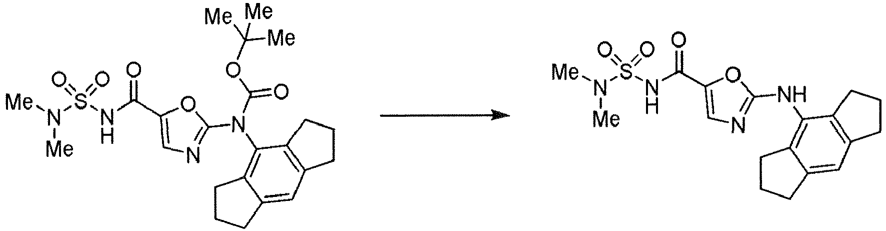

To tert-butyl (5-((N,N-dimethylsulfamoyl)carbamoyl)oxazol-2-yl)(1,2,3,5,6,7-hexahydro-s-indacen-4-yl)carbamate (45 mg) obtained in (4) was added trifluoroacetic acid (1 mL), and the mixture was stirred at room temperature for 20 minutes. Solvent was removed under reduced pressure, and then the residue was purified by column chromatography (ethyl acetate) to give the title compound (30 mg).

¹H-NMR (CDCl₃) δ: 8.06 (1H, br s), 7.63 (1H, s), 7.06 (1H, s), 6.75 (1H, s), 3.00 (6H, s), 2.91 (4H, t, J = 7.4 Hz), 2.77 (4H, t, J = 7.3 Hz), 2.12-2.05 (4H, m).

### [Preparation example 2]: Synthesis of N-(N,N-dimethylsulfamoyl)-2-((2-methyl-5-(trifluoromethyl)phenyl)amino)oxazole-4-carboxamide (Example 68)

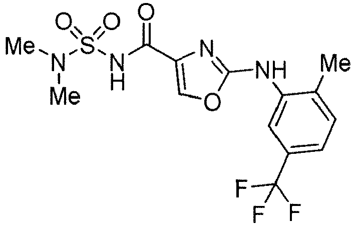

### (1) Ethyl 2-((2-methyl-5-(trifluoromethyl)phenyl)amino)oxazole-4-carboxylate

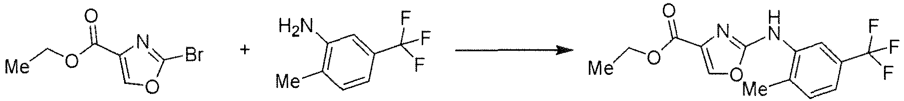

To a solution of ethyl 2-bromooxazole-4-carboxylate (4.5 g) in tert-butanol (30 mL) were added 2-methyl-5-(trifluoromethyl)aniline (3.0 g), [(2-di-tert-butylphosphino-3,6-dimethoxy-2',4',6'-triisopropyl-1,1'-biphenyl)-2-(2'-amino-1,1'-biphenyl)]palladium (II) methanesulfonic acid (0.37 g), and tripotassium phosphate (7.3 g) under an argon atmosphere, and the mixture was stirred at 100°C for 6 hours. To the reaction mixture was added water, and then the mixture was extracted with ethyl acetate. The resulted organic layer was washed with saturated brine, dried over anhydrous magnesium sulfate, and then solvent was removed under reduced pressure. The residue was purified by column chromatography (hexane/ethyl acetate) to give the title compound (3.0 g).

¹H-NMR (DMSO-D₆) δ: 9.64 (1H, s), 8.40 (1H, s), 8.26 (1H, s), 7.42 (1H, d, J = 7.6 Hz), 7.32 (1H, d, J = 7.6 Hz), 4.24 (2H, q, J = 7.2 Hz), 2.33 (3H, s), 1.26 (3H, t, J = 7.1 Hz).

### (2) Ethyl 2-((tert-butoxycarbonyl)(2-methyl-5-(trifluoromethyl)phenyl)amino)oxazole-4-carboxylate

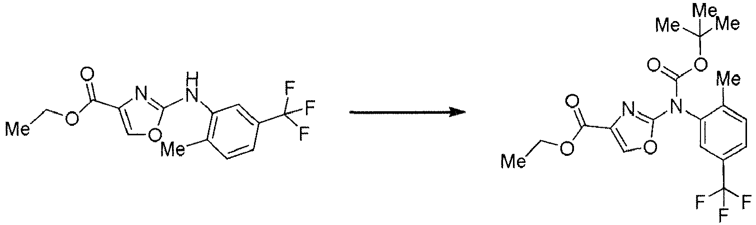

To a solution of ethyl 2-((2-methyl-5-(trifluoromethyl)phenyl)amino)oxazole-4-carboxylate (3.0 g) obtained in (1) in tetrahydrofuran (10 mL) were added di-tert-butyl dicarbonate (2.5 g) and 4-dimethylaminopyridine (1.4 g), and the mixture was stirred at 50°C for 2 hours. Then, solvent was removed under reduced pressure. The residue was purified by column chromatography (hexane/ethyl acetate) to give the title compound (4.0 g).

¹H-NMR (CDCl₃) δ: 8.06 (1H, s), 7.54 (1H, s), 7.50 (1H, d, J = 8.1 Hz), 7.38 (1H, d, J = 7.9 Hz), 4.35 (2H, q, J = 7.2 Hz), 2.35 (3H, s), 1.43 (9H, s), 1.34 (3H, t, J = 7.2 Hz).

### (3) 2-((tert-Butoxycarbonyl)(2-methyl-5-(trifluoromethyl)phenyl)amino)oxazole-4-carboxylic acid

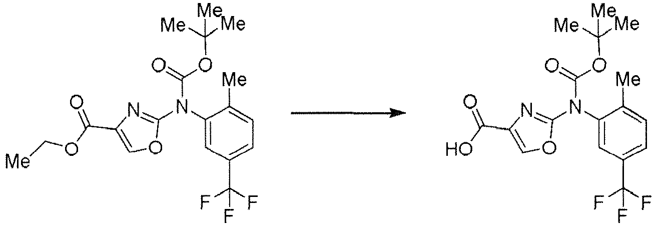

To a solution of ethyl 2-((tert-butoxycarbonyl)(2-methyl-5-(trifluoromethyl)phenyl)amino)oxazole-4-carboxylate (4.0 g) obtained in (2) in methanol (40 mL) was added a 2 M aqueous solution of sodium hydroxide (19 mL), and the mixture was stirred at room temperature for 20 minutes. To the reaction mixture were added 2 M hydrochloric acid (19 mL) and water, and the mixture was extracted with ethyl acetate. The resulted organic layer was washed with saturated brine, dried over anhydrous magnesium sulfate, and then solvent was removed under reduced pressure. To the residue were added diisopropyl ether and hexane, and the mixture was stirred, and then the resulted solid was filtered to give the title compound (3.2 g).

¹H-NMR (DMSO-D₆) δ: 13.17 (1H, br s), 8.63 (1H, s), 7.72 (1H, s), 7.70 (1H, d, J = 8.1 Hz), 7.59 (1H, d, J = 8.1 Hz), 2.25 (3H, s), 1.39 (9H, s).

### (4) tert-Butyl (4-((N,N-dimethylsulfamoyl)carbamoyl)oxazol-2-yl)(2-methyl-5-(trifluoromethyl)phenyl)carbamate

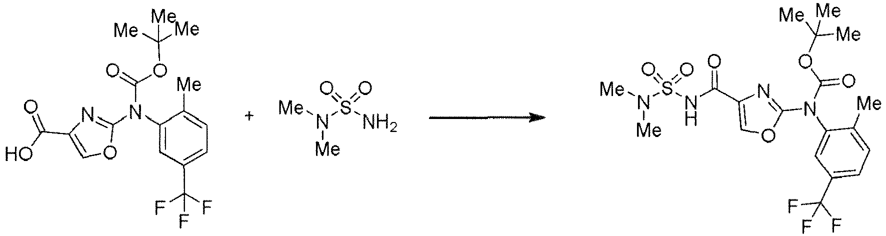

To a solution of 2-((tert-butoxycarbonyl)(2-methyl-5-(trifluoromethyl)phenyl)amino)oxazole-4-carboxylic acid (100 mg) obtained in (3) in tetrahydrofuran (1.0 mL) was added N,N'-carbonyldiimidazole (63 mg) under an argon atmosphere, and the mixture was stirred at 60°C for 2 hours. Then, the mixture was allowed to cool to room temperature.

To the reaction mixture were added N,N-dimethylsulfamide (48 mg) and 1,8-diazabicyclo[5.4.0]-7-undecene (59 µL), and the mixture was stirred at 60°C for 1 hour. Then, the mixture was allowed to cool to room temperature. To the reaction mixture was added acetic acid (0.1 mL), and then solvent was removed under reduced pressure. The residue was purified by column chromatography (hexane/ethyl acetate) to give the title compound (44 mg).

¹H-NMR (CDCl₃) δ: 8.82 (1H, s), 8.10 (1H, br s), 7.57 (1H, d, J = 8.1 Hz), 7.47 (1H, s), 7.43 (1H, d, J = 8.1 Hz), 2.98 (6H, s), 2.96 (3H, s), 2.32 (3H, s), 1.45 (9H, s).

### (5) N-(N,N-Dimethylsulfamoyl)-2-((2-methyl-5-(trifluoromethyl)phenyl)amino)oxazole-4-carboxamide

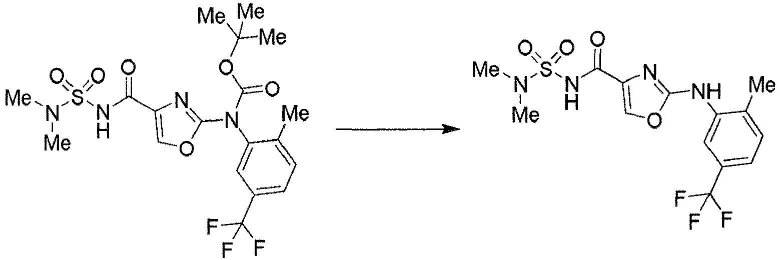

To tert-butyl (4-((N,N-dimethylsulfamoyl)carbamoyl)oxazol-2-yl)(2-methyl-5-(trifluoromethyl)phenyl)carbamate (44 mg) obtained in (4) was added trifluoroacetic acid (2.0 mL), and the mixture was stirred at room temperature for 20 minutes. Solvent was removed under reduced pressure, and then the residue was purified by column chromatography (hexane/ethyl acetate). To the resulted crude product were added isopropylmethyl ether and hexane, and the mixture was stirred, and then the resulted solid was filtered to give the title compound (26 mg).

¹H-NMR (DMSO-D₆) δ: 11.36 (1H, br s), 9.54 (1H, br s), 8.28 (1H, s), 7.42 (1H, d, J = 8.1 Hz), 7.31 (1H, d, J = 8.6 Hz), 2.77 (6H, br s), 2.34 (3H, s) (-NH).

### [Preparation example 3]: Synthesis of 2-((8-fluoro-1,2,3,5,6,7-hexahydro-s-indacen-4-yl)amino)-N-((3-hydroxy-3-methylpyrrolidin-1-yl)sulfonyl)oxazole-4-carboxamide (Example 33)

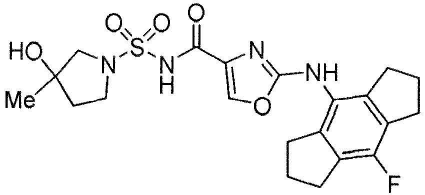

### (1) 4-Fluoro-1,2,3,5,6,7-hexahydro-s-indacene

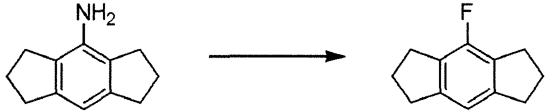

To a solution of 1,2,3,5,6,7-hexahydro-s-indacen-4-amine (40 g) in a mixture of tetrahydrofuran/water (v/v = 5/1, 720 mL) was added tetrafluoroboric acid (170 mL) under an argon atmosphere, and the internal temperature was cooled to -10°C or less. To the reaction mixture was added dropwise a solution of sodium nitrite (18 g) in water (40 mL) over 2 hours with the internal temperature maintained at -5°C or less. The reaction mixture was stirred overnight with the temperature spontaneously rising to room temperature, and then thereto was added water. The mixture was extracted with ethyl acetate. The resulted organic layer was washed sequentially with an aqueous solution of saturated sodium hydrogen carbonate and saturated brine, dried over anhydrous magnesium sulfate, and then solvent was removed under reduced pressure. The residue was purified by column chromatography (hexane/ethyl acetate) to give the title compound (20 g).

¹H-NMR (CDCl₃) δ: 6.85 (1H, s), 2.87-2.85 (8H, m), 2.12-2.05 (4H, m) .

### (2) 4-Fluoro-8-nitro-1,2,3,5,6,7-hexahydro-s-indacene

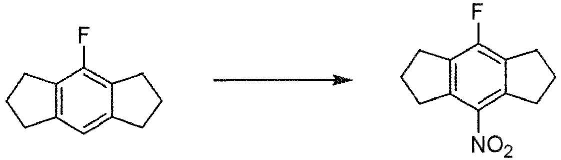

To a solution of 4-fluoro-1,2,3,5,6,7-hexahydro-s-indacene (20 g) obtained in (1) in acetonitrile (300 mL) was added dioxoammonium tetrafluoroborate (18 g) in a ice bath over 1 hour with the internal temperature maintained at 5°C or less. At the same temperature, the reaction mixture was stirred for additional 1 hour, and then thereto was added water. The resulted solid was filtered, and then washed with water to give the title compound (21 g).

¹H-NMR (DMSO-D₆) δ: 3.20 (4H, t, J = 7.5 Hz), 2.91 (4H, t, J = 7.5 Hz), 2.15-2.08 (4H, m).

### (3) 8-Fluoro-1,2,3,5,6,7-hexahydro-s-indacen-4-amine

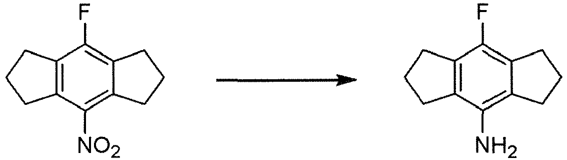

To a solution of 4-fluoro-8-nitro-1,2,3,5,6,7-hexahydro-s-indacene (20 g) obtained in (2) in methanol (400 mL) was added 10% Pd/C (4.0 g), and the mixture was stirred at room temperature overnight under a hydrogen atmosphere. The resulted insoluble matter was filtered off through Celite, and the resulted filtrate was concentrated under reduced pressure to give the title compound (17 g).

¹H-NMR (DMSO-D₆) δ: 4.39 (2H, br s), 2.74 (4H, t, J = 7.4 Hz), 2.61 (4H, t, J = 7.3 Hz), 2.01-1.99 (4H, m).

### (4) Ethyl 2-((tert-butoxycarbonyl)(8-fluoro-1,2,3,5,6,7-hexahydro-s-indacen-4-yl)amino)oxazole-4-carboxylate

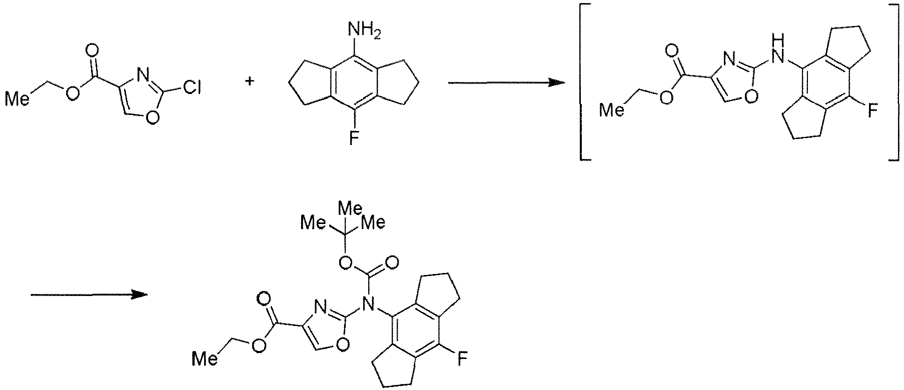

To a solution of ethyl 2-chlorooxazole-4-carboxylate (16 g) in 1-methylpyrrolidin-2-one (200 mL) was added 8-fluoro-1,2,3,5,6,7-hexahydro-s-indacen-4-amine (17 g) obtained in (3) under an argon atmosphere, and the mixture was stirred at 160°C for 5 hours. The reaction mixture was allowed to cool to room temperature, and then thereto were added di-tert-butyl dicarbonate (24 g), triethylamine (15 mL), and 4-dimethylaminopyridine (13 g). Then, the mixture was stirred at 50°C for 3 hours, and then thereto were added water and ethyl acetate. The resulted insoluble matter was filtered off through Celite, and then the filtrate was extracted with ethyl acetate. The resulted organic layer was washed sequentially with water and saturated brine, dried over anhydrous magnesium sulfate, and then solvent was removed under reduced pressure. The residue was purified by column chromatography (hexane/ethyl acetate) to give the title compound (21 g).

¹H-NMR (CDCl₃) δ: 8.00 (1H, s), 4.34 (2H, q, J = 7.2 Hz), 2.85-2.78 (8H, m), 2.14-2.06 (4H, m), 1.44 (9H, s), 1.34 (3H, t, J = 7.1 Hz).

### (5) 2-((tert-Butoxycarbonyl)(8-fluoro-1,2,3,5,6,7-hexahydro-s-indacen-4-yl)amino)oxazole-4-carboxylic acid

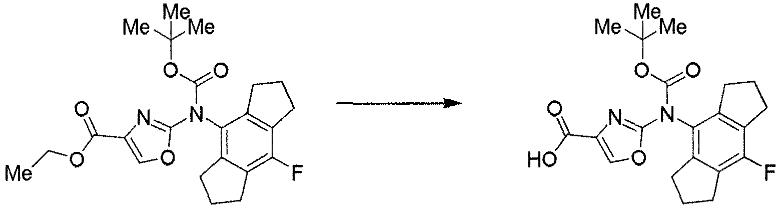

To a solution of ethyl 2-((tert-butoxycarbonyl)(8-fluoro-1,2,3,5,6,7-hexahydro-s-indacen-4-yl)amino)oxazole-4-carboxylate (21 g) obtained in (4) in methanol (210 mL) was added a 2 M aqueous solution of sodium hydroxide (98 mL), and the mixture was stirred at room temperature for 20 minutes. To the reaction mixture was added 2 M hydrochloric acid (98 mL), and the mixture was stirred, and then the resulted solid was filtered. The filtered solid was washed with water and hexane to give the title compound (14 g).

¹H-NMR (DMSO-D₆) δ: 13.15 (1H, br s), 8.61 (1H, s), 2.87-2.85 (4H, m), 2.75-2.71 (2H, m), 2.65-2.63 (2H, m), 2.08-2.05 (4H, m), 1.39 (9H, s).

### (6) tert-Butyl((3-hydroxy-3-methylpyrrolidin-1-yl)sulfonyl)carbamate

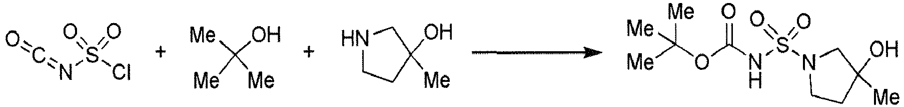

To a solution of chlorosulfonyl isocyanate (0.31 mL) in deuterated chloroform (1.5 mL) was added tert-butanol (0.34 mL) at 0°C under an argon atmosphere, and then the mixture was stirred at room temperature for 2 hours. The reaction mixture was cooled to 0°C, and then thereto were added 3-methylpyrrolidin-3-ol (360 mg) and triethylamine (0.59 mL). The mixture was stirred at room temperature for 1 hour. Solvent was removed under reduced pressure, and then the residue was purified by column chromatography (hexane/ethyl acetate) to give the title compound (200 mg).

¹H-NMR (CDCl₃) δ: 7.07 (1H, br s), 3.71-3.56 (3H, m), 3.37 (1H, d, J = 11.1 Hz), 2.69 (1H, s), 2.04-1.89 (2H, m), 1.47 (9H, s), 1.40 (3H, s).

### (7) 3-Hydroxy-3-methylpyrrolidine-1-sulfonamide

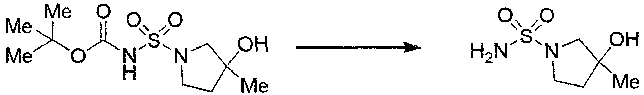

To tert-butyl ((3-hydroxy-3-methylpyrrolidin-1-yl)sulfonyl)carbamate (200 mg) obtained in (6) was added a 4 M solution (3 mL) of hydrogen chloride in ethyl acetate under an argon atmosphere, and the mixture was stirred at room temperature for 1 hour. Then, solvent was removed under reduced pressure. The residue was purified by column chromatography (hexane/ethyl acetate) to give the title compound (80 mg).

¹H-NMR (DMSO-D₆) δ: 6.61 (2H, br s), 4.77 (1H, s), 3.27-3.15 (2H, m), 3.00 (2H, dd, J = 15.5, 10.2 Hz), 1.80-1.67 (2H, m), 1.25 (3H, s).

### (8) tert-Butyl (8-fluoro-1,2,3,5,6,7-hexahydro-s-indacen-4-yl)(4-(((3-hydroxy-3-methylpyrrolidin-1-yl)sulfonyl)carbamoyl)oxazol-2-yl)carbamate

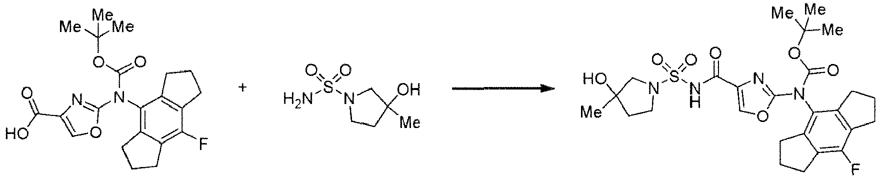

To a solution of 2-((tert-butoxycarbonyl)(8-fluoro-1,2,3,5,6,7-hexahydro-s-indacen-4-yl)amino)oxazole-4-carboxylic acid (120 mg) obtained in (5) in tetrahydrofuran (0.8 mL) was added N,N'-carbonyldiimidazole (72 mg) under an argon atmosphere, and the mixture was stirred at 60°C for 2 hours. Then, the mixture was allowed to cool to room temperature. To the reaction mixture were added a solution of 3-hydroxy-3-methylpyrrolidine-1-sulfonamide (80 mg) obtained in (7) in tetrahydrofuran (0.8 mL) and 1,8-diazabicyclo[5.4.0]-7-undecene (67 µL), and the mixture was stirred at 60°C for 1 hour. Then, the mixture was allowed to cool to room temperature. To the reaction mixture was added acetic acid (0.1 mL), and then solvent was removed under reduced pressure. The residue was purified by column chromatography (hexane/ethyl acetate) to give the title compound (94 mg).

MS (M+H): 686.

### (9) 2-((8-Fluoro-1,2,3,5,6,7-hexahydro-s-indacen-4-yl)amino)-N-((3-hydroxy-3-methylpyrrolidin-1-yl)sulfonyl)oxazole-4-carboxamide

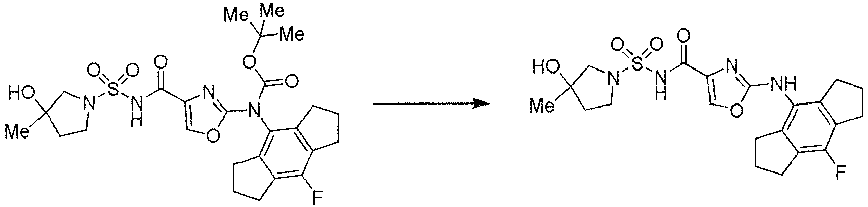

To tert-butyl (8-fluoro-1,2,3,5,6,7-hexahydro-s-indacen-4-yl)(4-(((3-hydroxy-3-methylpyrrolidin-1-yl)sulfonyl)carbamoyl)-oxazol-2-yl)carbamate (94 mg) obtained in (8) was added trifluoroacetic acid (2.0 mL), and the mixture was stirred at room temperature for 20 minutes. Solvent was removed under reduced pressure, and then the residue was purified by column chromatography (hexane/ethyl acetate). To the resulted crude product were added isopropylmethyl ether and hexane, and the mixture was stirred, and then the resulted solid was filtered to give the title compound (63 mg).

¹H-NMR (DMSO-D₆) δ: 11.09 (1H, s), 9.30 (1H, s), 8.25 (1H, br s), 4.88 (1H, br s), 3.53-3.51 (2H, m), 3.20 (2H, dd, J = 15.0, 9.7 Hz), 2.84 (4H, t, J = 7.2 Hz), 2.72 (4H, t, J = 7.3 Hz), 2.05-2.00 (4H, m), 1.80-1.65 (2H, m), 1.22 (3H, s).

### [Preparation example 4]: Synthesis of 2-((8-chloro-1,2,3,5,6,7-hexahydro-s-indacen-4-yl)amino)-N-(N,N-dimethylsulfamoyl)oxazole-4-carboxamide (Example 28)

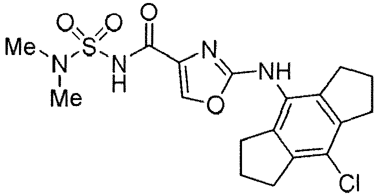

### (1) 8-Chloro-1,2,3,5,6,7-hexahydro-s-indacen-4-amine

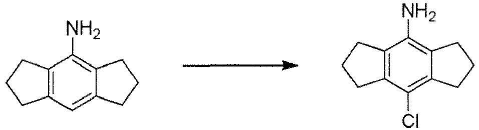

To a solution of 1,2,3,5,6,7-hexahydro-s-indacen-4-amine (10 g) in acetonitrile (100 mL) was added N-chlorosuccinimide (8.1 g) under an argon atmosphere, and the mixture was stirred at room temperature overnight. To the reaction mixture was added an aqueous solution of sodium sulfite, and then the mixture was extracted with ethyl acetate. The resulted organic layer was washed with an aqueous solution of saturated sodium hydrogen carbonate and saturated brine, dried over anhydrous magnesium sulfate, and then solvent was removed under reduced pressure. The residue was purified by column chromatography (hexane/ethyl acetate), and then to the resulted crude product was added hexane, and the mixture was stirred. Then, the resulted solid was filtered to give the title compound (7.8 g).

¹H-NMR (DMSO-D₆) δ: 4.65 (2H, br s), 2.74 (4H, t, J = 7.5 Hz), 2.66 (4H, t, J = 7.5 Hz), 2.00-1.98 (4H, m).

### (2) Ethyl 2-((tert-butoxycarbonyl)(8-chloro-1,2,3,5,6,7-hexahydro-s-indacen-4-yl)amino)oxazole-4-carboxylate

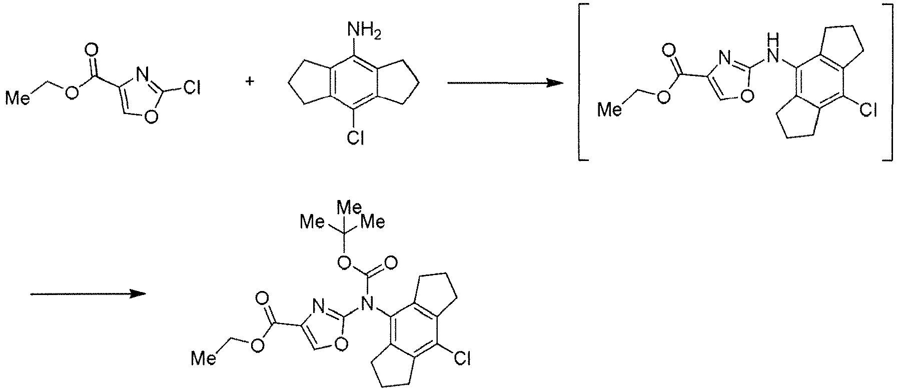

To a solution of ethyl 2-chlorooxazole-4-carboxylate (6.6 g) in 1-methylpyrrolidin-2-one (40 mL) was added 8-chloro-1,2,3,5,6,7-hexahydro-s-indacen-4-amine (7.8 g) obtained in (1) under an argon atmosphere, and the mixture was stirred at 120°C for 3 hours. To the reaction mixture was added water, and then the mixture was extracted with a mixed solution of ethyl acetate/tetrahydrofuran. The resulted organic layer was washed with water and saturated brine, dried over magnesium sulfate, and then solvent was removed under reduced pressure. To a solution of the residue in tetrahydrofuran (40 mL) were added di-tert-butyl dicarbonate (9.9 g) and 4-dimethylaminopyridine (5.5 g), and the mixture was stirred at 50°C for 3 hours. Then, solvent was removed under reduced pressure. The residue was purified by column chromatography (hexane/ethyl acetate) to give the title compound (9.0 g).

¹H-NMR (CDCl₃) δ: 8.00 (1H, s), 4.34 (2H, q, J = 7.2 Hz), 2.94-2.73 (8H, m), 2.13-2.04 (4H, m), 1.44 (9H, s), 1.34 (3H, t, J = 7.2 Hz).

### (3) 2-((tert-Butoxycarbonyl)(8-chloro-1,2,3,5,6,7-hexahydro-s-indacen-4-yl)amino)oxazole-4-carboxylic acid

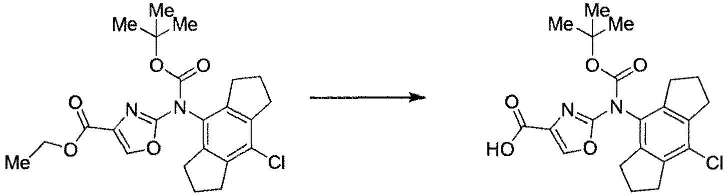

To a mixed solution of ethyl 2-((tert-butoxycarbonyl)(8-chloro-1,2,3,5,6,7-hexahydro-s-indacen-4-yl)amino)oxazole-4-carboxylate (8.1 g) obtained in (2) in methanol-tetrahydrofuran (v/v = 2/1, 150 mL) was added a 2 M aqueous solution of sodium hydroxide (36 mL), and the mixture was stirred at room temperature for 20 minutes. To the reaction mixture were added 2 M hydrochloric acid (36 mL) and water, and the mixture was stirred. The resulted solid was filtered, and then washed with water and hexane to give the title compound (6.3 g).

¹H-NMR (DMSO-D₆) δ: 8.60 (1H, s), 2.92-2.62 (8H, m), 2.11-1.98 (4H, m), 1.40 (9H, s).

### (4) tert-Butyl (8-chloro-1,2,3,5,6,7-hexahydro-s-indacen-4-yl)(4-((N,N-dimethylsulfamoyl)carbamoyl)oxazol-2-yl)carbamate

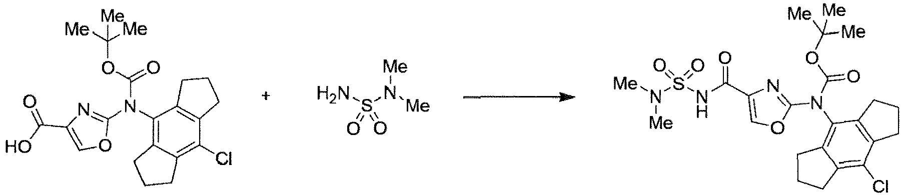

To a solution of 2-((tert-butoxycarbonyl)(8-chloro-1,2,3,5,6,7-hexahydro-s-indacen-4-yl)amino)oxazole-4-carboxylic acid (120 mg) obtained in (3) in tetrahydrofuran (1.2 mL) was added N,N'-carbonyldiimidazole (70 mg) under an argon atmosphere, and the mixture was stirred at 60°C for 2 hours. The reaction mixture was allowed to cool to room temperature, and then thereto were added N,N-dimethylsulfamide (53 mg) and 1,8-diazabicyclo[5.4.0]-7-undecene (65 µL). Then, the mixture was stirred at 60°C for an additional 1 hour. To the reaction mixture was added acetic acid (0.1 mL), and then solvent was removed under reduced pressure. The residue was purified by column chromatography (hexane/ethyl acetate) to give the title compound (110 mg).

¹H-NMR (DMSO-D₆) δ: 11.92-11.68 (1H, m), 8.71 (1H, s), 2.93-2.65 (14H, m), 2.10-2.03 (4H, m), 1.40 (9H, s).

### (5) 2-((8-Chloro-1,2,3,5,6,7-hexahydro-s-indacen-4-yl)amino)-N-(N,N-dimethylsulfamoyl)oxazole-4-carboxamide

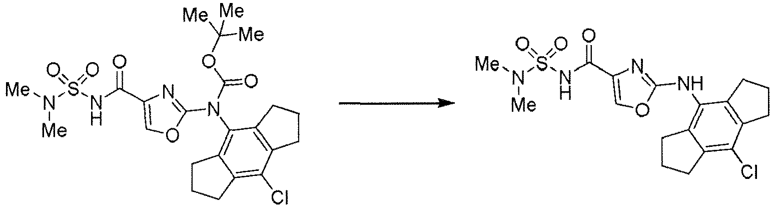

To tert-butyl (8-chloro-1,2,3,5,6,7-hexahydro-s-indacen-4-yl)(4-((N,N-dimethylsulfamoyl)carbamoyl)oxazol-2-yl)carbamate (110 mg) obtained in (4) was added trifluoroacetic acid (2.0 mL), and the mixture was stirred at room temperature for 20 minutes. Solvent was removed under reduced pressure, and then the residue was purified by column chromatography (hexane/ethyl acetate). Then, to the resulted crude product were added isopropylmethyl ether and hexane, and the mixture was stirred. The resulted solid was filtered to give the title compound (75 mg).

¹H-NMR (DMSO-D₆) δ: 11.22 (1H, br s), 9.42 (1H, s), 8.29 (1H, s), 2.86 (4H, t, J = 7.3 Hz), 2.81 (6H, s), 2.77 (4H, t, J = 7.4 Hz), 2.05-1.98 (4H, m).

### [Preparation example 5]: Synthesis of (S)-2-((4-fluoro-2,5-dimethylphenyl)amino)-N-((7-((2-methoxyethoxy)methyl)-1,4-oxazepan-4-yl)sulfonyl)oxazole-4-carboxamide (Example 3-005)

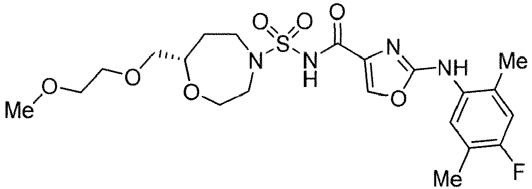

### (1) 4-Fluoro-2,5-dimethylaniline

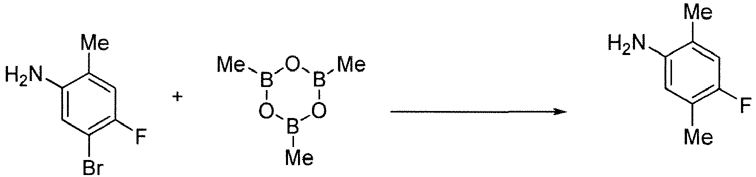

To a mixture of 5-bromo-4-fluoro-2-methylaniline (2.5 g) in 1,2-dimethoxyethane (25 mL) were added [1,1'-bis(diphenylphosphino)ferrocene]palladium(II)dichloride dichloromethane adduct (500 mg), 2 M aqueous solution of potassium carbonate (12 mL), and 2,4,6-trimethylboroxine (2.3 g) under an argon atmosphere, and the mixture was stirred at 90°C overnight. The reaction mixture was allowed to cool to room temperature, and then thereto was added water, and the mixture was extracted with ethyl acetate. The resulted organic layer was dried over anhydrous magnesium sulfate, and then solvent was removed under reduced pressure. The residue was purified by column chromatography (hexane/ethyl acetate= 1/0 to 4/1) to give the title compound (1.0 g).

¹H-NMR (DMSO-D₆) δ: 6.69 (1H, d, J = 10.6 Hz), 6.43 (1H, d, J = 7.4 Hz), 4.56 (2H, s), 2.06 (3H, br s), 1.99 (3H, s).

### (2) Ethyl 2-((4-fluoro-2,5-dimethylphenyl)amino)oxazole-4-carboxylate

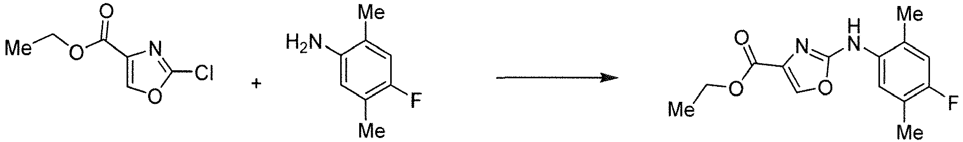

To a mixture of ethyl 2-chlorooxazole-4-carboxylate (1.4 g) in 1-methylpyrrolidin-2-one (10 mL) was added 4-fluoro-2,5-dimethylaniline (1.0 g) obtaied in (1) under an argon atmosphere, and the mixture was stirred at 150°C for 5.5 hours. The reaction mixture was allowed to cool to room temperature, and then thereto was added water, and the mixture was extracted with ethyl acetate. The resulted organic layer was washed with water, dried over anhydrous magnesium sulfate, and then solvent was removed under reduced pressure. The residue was purified by column chromatography (hexane/ethyl acetate= 9/1 to 2/3) to give a crude product of the title compound (3.5 g).

¹H-NMR (DMSO-D₆) δ: 9.28 (1H, s), 8.29 (1H, s), 7.44 (1H, d, J = 7.6 Hz), 7.02 (1H, d, J = 10.4 Hz), 4.23 (2H, q, J = 7.1 Hz), 2.19 (6H, s), 1.25 (3H, t, J = 7.1 Hz).

### (3) Ethyl 2-((tert-butoxycarbonyl)(4-fluoro-2,5-dimethylphenyl)amino)oxazole-4-carboxylate

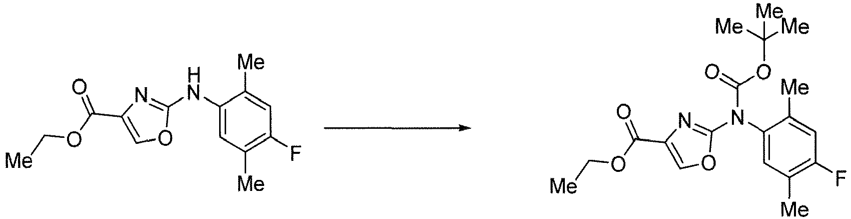

To a mixture of ethyl 2-((4-fluoro-2,5-dimethylphenyl)amino)oxazole-4-carboxylate (2.3 g) obtained in (2) in tetrahydrofuran (23 mL) were added 4-dimethylaminopyridine (0.20 g) and di-tert-butyl dicarbonate (3.6 g) under an argon atmosphere at room temperature, and the mixture was stirred at 60°C for 1 hour. Solvent was removed under reduced pressure, and then the residue was purified by column chromatography (hexane/ethyl acetate= 1/0 to 4/1) to give the title compound (1.9 g).

¹H-NMR (DMSO-D₆) δ: 8.74 (1H, s), 7.24 (1H, d, J = 7.4 Hz), 7.15 (1H, d, J = 10.4 Hz), 4.25 (2H, q, J = 7.1 Hz), 2.18 (6H, d, J = 6.5 Hz), 1.40 (9H, s), 1.25 (3H, t, J = 7.1 Hz).

### (4) 2-((tert-Butoxycarbonyl)(4-fluoro-2,5-dimethylphenyl)amino)oxazole-4-carboxylic acid

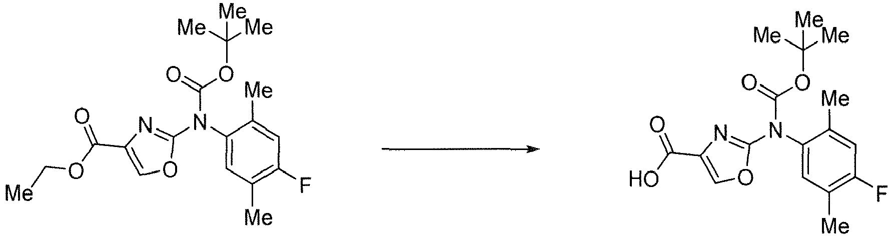

To a mixture of ethyl 2-((tert-butoxycarbonyl) (4-fluoro-2,5-dimethylphenyl)amino)oxazole-4-carboxylate (1.9 g) obtained in (3) in ethanol (19 mL) was added 2 M aqueous solution of sodium hydroxide (10 mL), and the mixture was stirred at room temperature for 30 minutes. The reaction mixture was neutralized with 2 M hydrochloric acid (10 mL), and then the mixture was extracted with ethyl acetate. The resulted organic layer was dried over anhydrous magnesium sulfate, and then solvent was removed under reduced pressure. To the residue was added diisopropyl ether, and the mixture was stirred. The resulted solid was collected by filtration to give the title compound (1.1 g).

¹H-NMR (DMSO-D₆) δ: 13.13 (1H, s), 8.64 (1H, s), 7.24 (1H, d, J = 7.4 Hz), 7.15 (1H, d, J = 10.4 Hz), 2.19 (6H, d, J = 3.9 Hz), 1.40 (9H, s).

### (5) (S)-4-Benzyl-3-oxo-1,4-oxazepane-7-carboxylic acid

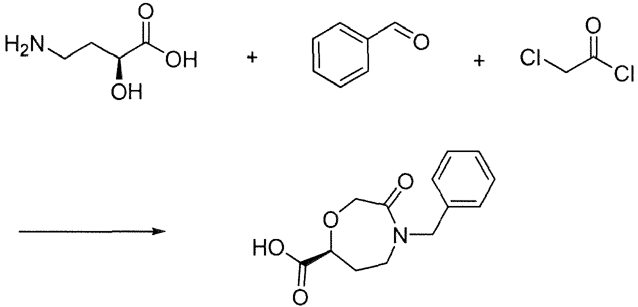

To a solution of (S)-4-amino-2-hydroxybutanoic acid (27 g) in water (240 mL) was added sodium hydroxide (9.7 g), and then thereto was added benzaldehyde (25.7 g). The mixture was stirred at room temperature for 30 minutes, and then the reaction mixture was cooled to 0°C in a ice bath. Thereto was added slowly sodium borohydride (5.84 g) over 15 minutes at the same temperature, and then the mixture was stirred at room temperature for 2 days. The reaction mixture was cooled to 0°C in a ice bath, and then neutralized with concentrated hydrochloric acid to pH = 6. To the mixture were added sequentially sodium hydroxide (27.1 g) and 2-chloroacetyl chloride (30.6 g), and then the mixture was stirred at room temperature for 1 hour. To the reaction mixture was added concentrated hydrochloric acid to pH = 1, and the mixture was extracted with ethyl acetate. The resulted organic layer was washed with saturated brine. The resulted organic layer was dried over anhydrous magnesium sulfate, and then solvent was removed under reduced pressure. To the residue was added 4 M aqueous solution of sodium hydroxide (250 mL). The mixture was stirred at room temperature for 30 minutes, and then washed with a mixed solvent of hexane/ethyl acetate (v/v = 1/1, 100 mL). To the aqueous layer was added concentrated sulfuric acid to pH = 1 in a ice bath, and then the mixture was extracted with ethyl acetate. The resulted organic layer was dried over anhydrous sodium sulfate, and then solvent was removed under reduced pressure. The residue was purified by column chromatography (hexane/ethyl acetate= 1/1 to 1/2) to give the title compound (29.4 g).

¹H-NMR (CDCl₃) δ: 7.39-7.22 (5H, m), 4.71-4.55 (3H, m), 4.37 (1H, d, J = 14.8 Hz), 4.29-4.23 (1H, m), 3.54-3.38 (2H, m), 2.35-2.25 (1H, m), 2.13-2.01 (1H, m).

### (6) (S)-(4-Benzyl-1,4-oxazepan-7-yl)methanol

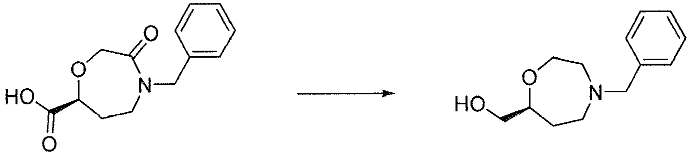

To a mixture of lithium aluminium hydride (3.81 g) in tetrahydrofuran (160 mL) was added dropwise a solution of (S)-4-benzyl-3-oxo-1,4-oxazepane-7-carboxylic acid (10.0 g) obtained in (5) in tetrahydrofuran (10 mL) with the internal temperature maintained at 20°C or less under an argon atmosphere at 0°C, and then the mixture was stirred at room temperature for 2 hours. To the reaction mixture were added water (3.81 mL), 15% aqueous solution of sodium hydroxide (3.81 mL), water (3.81 mL) in a ice bath, and then the mixture was stirred at room temperature for 20 minutes. The resulted insoluble matter was filtered off through Celite, and washed with tetrahydrofuran. The combined filtrate was concentrated under reduced pressure to give a crude product of the title compound (6.61 g). The crude product was used in the next step with no additional purification.

¹H-NMR (CDCl₃) δ: 7.36-7.22 (5H, m), 3.96-3.84 (2H, m), 3.70-3.61 (3H, m), 3.53-3.47 (2H, m), 2.77-2.63 (4H, m), 1.90-1.80 (1H, m), 1.70-1.60 (1H, m).

### (7) (S)-(1,4-Oxazepan-7-yl)methanol

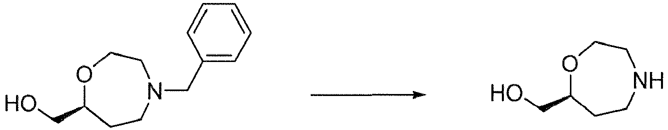

To (S)-(4-benzyl-1,4-oxazepan-7-yl)methanol (6.14 g) in methanol (61 mL) obtained in (6) was added 10% palladium carbon (3.0 g), and the mixture was stirred under a hydrogen atmosphere (4 atm) for 24 hours. The resulted insoluble matter was filtered off through Celite, and the resulted filtrate was concentrated under reduced pressure to give a crude product of the title compound (3.87 g). The crude product was used in the next step with no additional purification.

¹H-NMR (CDCl₃) δ: 4.04-3.97 (1H, m), 3.83-3.75 (1H, m), 3.63-3.46 (3H, m), 2.99-2.94 (4H, m), 1.90-1.80 (1H, m), 1.64-1.53 (1H, m).

### (8) tert-Butyl (S)-7-(hydroxymethyl)-1,4-oxazepane-4-carboxylate

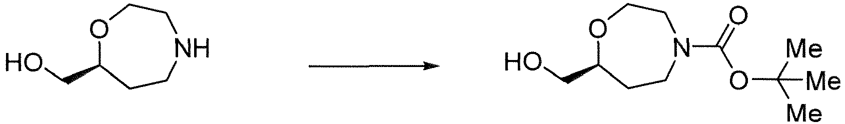

To a mixture of (S)-(1,4-oxazepan-7-yl)methanol (3.0 g) obtained in (7) in tetrahydrofuran (56 mL) were added di-tert-butyl dicarbonate (4.69 g) and triethylamine (3.0 mL). The mixture was stirred at room temperature for 2 hours, and then solvent was removed under reduced pressure. The residue was purified by column chromatography (hexane/ethyl acetate= 2/1 to 1/1) to give the title compound (3.78 g).

¹H-NMR (CDCl₃) δ: 4.13-3.13 (9H, m), 2.16-2.04 (1H, m), 1.96-1.83 (1H, m), 1.47 (9H, s).

### (9) tert-Butyl (S)-7-((2-methoxyethoxy)methyl)-1,4-oxazepane-4-carboxylate

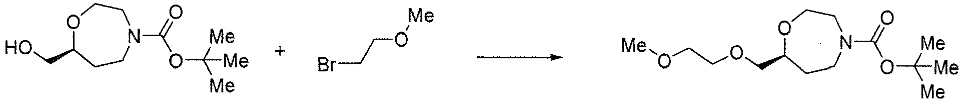

To a mixture of sodium hydride (60% in oil, 67 mg) in N,N-dimethylformamide (3.0 mL) was added tert-butyl (S)-7-(hydroxymethyl)-1,4-oxazepane-4-carboxylate (300 mg) obtained in (8) under an argon atmosphere at 0°C, and the mixture was stirred at room temperature for 2 hours. To the reaction mixture was added 1-bromo-2-methoxyethane (0.24 mL), and then the mixture was stirred for 22 hours. To the reaction mixture was added water, and then the mixture was extracted with ethyl acetate. The resulted organic layer was washed with saturated brine, dried over anhydrous magnesium sulfate, and then solvent was removed under reduced pressure. The residue was purified by column chromatography (hexane/ethyl acetate= 4/1 to 1/1) to give the title compound (110 mg).

¹H-NMR (CDCl₃) δ: 4.07-3.96 (1H, m), 3.91-3.15 (12H, m), 3.38 (3H, s), 2.04-1.91 (1H, m), 1.69-1.56 (1H, m), 1.46 (9H, s).

### (10) (S)-7-((2-Methoxyethoxy)methyl)-1,4-oxazepane trifluoroacetate

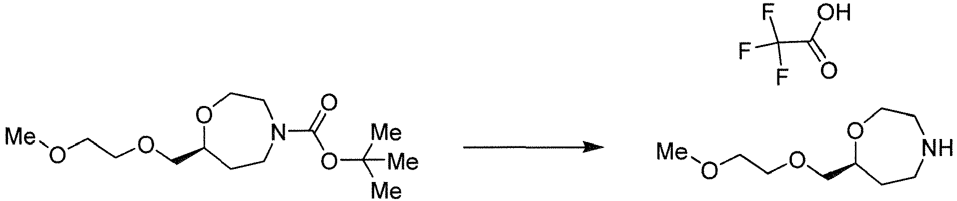

To tert-butyl (S)-7-((2-methoxyethoxy)methyl)-1,4-oxazepane-4-carboxylate (110 mg) obtained in (9) was added trifluoroacetic acid (2.0 mL), and the mixture was stirred at room temperature for 1 hour, and then solvent was removed under reduced pressure to give a crude product of the title compound (118 mg). The crude product was used in the next step with no additional purification.

### (11) (S)-7-((2-Methoxyethoxy)methyl)-1,4-oxazepane-4-sulfonamide

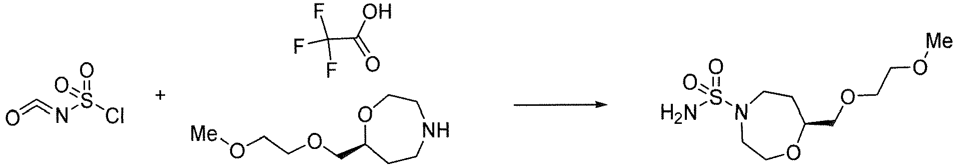

To a mixture of chlorosulfonyl isocyanate (0.044 mL) in deuterated chloroform (1.2 mL) was added tert-butanol (0.048 mL) under an argon atmosphere at 0°C, and the mixture was stirred at the same temperature for 2 hours. To the reaction mixture were added a mixture of (S)-7-((2-methoxyethoxy)methyl)-1,4-oxazepane trifluoroacetate (118 mg) obtained in (10) in deuterated chloroform (1.2 mL) and triethylamine (0.27 mL) at the same temperature, and then the mixture was stirred at room temperature for 1 hour. To the reaction mixture was added acetic acid (0.16 mL), and thereto was added water. The reaction mixture was extracted with ethyl acetate, and then the resulted organic layer was washed with saturated brine, dried over anhydrous magnesium sulfate, and then solvent was removed under reduced pressure. To the residue was added trifluoroacetic acid (2.4 mL). The mixture was stirred at room temperature for 1 hour, and then solvent was removed under reduced pressure. The residue was purified by column chromatography (hexane/ethyl acetate= 2/1 to 1/1) to give the title compound (80 mg).

¹H-NMR (CDCl₃) δ: 4.45 (2H, br s), 4.11-4.04 (1H, m), 3.90-3.83 (1H, m), 3.73-3.32 (11H, m), 3.38 (3H, s), 2.09-2.00 (1H, m), 1.91-1.81 (1H, m).

### (12) tert-Butyl (S)-(4-fluoro-2,5-dimethylphenyl)(4-(((7-((2-methoxyethoxy)methyl)-1,4-oxazepan-4-yl)sulfonyl)carbamoyl)oxazol-2-yl)carbamate

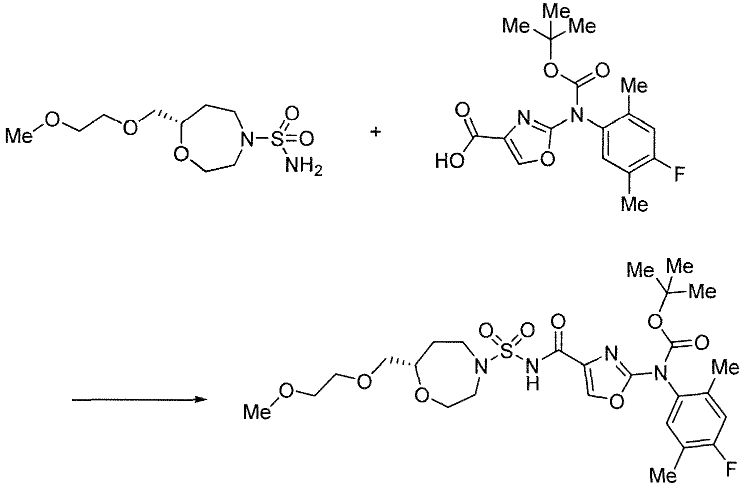

To a mixture of 2-((tert-butoxycarbonyl)(4-fluoro-2,5-dimethylphenyl)amino)oxazole-4-carboxylic acid (50 mg) obtained in (4) in tetrahydrofuran (1.0 mL) was added 1,1'-carbonyldiimidazole under an argon atmosphere at room temperature, and then the mixture was stirred at 60°C for 30 minutes. The reaction mixture was allowed to cool to room temperature, and then thereto were added (S)-7-((2-methoxyethoxy)methyl)-1,4-oxazepane-4-sulfonamide (38 mg) obtained in (11) and 1,8-diazabicyclo[5.4.0]undec-7-ene (65 mg), and the mixture was stirred at 60°C for 1 hour. The reaction mixture was allowed to cool to room temperature, and then thereto were added acetic acid (43 mg) and water. The residue was extracted with ethyl acetate, and the resulted organic layer was washed with saturated brine, dried over anhydrous magnesium sulfate, and then solvent was removed under reduced pressure. the residue was purified by thin-layer chromatography (hexane/ethyl acetate/acetic acid= 100/200/3) to give the title compound (45 mg).

### (13) (S)-2-((4-Fluoro-2,5-dimethylphenyl)amino)-N-((7-((2-methoxyethoxy)methyl)-1,4-oxazepan-4-yl)sulfonyl)oxazole-4-carboxamide

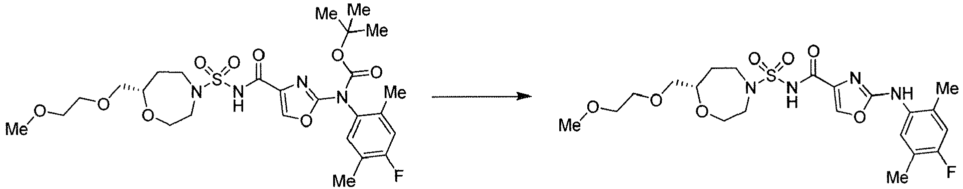

To tert-butyl (S)-(4-fluoro-2,5-dimethylphenyl)(4-(((7-((2-methoxyethoxy)methyl)-1,4-oxazepan-4-yl)sulfonyl)carbamoyl)oxazol-2-yl)carbamate (45 mg) obtained in (12) was added trifluoroacetic acid (2.5 mL), and the mixture was stirred at room temperature for 20 minutes, and then solvent was removed under reduced pressure. The residue was purified by column chromatography (hexane/ethyl acetate/acetic acid= 100/200/3 to 0/100/1) to give the title compound (27 mg).

¹H-NMR (CDCl₃) δ: 8.97 (1H, br s), 7.86 (1H, s), 7.47 (1H, d, J = 7.2 Hz), 6.89 (1H, d, J = 9.7 Hz), 6.35 (1H, s), 4.11-4.04 (1H, m), 3.92-3.61 (6H, m), 3.59-3.40 (6H, m), 3.37 (3H, s), 2.29 (3H, s), 2.25 (3H, s), 2.14-2.03 (1H, m), 1.85-1.75 (1H, m).

MS:501(M+1).

### [Preparation example 6]: Synthesis of (S)-2-((4-fluoro-2,5-dimethylphenyl)amino)-N-((7-(methoxymethyl)-1,4-oxazepan-4-yl)sulfonyl)oxazole-4-carboxamide (Example 2-347)

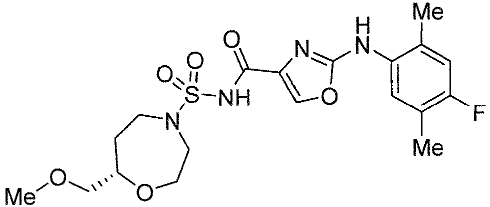

### (1) 4-(Benzyloxy)-3-hydroxybutanenitrile

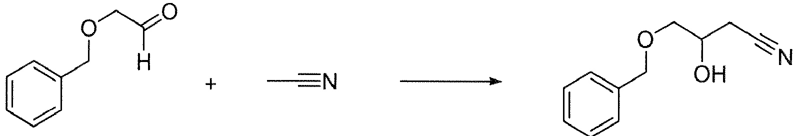

To a solution of n-butyllithium (1.58 M in hexane, 50.6 mL) in tetrahydrofuran (20 mL) was added a solution of acetonitrile (5.2 mL) in tetrahydrofuran (10 mL) under an argon atmosphere at -78°C, and the mixture was stirred at the same temperature for 1 hour. To the reaction mixture was added a solution of 2-(benzyloxy)acetaldehyde (10 g) in tetrahydrofuran (20 mL), and then the mixture was stirred at 0°C for 30 minutes. To the reaction mixture was added an aqueous solution of saturated ammonium chloride, and then the mixture was extracted with ethyl acetate. The resulted organic layer was washed with saturated brine, dried over anhydrous magnesium sulfate, and then solvent was removed under reduced pressure. The residue was purified by column chromatography (hexane/ethyl acetate= 1/0 to 0/1) to give the title compound (9.97 g).

¹H-NMR (DMSO-D₆) δ: 7.41-7.26 (5H, m), 5.51 (1H, d, J = 5.2 Hz), 4.51 (2H, s), 3.92 (1H, td, J = 11.2, 5.2 Hz), 3.44 (1H, dd, J = 9.7, 5.2 Hz), 3.35 (1H, dd, J = 9.7, 6.0 Hz), 2.69 (1H, dd, J = 17.2, 4.5 Hz), 2.57 (1H, dd, J = 17.2, 6.7 Hz).

### (2) 4-Amino-1-(benzyloxy)butan-2-ol

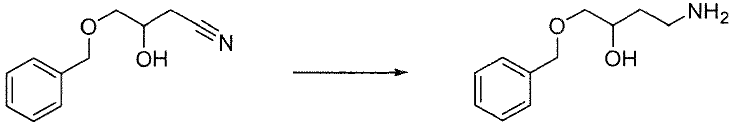

To a mixture of lithium aluminium hydride (6.9 g) in diethyl ether (200 mL) was added dropwise a mixture of 4-(benzyloxy)-3-hydroxybutanenitrile (9.97 g) obtained in (1) in diethyl ether (100 mL) under an argon atmosphere at 0°C. The reaction mixture was stirred at the same temperature for 2 hours, and then thereto were added sequentially water (7 mL), 4 M aqueous solution of sodium hydroxide (7 mL), and water (21 mL). The mixture was stirred at room temperature for 1 hour, and then the resulted insoluble matter was filtered off through Celite, and washed with tetrahydrofuran. The combined filtrate was concentrated under reduced pressure to give a crude product of the title compound (9.7 g). The crude product was used in the next step with no additional purification.

¹H-NMR (DMSO-D₆) δ: 7.37-7.25 (5H, m), 4.48 (2H, s), 3.76-3.70 (1H, m), 3.34-3.30 (5H, m), 2.70-2.60 (2H, m), 1.55-1.47 (1H, m), 1.40-1.31 (1H, m).

### (3) 6-((Benzyloxy)methyl)-2-phenyl-1,3-oxazinane

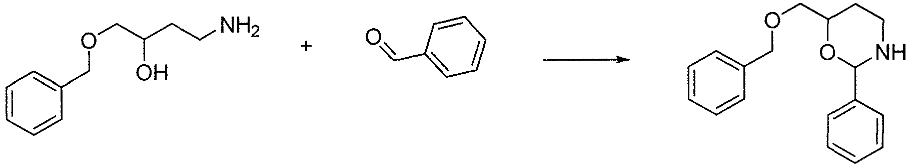

To a mixture of 4-amino-1-(benzyloxy)butan-2-ol (9.6 g) obtained in (2) in tetrahydrofuran (96 mL) was added benzaldehyde (5.0 mL), and the mixture was stirred at room temperature for 50 minutes. Solvent was removed under reduced pressure to give a crude product of the title compound (13.9 g). The crude product was used in the next step with no additional purification.

### (4) 4-(Benzylamino)-1-(benzyloxy)butan-2-ol

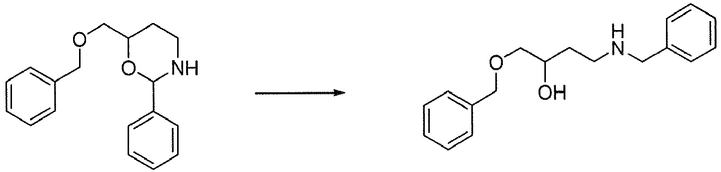

To a solution of 6-((benzyloxy)methyl)-2-phenyl-1,3-oxazinane (13.9 g) obtained in (3) in methanol (210 mL) was added slowly in four portions sodium borohydride (4.1 g) under an argon atmosphere at 0°C. The reaction mixture was stirred at room temperature overnight, and then thereto was added water and ethyl acetate. The organic layer was separated, and then the aqueous layer was extracted with ethyl acetate. The resulted organic layer was washed with saturated brine, dried over anhydrous magnesium sulfate, and then solvent was removed under reduced pressure to give a crude product of the title compound (13.3 g). The crude product was used in the next step with no additional purification.

¹H-NMR (DMSO-D₆) δ: 7.36-7.18 (10H, m), 4.48 (3H, t, J = 4.9 Hz), 3.74-3.71 (1H, m), 3.66 (2H, s), 3.30 (3H, ddd, J = 20.0, 9.6, 5.7 Hz), 2.59 (2H, t, J = 6.9 Hz), 1.66-1.58 (1H, m), 1.45 (1H, dq, J = 18.0, 5.1 Hz).

### (5) 4-Benzyl-7-((benzyloxy)methyl)-1,4-oxazepan-3-one

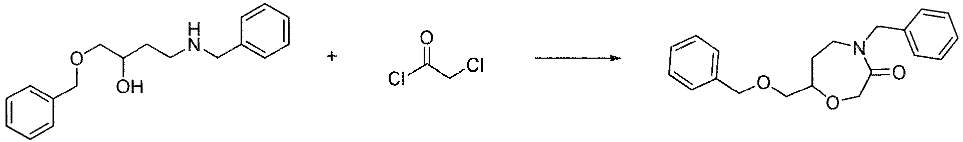

To a mixture of 4-(benzylamino)-1-(benzyloxy)butan-2-ol (13.3 g) obtained in (4) in tetrahydrofuran (130 mL) were added 4 M aqueous solution of sodium hydroxide (47 mL) and chloroacetyl chloride (7.5 mL) in a ice bath, and then the mixture was stirred at room temperature for 90 minutes. To the reaction mixture were added again 4 M aqueous solution of sodium hydroxide (46 mL) and chloroacetyl chloride (2.0 mL), and the mixture was stirred for 1 hour, and then thereto was added 4 M aqueous solution of sodium hydroxide (46 mL). The mixture was stirred at room temperature overnight. To the reaction mixture was added 4 M aqueous solution of sodium hydroxide (23 mL), and the mixture was stirred at room temperature overnight. To the reaction mixture was further added 4 M aqueous solution of sodium hydroxide (23 mL), and then the mixture was stirred at room temperature for 5 days. To the reaction mixture was added 4 M aqueous solution of sodium hydroxide (23 mL), and the mixture was stirred at room temperature overnight. To the reaction mixture was added water, and then the mixture was extracted with ethyl acetate. The resulted organic layer was dried over anhydrous magnesium sulfate, and then solvent was removed under reduced pressure. The residue was purified by column chromatography (hexane/ethyl acetate= 39/11 to 57/43) to give the title compound (11.4 g).

¹H-NMR (DMSO-D₆) δ: 7.36-7.21 (10H, m), 4.55 (1H, d, J = 14.8 Hz), 4.48-4.47 (3H, m), 4.32 (1H, d, J = 14.8 Hz), 4.20 (1H, d, J = 14.8 Hz), 3.78 (1H, td, J = 9.7, 4.3 Hz), 3.54 (1H, dd, J = 14.9, 9.1 Hz), 3.45 (1H, dd, J = 10.4, 6.2 Hz), 3.36-3.31 (2H, m), 1.84-1.79 (1H, m), 1.53 (1H, ddd, J = 16.8, 7.7, 6.0 Hz).

### (6) 4-Benzyl-7-(hydroxymethyl)-1,4-oxazepan-3-one

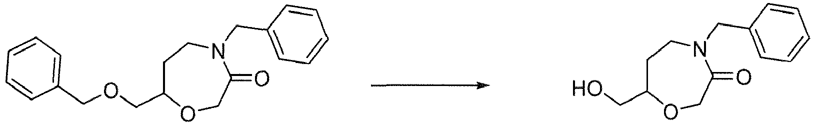

To a mixture of 4-benzyl-7-((benzyloxy)methyl)-1,4-oxazepan-3-one (4.0 g) obtained in (5) in ethanol (80 mL) was added palladium carbon (0.80 g), and the mixture was stirred under a hydrogen atmosphere overnight. The resulted insoluble matter was filtered off through Celite, and washed with ethanol. The combined filtrate was concentrated under reduced pressure. The residue was purified by column chromatography (hexane/ethyl acetate= 1/0 to 0/1) to give the title compound (1.96 g).

¹H-NMR (DMSO-D₆) δ: 7.30 (5H, dt, J = 30.4, 7.5 Hz), 4.69 (1H, t, J = 5.8 Hz), 4.55 (1H, d, J = 14.8 Hz), 4.47 (1H, d, J = 14.8 Hz), 4.31 (1H, d, J = 14.8 Hz), 4.20 (1H, d, J = 14.8 Hz), 3.57-3.48 (2H, m), 3.39-3.36 (2H, m), 3.26 (1H, dd, J = 11.0, 5.7 Hz), 1.82-1.79 (1H, m), 1.51-1.43 (1H, m).

### (7) (R)-4-Benzyl-7-(hydroxymethyl)-1,4-oxazepan-3-one, and (S)-4-benzyl-7-(hydroxymethyl)-1,4-oxazepan-3-one

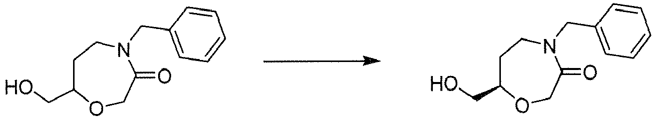

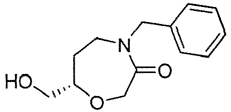

4-Benzyl-7-(hydroxymethyl)-1,4-oxazepan-3-one (1.8 g) obtained in (6) was purified by using an automated recycling preparative HPLC device (device name: Japan Analytical Industry LaboACE LC-7080, column: Daicel CHIRALPAK IG, 20 mm (I.D.) x 250 mm (L), 5 µm, flow rate of mobile phase:20 mL/min, mixture ratio of mobile phase: isocratic, MeOH/CH₃CN = 10/90) to give (R)-4-benzyl-7-(hydroxymethyl)-1,4-oxazepan-3-one (810 mg, Peak with longer retention time, 85.7%ee) and (S)-4-benzyl-7-(hydroxymethyl)-1,4-oxazepan-3-one (685 mg, Peak with shorter retention time, 95.7%ee). Absolute configuration of (S)-4-benzyl-7-(hydroxymethyl)-1,4-oxazepan-3-one was determined by X-ray crystallography.

### (8) (S)-4-Benzyl-7-(methoxymethyl)-1,4-oxazepan-3-one

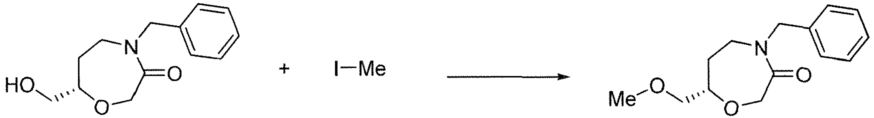

To a mixture of (S)-4-benzyl-7-(hydroxymethyl)-1,4-oxazepan-3-one (500 mg)obtained in (7) in tetrahydrofuran (5.0 mL) was added sodium hydride (60% in oil, 100 mg) under an argon atmosphere at 0°C, and the mixture was stirred at the same temperature for 1 hour. To the reaction mixture was added methyl iodide (0.2 mL), and then the mixture was stirred at room temperature for 1 hour. Then thereto was added an aqueous solution of saturated ammonium chloride. The mixture was extracted with ethyl acetate, and the resulted organic layer wasdried over anhydrous magnesium sulfate, and then solvent was removed under reduced pressure. The residue was purified by column chromatography (hexane/ethyl acetate= 4/1 to 0/1) to give the title compound (460 mg).

¹H-NMR (DMSO-D₆) δ: 7.35-7.33 (2H, m), 7.27-7.25 (3H, m), 4.55 (1H, d, J = 15.0 Hz), 4.47 (1H, d, J = 14.8 Hz), 4.31 (1H, d, J = 14.6 Hz), 4.19 (1H, d, J = 14.8 Hz), 3.74-3.71 (1H, m), 3.53 (1H, ddd, J = 14.7, 10.2, 1.4 Hz), 3.38-3.29 (2H, m), 3.26-3.23 (4H, m), 1.79-1.77 (1H, m), 1.50-1.46 (1H, m).

### (9) (S)-4-Benzyl-7-(methoxymethyl)-1,4-oxazepane

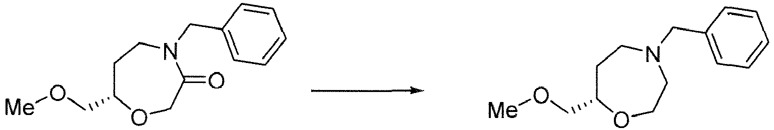

To a mixture of (S)-4-benzyl-7-(methoxymethyl)-1,4-oxazepan-3-one (460 mg) obtained in (8) in tetrahydrofuran (10 mL) was added lithium aluminium hydride (230 mg) under an argon atmosphere at 0°C, and the mixture was stirred at 60°C for 3 hours. To the reaction mixture were added sequentially water (0.25 mL), 4 M aqueous solution of sodium hydroxide (0.25 mL), water (0.75 mL) in a ice bath, and then the mixture was stirred at room temperature for 2 hours. The resulted insoluble matter was filtered off through Celite, and washed with tetrahydrofuran. The combined filtrate was concentrated under reduced pressure to give a crude product of the title compound (460 mg). The crude product was used in the next step with no additional purification. ¹H-NMR (DMSO-D₆) δ: 7.34-7.29 (4H, m), 7.25-7.22 (1H, m), 3.84-3.72 (2H, m), 3.59 (2H, d, J = 1.2 Hz), 3.51-3.48 (1H, m), 3.31 (1H, s), 3.29-3.27 (1H, m), 3.23 (3H, d, J = 2.5 Hz), 3.19 (1H, dd, J = 10.1, 5.0 Hz), 2.62 (1H, ddd, J = 16.2, 8.4, 4.2 Hz), 2.57-2.54 (2H, m), 1.86-1.78 (1H, m), 1.64-1.60 (1H, m).

### (10) (S)-7-(Methoxymethyl)-1,4-oxazepane

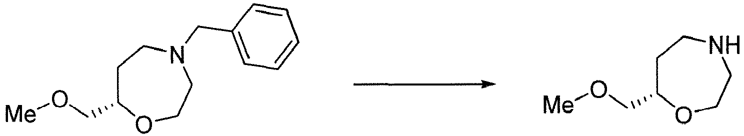

To a mixture of (S)-4-benzyl-7-(methoxymethyl)-1,4-oxazepane (460 mg) obtained in (9) in tetrahydrofuran-methanol (v/v = 1/1, 10 mL) was added palladium hydroxide (460 mg), and then the mixture was stirred under a hydrogen atmosphere at room temperature overnight. The resulted insoluble matter was filtered off through Celite, and then solvent was removed under reduced pressure to give a crude product of the title compound (270 mg). The crude product was used in the next step with no additional purification.

¹H-NMR (DMSO-D₆) δ: 3.80-3.72 (2H, m), 3.42 (2H, ddd, J = 12.5, 8.8, 2.8 Hz), 3.28 (2H, dd, J = 10.2, 6.7 Hz), 3.22-3.18 (4H, m), 2.87-2.64 (3H, m), 1.82-1.78 (1H, m), 1.57-1.48 (1H, m).

### (11) Benzyl (S)-((7-(methoxymethyl)-1,4-oxazepan-4-yl)sulfonyl)carbamate

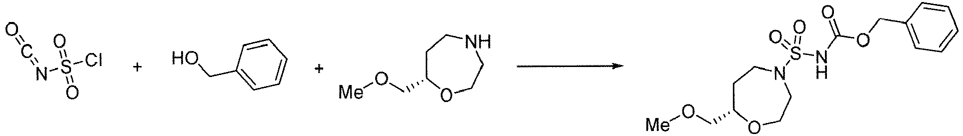

To a mixture of chlorosulfonyl isocyanate (0.11 mL) in deuterated chloroform (1.5 mL) was added benzyl alcohol (0.13 mL) under an argon atmosphere at 0°C, and then the mixture was stirred at the same temperature for 2 hours. To the reaction mixture was added a mixture of (S)-7-(methoxymethyl)-1,4-oxazepane (150 mg) obtained in (10) in deuterated chloroform (1.5 mL), and the mixture was stirred at room temperature for 1 hour. To the reaction mixture was added water, and the mixture was extracted with ethyl acetate. The resulted organic layer was dried over anhydrous magnesium sulfate, and then solvent was removed under reduced pressure to give a crude product of the title compound (370 mg). The crude product was used in the next step with no additional purification.

¹H-NMR (DMSO-D₆) δ: 11.93 (1H, s), 7.39-7.34 (5H, m), 5.14 (2H, s), 3.89 (1H, dt, J = 12.7, 3.4 Hz), 3.64 (1H, td, J = 10.0, 4.2 Hz), 3.55-3.35 (4H, m), 3.33-3.27 (3H, m), 3.24 (3H, s), 1.87-1.84 (1H, m), 1.58-1.54 (1H, m).

### (12) (S)-7-(Methoxymethyl)-1,4-oxazepane-4-sulfonamide

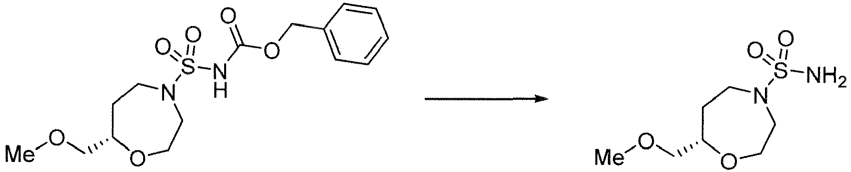

To a mixture of benzyl (S)-((7-(methoxymethyl)-1,4-oxazepan-4-yl)sulfonyl)carbamate (370 mg) obtained in (11) in ethanol (7.4 mL) was added palladium hydroxide (0.11 g) and the mixture was stirred under a hydrogen atmosphere at room temperature overnight. The resulted insoluble matter was filtered off through Celite, and washed with ethanol. The combined filtrate was concentrated under reduced pressure. The residue was purified by column chromatography (hexane/ethyl acetate= 2/3 to 0/1) to give the title compound (185 mg).

¹H-NMR (DMSO-D₆) δ: 6.72 (2H, s), 3.92-3.87 (1H, m), 3.74-3.71 (1H, m), 3.56-3.50 (1H, m), 3.41-3.29 (3H, m), 3.25-3.18 (5H, m), 3.14-3.08 (1H, m), 1.91-1.88 (1H, m), 1.66-1.57 (1H, m).

### (13) tert-Butyl (S)-(4-fluoro-2,5-dimethylphenyl)(4-(((7-(methoxymethyl)-1,4-oxazepan-4-yl)sulfonyl)carbamoyl)oxazol-2-yl)carbamate

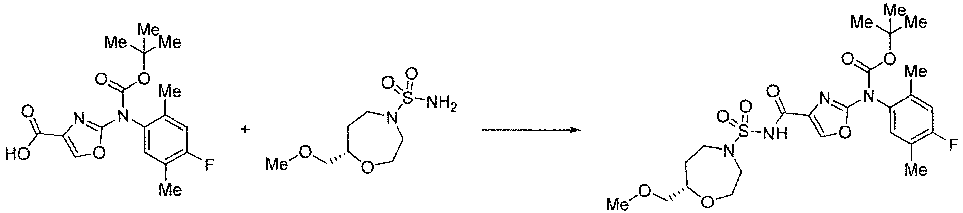

To a mixture of 2-((tert-butoxycarbonyl)(4-fluoro-2,5-dimethylphenyl)amino)oxazole-4-carboxylic acid (80 mg) obtained in (4) of [Preparation example 5] in tetrahydrofuran (1.6 mL) was added 1,1'-carbonyldiimidazole (41 mg) under an argon atmosphere, and the mixture was stirred at 60 °C for 6 hours. The reaction mixture was allowed to cool to room temperature, and then thereto were added (S)-7-(methoxymethyl)-1,4-oxazepane-4-sulfonamide (72 mg) obtained in (12) and 1,8-diazabicyclo[5.4.0]undec-7-ene (0.069 mL), and the mixture was stirred at 60 °C for 1 hour, and then solvent was removed under reduced pressure. The residue was purified by column chromatography (hexane/ethyl acetate= 4/1 to 1/4) to give a crude product of the title compound (0.13 g). The crude product was used in the next step with no additional purification.

LC-MS (MH+): 557

### (14) (S)-2-((4-Fluoro-2,5-dimethylphenyl)amino)-N-((7-(methoxymethyl)-1,4-oxazepan-4-yl)sulfonyl)oxazole-4-carboxamide

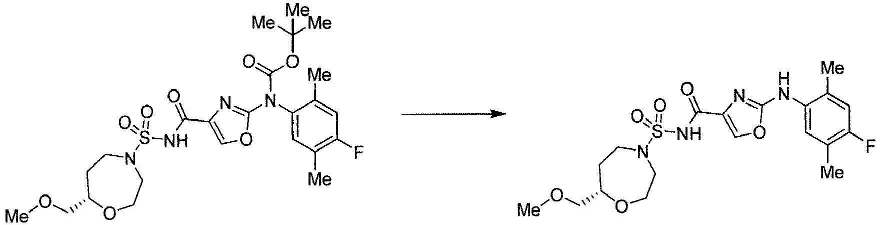

To tert-butyl (S)-(4-fluoro-2,5-dimethylphenyl)(4-(((7-(methoxymethyl)-1,4-oxazepan-4-yl)sulfonyl)carbamoyl)oxazol-2-yl)carbamate (0.13 g) obtained in (13) was added trifluoroacetic acid (1.3 mL), and the mixture was stirred at room temperature for 30 minutes, and then solvent was removed under reduced pressure. The residue was purified by column chromatography (hexane/ethyl acetate= 3/2 = 0/1) to give the title compound (25 mg).

¹H-NMR (DMSO-D₆) δ: 11.39 (1H, s), 9.30 (1H, s), 8.34 (1H, s), 7.51 (1H, d, J = 7.6 Hz), 7.02 (1H, d, J = 10.2 Hz), 3.93 (1H, dt, J = 12.9, 3.4 Hz), 3.68-3.64 (2H, m), 3.57-3.29 (5H, m), 3.25-3.22 (4H, m), 2.19 (6H, s), 1.91-1.89 (1H, m), 1.61-1.58 (1H, m).

### [Preparation example 7]: Synthesis of (R)-2-((4-fluoro-2,5-dimethylphenyl)amino)-N-((7-(methoxymethyl)-1,4-oxazepan-4-yl)sulfonyl)oxazole-4-carboxamide (Example 3-001)

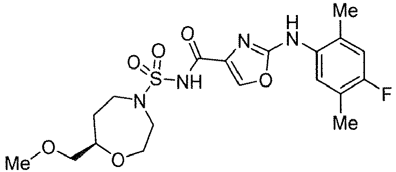

### (1) (R)-4-benzyl-7-(methoxymethyl)-1,4-oxazepan-3-one

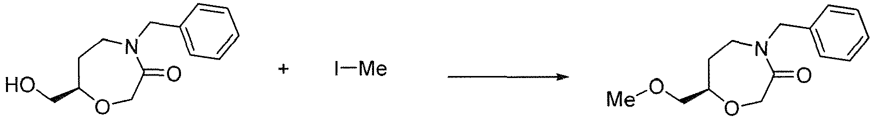

To a mixture of (R)-4-benzyl-7-(hydroxymethyl)-1,4-oxazepan-3-one (500 mg) obtained in (7) of [Preparation example 6] in tetrahydrofuran (5.0 mL) was added sodium hydride (60% in oil, 100 mg) under an argon atmosphere at 0°C, and the mixture was stirred at the same temperature for 1 hour. To the reaction mixture was added methyl iodide (0.2 mL), and then the mixture was stirred at room temperature for 1 hour. Then, thereto was added an aqueous solution of saturated ammonium chloride. The mixture was extracted with ethyl acetate, and the resulted organic layer wasdried over anhydrous magnesium sulfate, and then solvent was removed under reduced pressure. The residue was purified by column chromatography (hexane/ethyl acetate= 4/1 to 0/1) to give the title compound (520 mg).

¹H-NMR (DMSO-D₆) δ: 7.35-7.33 (2H, m), 7.27-7.25 (3H, m), 4.55 (1H, d, J = 15.0 Hz), 4.47 (1H, d, J = 14.8 Hz), 4.31 (1H, d, J = 14.8 Hz), 4.19 (1H, d, J = 14.8 Hz), 3.76-3.70 (1H, m), 3.56-3.50 (1H, m), 3.38-3.29 (2H, m), 3.26-3.23 (4H, m), 1.82-1.75 (1H, m), 1.50-1.46 (1H, m).

### (2) (R)-4-Benzyl-7-(methoxymethyl)-1,4-oxazepane

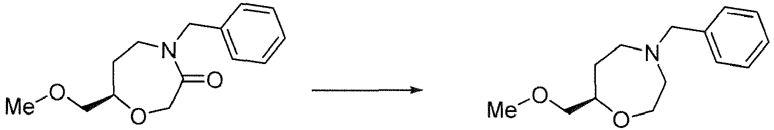

To a mixture of (R)-4-benzyl-7-(methoxymethyl)-1,4-oxazepan-3-one (520 mg)obtained in (1) in tetrahydrofuran (10 mL) was added lithium aluminium hydride (240 mg) under an argon atmosphere at 0°C, and then the mixture was stirred at 60°C for 3 hours. To the reaction mixture were added sequentially water (0.25 mL), 4 M aqueous solution of sodium hydroxide (0.25 mL), and water (0.75 mL) in a ice bath, and then the mixture was stirred at room temperature for 2 hours. The resulted solid was filtered off through Celite, and washed with tetrahydrofuran. The combined filtrate was concentrated under reduced pressure to give a crude product of the title compound (430 mg). The crude product was used in the next step with no additional purification. ¹H-NMR (DMSO-D₆) δ: 7.31-7.24 (5H, m), 3.84-3.72 (2H, m), 3.59 (2H, d, J = 1.2 Hz), 3.51-3.48 (1H, m), 3.32-3.30 (1H, m), 3.29-3.27 (1H, m), 3.23 (3H, s), 3.19 (1H, dd, J = 10.1, 5.0 Hz), 2.62 (1H, ddd, J = 16.1, 8.3, 4.1 Hz), 2.56-2.53 (2H, m), 1.86-1.78 (1H, m), 1.64-1.60 (1H, m).

### (3) (R)-7-(Methoxymethyl)-1,4-oxazepane

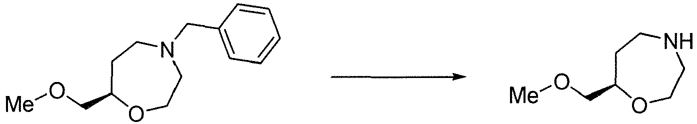

To a mixture of (R)-4-benzyl-7-(methoxymethyl)-1,4-oxazepane (430 mg) obtained in (2) in tetrahydrofuran-methanol (v/v = 1/1, 8.6 mL) was added palladium hydroxide (220 mg), and then the mixture was stirred under a hydrogen atmosphere at room temperature overnight. The resulted insoluble matter was filtered off through Celite, and then solvent was removed under reduced pressure to give a crude product of the title compound (250 mg). The crude product was used in the next step with no additional purification.

¹H-NMR (DMSO-D₆) δ: 3.78-3.74 (2H, m), 3.41 (2H, ddd, J = 12.3, 8.7, 2.8 Hz), 3.30-3.26 (2H, m), 3.23-3.17 (4H, m), 2.86-2.64 (3H, m), 1.82-1.78 (1H, m), 1.56-1.47 (1H, m).

### (4) Benzyl (R)-((7-(methoxymethyl)-1,4-oxazepan-4-yl)sulfonyl)carbamate

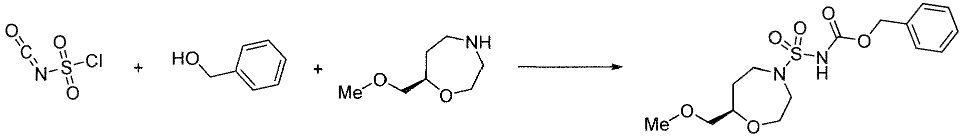

To a mixture of chlorosulfonyl isocyanate (0.11 mL) in deuterated chloroform (1.5 mL) was added benzyl alcohol (0.13 mL) under an argon atmosphere at 0°C, and then the mixture was stirred at the same temperature for 2 hours. To the reaction mixture was added a mixture of (R)-7-(methoxymethyl)-1,4-oxazepane (150 mg) obtained in (3) in deuterated chloroform (1.5 mL), and the mixture was stirred at room temperature for 1 hour. To the reaction mixture was added water. The mixture was extracted with ethyl acetate, and the resulted organic layer wasdried over anhydrous magnesium sulfate, and then solvent was removed under reduced pressure to give a crude product of the title compound (370 mg). The crude product was used in the next step with no additional purification.

¹H-NMR (DMSO-D₆) δ: 11.43 (1H, s), 7.40-7.32 (5H, m), 5.14 (2H, s), 3.89 (1H, dt, J = 12.6, 3.4 Hz), 3.64 (1H, d, J = 4.4 Hz), 3.55-3.20 (10H, m), 1.89-1.83 (1H, m), 1.57-1.55 (1H, m).

### (5) (R)-7-(Methoxymethyl)-1,4-oxazepane-4-sulfonamide

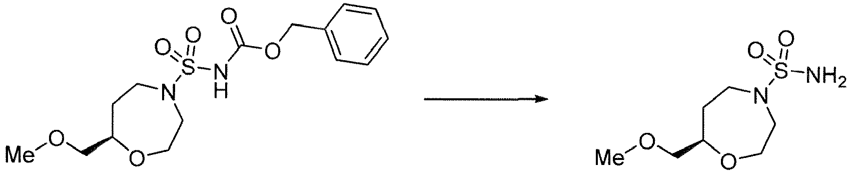

To a mixture of the crude benzyl (R)-((7-(methoxymethyl)-1,4-oxazepan-4-yl)sulfonyl)carbamate (370 mg) obtained in (4) in ethanol (7.4 mL) was added palladium hydroxide (0.11 g), and the mixture was stirred under a hydrogen atmosphere at room temperature overnight. The resulted insoluble matter was filtered off through Celite, and washed with ethanol. The combined filtrate was concentrated under reduced pressure. The residue was purified by column chromatography (hexane/ethyl acetate= 2/3 to 0/1) to give the title compound (200 mg).

¹H-NMR (DMSO-D₆) δ: 6.72 (2H, s), 3.89 (1H, dt, J = 12.9, 3.5 Hz), 3.76-3.70 (1H, m), 3.56-3.50 (1H, m), 3.42-3.29 (4H, m), 3.24-3.20 (4H, m), 3.13-3.08 (1H, m), 1.91-1.88 (1H, m), 1.64-1.59 (1H, m).

### (6) tert-Butyl (R)-(4-fluoro-2,5-dimethylphenyl)(4-(((7-(methoxymethyl)-1,4-oxazepan-4-yl)sulfonyl)carbamoyl)oxazol-2-yl)carbamate

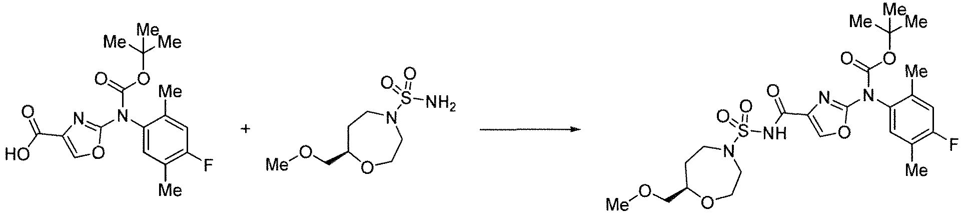

To a mixture of 2-((tert-butoxycarbonyl)(4-fluoro-2,5-dimethylphenyl)amino)oxazole-4-carboxylic acid (80 mg) obtained in (4) of [Preparation example 5] in tetrahydrofuran (1.6 mL) was added 1,1'-carbonyldiimidazole (41 mg) under an argon atmosphere, and the mixture was stirred at 60 °C for 6 hours. The reaction mixture was allowed to cool to room temperature, and then thereto were added (R)-7-(methoxymethyl)-1,4-oxazepane-4-sulfonamide (72 mg) obtained in (5) and 1,8-diazabicyclo[5.4.0]undec-7-ene (0.069 mL), and the mixture was stirred at 60°C for 1 hour, and then solvent was removed under reduced pressure. The residue was purified by column chromatography (hexane/ethyl acetate= 4/1 to 1/4) to give a crude product of the title compound (0.13 g). The crude product was used in the next step with no additional purification.

LC-MS (MH+): 557

### (7) (R)-2-((4-Fluoro-2,5-dimethylphenyl)amino)-N-((7-(methoxymethyl)-1,4-oxazepan-4-yl)sulfonyl)oxazole-4-carboxamide

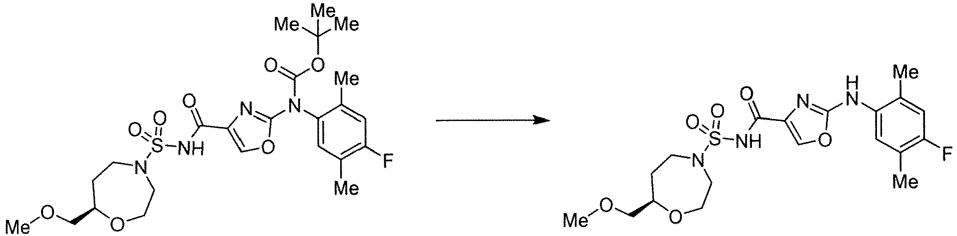

To tert-butyl (R)-(4-fluoro-2,5-dimethylphenyl)(4-(((7-(methoxymethyl)-1,4-oxazepan-4-yl)sulfonyl)carbamoyl)oxazol-2-yl)carbamate (0.13 g) obtained in (6) was added trifluoroacetic acid (1.3 mL), and the mixture was stirred at room temperature for 30 minutes, and then solvent was removed under reduced pressure. The residue was purified by column chromatography (hexane/ethyl acetate= 3/2 to 0/1) to give the title compound (39 mg) .

¹H-NMR (DMSO-D₆) δ: 11.40 (1H, s), 9.29 (1H, s), 8.33 (1H, s), 7.51 (1H, d, J = 7.6 Hz), 7.02 (1H, d, J = 10.4 Hz), 3.92 (1H, dt, J = 12.9, 3.5 Hz), 3.71-3.45 (5H, m), 3.32-3.30 (3H, m), 3.23 (3H, s), 2.19 (6H, s), 1.94-1.86 (1H, m), 1.64-1.54 (1H, m).

### [Preparation example 8]: Synthesis of N-(((6S,7R)-7-(ethoxymethyl)-6-methoxy-1,4-oxazepan-4-yl)sulfonyl)-2-((4-fluoro-2,5-dimethylphenyl)amino)oxazole-4-carboxamide (Example 3-006)

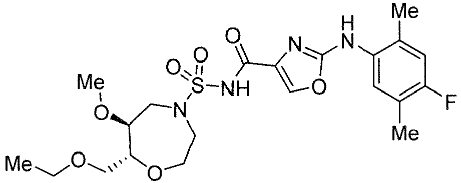

### (1) (2R,4R,5R)-5-Hydroxy-2-phenyl-1,3-dioxane-4-carbaldehyde

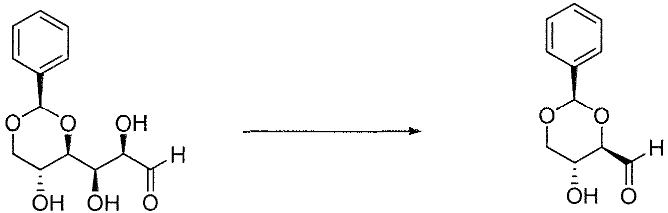

To a solution of sodium periodate (13 g) in water (160 mL) was added 8 M aqueous solution of sodium hydroxide (3.4 mL), and the reaction mixture was cooled to 0°C. To the reaction mixture was added dropwise a mixture of (2R,3R)-2,3-dihydroxy-3-((2R,4R,5R)-5-hydroxy-2-phenyl-1,3-dioxan-4-yl)propanal (8.0 g) in tetrahydrofuran (40 mL), and the mixture was stirred at room temperature for 3 hours. To the reaction mixture were added an aqueous solution of sodium thiosulfate and an aqueous solution of saturated sodium hydrogen carbonate, and then the mixture was extracted with ethyl acetate. The resulted organic layer was dried over anhydrous magnesium sulfate, and then solvent was removed under reduced pressure to give a crude product of the title compound (5.4 g). The crude product was used in the next step with no additional purification.

### (2) (2R,5R)-4-((E)-((Naphthalen-1-ylmethyl)imino)methyl)-2-phenyl-1,3-dioxan-5-ol

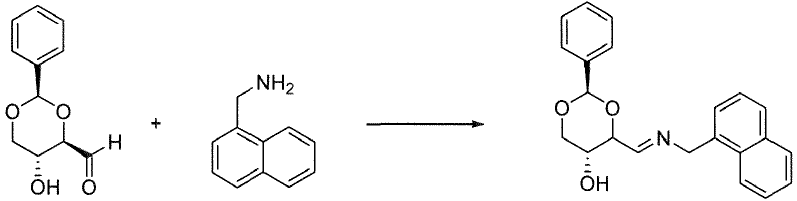

To a mixture of (2R,4R,5R)-5-hydroxy-2-phenyl-1,3-dioxane-4-carbaldehyde (5.4 g) obtained in (1) in tetrahydrofuran (54 mL) was added naphthalen-1-ylmethylamine, and the mixture was stirred at room temperature for 1 hour, and then solvent was removed under reduced pressure to give a crude product of the title compound (9.0 g). The crude product was used in the next step with no additional purification.

¹H-NMR (DMSO-D₆) δ: 8.14 (1H, d, J = 7.9 Hz), 7.94 (2H, t, J = 6.6 Hz), 7.85 (1H, dd, J = 6.2, 3.2 Hz), 7.58-7.52 (2H, m), 7.49-7.34 (7H, m), 5.60 (1H, s), 5.30 (1H, d, J = 6.0 Hz), 5.09 (2H, s), 4.18-4.15 (2H, m), 3.77-3.70 (1H, m).

### (3) (2R,4S,5R)-4-(((Naphthalen-1-ylmethyl)amino)methyl)-2-phenyl-1,3-dioxan-5-ol

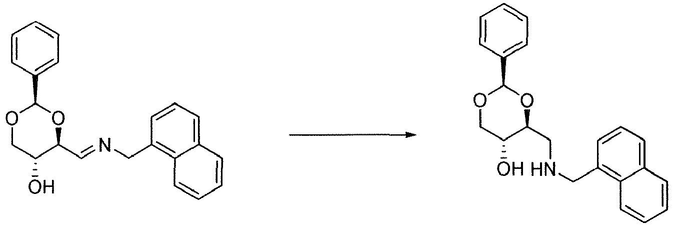

To a mixture of (2R,5R)-4-((E)-((naphthalen-1-ylmethyl)imino)methyl)-2-phenyl-1,3-dioxan-5-ol (9.0 g) obtained in (2) in methanol (90 mL) was added sodium borohydride (2.2 g) under an argon atmosphere at 0°C, and the mixture was stirred at the same temperature for 2 hours. To the reaction mixture was added water, and the mixture was extracted with ethyl acetate. The resulted organic layer was washed with saturated brine, dried over anhydrous magnesium sulfate, and then solvent was removed under reduced pressure to give a crude product of the title compound (9.0 g). The crude product was used in the next step with no additional purification.

¹H-NMR (DMSO-D₆) δ: 8.20-8.17 (1H, m), 7.91 (1H, dt, J = 6.8, 2.7 Hz), 7.81 (1H, d, J = 8.3 Hz), 7.57-7.32 (9H, m), 5.53 (1H, s), 5.26 (1H, s), 4.21 (2H, dd, J = 18.5, 13.6 Hz), 4.09 (1H, d, J = 5.3 Hz), 3.71 (1H, td, J = 7.9, 2.9 Hz), 3.53-3.48 (2H, m), 3.06 (1H, dd, J = 12.5, 3.0 Hz), 2.81 (1H, dd, J = 12.5, 7.4 Hz), 2.17 (1H, s).

### (4) (2R,4aR,9aS)-8-(Naphthalen-1-ylmethyl)-2-phenyltetrahydro-4H-[1,3]dioxino[4,5-f][1,4]oxazepin-7(6H)-one

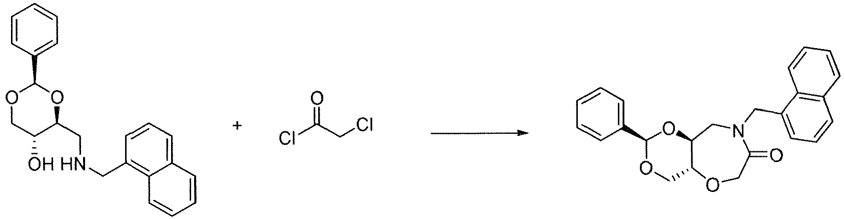

To a mixture of (2R,4S,5R)-4-(((naphthalen-1-ylmethyl)amino)methyl)-2-phenyl-1,3-dioxan-5-ol (9.0 g) obtained in (3) in tetrahydrofuran (90 mL) were added 4 M aqueous solution of sodium hydroxide (26 mL) and 2-chloroacetyl chloride (4.2 mL) in a ice bath, and the mixture was stirred at room temperature for 1 hour. To the reaction mixture were added 4 M aqueous solution of sodium hydroxide (25 mL) and 2-chloroacetyl chloride (2.0 mL), and the mixture was stirred at room temperature overnight. To the reaction mixture was added 4 M aqueous solution of sodium hydroxide (25 mL), and the mixture was stirred for 3 hours. Then thereto was further added 4 M aqueous solution of sodium hydroxide (25 mL), and the mixture was stirred for 3 hours. To the reaction mixture was added an aqueous solution of saturated ammonium chloride, and then the mixture was extracted with ethyl acetate. The resulted organic layer was dried over anhydrous magnesium sulfate, and then solvent was removed under reduced pressure to give a crude product of the title compound (9.5 g). The crude product was used in the next step with no additional purification.

¹H-NMR (DMSO-D₆) δ: 8.09 (1H, t, J = 4.9 Hz), 7.97 (1H, td, J = 4.9, 1.9 Hz), 7.90 (1H, t, J = 4.7 Hz), 7.58-7.47 (4H, m), 7.33 (5H, dd, J = 9.7, 5.8 Hz), 5.44 (1H, s), 5.17 (1H, d, J = 15.0 Hz), 4.98 (1H, d, J = 15.0 Hz), 4.62 (1H, d, J = 14.1 Hz), 4.24 (1H, d, J = 14.1 Hz), 4.16 (1H, dd, J = 8.7, 3.1 Hz), 3.91 (1H, dd, J = 14.6, 9.9 Hz), 3.60-3.57 (2H, m), 3.48 (1H, t, J = 8.2 Hz), 3.29 (1H, s).

### (5) (2R,4aR,9aS)-8-(Naphthalen-1-ylmethyl)-2-phenylhexahydro-4H-[1,3]dioxino[4,5-f][1,4]oxazepine

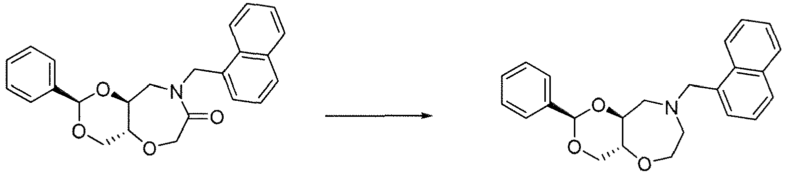

To a mixture of (2R,4aR,9aS)-8-(naphthalen-1-ylmethyl)-2-phenyltetrahydro-4H-[1,3]dioxino[4,5-f][1,4]oxazepin-7 (6H)-one (4.5 g) obtained in (4) in tetrahydrofuran (90 mL) was added in three portions lithium aluminium hydride (1.8 g) under an argon atmosphere at 0°C, and then the mixture was stirred at room temperature for 2 days. To the reaction mixture were added water (1.7 mL), 4 M aqueous solution of sodium hydroxide (1.7 mL), water (5.1 mL), and then the mixture was stirred for 3 hours. The resulted solid was filtered off through Celite, and washed with tetrahydrofuran. The combined filtrate was concentrated under reduced pressure. The residue was purified by column chromatography (hexane/ethyl acetate= 24/1 to 71/29) to give the title compound (3.4 g).

¹H-NMR (DMSO-D₆) δ: 8.30 (1H, d, J = 8.3 Hz), 7.93-7.83 (2H, m), 7.57-7.43 (4H, m), 7.36-7.32 (5H, m), 5.50 (1H, s), 4.18-4.13 (3H, m), 3.88-3.82 (2H, m), 3.73-3.53 (3H, m), 3.13 (1H, dd, J = 12.8, 5.4 Hz), 2.96-2.89 (1H, m), 2.80 (1H, dt, J = 9.5, 4.8 Hz), 2.69 (1H, dd, J = 12.9, 8.3 Hz).

### (6) ((6S,7R)-6-(Benzyloxy)-4-(naphthalen-1-ylmethyl)-1,4-oxazepan-7-yl)methanol

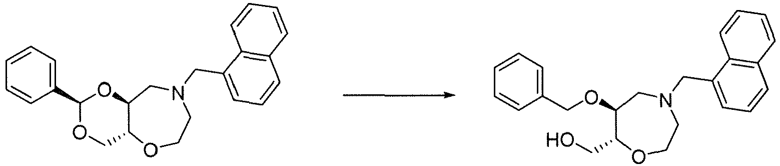

To a mixture of (2R,4aR,9aS)-8-(naphthalen-1-ylmethyl)-2-phenylhexahydro-4H-[1,3]dioxino[4,5-f][1,4]oxazepine (3.4 g) obtained in (5) in toluene (34 mL) was added diisobutylaluminium hydride (1.0 M in toluene, 27 mL) under an argon atmosphere at 0°C, and the mixture was stirred at room temperature for 2 hours. To the residue was added an aqueous solution of potassium sodium (+)-tartrate, and the mixture was stirred for 1 hour. Then, the mixture was extracted with ethyl acetate. The resulted organic layer was dried over anhydrous magnesium sulfate, and then solvent was removed under reduced pressure. The residue was purified by column chromatography (hexane/ethyl acetate=9/1 to 1/1) to give the title compound (2.67 g).

¹H-NMR (DMSO-D₆) δ: 8.39 (1H, d, J = 8.3 Hz), 7.90 (1H, d, J = 8.1 Hz), 7.83 (1H, d, J = 8.3 Hz), 7.49 (2H, dd, J = 11.9, 6.8 Hz), 7.43-7.38 (2H, m), 7.28-7.23 (3H, m), 7.03 (2H, t, J = 3.8 Hz), 4.59 (1H, t, J = 5.7 Hz), 4.19 (1H, d, J = 11.6 Hz), 4.06 (3H, d, J = 9.5 Hz), 3.85 (1H, dt, J = 12.8, 3.1 Hz), 3.43-3.34 (5H, m), 3.17 (1H, d, J = 14.6 Hz), 2.78 (1H, d, J = 12.9 Hz), 2.67 (1H, dd, J = 13.9, 2.3 Hz), 2.56-2.52 (1H, m).

### (7) (6S,7R)-6-(Benzyloxy)-7-(ethoxymethyl)-4-(naphthalen-1-ylmethyl)-1,4-oxazepane

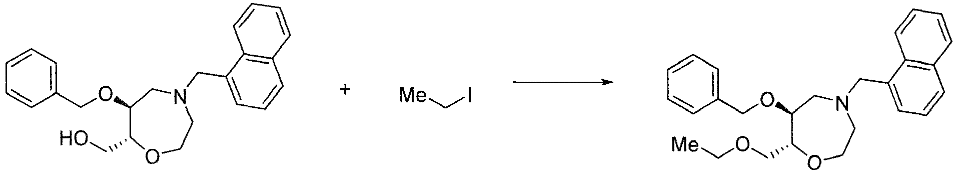

To a mixture of ((6S,7R)-6-(benzyloxy)-4-(naphthalen-1-ylmethyl)-1,4-oxazepan-7-yl)methanol (600 mg) obtained in (6) in tetrahydrofuran/N,N-dimethylformamide (v/v = 1/1, 12 mL) was added sodium hydride (60% in oil, 100 mg) under an argon atmosphere at 0°C, and the mixture was stirred at the same temperature for 1 hour, and then thereto was added ethyl iodide (0.21 mL), and the mixture was stirred at room temperature for 3 hours. To the reaction mixture was added an aqueous solution of saturated ammonium chloride, and then the mixture was extracted with ethyl acetate. The resulted organic layer was dried over anhydrous magnesium sulfate, and then solvent was removed under reduced pressure. The residue was purified by column chromatography (hexane/ethyl acetate) to give the title compound (570 mg).

¹H-NMR (DMSO-D₆) δ: 8.38 (1H, d, J = 8.3 Hz), 7.90 (1H, d, J = 8.3 Hz), 7.83 (1H, d, J = 8.1 Hz), 7.46 (4H, tt, J = 19.7, 7.1 Hz), 7.25 (3H, dd, J = 11.7, 5.0 Hz), 7.03 (2H, t, J = 3.8 Hz), 4.21 (1H, d, J = 11.6 Hz), 4.05 (3H, t, J = 5.7 Hz), 3.84 (1H, dt, J = 12.6, 2.9 Hz), 3.53-3.49 (1H, m), 3.47-3.43 (1H, m), 3.40-3.29 (5H, m), 3.15 (1H, dd, J = 14.1, 3.0 Hz), 2.76 (1H, d, J = 12.7 Hz), 2.69 (1H, dd, J = 14.2, 2.4 Hz), 2.57-2.53 (1H, m), 1.05 (3H, t, J = 6.9 Hz).

### (8) (6S,7R)-7-(Ethoxymethyl)-1,4-oxazepan-6-ol hydrochloride

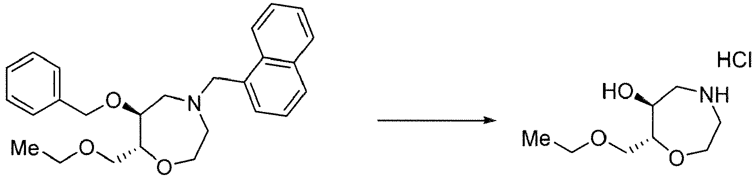

To a mixture of (6S,7R)-6-(benzyloxy)-7-(ethoxymethyl)-4-(naphthalen-1-ylmethyl)-1,4-oxazepane (570 mg) obtained in (7) in tetrahydrofuran/methanol (v/v = 1/1, 12 mL) were added acetic acid (0.40 mL) and palladium hydroxide (280 mg), and the mixture was stirred under a hydrogen atmosphere at room temperature overnight. The resulted insoluble matter was filtered off through Celite, and washed with methanol. The combined filtrate was concentrated under reduced pressure. To a mixture of the residue in tetrahydrofuran/methanol (v/v = 1/1, 8.0 mL) were added 2 M hydrochloric acid (1.4 mL) and palladium hydroxide (190 mg), and the mixture was stirred under a hydrogen atmosphere at room temperature overnight. The resulted insoluble matter was filtered off through Celite, and washed with methanol. The combined filtrate was concentrated under reduced pressure to give a crude product of the title compound (296 mg). The crude product was used in the next step with no additional purification.

¹H-NMR (DMSO-D₆) δ: 9.37 (1H, s), 8.65 (1H, s), 4.00-3.92 (2H, m), 3.72 (1H, t, J = 11.0 Hz), 3.57 (1H, q, J = 5.0 Hz), 3.46 (1H, d, J = 6.9 Hz), 3.43-3.41 (2H, m), 3.19-3.14 (4H, m), 2.69 (1H, t, J = 6.2 Hz), 2.56 (1H, t, J = 6.2 Hz), 1.11 (3H, t, J = 6.9 Hz) .

### (9) Benzyl (6S,7R)-7-(ethoxymethyl)-6-hydroxy-1,4-oxazepane-4-carboxylate

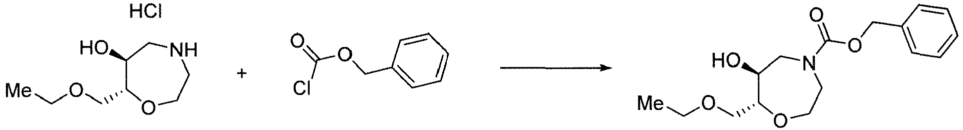

To a mixture of (6S,7R)-7-(ethoxymethyl)-1,4-oxazepan-6-ol hydrochloride (296 mg) obtained in (8) in tetrahydrofuran (4.9 mL) were added triethylamine (0.98 mL) and benzyl chloroformate (0.36 mL) under an argon atmosphere at 0°C, and the mixture was stirred at room temperature for 2 hours. To the reaction mixture was added an aqueous solution of saturated sodium hydrogen carbonate, and then the mixture was extracted with ethyl acetate. The resulted organic layer was dried over anhydrous magnesium sulfate, and then solvent was removed under reduced pressure. The residue was purified by column chromatography (hexane/ethyl acetate= 3/7 to 1/99) to give the title compound (360 mg).

¹H-NMR (DMSO-D₆) δ: 7.36-7.31 (5H, m), 5.11-5.09 (3H, m), 3.96-3.94 (1H, m), 3.71 (1H, dd, J = 35.0, 12.8 Hz), 3.54-3.51 (3H, m), 3.43-3.37 (5H, m), 3.28-3.24 (2H, m), 1.09 (3H, t, J = 7.1 Hz) .

### (10) Benzyl (6S,7R)-7-(ethoxymethyl)-6-methoxy-1,4-oxazepane-4-carboxylate

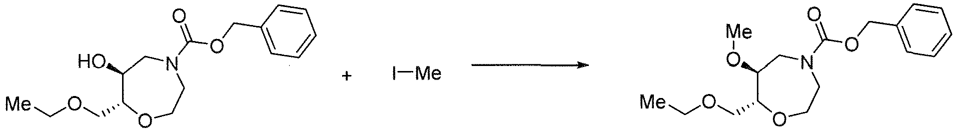

To a mixture of benzyl (6S,7R)-7-(ethoxymethyl)-6-hydroxy-1,4-oxazepane-4-carboxylate (360 mg) obtained in (9) in tetrahydrofuran/N,N-dimethylformamide (v/v = 1/1, 7 mL) was added sodium hydride (60% in oil, 70 mg) under an argon atmosphere at 0°C, and the mixture was stirred for 40 minutes, and then thereto was added dropwise methyl iodide (0.15 mL), and the mixture was stirred at the same temperature for 1 hour. To the reaction mixture was added an aqueous solution of saturated sodium hydrogen carbonate, and then the mixture was extracted with ethyl acetate. The resulted organic layer was dried over anhydrous magnesium sulfate, and then solvent was removed under reduced pressure. The residue was purified by column chromatography (hexane/ethyl acetate= 4/1 to 1/1) to give the title compound (210 mg).

¹H-NMR (DMSO-D₆) δ: 7.37-7.32 (5H, m), 5.09-5.06 (2H, m), 3.96-3.94 (1H, m), 3.88-3.60 (2H, m), 3.49-3.41 (6H, m), 3.33-3.23 (5H, m), 3.13 (1H, s), 1.09 (3H, t, J = 6.9 Hz).

### (11) (6S,7R)-7-(Ethoxymethyl)-6-methoxy-1,4-oxazepane

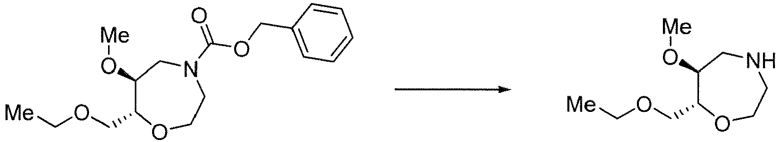

To benzyl (6S,7R)-7-(ethoxymethyl)-6-methoxy-1,4-oxazepane-4-carboxylate (210 mg) obtained in (10) in tetrahydrofuran-methanol (v/v = 1/1, 4.0 mL) was added palladium hydroxide (64 mg), and the mixture was stirred under a hydrogen atmosphere at room temperature for 2.5 hours. The resulted insoluble matter was filtered off through Celite, and washed with methanol. The combined filtrate was concentrated under reduced pressure to give a crude product of the title compound (126 mg). The crude product was used in the next step with no additional purification. ¹H-NMR (DMSO-D₆) δ: 3.85 (1H, ddd, J = 12.2, 3.1, 2.1 Hz), 3.44-3.34 (7H, m), 3.21 (3H, s), 3.15-3.12 (2H, m), 2.82 (1H, d, J = 13.4 Hz), 2.66-2.56 (2H, m), 1.10 (3H, t, J = 7.1 Hz).

### (12) Benzyl(((6S,7R)-7-(ethoxymethyl)-6-methoxy-1,4-oxazepan-4-yl)sulfonyl)carbamate

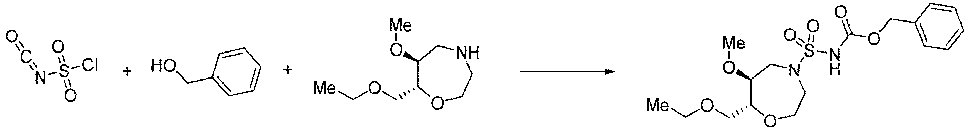

To a mixture of chlorosulfonyl isocyanate (0.069 mL) in deuterated chloroform (1.9 mL) was added benzyl alcohol (0.083 mL) under an argon atmosphere at 0°C, and the mixture was stirred at the same temperature for 3 hours. To the reaction mixture were added (6S,7R)-7-(ethoxymethyl)-6-methoxy-1,4-oxazepane (126 mg) obtained in (11) and a mixture of triethylamine (0.22 mL) in deuterated chloroform (1.0 mL), and the mixture was stirred at room temperature for 1 hour. To the reaction mixture were added acetic acid (0.19 mL) and water, and then the mixture was extracted with ethyl acetate. The resulted organic layer was dried over anhydrous magnesium sulfate, and then solvent was removed under reduced pressure to give a crude product of the title compound (267 mg). The crude product was used in the next step with no additional purification.

LC-MS (MH+): 403

### (13) (6S,7R)-7-(Ethoxymethyl)-6-methoxy-1,4-oxazepane-4-sulfonamide

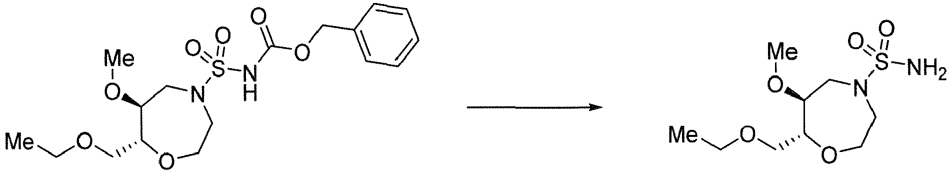

To a mixture of benzyl(((6S,7R)-7-(ethoxymethyl)-6-methoxy-1,4-oxazepan-4-yl)sulfonyl)carbamate (267 mg) obtained in (12) in ethanol (8.0 mL) was added palladium hydroxide (80 mg), and the mixture was stirred under a hydrogen atmosphere at room temperature overnight. The resulted insoluble matter was filtered off through Celite, and washed with ethanol. The combined filtrate was concentrated under reduced pressure to give a crude product of the title compound (178 mg).The crude product was used in the next step with no additional purification.

### (14) tert-Butyl (4-((((6S,7R)-7-(ethoxymethyl)-6-methoxy-1,4-oxazepan-4-yl)sulfonyl)carbamoyl)oxazol-2-yl)(4-fluoro-2,5-dimethylphenyl)carbamate

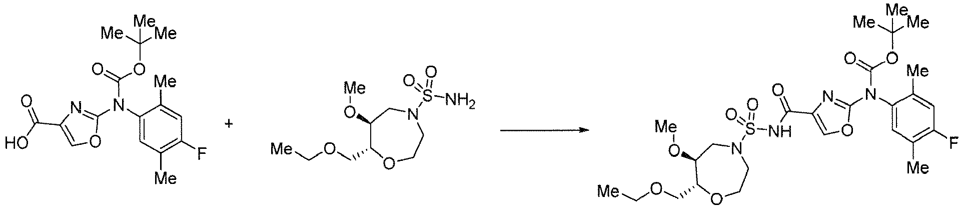

To a mixture of 2-((tert-butoxycarbonyl)(4-fluoro-2,5-dimethylphenyl)amino)oxazole-4-carboxylic acid (80 mg) obtained in (4) of [Preparation example 5] in tetrahydrofuran (1.0 mL) was added 1,1'-carbonyldiimidazole (43 mg) under an argon atmosphere, and the mixture was stirred at 60 °C for 4 hours. The reaction mixture was allowed to cool to room temperature, and then thereto were added (6S,7R)-7-(ethoxymethyl)-6-methoxy-1,4-oxazepane-4-sulfonamide (80 mg) obtained in (13) and a mixture of 1,8-diazabicyclo[5.4.0]undec-7-ene (0.086 mL) in tetrahydrofuran (0.6 mL), and the mixture was stirred at 60°C for 2 hours. The reaction mixture was allowed to cool to room temperature, and then solvent was removed under reduced pressure. The residue was purified by column chromatography (hexane/ethyl acetate=4/1 to 1/4) to give a crude product of the title compound (137 mg). The crude product was used in the next step with no additional purification.

LC-MS (MH+): 601

### (15) N-(((6S,7R)-7-(Ethoxymethyl)-6-methoxy-1,4-oxazepan-4-yl)sulfonyl)-2-((4-fluoro-2,5-dimethylphenyl)amino)oxazole-4-carboxamide

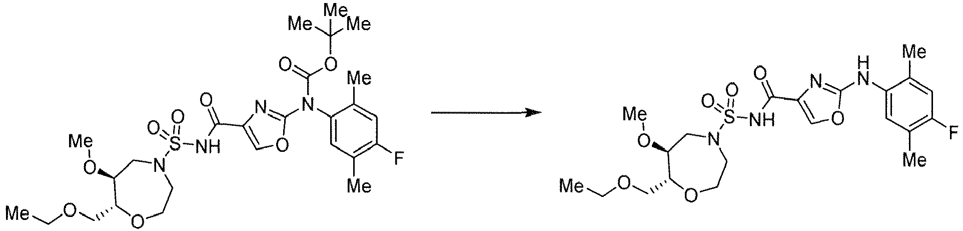

To tert-butyl (4-((((6S,7R)-7-(ethoxymethyl)-6-methoxy-1,4-oxazepan-4-yl)sulfonyl)carbamoyl)oxazol-2-yl)(4-fluoro-2,5-dimethylphenyl)carbamate (137 mg) obtained in (14) was added trifluoroacetic acid (1.4 mL), the mixture was stirred at room temperature for 30 minutes, and then solvent was removed under reduced pressure. The residue was purified by column chromatography (hexane/ethyl acetate= 4/1 to 1/4) to give the title compound (30 mg).

¹H-NMR (DMSO-D₆) δ: 11.37 (1H, s), 9.31 (1H, s), 8.34 (1H, s), 7.50 (1H, d, J = 7.2 Hz), 7.02 (1H, d, J = 10.2 Hz), 4.00 (2H, d, J = 11.8 Hz), 3.60-3.55 (2H, m), 3.45-3.36 (5H, m), 3.24-3.22 (5H, m), 3.15-3.08 (1H, m), 2.20 (6H, s), 1.09 (3H, t, J = 6.9 Hz).

### [Reference preparation example 1]: Synthesis of 5-methyl-2,3-dihydro-1H-inden-4-amine

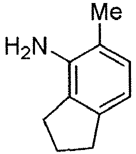

### (1) N-(2,3-Dihydro-1H-inden-4-yl)pivalamide

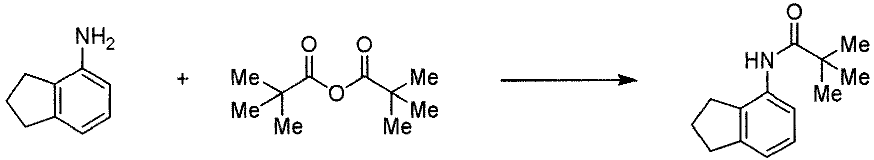

To a solution of 2,3-dihydro-1H-inden-4-amine (5.0 g) in dichloromethane (50 mL) were added triethylamine (7.9 mL), 2,2-dimethylpropionic anhydride (23 mL), and 4-dimethylaminopyridine (1.4 g), and the mixture was stirred at room temperature for 2 hours. To the reaction mixture was added water, and then the mixture was extracted with ethyl acetate. The resulted organic layer was dried over anhydrous sodium sulfate, and then solvent was removed under reduced pressure. The residue was purified by column chromatography (hexane/ethyl acetate) to give the title compound (4.1 g).

¹H-NMR (DMSO-D₆) δ: 8.82 (1H, s), 7.09-7.01 (3H, m), 2.86 (2H, t, J = 7.5 Hz), 2.71 (2H, t, J = 7.4 Hz), 1.99-1.91 (2H, m), 1.20 (9H, s).

### (2) N-(5-Bromo-2,3-dihydro-1H-inden-4-yl)pivalamide

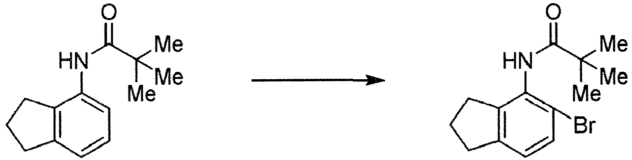

To a solution of N-(2,3-dihydro-1H-inden-4-yl)pivalamide (4.1 g) obtained in (1) in toluene (30 mL) were added palladium (II) acetate (0.21 g), p-toluenesulfonic acid (1.6 g), and N-bromosuccinimide (4.1 g), and the mixture was stirred at room temperature overnight. To the reaction mixture was added water, and then the mixture was extracted with ethyl acetate. The resulted organic layer was dried over anhydrous sodium sulfate, and then solvent was removed under reduced pressure. The residue was purified by column chromatography (hexane/ethyl acetate) to give the title compound (5.1 g).

MS (M+H): 296, 298.

### (3) 5-Bromo-2,3-dihydro-1H-inden-4-amine

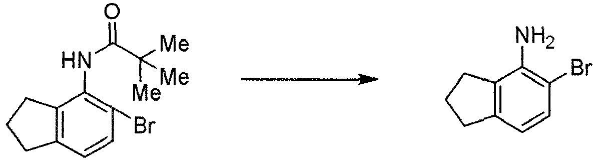

To N-(5-bromo-2,3-dihydro-1H-inden-4-yl)pivalamide (5.0 g) obtained in (2) was added 35% hydrochloric acid (50 mL), and the mixture was stirred at 150°C for 1 hour in a microwave reactor. The reaction mixture was cooled, and then thereto was added a 4 M aqueous solution of sodium hydroxide (142 mL). Then, the mixture was extracted with ethyl acetate. The resulted organic layer was dried over anhydrous sodium sulfate, and then solvent was removed under reduced pressure. The residue was purified by column chromatography (hexane/ethyl acetate) to give the title compound (2.9 g).

¹H-NMR (CDCl₃) δ: 7.19 (1H, d, J = 7.9 Hz), 6.53 (1H, d, J = 7.9 Hz), 3.95 (2H, br s), 2.85 (2H, t, J = 7.5 Hz), 2.73 (2H, t, J = 7.3 Hz), 2.15-2.07 (2H, m).

### (4) 5-Methyl-2,3-dihydro-1H-inden-4-amine

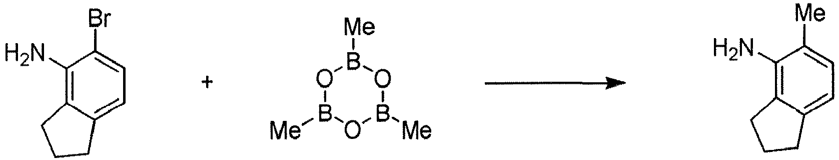

To a solution of 5-bromo-2,3-dihydro-1H-inden-4-amine (500 mg) obtained in (3) in 1,2-dimethoxyethane (10 mL) were added 2,4,6-trimethylboroxine (590 mg), [1,1'-bis(diphenylphosphino)ferrocene]palladium (II) dichloromethane complex (90 mg), and a 2 M aqueous solution of tripotassium phosphate (3.5 mL), and the mixture was stirred at 90°C for 5 hours. To the reaction mixture was added water, and then the mixture was extracted with ethyl acetate. The resulted organic layer was dried over anhydrous sodium sulfate, and then solvent was removed under reduced pressure. The residue was purified by column chromatography (hexane/ethyl acetate) to give the title compound (200 mg).

¹H-NMR (DMSO-D₆) δ: 6.69 (1H, d, J = 7.2 Hz), 6.35 (1H, d, J = 7.4 Hz), 4.47 (2H, br s), 2.73 (2H, t, J = 7.5 Hz), 2.61 (2H, t, J = 7.4 Hz), 2.01 (3H, s), 1.96-1.93 (2H, m).

### [Reference preparation example 2]: Synthesis of N,N-dimethyl-1-sulfamoylpiperidine-4-carboxamide

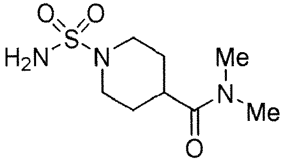

### (1) tert-Butyl-1-(N-(tert-butoxycarbonyl)sulfamoyl)piperidine-4-carboxylate

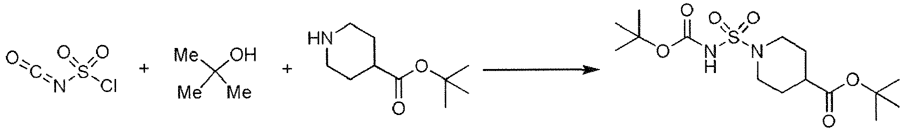

tert-Butylpiperidine-4-carboxylate (2.6 g) was used instead of 3-methylpyrrolidin-3-ol in a similar manner to [Preparation example 3] (6) to give the title compound (3.7 g).

¹H-NMR (CDCl₃) δ: 6.93 (1H, br s), 3.75 (2H, dt, J = 12.9, 3.9 Hz), 3.06-3.00 (2H, m), 2.33 (1H, tt, J = 10.4, 4.0 Hz), 1.95-1.91 (2H, m), 1.82-1.72 (2H, m), 1.47 (9H, s), 1.43 (9H, s).

### (2) 1-Sulfamoylpiperidine-4-carboxylic acid

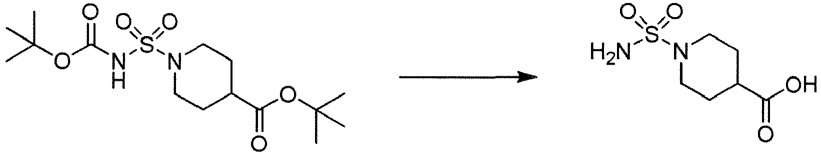

To tert-butyl-1-(N-(tert-butoxycarbonyl)sulfamoyl)piperidine-4-carboxylate (3.7 g) obtained in (1) was added trifluoroacetic acid (18 mL), and the mixture was stirred at room temperature for 1 hour. Solvent was removed under reduced pressure to give the title compound (1.5 g).

¹H-NMR (DMSO-D₆) δ: 12.25 (1H, br s), 6.70 (2H, s), 3.40-3.35 (2H, m), 2.68-2.57 (2H, m), 2.36-2.27 (1H, m), 1.89-1.85 (2H, m), 1.62-1.50 (2H, m).

### (3) N,N-Dimethyl-1-sulfamoylpiperidine-4-carboxamide

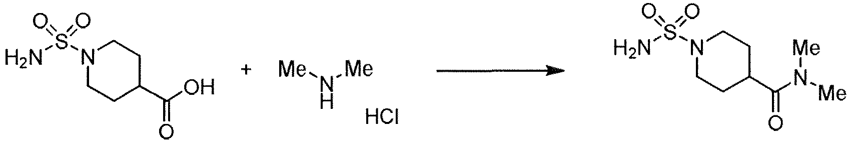

To a solution of 1-sulfamoylpiperidine-4-carboxylic acid (300 mg) obtained in (2) in N,N-dimethylformamide (3.0 mL) were added dimethylamine hydrochloride (240 mg), 1-ethyl-3-(3-dimethylaminopropyl)carbodiimide hydrochloride (330 mg), and triethylamine (0.40 mL), and the mixture was stirred at room temperature overnight. The reaction mixture was purified by column chromatography (ethyl acetate), and then thereto was added a mixed solution of ethyl acetate/hexane (v/v/ = 1/1). Then, the mixture was stirred. The resulted solid was filtered to give the title compound (110 mg).

¹H-NMR (DMSO-D₆) δ: 6.71 (2H, s), 3.46-3.43 (2H, m), 3.00 (3H, s), 2.79 (3H, s), 2.65 (1H, tt, J = 11.2, 3.8 Hz), 2.55 (2H, td, J = 11.9, 2.6 Hz), 1.71-1.68 (2H, m), 1.54 (2H, ddd, J = 24.6, 12.3, 3.8 Hz).

### [Reference preparation example 3]: Synthesis of N⁴,N⁴-dimethylpiperidine-1,4-disulfonamide

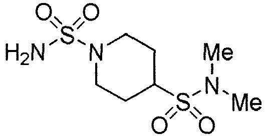

### (1) Benzyl-4-(N,N-dimethylsulfamoyl)piperidine-1-carboxylate

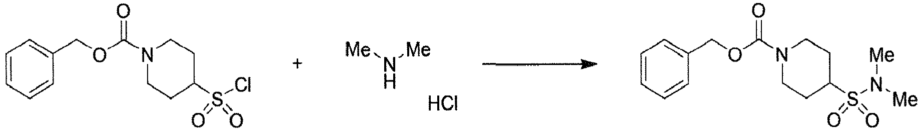

To a solution of benzyl-4-(chlorosulfonyl)piperidine-1-carboxylate (3.3 g) in chloroform (10 mL) were added sequentially triethylamine (2.1 mL) and dimethylamine hydrochloride (1.0 g) at 0°C under an argon atmosphere, and the mixture was stirred at room temperature for 3 days. The reaction mixture was purified by column chromatography (hexane/ethyl acetate) to give the title compound (2.6 g).

¹H-NMR (CDCl₃) δ: 7.37-7.26 (5H, m), 5.12 (2H, s), 4.31-4.30 (2H, m), 3.10-3.08 (1H, m), 2.91 (6H, s), 2.80-2.77 (2H, m), 2.03-2.01 (2H, m), 1.80-1.70 (2H, m).

### (2) N,N-Dimethylpiperidine-4-sulfonamide

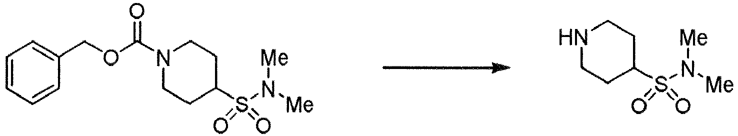

To a solution of benzyl 4-(N,N-dimethylsulfamoyl)piperidine-1-carboxylate (2.6 g) obtained in (1) in ethanol (1.0 L) was added 10% palladium carbon (500 mg), and the mixture was stirred at room temperature for 20 hours under a hydrogen atmosphere. The insoluble matter was filtered off through Celite, and the resulted filtrate was concentrated under reduced pressure to give the title compound (1.4 g).

¹H-NMR (CDCl₃) δ: 5.07 (1H, s), 3.22-3.18 (2H, m), 3.11-3.03 (1H, m), 2.92 (6H, s), 2.59 (2H, td, J = 12.5, 2.5 Hz), 2.02-2.00 (2H, m), 1.74-1.67 (2H, m).

### (3) N⁴,N⁴-Dimethylpiperidine-1,4-disulfonamide

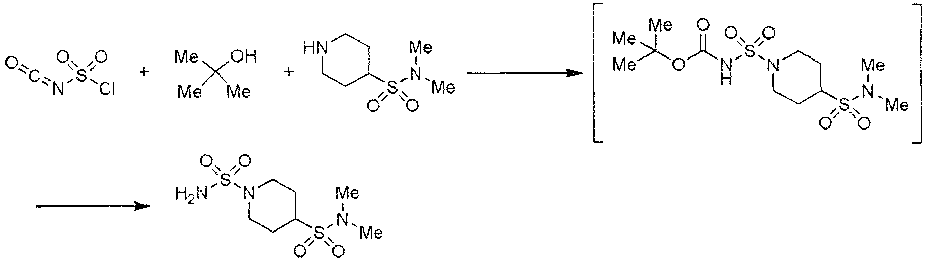

N,N-Dimethylpiperidine-4-sulfonamide (1.0 g) obtained in (2) was used instead of 3-methylpyrrolidin-3-ol in a similar manner to [Preparation example 3] (6) and (7) to give the title compound (1.0 g).

¹H-NMR (DMSO-D₆) δ: 6.81 (2H, s), 3.55-3.52 (2H, m), 3.41-3.30 (1H, m), 2.82 (6H, s), 2.56 (2H, td, J = 12.2, 2.5 Hz), 2.01-1.99 (2H, m), 1.63 (2H, ddd, J = 25.0, 12.5, 4.2 Hz).

Other example compounds were obtained in a similar manner to the above Preparation methods and Preparation examples, or if necessary by known methods. The structures and physical property data of the compounds of Examples 1 to 77, Examples 2-001 to 2-353 and Examples 3-001 to 3-007 are shown in the following tables.

**[Table 1]**

| Example | Structure | Note |
|---|---|---|
| 1 | 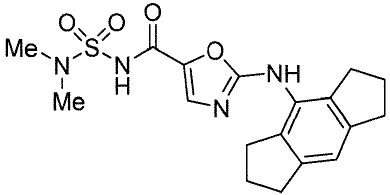 | |
| 2 | 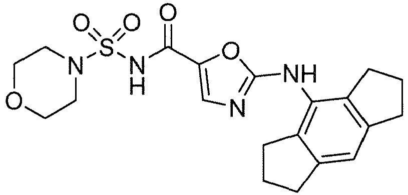 | |
| 3 | 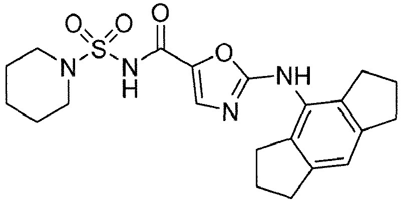 | |
| 4 | 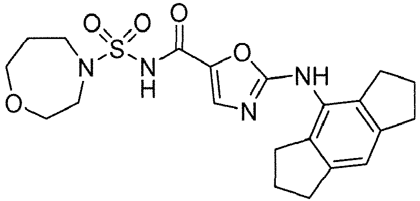 | |
| 5 | 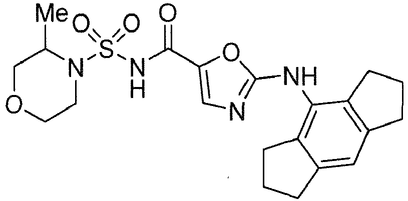 | Racemate |
| 6 | 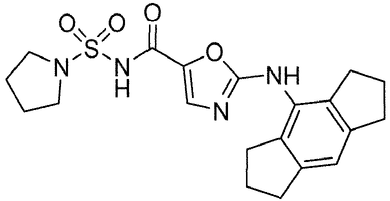 | |
| 7 | 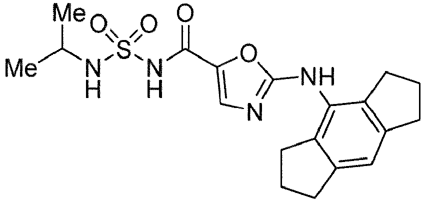 | |
| 8 | 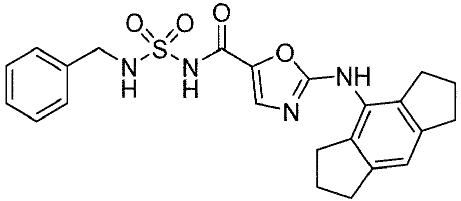 | |
| 9 | 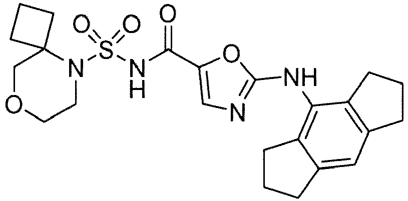 | |
| 10 | 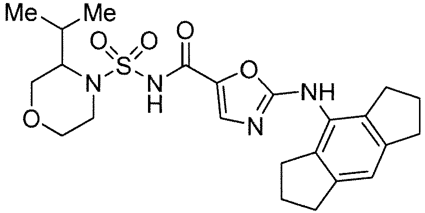 | Racemate |
| 11 | 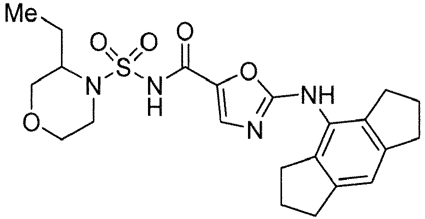 | Racemate |
| 12 | 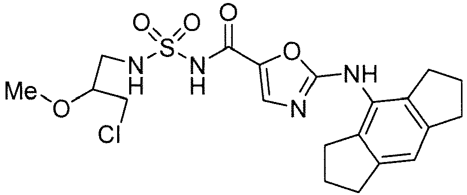 | Racemate |
| 13 | 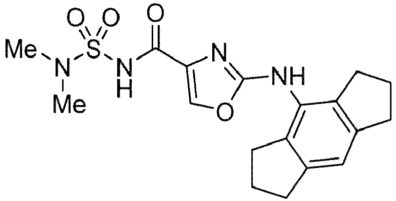 | |
| 14 | 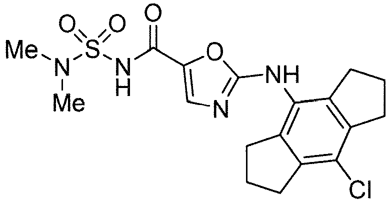 | |
| 15 | 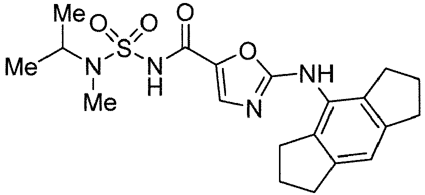 | |
| 16 | 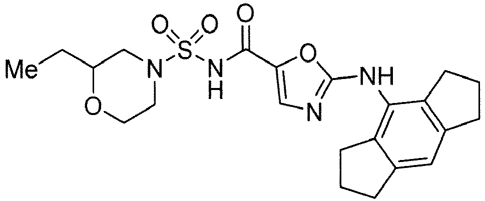 | Racemate |
| 17 | 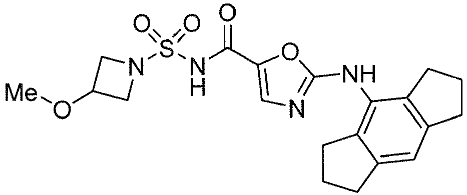 | |
| 18 | 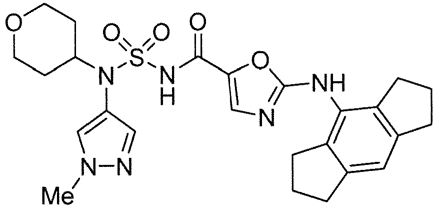 | |
| 19 | 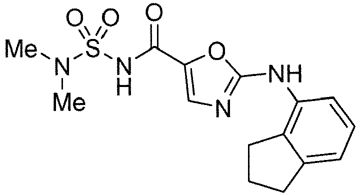 | |
| 20 | 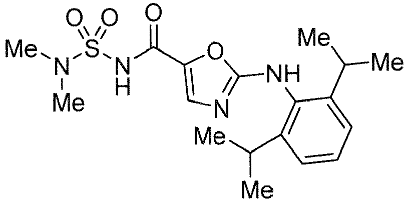 | |
| 21 | 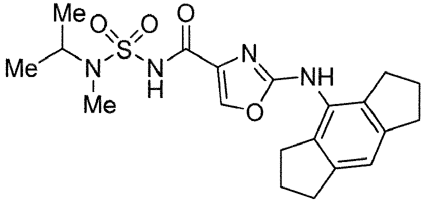 | |
| 22 | 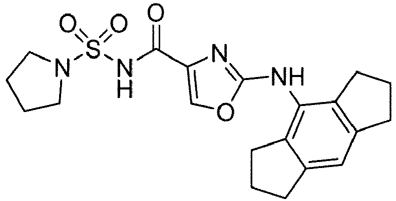 | |
| 23 | 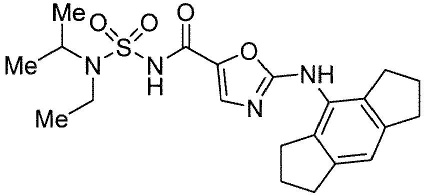 | |
| 24 | 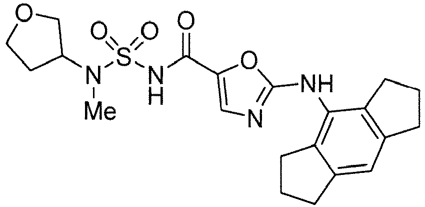 | Racemate |
| 25 | 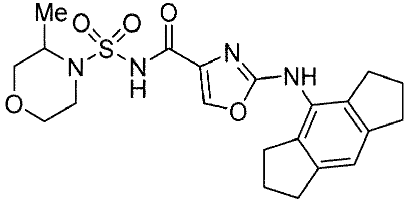 | Racemate |
| 26 | 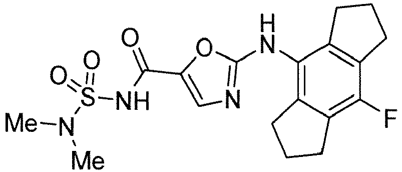 | |
| 27 | 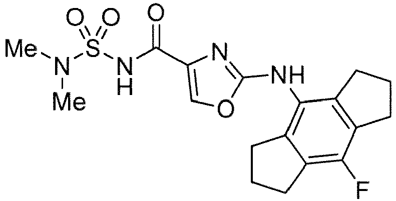 | |
| 28 | 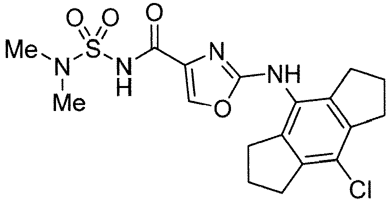 | |
| 29 | 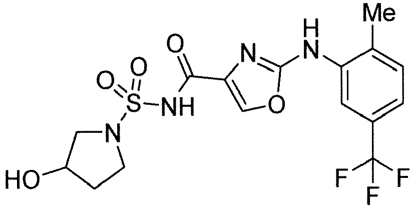 | Racemate |
| 30 | 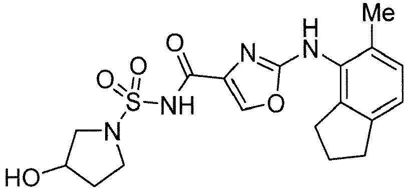 | Racemate |
| 31 | 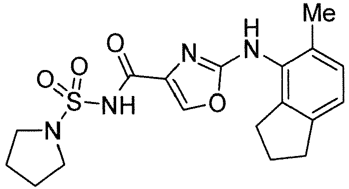 | |
| 32 | 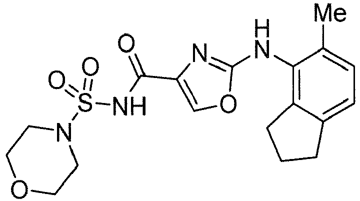 | |
| 33 | 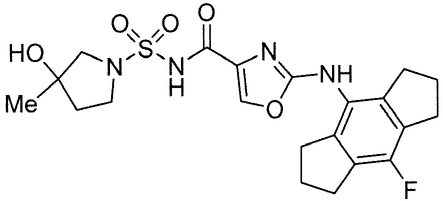 | Racemate |
| 34 | 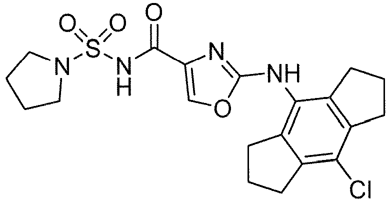 | |
| 35 | 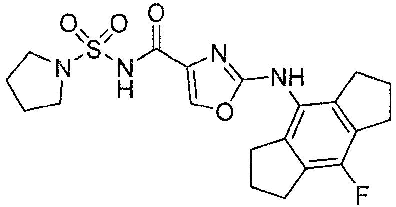 | |
| 36 | 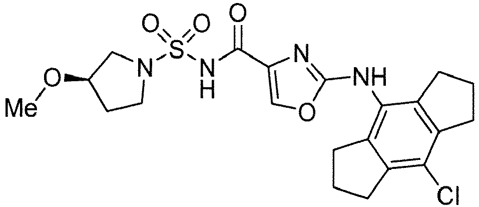 | |
| 37 | 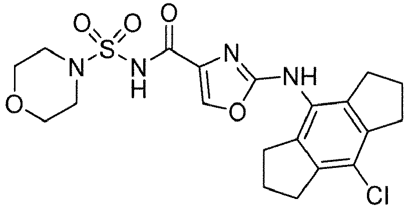 | |
| 38 | 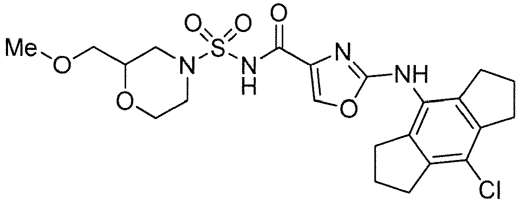 | Racemate |
| 39 | 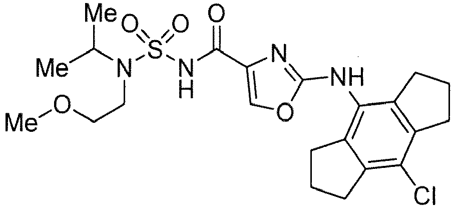 | |
| 40 | 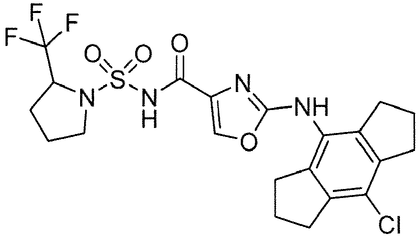 | Racemate |
| 41 | 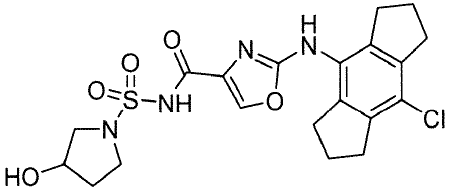 | Racemate |
| 42 | 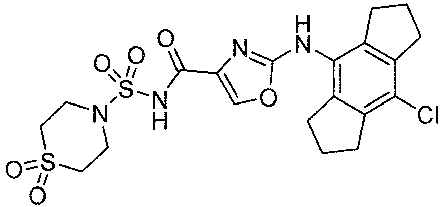 | |
| 43 | 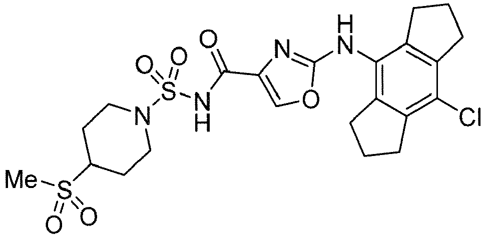 | |
| 44 | 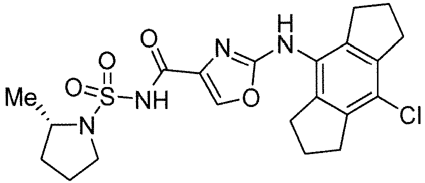 | |
| 45 | 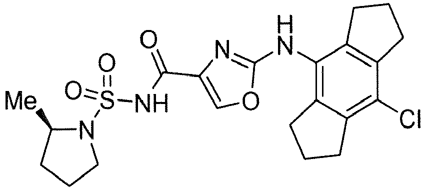 | |
| 46 | 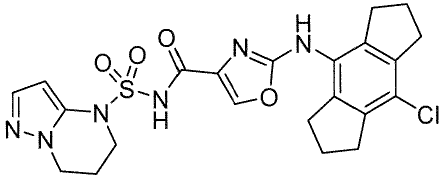 | |
| 47 | 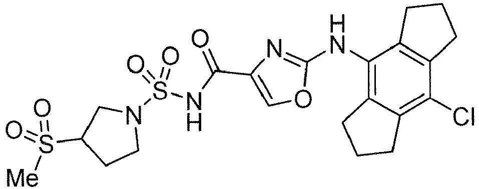 | Racemate |
| 48 | 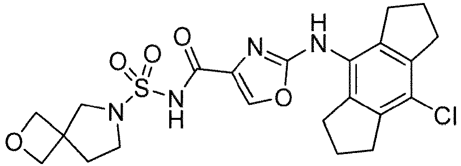 | |
| 49 | 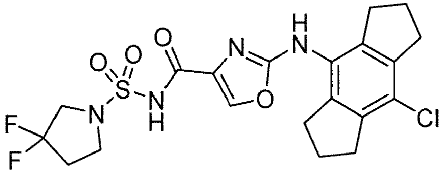 | |
| 50 | 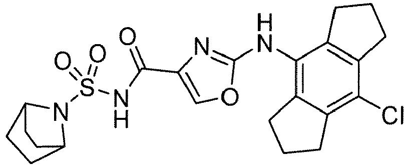 | |
| 51 | 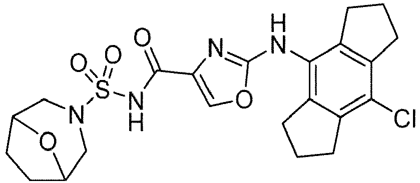 | |
| 52 | 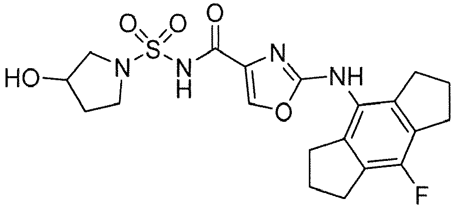 | Racemate |
| 53 | 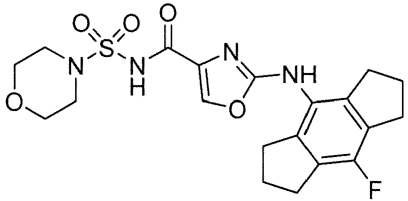 | |
| 54 | 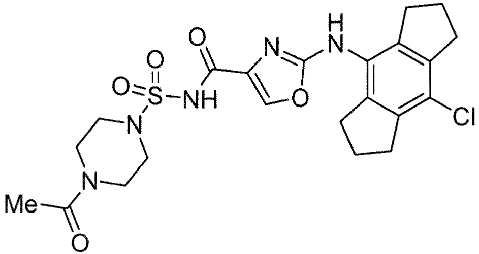 | |
| 55 | 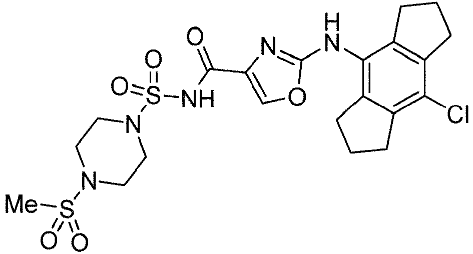 | |
| 56 | 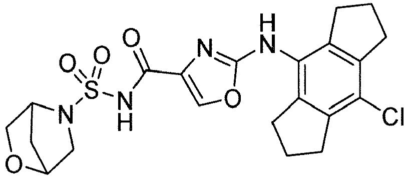 | Racemate |
| 57 | 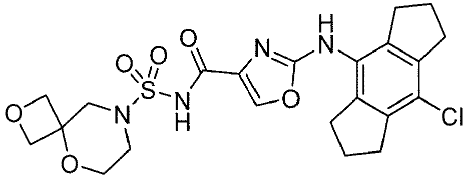 | |
| 58 | 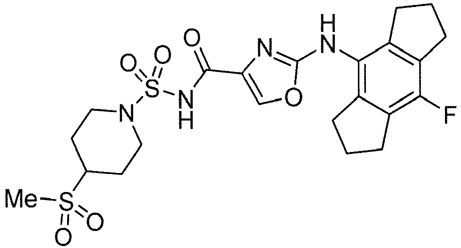 | |
| 59 | 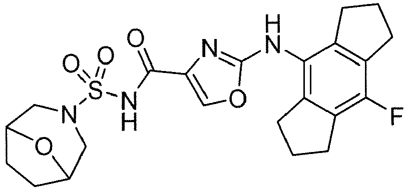 | |
| 60 | 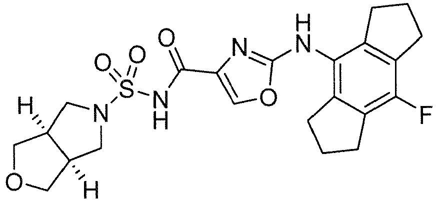 | |
| 61 | 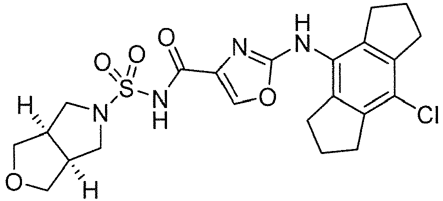 | |
| 62 | 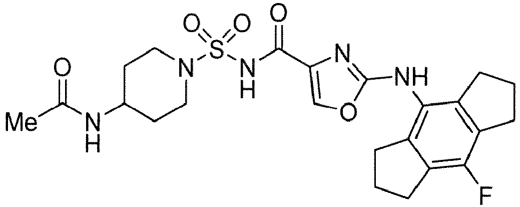 | |
| 63 | 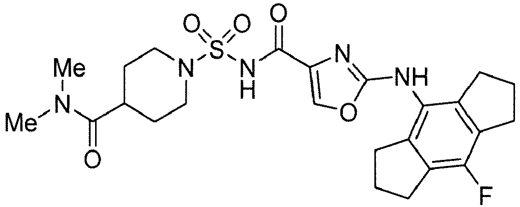 | |
| 64 | 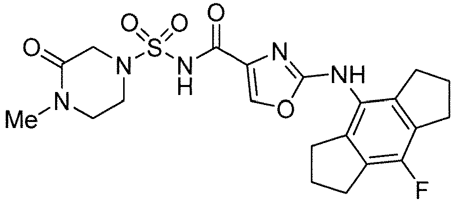 | |
| 65 | 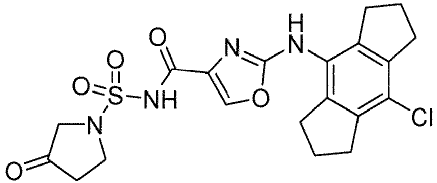 | |
| 66 | 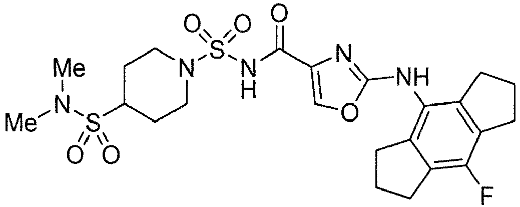 | |
| 67 | 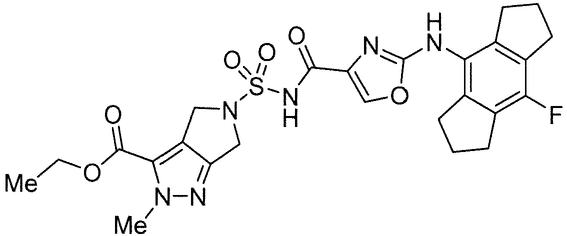 | |
| 68 | 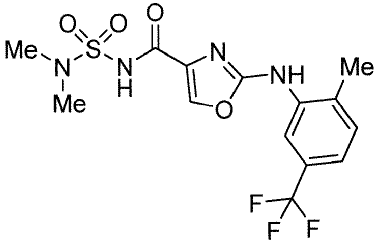 | |
| 69 | 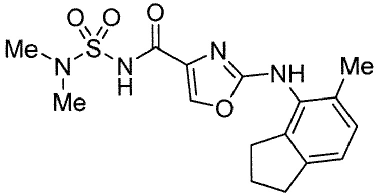 | |
| 70 | 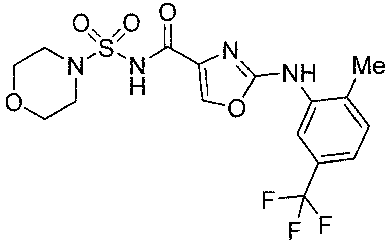 | |
| 71 | 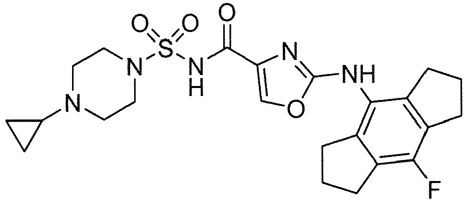 | |
| 72 | 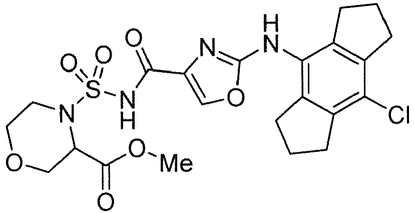 | Racemate |
| 73 | 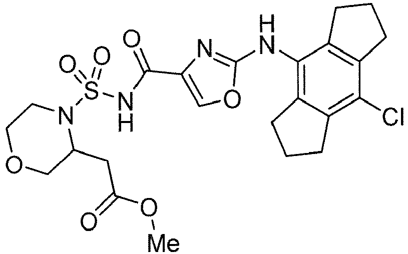 | Racemate |
| 74 | 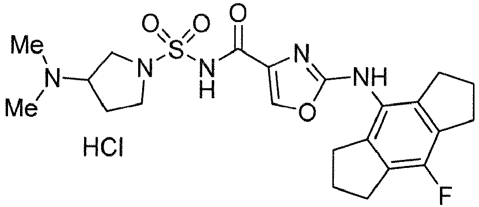 | Racemate |
| 75 | 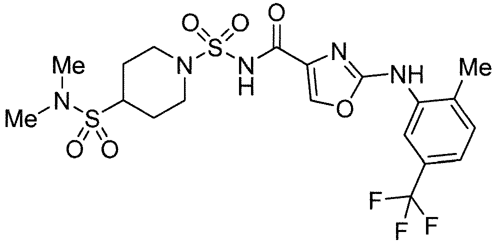 | |
| 76 | 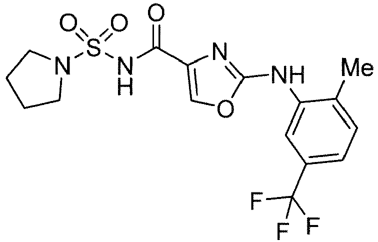 | |
| 77 | 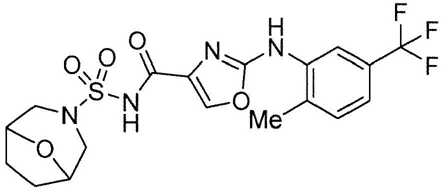 | |

**[Table 2]**

| Example | Structure | Note |
|---|---|---|
| 2-001 | 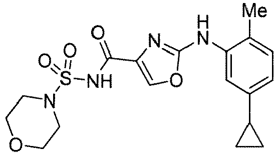 | |
| 2-002 | 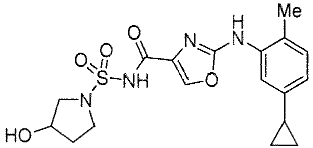 | Racemate |
| 2-003 | 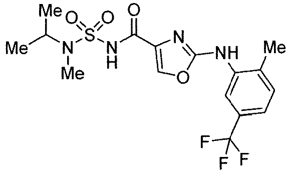 | |
| 2-004 | 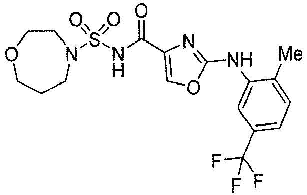 | |
| 2-005 | 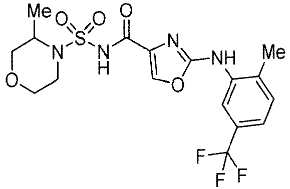 | Racemate |
| 2-006 | 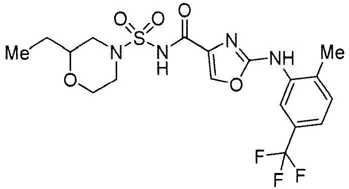 | Racemate |
| 2-007 | 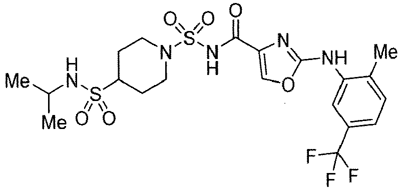 | |
| 2-008 | 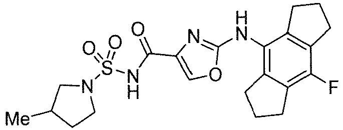 | Racemate |
| 2-009 | 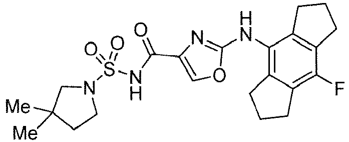 | |
| 2-010 | 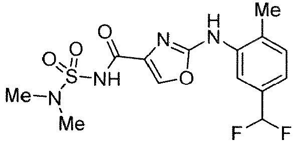 | |
| 2-011 | 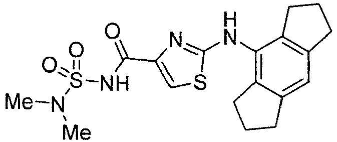 | |
| 2-012 | 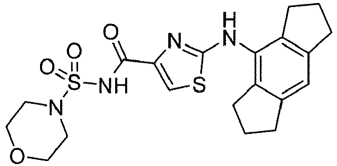 | |
| 2-013 | 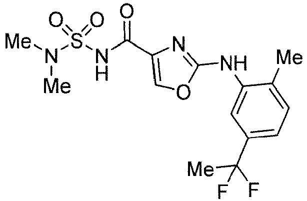 | |
| 2-014 | 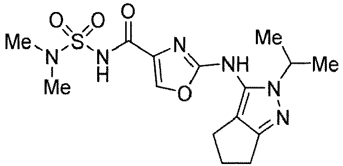 | |
| 2-015 | 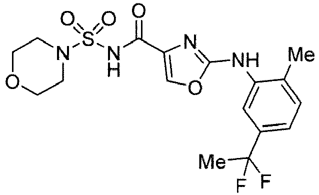 | |
| 2-016 | 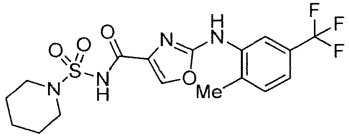 | |
| 2-017 | 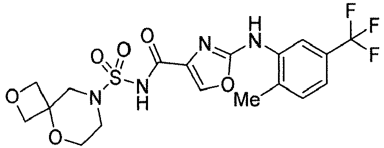 | |
| 2-018 | 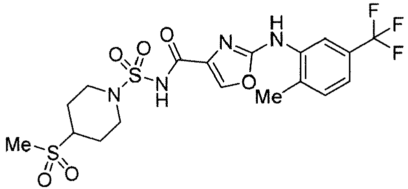 | |
| 2-019 | 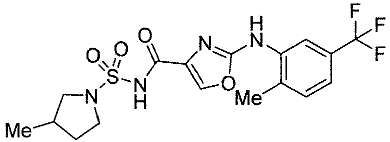 | Racemate |
| 2-020 | 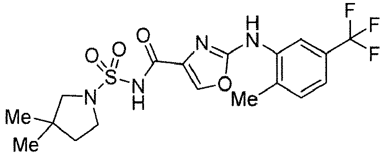 | |
| 2-021 | 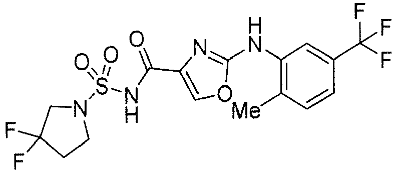 | |
| 2-022 | 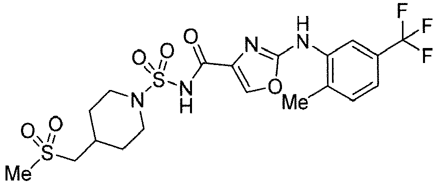 | |
| 2-023 | 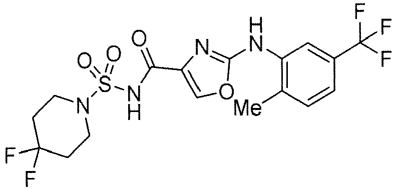 | |
| 2-024 | 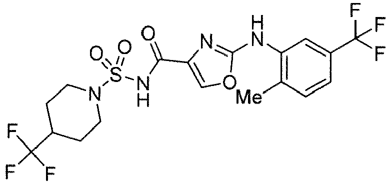 | |
| 2-025 | 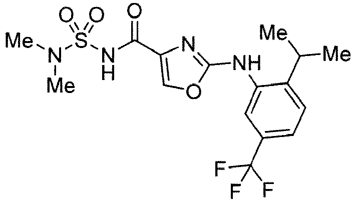 | |
| 2-026 | 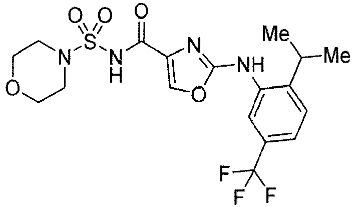 | |
| 2-027 | 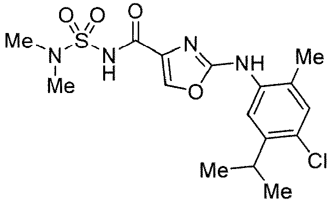 | |
| 2-028 | 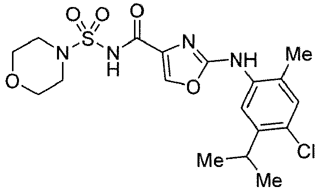 | |
| 2-029 | 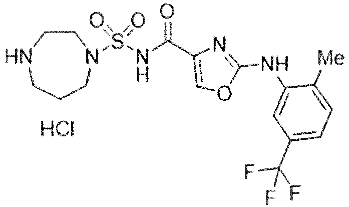 | |
| 2-030 | 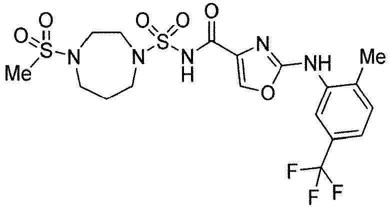 | |
| 2-031 | 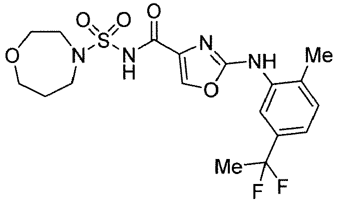 | |
| 2-032 | 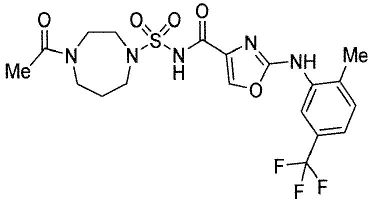 | |
| 2-033 | 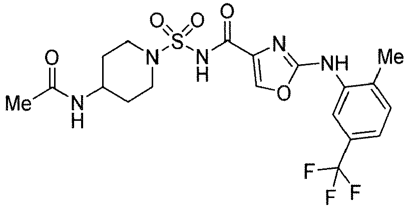 | |
| 2-034 | 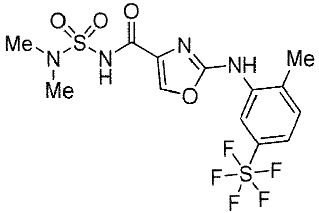 | |
| 2-035 | 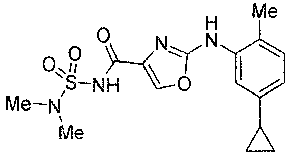 | |
| 2-036 | 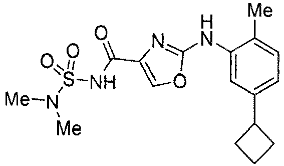 | |
| 2-037 | 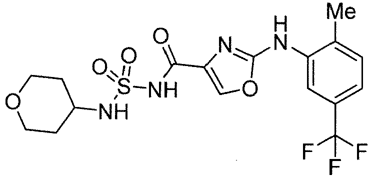 | |
| 2-038 | 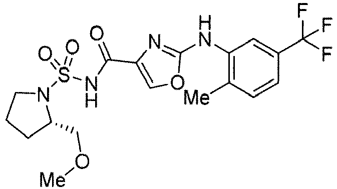 | |
| 2-039 | 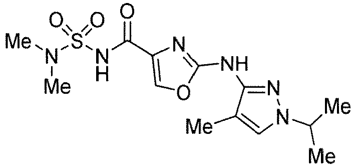 | |
| 2-040 | 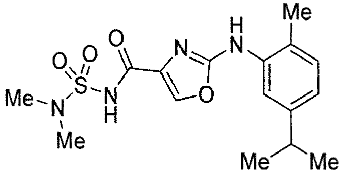 | |
| 2-041 | 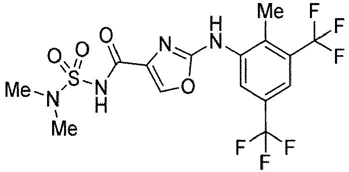 | |
| 2-042 | 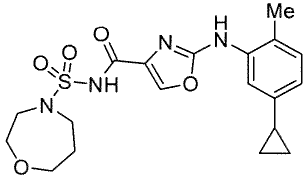 | |
| 2-043 | 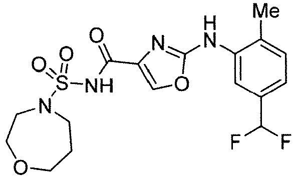 | |
| 2-044 | 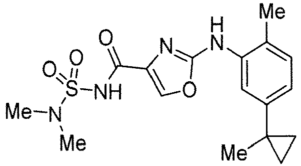 | |
| 2-045 | 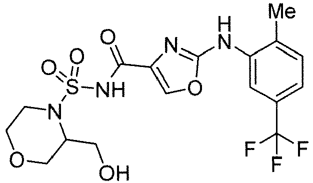 | Racemate |
| 2-046 | 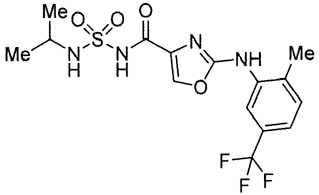 | |
| 2-047 | 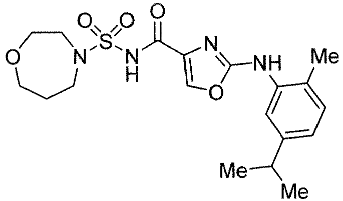 | |
| 2-048 | 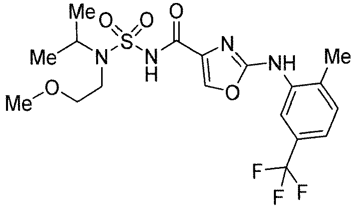 | |
| 2-049 | 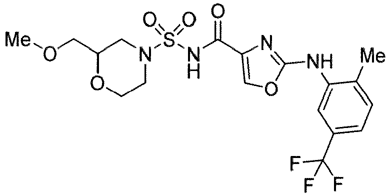 | Racemate |
| 2-050 | 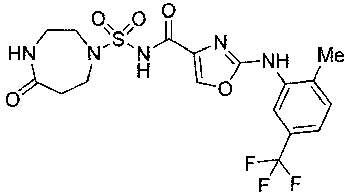 | |
| 2-051 | 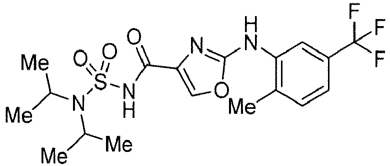 | |
| 2-052 | 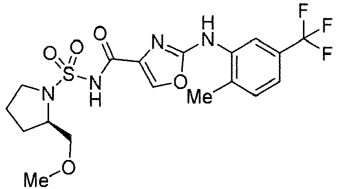 | Optically-active compound |
| 2-053 | 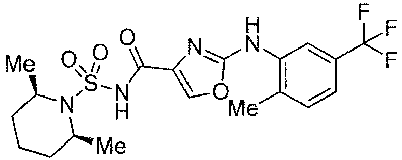 | Cis-isomer |
| 2-054 | 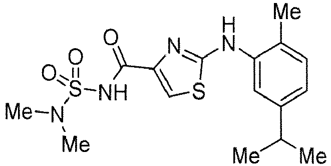 | |
| 2-055 | 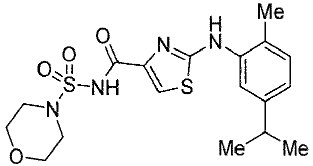 | |
| 2-056 | 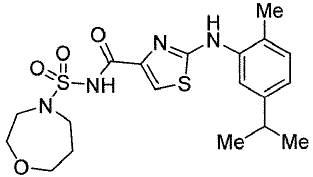 | |
| 2-057 | 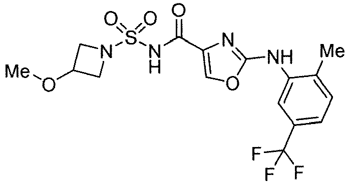 | |
| 2-058 | 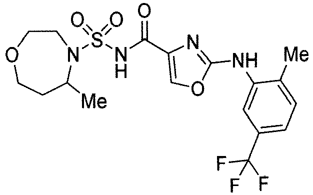 | Racemate |
| 2-059 | 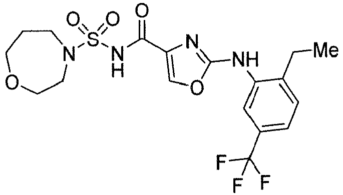 | |
| 2-060 | 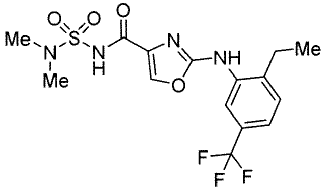 | |
| 2-061 | 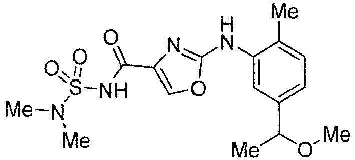 | Racemate |
| 2-062 | 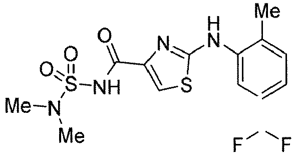 | |
| 2-063 | 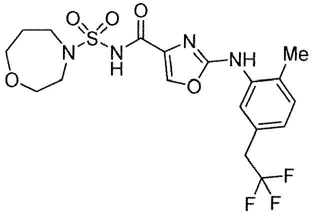 | |
| 2-064 | 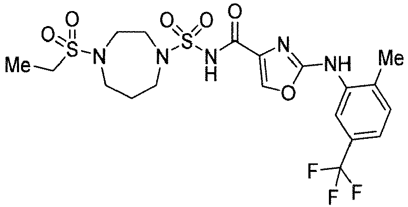 | |
| 2-065 | 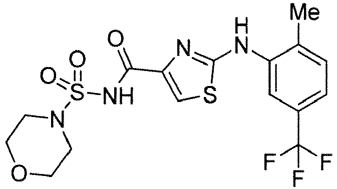 | |
| 2-066 | 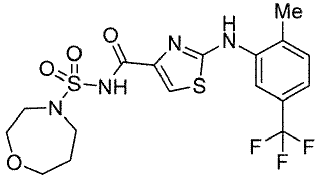 | |
| 2-067 | 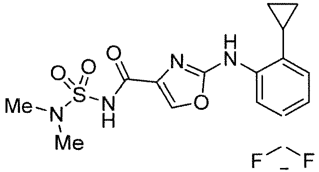 | |
| 2-068 | 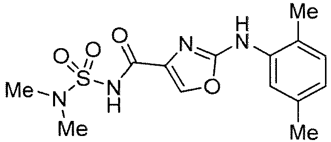 | |
| 2-069 | 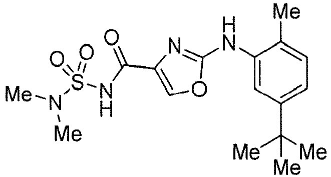 | |
| 2-070 | 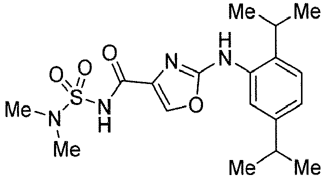 | |
| 2-071 | 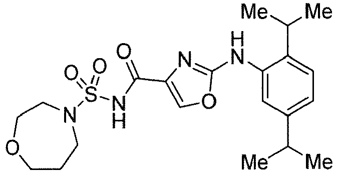 | |
| 2-072 | 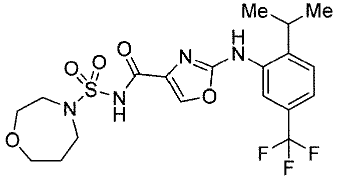 | |
| 2-073 | 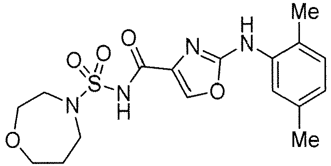 | |
| 2-074 | 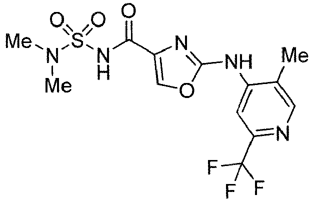 | |
| 2-075 | 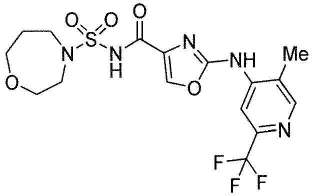 | |
| 2-076 | 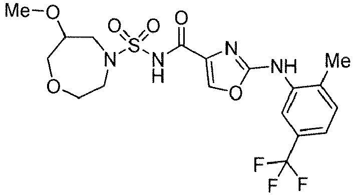 | Racemate |
| 2-077 | 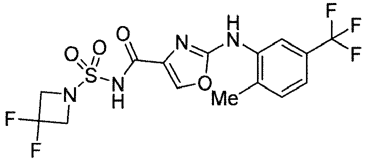 | |
| 2-078 | 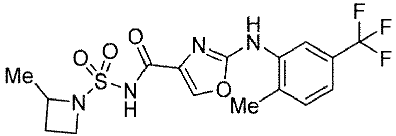 | Racemate |
| 2-079 | 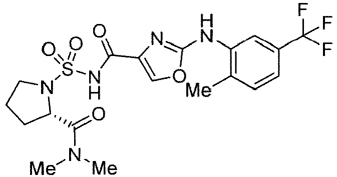 | |
| 2-080 | 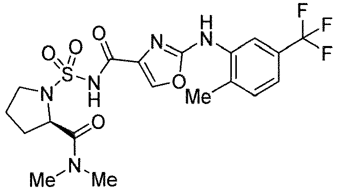 | |
| 2-081 | 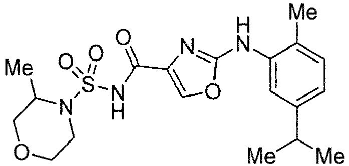 | Racemate |
| 2-082 | 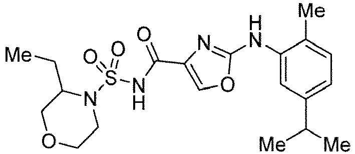 | Racemate |
| 2-083 | 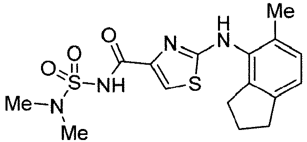 | |
| 2-084 | 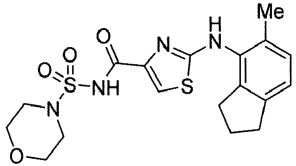 | |
| 2-085 | 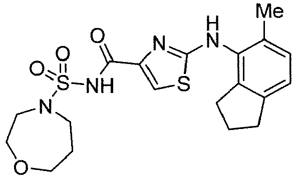 | |
| 2-086 | 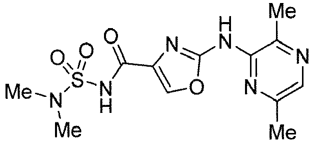 | |
| 2-087 | 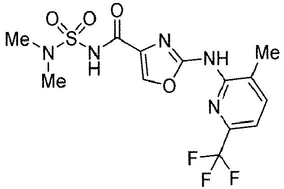 | |
| 2-088 | 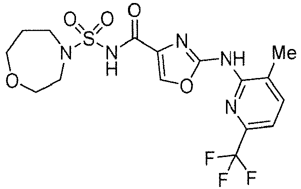 | |
| 2-089 | 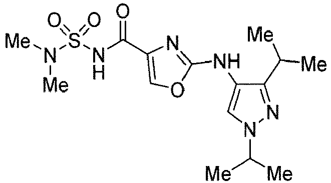 | |
| 2-090 | 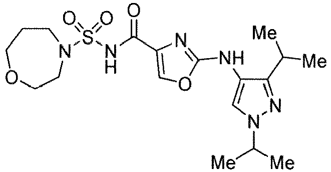 | |
| 2-091 | 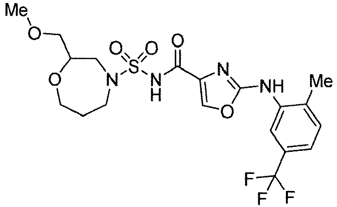 | Racemate |
| 2-092 | 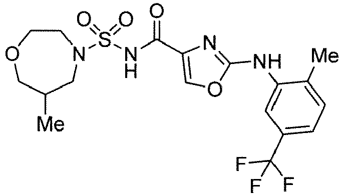 | Racemate |
| 2-093 | 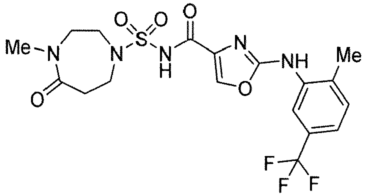 | |
| 2-094 | 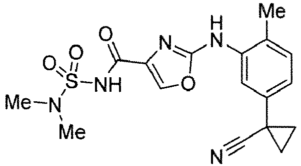 | |
| 2-095 | 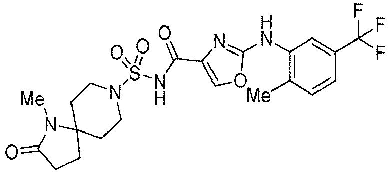 | |
| 2-096 | 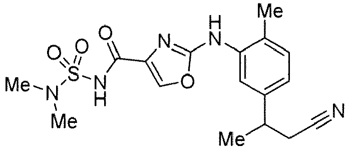 | Racemate |
| 2-097 | 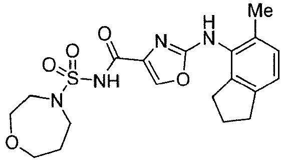 | |
| 2-098 | 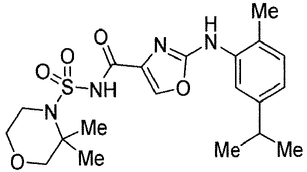 | |
| 2-099 | 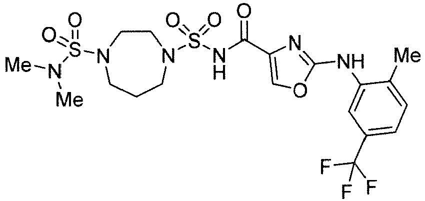 | |
| 2-100 | 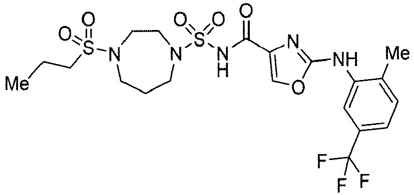 | |
| 2-101 | 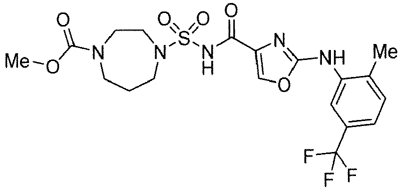 | |
| 2-102 | 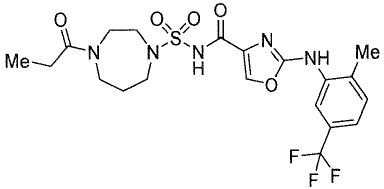 | |
| 2-103 | 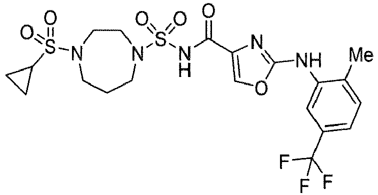 | |
| 2-104 | 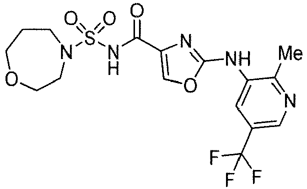 | |
| 2-105 | 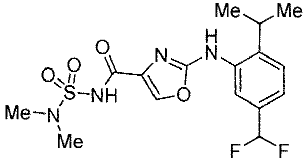 | |
| 2-106 | 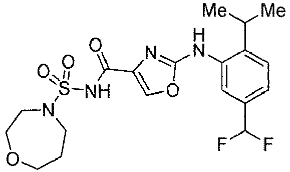 | |
| 2-107 | 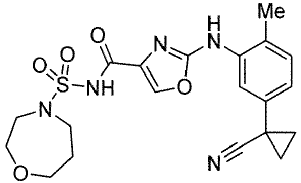 | |
| 2-108 | 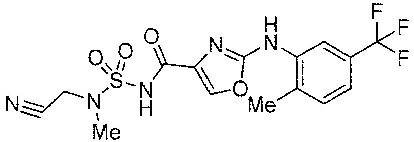 | |
| 2-109 | 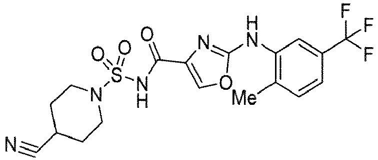 | |
| 2-110 | 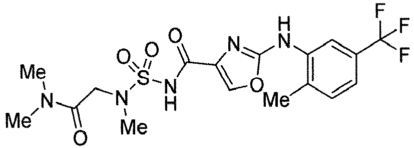 | |
| 2-111 | 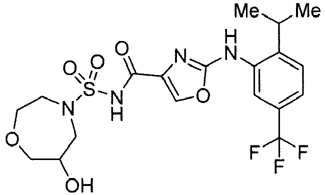 | Racemate |
| 2-112 | 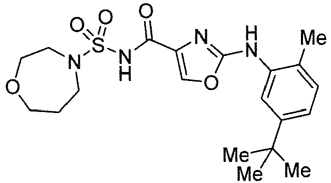 | |
| 2-113 | 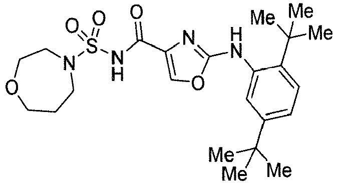 | |
| 2-114 | 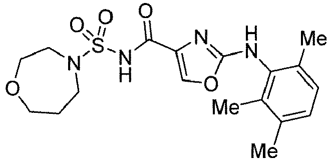 | |
| 2-115 | 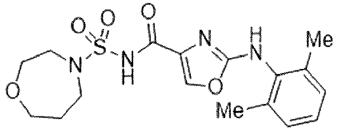 | |
| 2-116 | 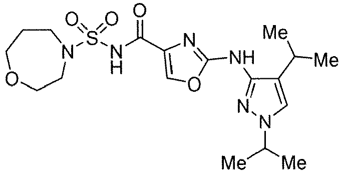 | |
| 2-117 | 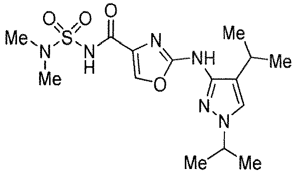 | |
| 2-118 | 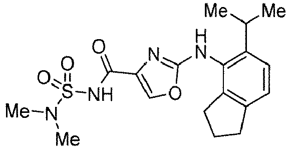 | |
| 2-119 | 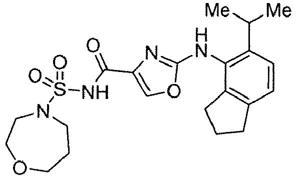 | |
| 2-120 | 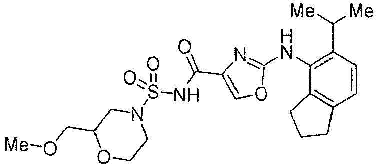 | Racemate |
| 2-121 | 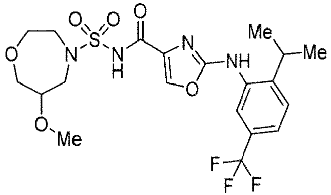 | Racemate |
| 2-122 | 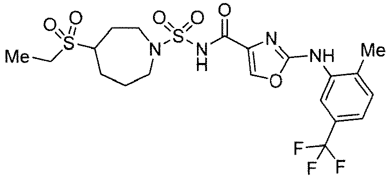 | Racemate |
| 2-123 | 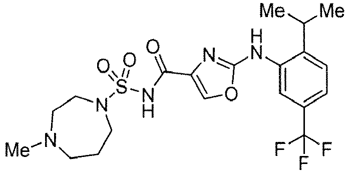 | |
| 2-124 | 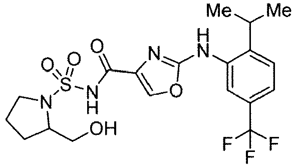 | Racemate |
| 2-125 | 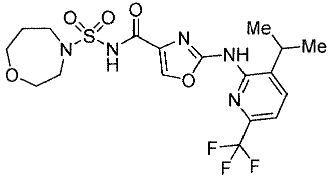 | |
| 2-126 | 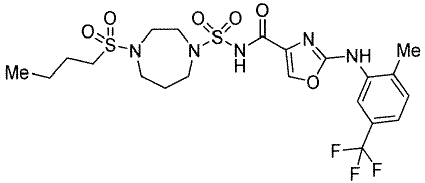 | |
| 2-127 | 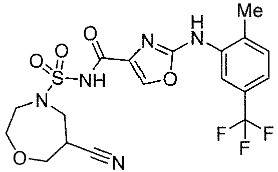 | Racemate |
| 2-128 | 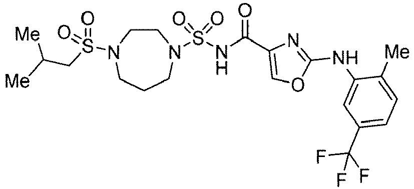 | |
| 2-129 | 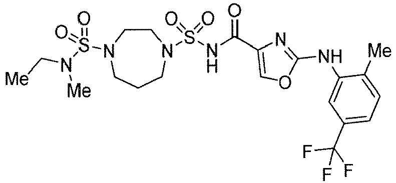 | |
| 2-130 | 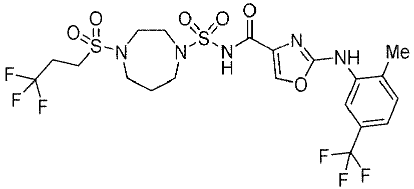 | |
| 2-131 | 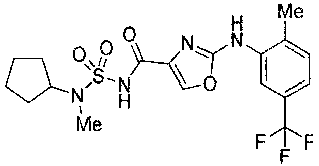 | |
| 2-132 | 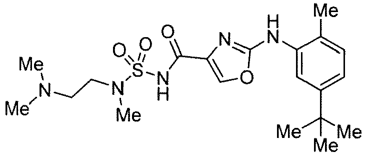 | |
| 2-133 | 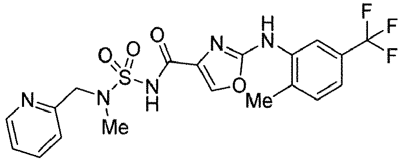 | |
| 2-134 | 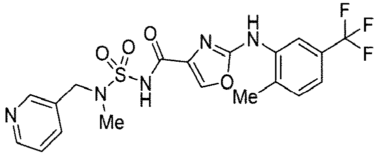 | |
| 2-135 | 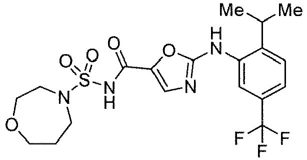 | |
| 2-136 | 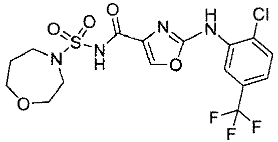 | |
| 2-137 | 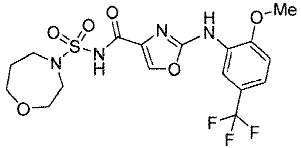 | |
| 2-138 | 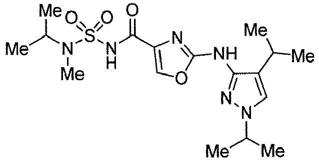 | |
| 2-139 | 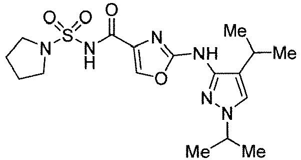 | |
| 2-140 | 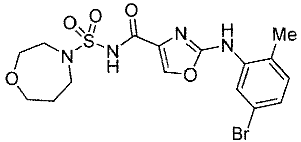 | |
| 2-141 | 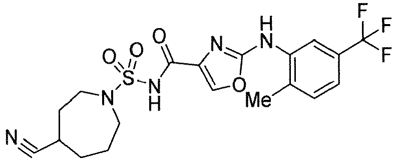 | Racemate |
| 2-142 | 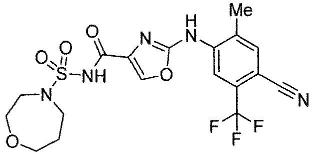 | |
| 2-143 | 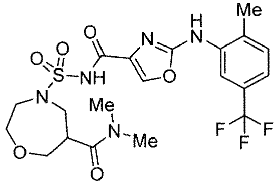 | Racemate |
| 2-144 | 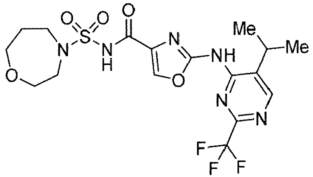 | |
| 2-145 | 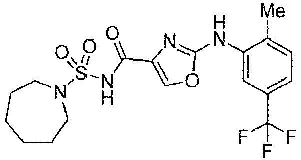 | |
| 2-146 | 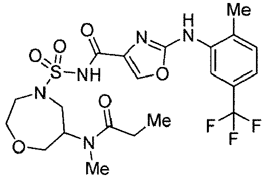 | Racemate |
| 2-147 | 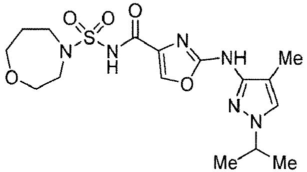 | |
| 2-148 | 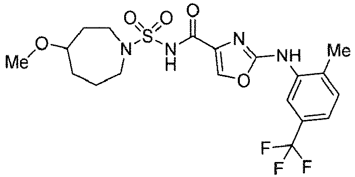 | Racemate |
| 2-149 | 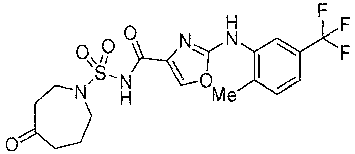 | |
| 2-150 | 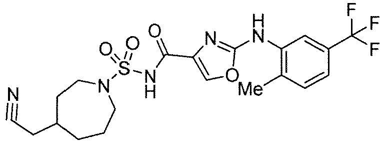 | Racemate |
| 2-151 | 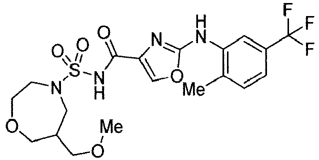 | Racemate |
| 2-152 | 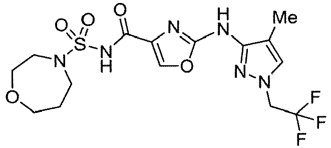 | |
| 2-153 | 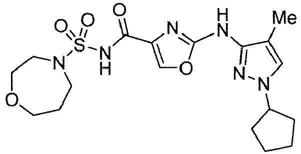 | |
| 2-154 | 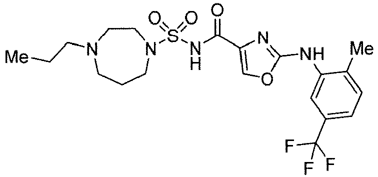 | |
| 2-155 | 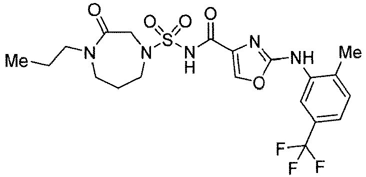 | |
| 2-156 | 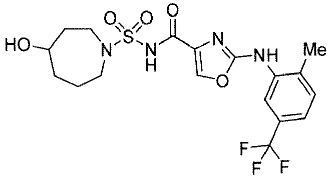 | Racemate |
| 2-157 | 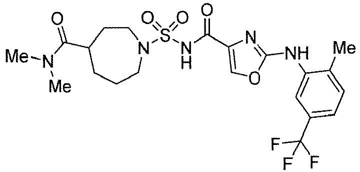 | Racemate |
| 2-158 | 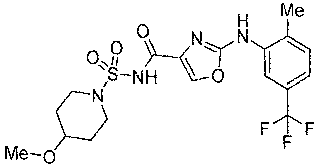 | |
| 2-159 | 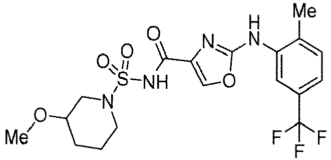 | Racemate |
| 2-160 | 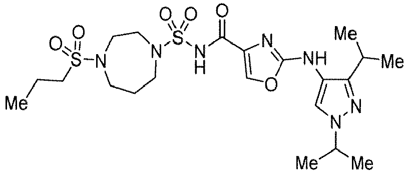 | |
| 2-161 | 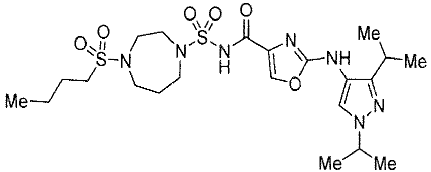 | |
| 2-162 | 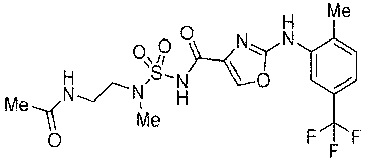 | |
| 2-163 | 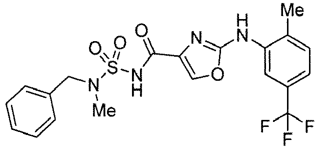 | |
| 2-164 | 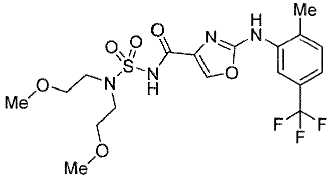 | |
| 2-165 | 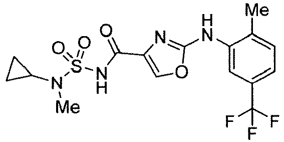 | |
| 2-166 | 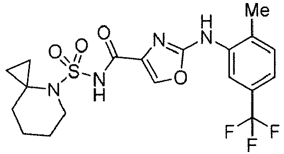 | |
| 2-167 | 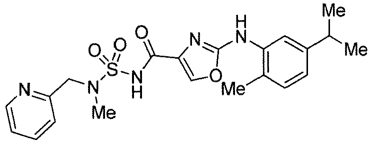 | |
| 2-168 | 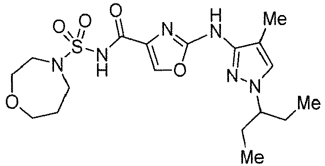 | |
| 2-169 | 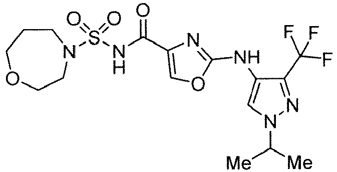 | |
| 2-170 | 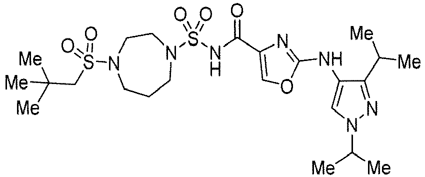 | |
| 2-171 | 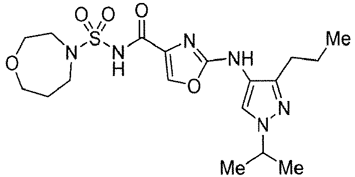 | |
| 2-172 | 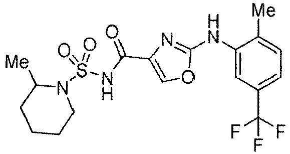 | Racemate |
| 2-173 | 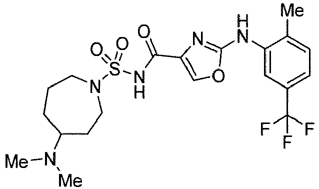 | Racemate |
| 2-174 | 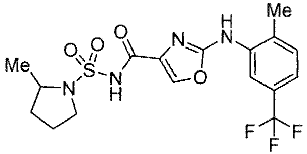 | Racemate |
| 2-175 | 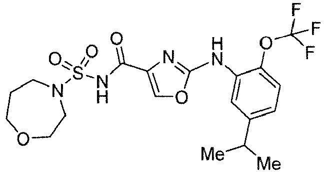 | |
| 2-176 | 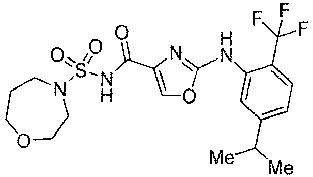 | |
| 2-177 | 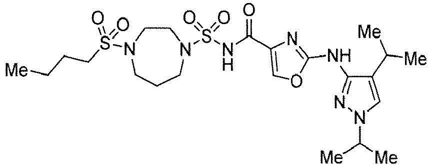 | |
| 2-178 | 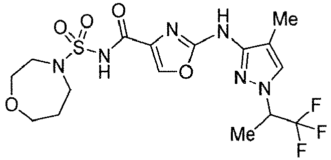 | Racemate |
| 2-179 | 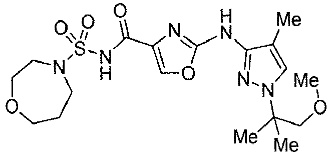 | |
| 2-180 | 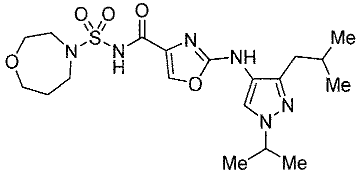 | |
| 2-181 | 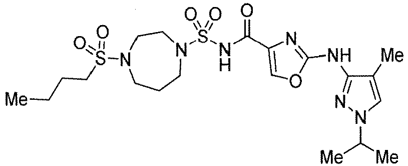 | |
| 2-182 | 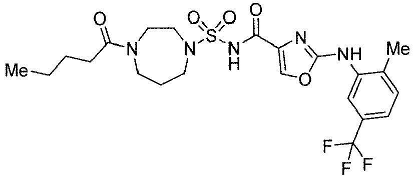 | |
| 2-183 | 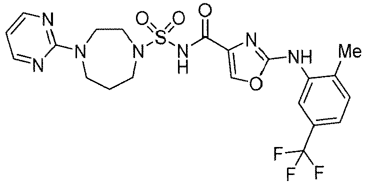 | |
| 2-184 | 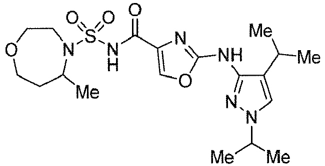 | Racemate |
| 2-185 | 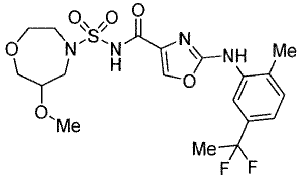 | Racemate |
| 2-186 | 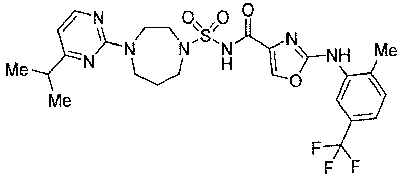 | |
| 2-187 | 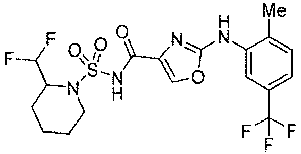 | Racemate |
| 2-188 | 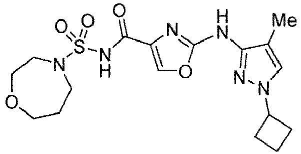 | |
| 2-189 | 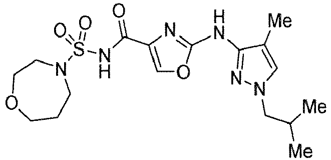 | |
| 2-190 | 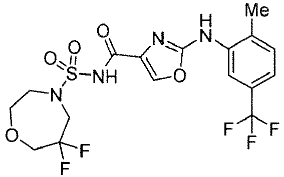 | |
| 2-191 | 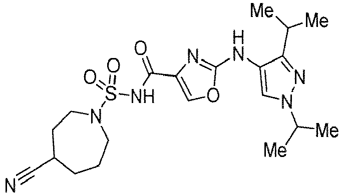 | Racemate |
| 2-192 | 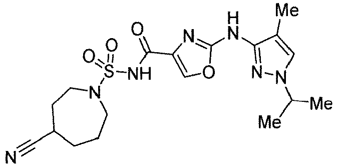 | Racemate |
| 2-193 | 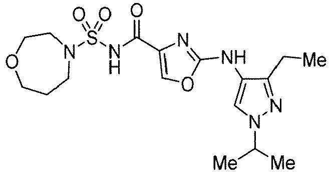 | |
| 2-194 | 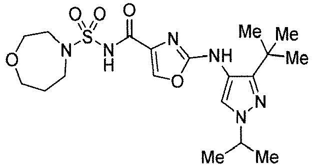 | |
| 2-195 | 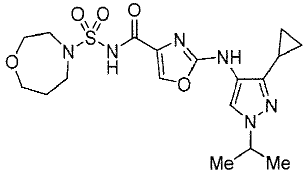 | |
| 2-196 | 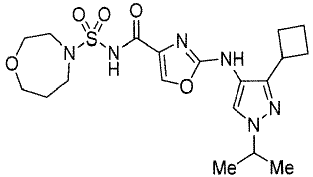 | |
| 2-197 | 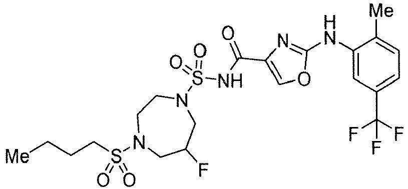 | Racemate |
| 2-198 | 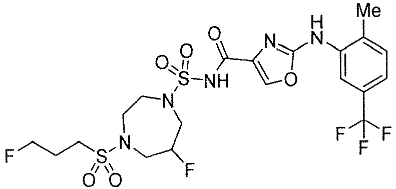 | Racemate |
| 2-199 | 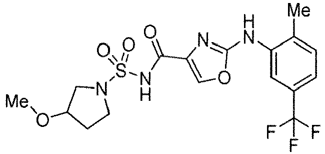 | Racemate |
| 2-200 | 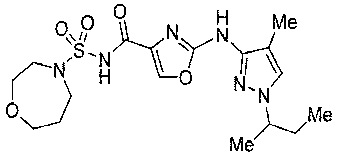 | Racemate |
| 2-201 | 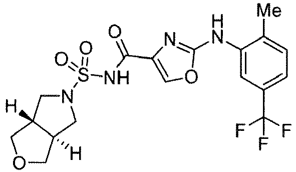 | Racemate Relative configuration : Trans |
| 2-202 | 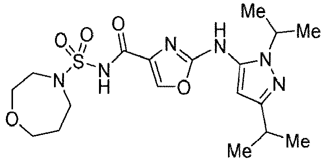 | |
| 2-203 | 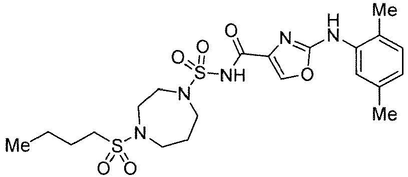 | |
| 2-204 | 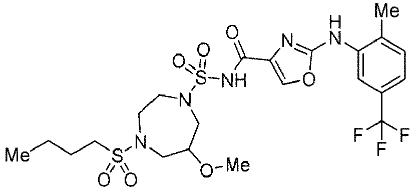 | Racemate |
| 2-205 | 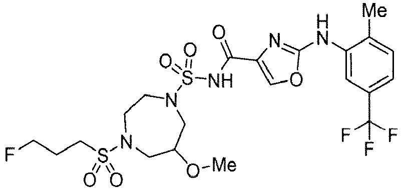 | Racemate |
| 2-206 | 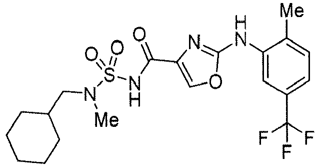 | |
| 2-207 | 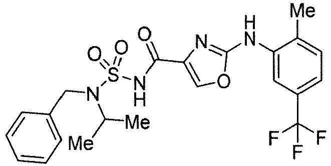 | |
| 2-208 | 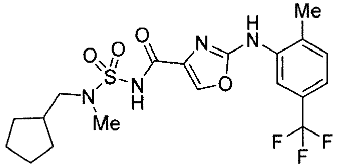 | |
| 2-209 | 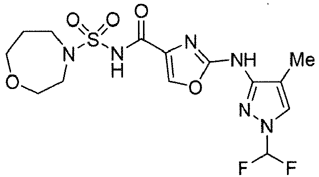 | |
| 2-210 | 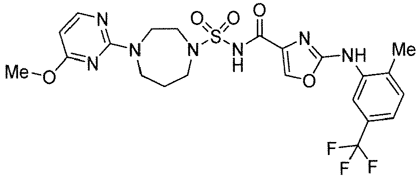 | |
| 2-211 | 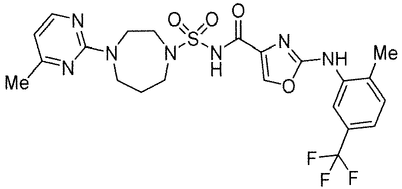 | |
| 2-212 | 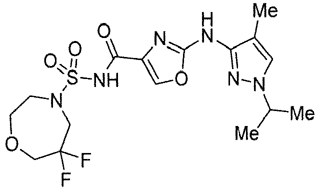 | |
| 2-213 | 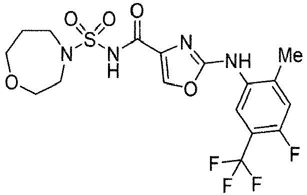 | |
| 2-214 | 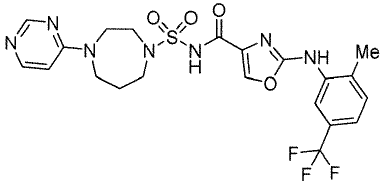 | |
| 2-215 | 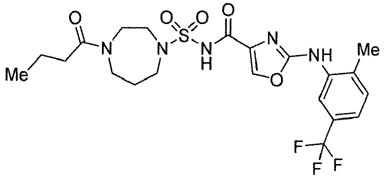 | |
| 2-216 | 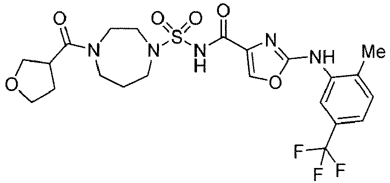 | Racemate |
| 2-217 | 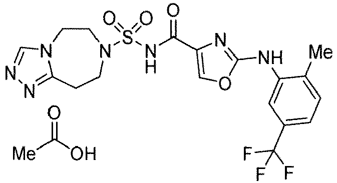 | |
| 2-218 | 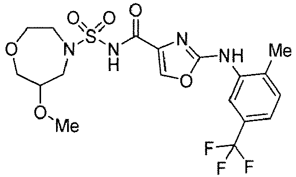 | Enantiomer of 2-219 |
| 2-219 | 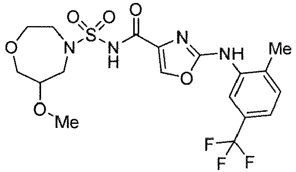 | Enantiomer of 2-218 |
| 2-220 | 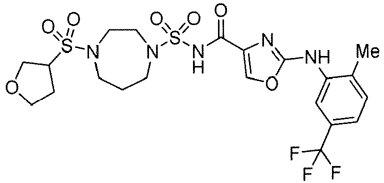 | Racemate |
| 2-221 | 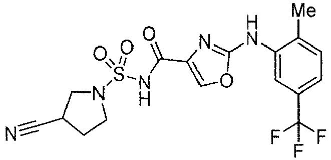 | Racemate |
| 2-222 | 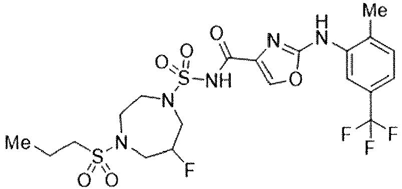 | Racemate |
| 2-223 | 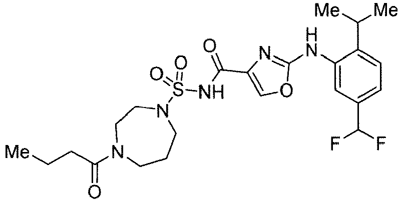 | |
| 2-224 | 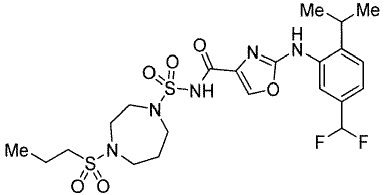 | |
| 2-225 | 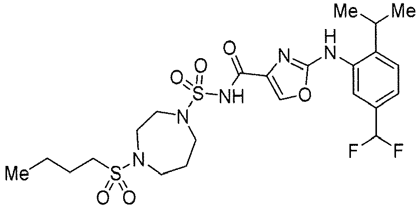 | |
| 2-226 | 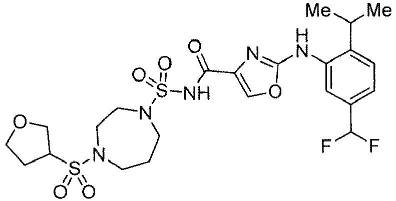 | Racemate |
| 2-227 | 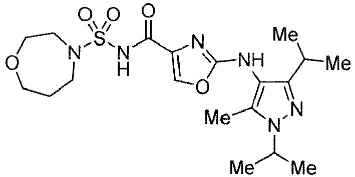 | |
| 2-228 | 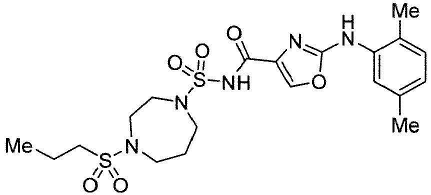 | |
| 2-229 | 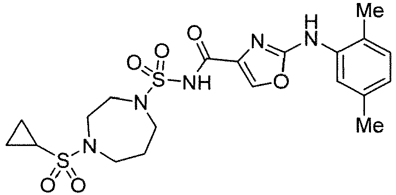 | |
| 2-230 | 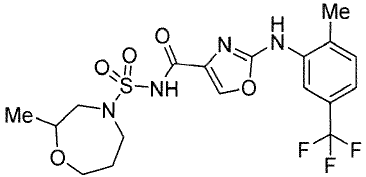 | Racemate |
| 2-231 | 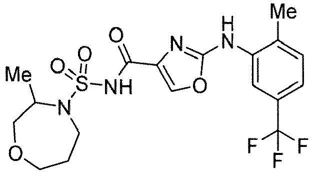 | Racemate |
| 2-232 | 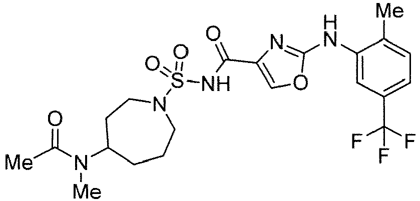 | Racemate |
| 2-233 | 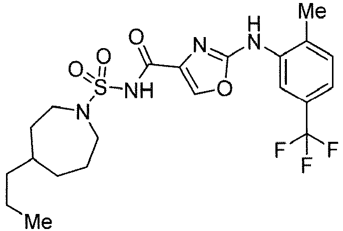 | Racemate |
| 2-234 | 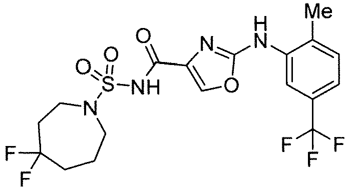 | |
| 2-235 | 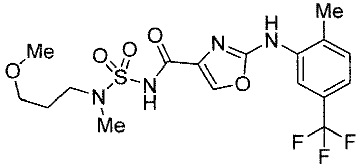 | |
| 2-236 | 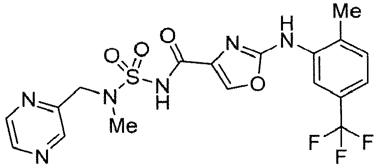 | |
| 2-237 | 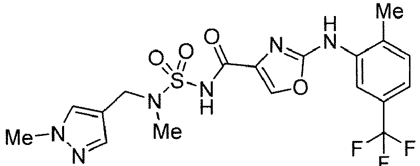 | |
| 2-238 | 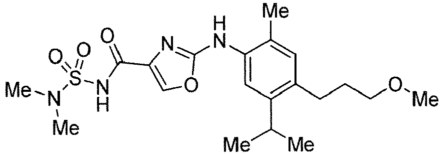 | |
| 2-239 | 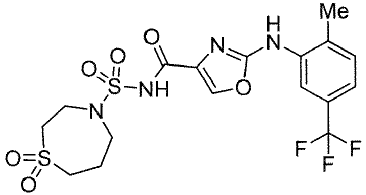 | |
| 2-240 | 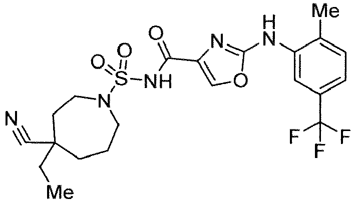 | Racemate |
| 2-241 | 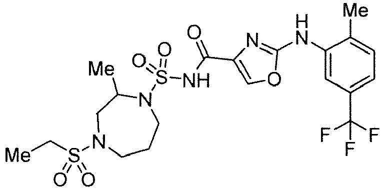 | Racemate |
| 2-242 | 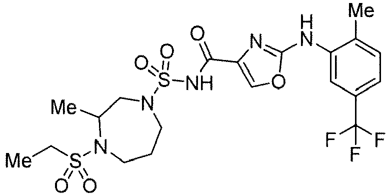 | Racemate |
| 2-243 | 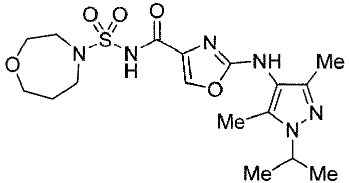 | |
| 2-244 | 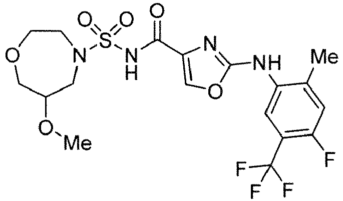 | Racemate |
| 2-245 | 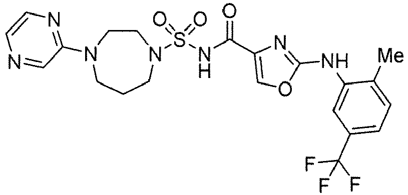 | |
| 2-246 | 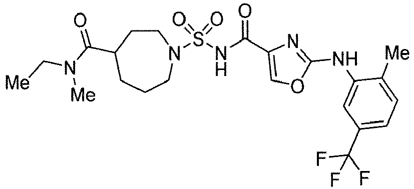 | Racemate |
| 2-247 | 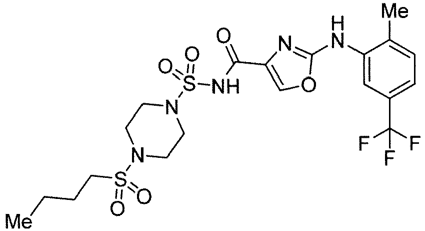 | |
| 2-248 | 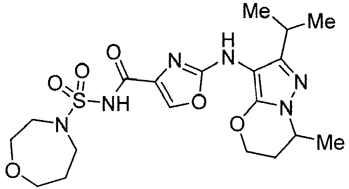 | Racemate |
| 2-249 | 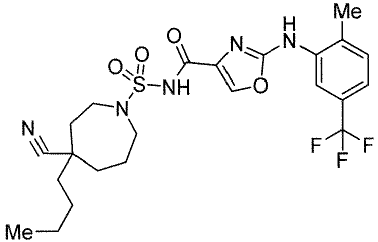 | Racemate |
| 2-250 | 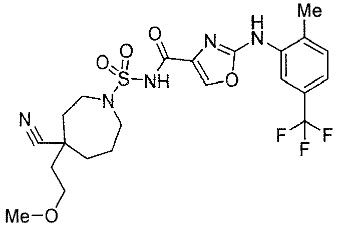 | Racemate |
| 2-251 | 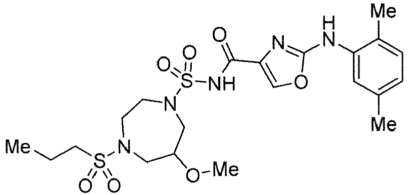 | Racemate |
| 2-252 | 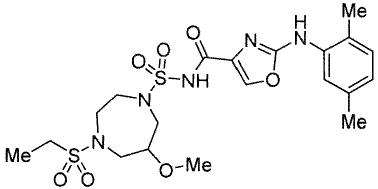 | Racemate |
| 2-253 | 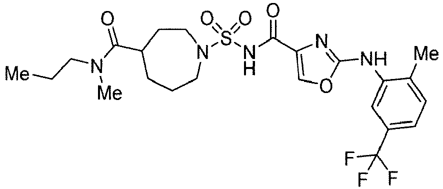 | Racemate |
| 2-254 | 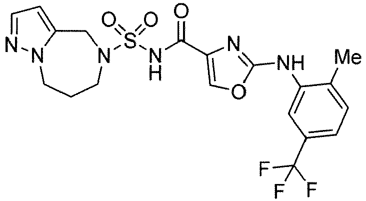 | |
| 2-255 | 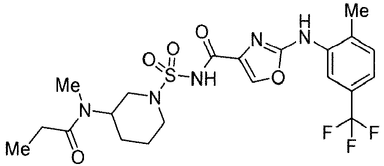 | Racemate |
| 2-256 | 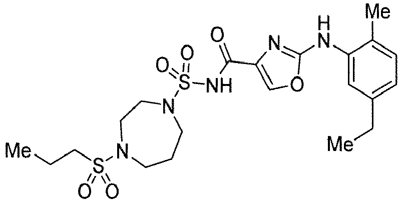 | |
| 2-257 | 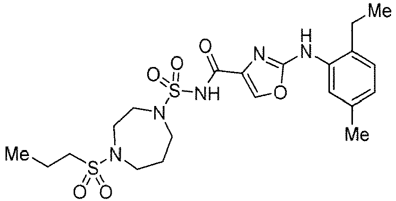 | |
| 2-258 | 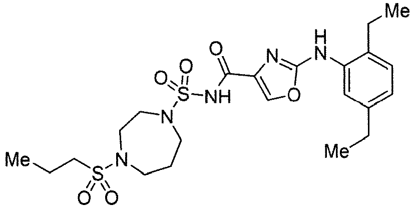 | |
| 2-259 | 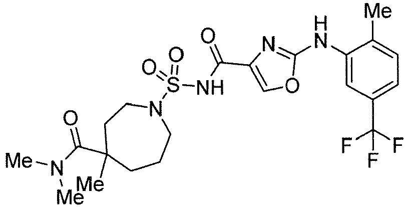 | Racemate |
| 2-260 | 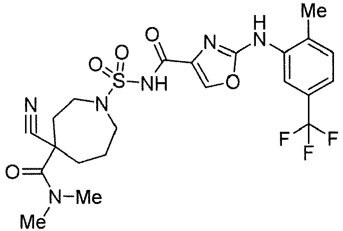 | Racemate |
| 2-261 | 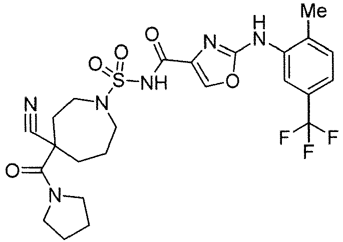 | Racemate |
| 2-262 | 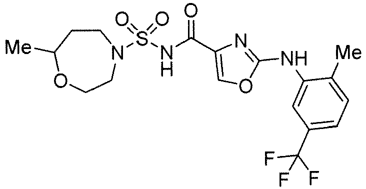 | Racemate |
| 2-263 | 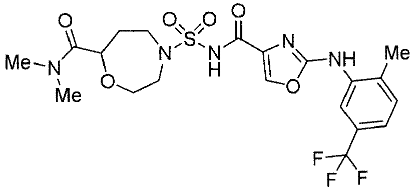 | Putative absolute configuration of substituents on oxazepane ring: (S) form |
| 2-264 | 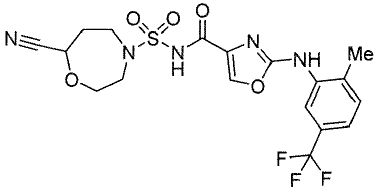 | Putative absolute configuration of substituents on oxazepane ring: (S) form |
| 2-265 | 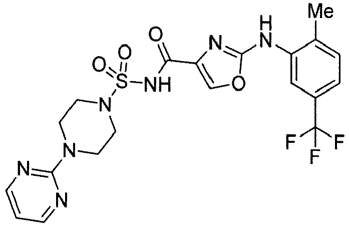 | |
| 2-266 | 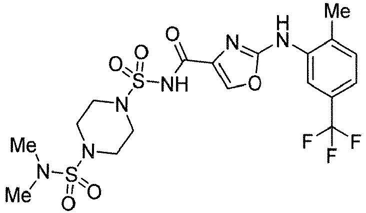 | |
| 2-267 | 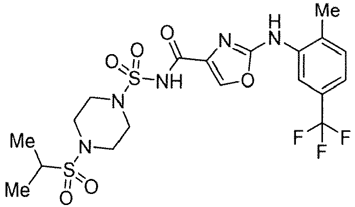 | |
| 2-268 | 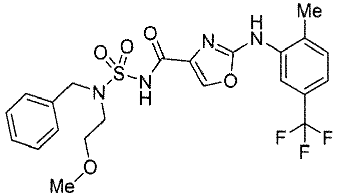 | |
| 2-269 | 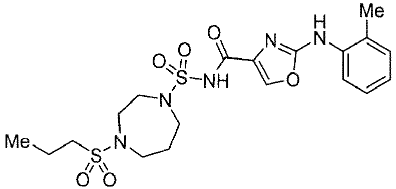 | |
| 2-270 | 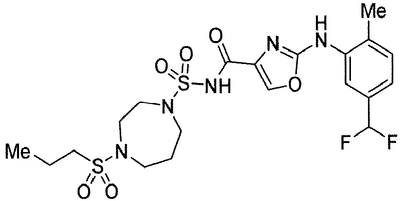 | |
| 2-271 | 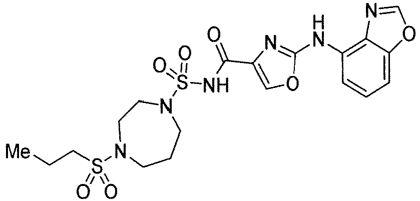 | |
| 2-272 | 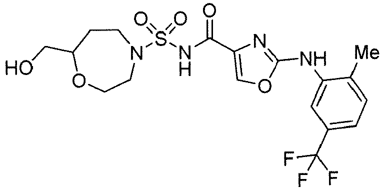 | Putative absolute configuration of substituents on oxazepane ring: (S) form |
| 2-273 | 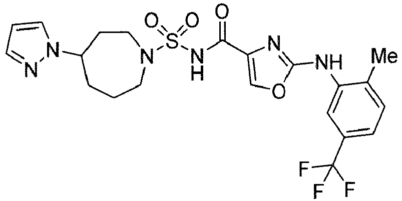 | Racemate |
| 2-274 | 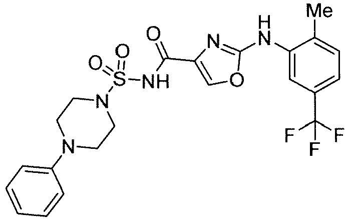 | |
| 2-275 | 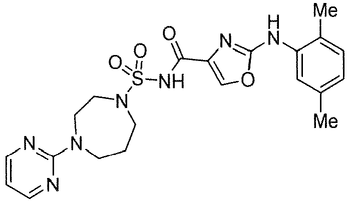 | |
| 2-276 | 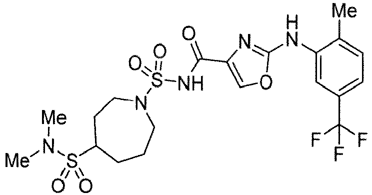 | Racemate |
| 2-277 | 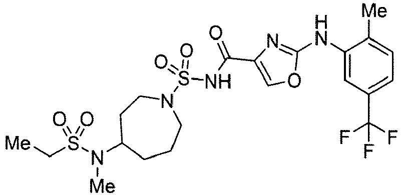 | Racemate |
| 2-278 | 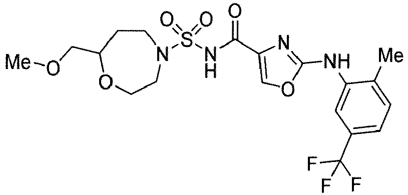 | Putative absolute configuration of substituents on oxazepane ring: (S) form |
| 2-279 | 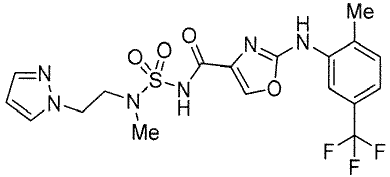 | |
| 2-280 | 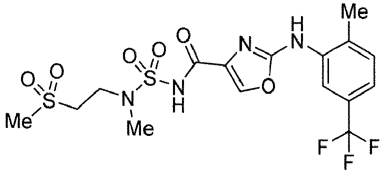 | |
| 2-281 | 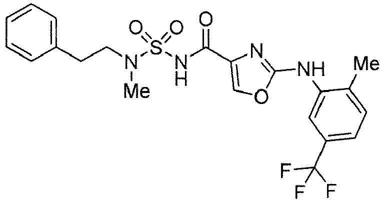 | |
| 2-282 | 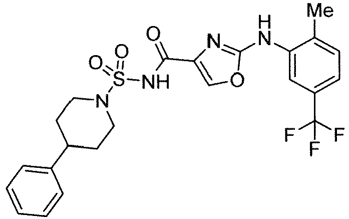 | |
| 2-283 | 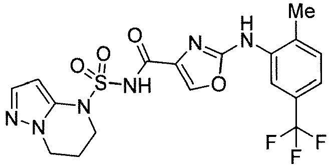 | |
| 2-284 | 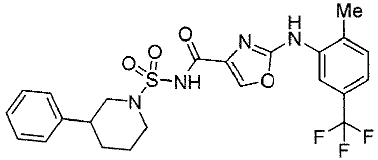 | Racemate |
| 2-285 | 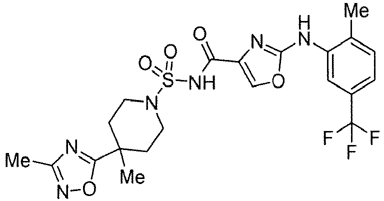 | |
| 2-286 | 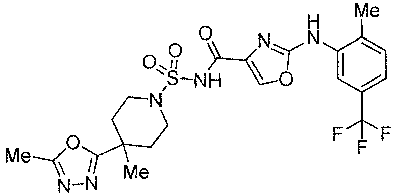 | |
| 2-287 | 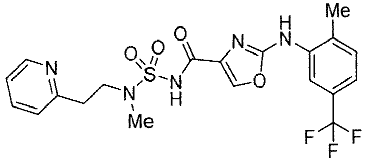 | |
| 2-288 | 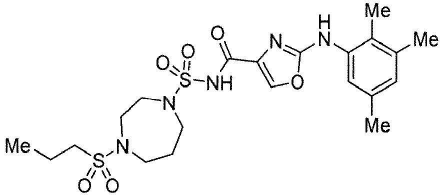 | |
| 2-289 | 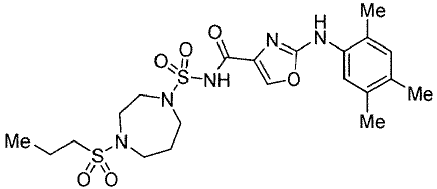 | |
| 2-290 | 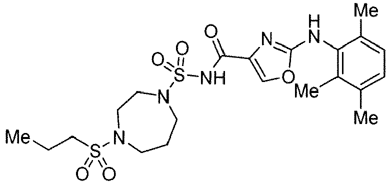 | |
| 2-291 | 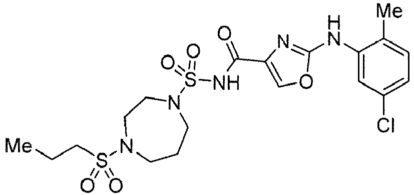 | |
| 2-292 | 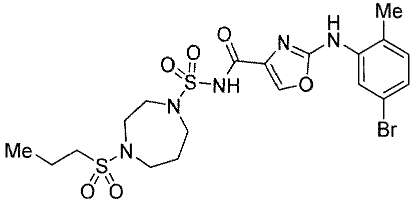 | |
| 2-293 | 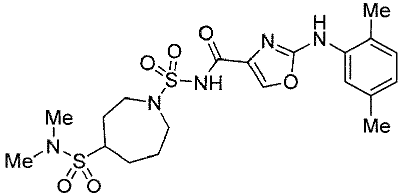 | Racemate |
| 2-294 | 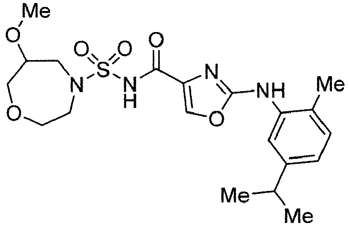 | Racemate |
| 2-295 | 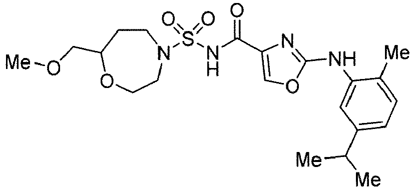 | Putative absolute configuration of substituents on oxazepane ring: (S) form |
| 2-296 | 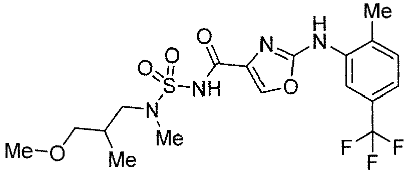 | Racemate |
| 2-297 | 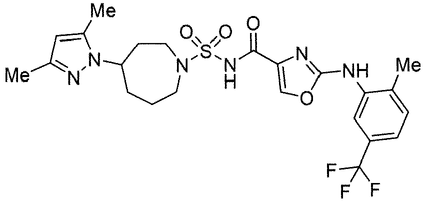 | Racemate |
| 2-298 | 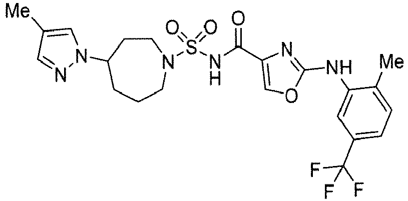 | Racemate |
| 2-299 | 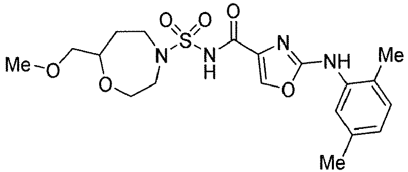 | Putative absolute configuration of substituents on oxazepane ring: (S) form |
| 2-300 | 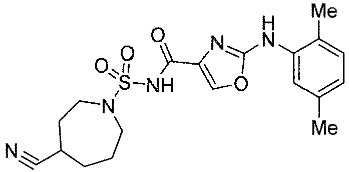 | Racemate |
| 2-301 | 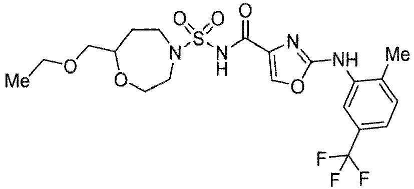 | Putative absolute configuration of substituents on oxazepane ring: (S) form |
| 2-302 | 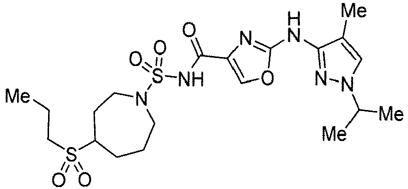 | Racemate |
| 2-303 | 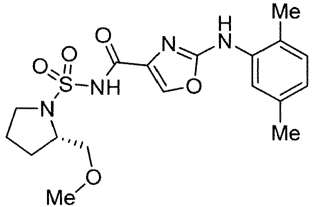 | |
| 2-304 | 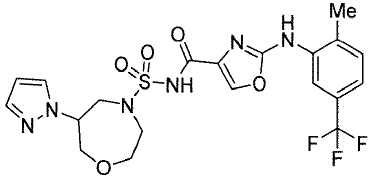 | Racemate |
| 2-305 | 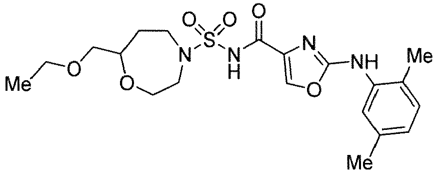 | Putative absolute configuration of substituents on oxazepane ring: (S) form |
| 2-306 | 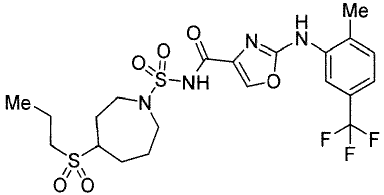 | Racemate |
| 2-307 | 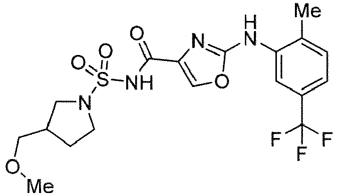 | Racemate |
| 2-308 | 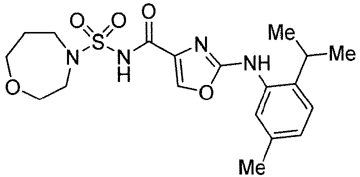 | |
| 2-309 | 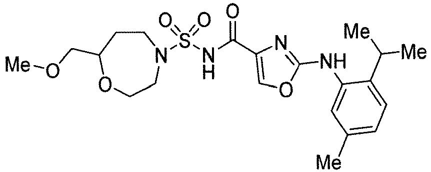 | Putative absolute configuration of substituents on oxazepane ring: (S) form |
| 2-310 | 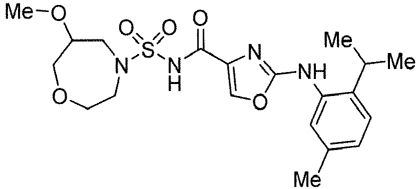 | Racemate |
| 2-311 | 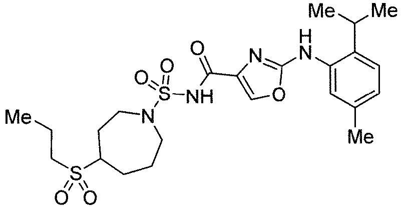 | Racemate |
| 2-312 | 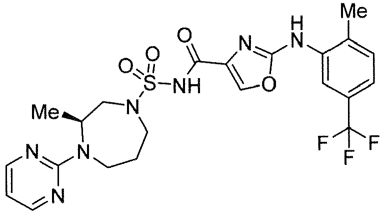 | |
| 2-313 | 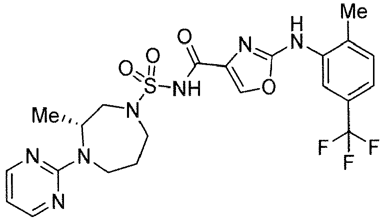 | |
| 2-314 | 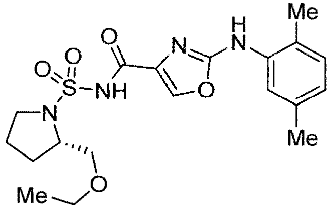 | |
| 2-315 | 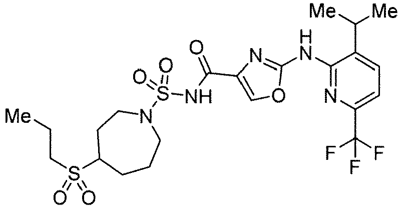 | Racemate |
| 2-316 | 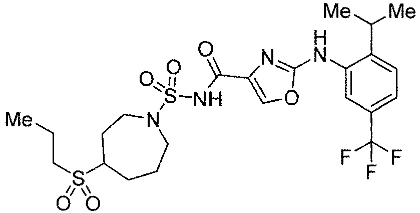 | Racemate |
| 2-317 | 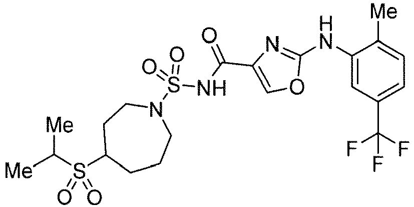 | Racemate |
| 2-318 | 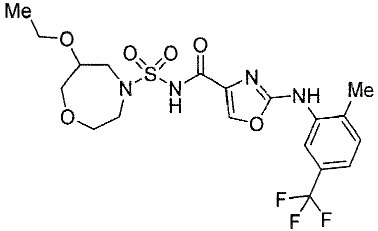 | Racemate |
| 2-319 | 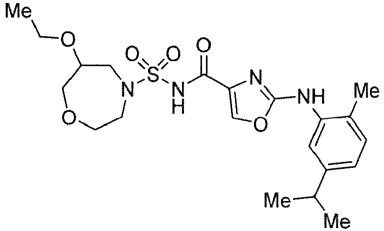 | Racemate |
| 2-320 | 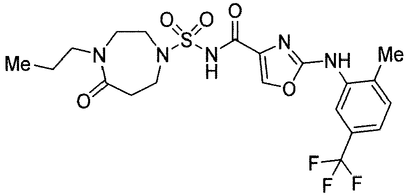 | |
| 2-321 | 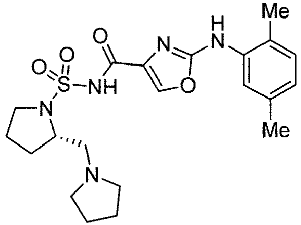 | |
| 2-322 | 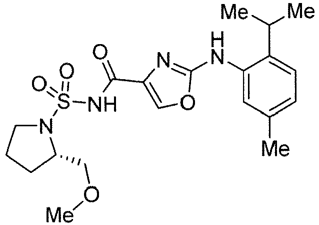 | |
| 2-323 | 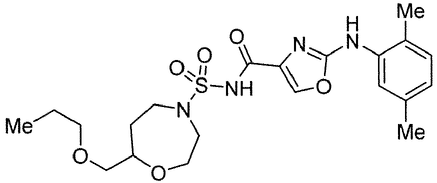 | Putative absolute configuration of substituents on oxazepane ring: (S) form |
| 2-324 | 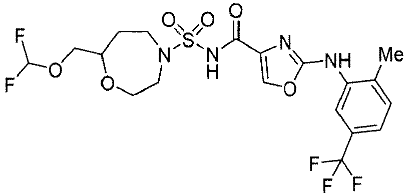 | Putative absolute configuration of substituents on oxazepane ring: (S) form |
| 2-325 | 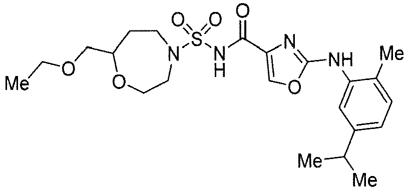 | Putative absolute configuration of substituents on oxazepane ring: (S) form |
| 2-326 | 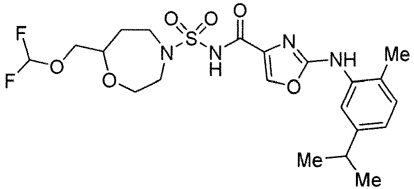 | Putative absolute configuration of substituents on oxazepane ring: (S) form |
| 2-327 | 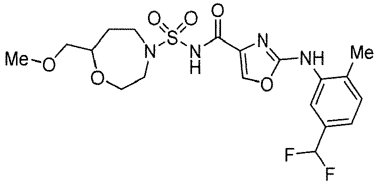 | Putative absolute configuration of substituents on oxazepane ring: (S) form |
| 2-328 | 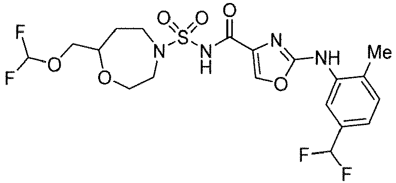 | Putative absolute configuration of substituents on oxazepane ring: (S) form |
| 2-329 | 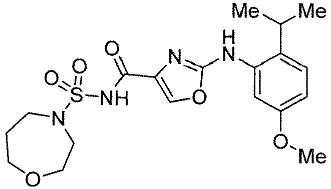 | |
| 2-330 | 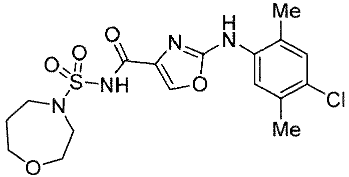 | |
| 2-331 | 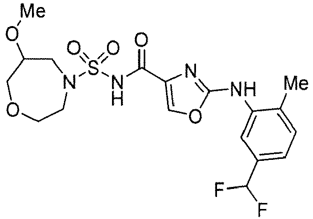 | Racemate |
| 2-332 | 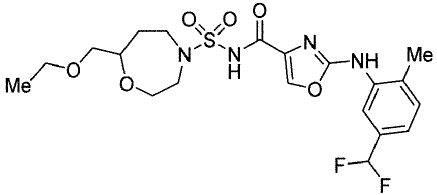 | Putative absolute configuration of substituents on oxazepane ring: (S) form |
| 2-333 | 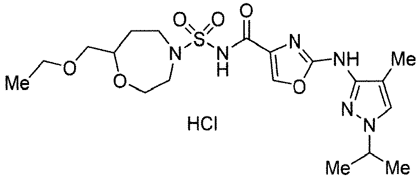 | Putative absolute configuration of substituents on oxazepane ring: (S) form |
| 2-334 | 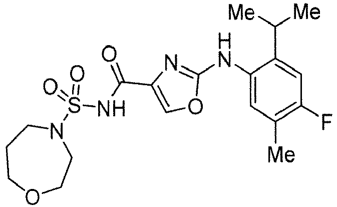 | |
| 2-335 | 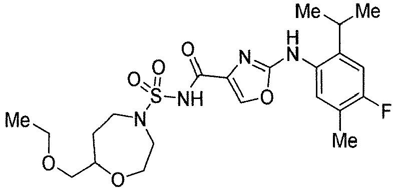 | Putative absolute configuration of substituents on oxazepane ring: (S) form |
| 2-336 | 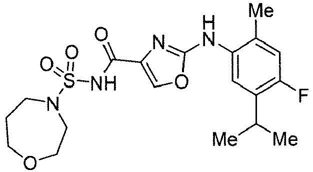 | |
| 2-337 | 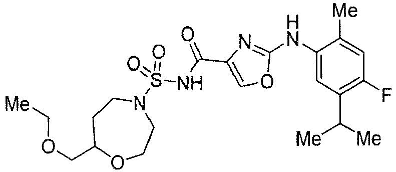 | Putative absolute configuration of substituents on oxazepane ring: (S) form |
| 2-338 | 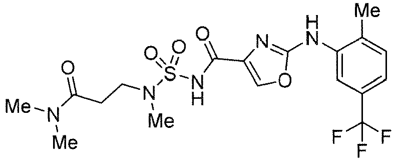 | |
| 2-339 | 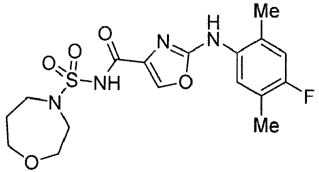 | |
| 2-340 | 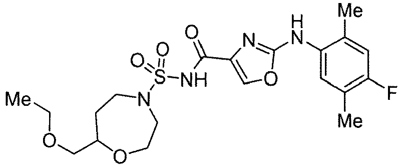 | Putative absolute configuration of substituents on oxazepane ring: (S) form |
| 2-341 | 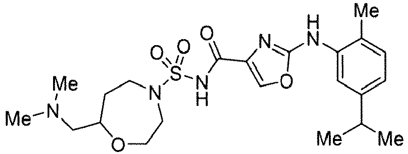 | Putative absolute configuration of substituents on oxazepane ring: (S) form |
| 2-342 | 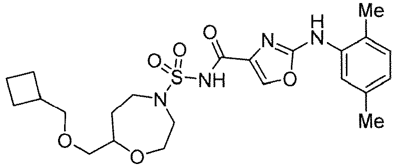 | Putative absolute configuration of substituents on oxazepane ring: (S) form |
| 2-343 | 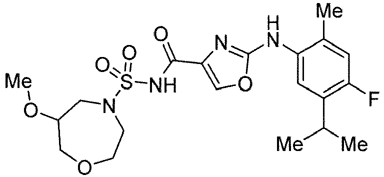 | Racemate |
| 2-344 | 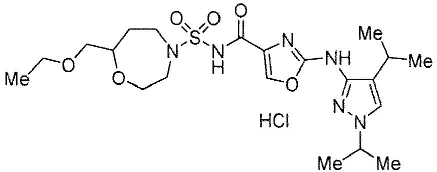 | Putative absolute configuration of substituents on oxazepane ring: (S) form |
| 2-345 | 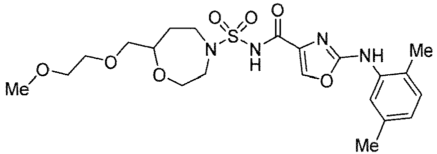 | Putative absolute configuration of substituents on oxazepane ring: (S) form |
| 2-346 | 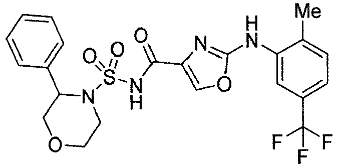 | Racemate |
| 2-347 | 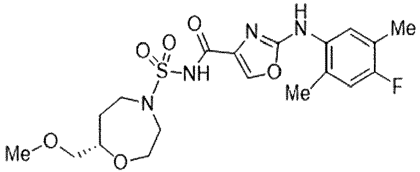 | |
| 2-348 | 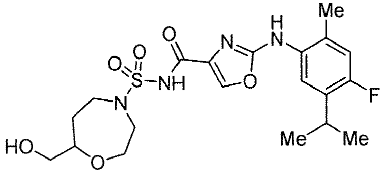 | Putative absolute configuration of substituents on oxazepane ring: (S) form |
| 2-349 | 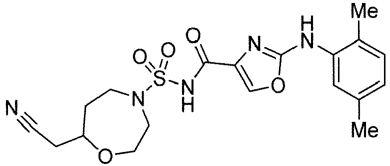 | Putative absolute configuration of substituents on oxazepane ring: (S) form |
| 2-350 | 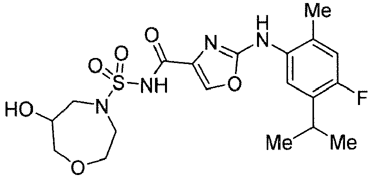 | Racemate |
| 2-351 | 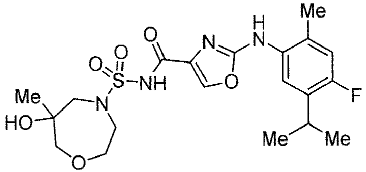 | Racemate |
| 2-352 | 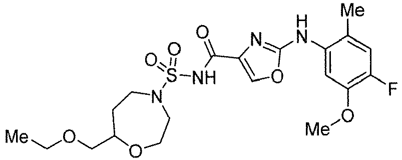 | Putative absolute configuration of substituents on oxazepane ring: (S) form |
| 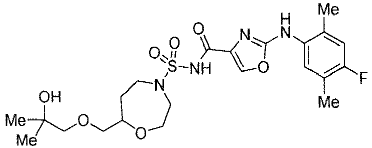 | | |
| 2-353 | 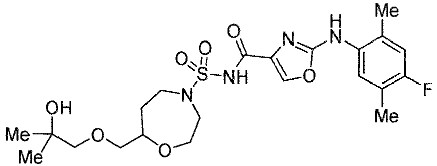 | Putative absolute configuration of substituents on oxazepane ring: (S) form |

**[Table 3]**

| Example | Structure | Note |
|---|---|---|
| 3-001 | 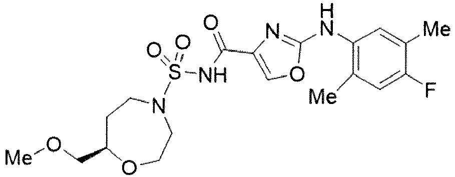 | |
| 3-002 | 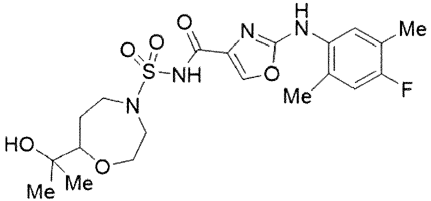 | oxazepane 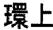 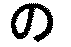 Absolute configuration 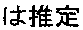 (s) 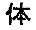 |
| 3-003 | 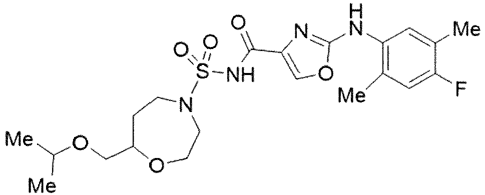 | oxazepane 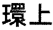 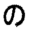 Absolute configuration 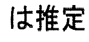 (s) 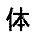 |
| 3-004 | 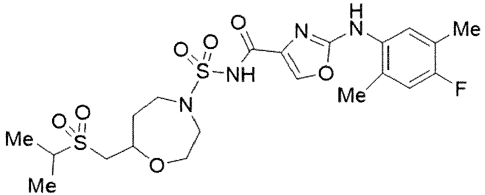 | oxazepane Absolute 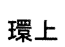 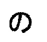 configuration 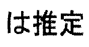 (s) 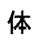 |
| 3-005 | 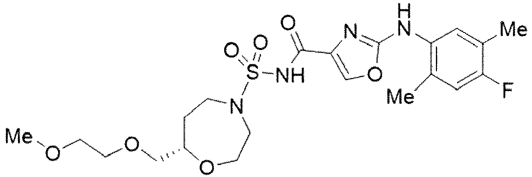 | |
| 3-006 | 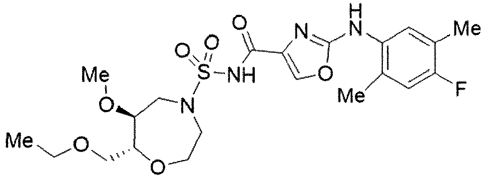 | |
| 3-007 | 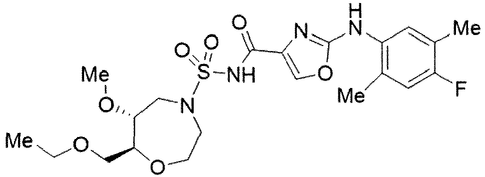 | |

**[Table 4]**

| Example | NMR | MS (M+H) |
|---|---|---|
| 1 | ¹H-NMR (CDCl₃) δ: 8.06 (1H, br s), 7.63 (1H, s), 7.06 (1H, s), 6.75 (1H, s), 3.00 (6H, s), 2.91 (4H, t, J = 7.4 Hz), 2.77 (4H, t, J = 7.3 Hz), 2.12-2.05 (4H, m). | 391 |
| 2 | ¹H-NMR (DMSO-D₆) δ: 11.69 (1H, s), 9.84 (1H, br s), 7.76 (1H, br s), 6.96 (1H, s), 3.61-3.59 (4H, m), 3.17 (3H, br s), 2.81 (4H, t, J = 7.3 Hz), 2.68 (4H, t, J = 7.4 Hz), 1.96 (5H, dt, J = 16.6, 6.1 Hz). | 433 |
| 3 | ¹H-NMR (CDCl₃) δ: 8.09 (1H, br s), 7.62 (1H, s), 7.05 (1H, s), 6.81 (1H, s), 3.40-3.39 (4H, m), 3.34-3.32 (1H, m), 2.91 (4H, t, J = 7.4 Hz), 2.76 (4H, t, J = 7.3 Hz), 2.12-2.05 (4H, m), 1.67-1.65 (5H, m). | 431 |
| 4 | ¹H-NMR (CDCl₃) δ: 7.63 (1H, s), 7.06 (1H, s), 6.75 (1H, s), 3.81-3.79 (4H, m), 3.66-3.62 (4H, m), 2.91 (4H, t, J = 7.4 Hz), 2.76 (4H, t, J = 7.3 Hz), 2.10-2.05 (4H, m), 2.00-1.97 (2H, m). (-NH) | 447 |
| 5 | ¹H-NMR (CDCl₃) δ: 8.13 (1H, br s), 7.63 (1H, s), 7.06 (1H, s), 6.73 (1H, s), 4.00-3.98 (1H, m), 3.85-3.83 (1H, m), 3.69-3.52 (5H, m), 2.91 (4H, t, J = 7.4 Hz), 2.76 (4H, t, J = 7.3 Hz), 2.10-2.06 (4H, m), 1.37 (3H, d, J = 6.9 Hz). | 447 |
| 6 | ¹H-NMR (CDCl₃) δ: 8.12 (1H, s), 7.62 (1H, s), 7.06 (1H, s), 6.77 (1H, s), 3.55-3.52 (4H, m), 2.91 (4H, t, J = 7.4 Hz), 2.77 (4H, t, J = 7.4 Hz), 2.12-2.05 (4H, m), 1.96-1.89 (4H, m). | 417 |
| 7 | ¹H-NMR (DMSO-D₆) δ: 11.49 (1H, s), 9.90 (1H, s), 7.89 (1H, s), 7.70 (1H, d, J = 7.6 Hz), 6.97 (1H, s), 3.45-3.42 (1H, m), 2.81 (4H, t, J = 7.3 Hz), 2.67 (4H, t, J = 7.4 Hz), 1.96-1.92 (4H, m), 1.06 (6H, d, J = 6.5 Hz). | 405 |
| 8 | ¹H-NMR (DMSO-D₆) δ: 11.48 (1H, s), 9.89 (1H, s), 8.34 (1H, s), 7.79 (1H, br s), 7.26-7.20 (5H, m), 6.97 (1H, s), 4.13 (2H, d, J = 6.2 Hz), 2.82 (4H, t, J = 7.3 Hz), 2.68 (4H, t, J = 7.4 Hz), 2.01-1.93 (4H, m). | 453 |
| 9 | ¹H-NMR (CDCl₃) δ: 8.36 (1H, br s), 7.65 (1H, s), 7.06 (1H, s), 6.93 (1H, s), 3.74-3.73 (2H, m), 3.64 (2H, s), 3.60-3.59 (2H, m), 2.91 (4H, t, J = 7.3 Hz), 2.77 (4H, t, J = 7.3 Hz), 2.59-2.51 (2H, m), 2.14-2.03 (6H, m), 1.88-1.79 (1H, m), 1.75-1.65 (1H, m). | 473 |
| 10 | ¹H-NMR (CDCl₃) δ: 8.31 (1H, br s), 7.64 (1H, s), 7.06 (1H, s), 6.88 (1H, s), 3.92 (1H, d, J = 11.8 Hz), 3.78-3.51 (4H, m), 3.45-3.28 (2H, m), 2.91 (4H, t, J = 7.4 Hz), 2.77 (4H, t, J = 7.4 Hz), 2.35-2.32 (1H, m), 2.10-2.05 (4H, m), 1.00 (6H, t, J = 6.7 Hz). | 475 |
| 11 | ¹H-NMR (CDCl₃) δ: 8.37 (1H, br s), 7.64 (1H, s), 7.06 (2H, s), 3.81-3.61 (5H, m), 3.57-3.39 (2H, m), 2.91 (4H, t, J = 7.4 Hz), 2.77 (4H, t, J = 7.4 Hz), 2.12-2.05 (4H, m), 1.87-1.80 (2H, m), 0.95 (3H, t, J = 7.4 Hz). | 461 |
| 12 | ¹H-NMR (DMSO-D₆) δ: 11.60 (1H, s), 9.93 (1H, s), 7.90 (1H, s), 7.86 (1H, br s), 6.97 (1H, s), 3.77 (1H, dd, J = 11.8, 4.2 Hz), 3.62 (1H, dd, J = 11.8, 4.6 Hz), 3.51-3.48 (1H, m), 3.28 (3H, s), 3.07-3.06 (2H, m), 2.81 (4H, t, J = 7.4 Hz), 2.67 (4H, t, J = 7.4 Hz), 1.99-1.92 (4H, m). | 469 |
| 13 | ¹H-NMR (DMSO-D₆) δ: 11.18 (1H, br s), 9.32-9.29 (1H, m), 8.26-8.23 (1H, m), 6.94 (1H, s), 2.82-2.80 (10H, m), 2.67-2.65 (4H, m), 2.00-1.92 (4H, m). | 391 |
| 14 | ¹H-NMR (DMSO-D₆) δ: 11.58 (1H, br s), 10.01 (1H, s), 7.92 (1H, s), 2.86 (4H, t, J = 7.3 Hz), 2.82 (6H, s), 2.78 (4H, t, J = 7.5 Hz), 2.05-1. 98 (4H, m). | 425 |
| 15 | ¹H-NMR (CDCl₃) δ: 8.13 (1H, br s), 7.61 (1H, s), 7.06 (1H, s), 6.77 (1H, s), 4.26-4.19 (1H, m), 2.92-2.90 (7H, m), 2.76 (4H, t, J = 7.4 Hz), 2.12-2.05 (4H, m), 1.17 (6H, d, J = 6.9 Hz). | 419 |
| 16 | ¹H-NMR (CDCl₃) δ: 8.09 (1H, br s), 7.64 (1H, s), 7.06 (1H, s), 6.85 (1H, s), 3.93 (1H, dd, J = 11.1, 2.8 Hz), 3.70-3.60 (3H, m), 3.41-3.39 (1H, m), 3.14 (1H, td, J = 12.5, 3.3 Hz), 2.91 (4H, t, J = 7.4 Hz), 2.84-2.82 (1H, m), 2.76 (4H, t, J = 7.3 Hz), 2.10-2.07 (4H, m), 1.49-1.46 (2H, m), 0.95 (3H, t, J = 7.5 Hz). | 461 |
| 17 | ¹H-NMR (DMSO-D₆) δ: 11.67 (1H, s), 9.98 (1H, s), 7.95 (1H, s), 6.98 (1H, s), 4.17-4.10 (3H, m), 3.93-3.87 (2H, m), 3.15 (3H, s), 2.82 (4H, t, J = 7.5 Hz), 2.68 (4H, t, J = 7.4 Hz), 1.99-1.95 (4H, m). | 433 |
| 18 | ¹H-NMR (CDCl₃) δ: 8.01 (1H, br s), 7.66 (1H, s), 7.41 (1H, s), 7.32 (1H, br s), 7.05-6.98 (2H, m), 4.50-4.47 (1H, m), 3.94 (2H, dd, J = 11.2, 4.5 Hz), 3.88 (3H, s), 3.49 (2H, dd, J = 11.8, 10.6 Hz), 2.90 (4H, t, J = 7.3 Hz), 2.75 (4H, t, J = 7.3 Hz), 2.09-2.05 (4H, m), 1.89 (2H, dd, J = 12.1, 2.7 Hz), 1.49 (2H, ddd, J = 24.6, 12.5, 4.7 Hz). | 527 |
| 19 | ¹H-NMR (DMSO-D₆) δ: 11.65 (1H, br s), 10.05 (1H, s), 7.96 (1H, br s), 7.60 (1H, d, J = 8.3 Hz), 7.11 (1H, t, J = 7.7 Hz), 6.97 (1H, d, J = 7.6 Hz), 2.86-2.84 (10H, m), 2.02-1.95 (2H, m). | 351 |
| 20 | ¹H-NMR (DMSO-D₆) δ: 11.50 (1H, s), 9.72 (1H, s), 7.86 (1H, s), 7.30 (1H, t, J = 7.7 Hz), 7.20 (2H, d, J = 7.6 Hz), 3.06-2.99 (2H, m), 2.81 (6H, s), 1.09 (12H, d, J = 6.7 Hz). | 395 |
| 21 | ¹H-NMR (CDCI₃+TFA) δ: 7.87 (2H, s), 7.11 (1H, s), 4.19-4.17 (1H, m), 2.90-2.88 (7H, m), 2.75 (4H, t, J = 7.4 Hz), 2.12-2.05 (4H, m), 1.15 (6H, d, J = 6.5 Hz). (-NH) | 419 |
| 22 | ¹H-NMR (CDCI₃+TFA) δ: 7.89-7.86 (2H, m), 7.11 (1H, s), 3.49 (4H, br s), 2.91 (4H, t, J = 7.4 Hz), 2.75 (4H, t, J = 7.3 Hz), 2.12-2.05 (4H, m), 1.90 (4H, br s). (-NH) | 417 |
| 23 | ¹H-NMR (CDCl₃) δ: 8.17 (1H, s), 7.60 (1H, s), 7.05 (1H, s), 6.74 (1H, s), 4.24-4.17 (1H, m), 3.45 (2H, q, J = 7.2 Hz), 2.91 (4H, t, J = 7.4 Hz), 2.76 (4H, t, J = 7.3 Hz), 2.12-2.05 (4H, m), 1.26 (3H, q, J = 5.5 Hz), 1.21 (6H, d, J = 6.7 Hz). | 433 |
| 24 | ¹H-NMR (CDCl₃) δ: 8.17 (1H, br s), 7.63 (1H, s), 7.06 (1H, s), 6.85 (1H, s), 4.83-4.77 (1H, m), 4.00 (1H, td, J = 8.7, 4.5 Hz), 3.83 (1H, dd, J = 10.4, 3.5 Hz), 3.75 (1H, dd, J = 10.4, 6.9 Hz), 3.61 (1H, q, J = 8.3 Hz), 2.95 (3H, s), 2.91 (4H, t, J = 7.4 Hz), 2.76 (4H, t, J = 7.4 Hz), 2.31-2.23 (1H, m), 2.12-2.05 (4H, m), 1.99-1.90 (1H, m). | 447 |
| 25 | ¹H-NMR (CDCl₃) δ: 8.98 (1H, br s), 7.83 (1H, s), 7.06 (1H, s), 6.20 (1H, s), 3.99-3.98 (1H, m), 3.84-3.81 (1H, m), 3.69-3.46 (5H, m), 2.92 (4H, t, J = 7.4 Hz), 2.76 (4H, t, J = 7.3 Hz), 2.13-2.03 (4H, m), 1.36 (3H, d, J = 6.7 Hz). | 447 |
| 26 | ¹H-NMR (DMSO-D₆) δ: 11.56 (1H, s), 9.91 (1H, s), 7.91 (1H, s), 2.85-2.80 (10H, m), 2.72 (4H, t, J = 7.4 Hz), 2.06-2.00 (4H, m). | 409 |
| 27 | ¹H-NMR (DMSO-D₆) δ: 11.19 (1H, br s), 9.30 (1H, s), 8.26 (1H, s), 2.85 (4H, t, J = 7.3 Hz), 2.80 (6H, s), 2.72 (4H, t, J = 7.4 Hz), 2.06-1.99 (4H, m). | 409 |
| 28 | ¹H-NMR (DMSO-D₆) δ: 11.22 (1H, br s), 9.42 (1H, s), 8.29 (1H, s), 2.86 (4H, t, J = 7.3 Hz), 2.81 (6H, s), 2.77 (4H, t, J = 7.4 Hz), 2.05-1.98 (4H, m). | 425 |
| 29 | ¹H-NMR (DMSO-D₆) δ: 11.34 (1H, s), 9.67 (1H, s), 8.45 (1H, s), 8.23 (1H, s), 7.44 (1H, d, J = 8.1 Hz), 7.35 (1H, d, J = 8.1 Hz), 5.07 (1H, br s), 4.28 (1H, br s), 3.58-3.48 (3H, m), 3.19 (1H, dd, J = 10.1, 1.6 Hz), 2.35 (3H, s), 1.89-1.86 (1H, m), 1.78-1.73 (1H, m). | 435 |
| 30 | ¹H-NMR (DMSO-D₆) δ: 11.15 (1H, br s), 9.25 (1H, s), 8.27 (1H, s), 7.03 (2H, s), 5.04 (1H, br s), 4.26 (1H, br s), 3.53-3.46 (3H, m), 3.15 (1H, dd, J = 9.7, 2.2 Hz), 2.86 (2H, t, J = 7.4 Hz), 2.69 (2H, t, J = 7.4 Hz), 2.16 (3H, s), 1.99-1.97 (2H, m), 1.87-1.82 (1H, m), 1.74 (1H, s) . | 407 |
| 31 | ¹H-NMR (DMSO-D₆) δ: 11.17 (1H, br s), 9.26 (1H, s), 8.29 (1H, s), 7.03 (2H, s), 3.38-3.36 (4H, m), 2.86 (2H, t, J = 7.3 Hz), 2.70 (2H, t, J = 7.3 Hz), 2.16 (3H, s), 2.01-1.93 (2H, m), 1.80-1.77 (4H, m). | 391 |
| 32 | ¹H-NMR (DMSO-D₆) δ: 11.39 (1H, br s), 9.28 (1H, s), 8.31 (1H, s), 7.03 (2H, s), 3.62-3.61 (4H, m), 3.23-3.22 (4H, m), 2.86 (2H, t, J = 7.4 Hz), 2.70 (2H, t, J = 7.5 Hz), 2.16 (3H, s), 2.01-1.94 (2H, m). | 407 |
| 33 | ¹H-NMR (DMSO-D₆) δ: 11.09 (1H, s), 9.30 (1H, s), 8.25 (1H, br s), 4.88 (1H, br s), 3.53-3.51 (2H, m), 3.20 (2H, dd, J = 15.0, 9.7 Hz), 2.84 (4H, t, J = 7.2 Hz), 2.72 (4H, t, J = 7.3 Hz), 2.05-2.00 (4H, m), 1.80-1.65 (2H, m), 1.22 (3H, s) . | 465 |
| 34 | ¹H-NMR (DMSO-D₆) δ: 11.17 (1H, s), 9.43 (1H, s), 8.31 (1H, s), 3.36-3.35 (4H, m), 2.86 (4H, t, J = 7.4 Hz), 2.77 (4H, t, J = 7.4 Hz), 2.05-1.98 (4H, m), 1.80-1.77 (4H, m). | 451 |
| 35 | ¹H-NMR (DMSO-D₆+TFA) δ: 11.14 (1H, s), 9.32 (1H, s), 3.36-3.35 (4H, m), 2.85 (4H, t, J = 7.4 Hz), 2.72 (4H, t, J = 7.4 Hz), 2.06-1.99 (4H, m), 1.79-1.76 (4H, m). (-NH) | 435 |
| 36 | ¹H-NMR (CDC1₃) δ: 8.91 (1H, s), 7.82 (1H, s), 6.11 (1H, s), 3.96 (1H, s), 3.66-3.54 (3H, m), 3.51-3.33 (1H, m), 3.26 (3H, s), 2.97-2.95 (4H, m), 2.84-2.82 (4H, m), 2.16-1.96 (6H, m). | 481 |
| 37 | ¹H-NMR (DMSO-D₆+TFA) δ: 9.45 (1H, s), 8.32 (1H, s), 3.61-3.59 (4H, m), 3.22-3.21 (4H, m), 2.86 (4H, t, J = 7.4 Hz), 2.78 (4H, t, J = 7.4 Hz), 2.06-1.98 (4H, m). | 467 |
| 38 | ¹H-NMR (CDCl₃+TFA) δ: 8.94 (1H, s), 7.87 (1H, s), 3.91-3.40 (10H, m), 3.10-3.08 (1H, m), 2.96 (4H, t, J = 7.5 Hz), 2.93-2.90 (1H, m), 2.83 (4H, t, J = 7.3 Hz), 2.15-2.08 (4H, m). (-NH) | 511 |
| 39 | ¹H-NMR (CDCl₃+TFA) δ: 8.85 (1H, br s), 7.84 (1H, s), 4.18-4.15 (1H, m), 3.61-3.52 (4H, m), 3.38 (3H, s), 2.96 (4H, t, J = 7.4 Hz), 2.83 (4H, t, J = 7.4 Hz), 2.15-2.08 (4H, m), 1.17 (6H, d, J = 6.7 Hz). (-NH) | 497 |
| 40 | ¹H-NMR (CDCl₃+TFA) δ: 8.95 (1H, br s), 7.87 (1H, s), 4.60 (1H, br s), 3.60-3.56 (1H, m), 3.43-3.39 (1H, m), 2.96 (4H, t, J = 7.4 Hz), 2.82 (4H, t, J = 7.3 Hz), 2.12-2.07 (8H, m). (-NH) | 519 |
| 41 | ¹H-NMR (DMSO-D₆+TFA) δ: 11.14 (1H, s), 9.43 (1H, s), 8.30 (1H, s), 4.26-4.22 (1H, m), 3.54-3.44 (3H, m), 3.14 (1H, dd, J = 10.1, 1.7 Hz), 2.86 (4H, t, J = 7.4 Hz), 2.77 (4H, t, J = 7.4 Hz), 2.03-2.00 (4H, m), 1.89-1.69 (2H, m). (-NH) | 467 |
| 42 | ¹H-NMR (DMSO-D₆) δ: 9.43 (1H, br s), 8.29 (1H, br s), 3.71 (4H, br s), 3.23 (4H, br s), 2.86 (4H, t, J = 7.3 Hz), 2.77 (4H, t, J = 7.4 Hz), 2.03-2.00 (4H, m). (-NH) | 515 |
| 43 | ¹H-NMR (DMSO-D₆) δ: 11.37 (1H, br s), 9.39 (1H, s), 8.24 (1H, br s), 3.80 (2H, d, J = 12.5 Hz), 3.21 (1H, t, J = 11.9 Hz), 2.91 (3H, s), 2.87-2.85 (6H, m), 2.77 (4H, t, J = 7.4 Hz), 2.07-2.00 (6H, m), 1.61-1.55 (2H, m). | 543 |
| 44 | ¹H-NMR (CDCl₃+TFA) δ: 7.87 (1H, s), 7.20 (1H, br s), 4.14 (1H, br s), 3.49 (2H, br s), 2.96 (4H, t, J = 7.3 Hz), 2.83 (4H, t, J = 7.4 Hz), 2.16-2.08 (4H, m), 1.97-1.85 (2H, m), 1.59-1.57 (1H, m), 1.26-1.23 (4H, m). | 465 |
| 45 | ¹H-NMR (CDCl₃+TFA) δ: 7.87 (1H, s), 7.20 (1H, br s), 4.15-4.13 (1H, m), 3.50-3.47 (2H, m), 2.96 (4H, t, J = 7.3 Hz), 2.83 (4H, t, J = 7.4 Hz), 2.16-2.08 (4H, m), 1.97-1.85 (2H, m), 1.59-1.57 (1H, m), 1.26-1.23 (4H, m). (-NH) | 465 |
| 46 | ¹H-NMR (DMSO-D₆) δ: 9.36 (1H, br s), 8.19 (1H, br s), 7.22 (1H, s), 5.99 (1H, s), 4.02-4.00 (2H, m), 3.90-3.88 (2H, m), 2.85 (4H, t, J = 7.2 Hz), 2.74 (4H, t, J = 7.6 Hz), 2.05-1.99 (6H, m). (-NH) | 503 |
| 47 | ¹H-NMR (DMSO-D₆) δ: 11.42 (1H, br s), 9.44 (1H, s), 8.32 (1H, s), 4.00-3.94 (1H, m), 3.85-3.82 (1H, m), 3.63 (1H, dd, J = 10.9, 6.2 Hz), 3.56-3.53 (1H, m), 3.48-3.45 (1H, m), 3.00 (3H, s), 2.86 (4H, t, J = 7.4 Hz), 2.77 (4H, t, J = 7.4 Hz), 2.21 (2H, q, J = 7.1 Hz), 2.05-1.98 (4H, m). | 529 |
| 48 | ¹H-NMR (DMSO-D₆) δ: 9.46 (1H, s), 8.33 (1H, s), 7.23 (1H, t, J = 6.6 Hz), 4.21 (2H, dd, J = 13.5, 11.4 Hz), 3.84 (1H, d, J = 12.0 Hz), 3.50 (1H, dd, J = 18.5, 8.3 Hz), 3.27-3.13 (2H, m), 3.00 (1H, dd, J = 12.1, 3.6 Hz), 2.86 (4H, t, J = 7.4 Hz), 2.77 (4H, t, J = 7.4 Hz), 2.05-1.97 (4H, m), 1.78-1.72 (1H, m), 1.32-1.19 (1H, m), 0.93-0.74 (1H, m). | 493 |
| 49 | ¹H-NMR (DMSO-D₆) δ: 11.53 (1H, br s), 9.43 (1H, s), 8.31 (1H, s), 3.81 (2H, t, J = 13.1 Hz), 3.61 (2H, t, J = 7.3 Hz), 2.86 (4H, t, J = 7.4 Hz), 2.77 (4H, t, J = 7.5 Hz), 2.45-2.34 (2H, m), 2.05-2.00 (4H, m). | 487 |
| 50 | ¹H-NMR (DMSO-D₆) δ: 11.23 (1H, br s), 9.43 (1H, s), 8.27 (1H, s), 4.19-4.19 (2H, m), 2.86 (4H, t, J = 7.3 Hz), 2.77 (4H, t, J = 7.5 Hz), 2.07-1.96 (4H, m), 1.76-1.75 (4H, m), 1.42-1.40 (4H, m). | 477 |
| 51 | ¹H-NMR (DMSO-D₆) δ: 11.33 (1H, br s), 9.39 (1H, s), 8.24 (1H, br s), 4.33 (2H, br s), 3.23 (2H, d, J = 11.8 Hz), 3.04 (2H, d, J = 10.6 Hz), 2.86 (4H, t, J = 7.4 Hz), 2.77 (4H, t, J = 7.4 Hz), 2.03-2.01 (4H, m), 1.78-1.70 (4H, m). | 493 |
| 52 | ¹H-NMR (CDCl₃+TFA) δ: 9.62 (1H, br s), 7.86 (1H, s), 4.59 (1H, s), 3.91 (1H, d, J = 11.6 Hz), 3.65-3.58 (3H, m), 2.93 (4H, t, J = 7.4 Hz), 2.77 (4H, t, J = 7.4 Hz), 2.16-2.11 (6H, m), 1.34-1.16 (1H, m). (-NH) | 451 |
| 53 | ¹H-NMR (CDCl₃+TFA) δ: 8.80 (1H, br s), 7.86 (1H, s), 3.75 (4H, s), 3.38 (4H, s), 2.94 (4H, t, J = 7.3 Hz), 2.77 (4H, t, J = 7.3 Hz), 2.16-2.09 (4H, m). (-NH) | 451 |
| 54 | ¹H-NMR (DMSO-D₆+TFA) δ: 11.41 (1H, s), 9.44 (1H, s), 3.48-3.47 (4H, m), 3.27-3.25 (2H, m), 3.20-3.19 (2H, m), 2.86 (4H, t, J = 7.4 Hz), 2.77 (4H, t, J = 7.4 Hz), 2.03-2.02 (4H, m), 1.98 (3H, s). (-NH) | 508 |
| 55 | ¹H-NMR (DMSO-D₆+TFA) δ: 11.49 (1H, br s), 9.45 (1H, s), 3.35-3.33 (4H, m), 3.21-3.18 (4H, m), 2.89-2.85 (7H, m), 2.78-2.76 (4H, m), 2.03-2.00 (4H, m). (-NH) | 544 |
| 56 | ¹H-NMR (DMSO-D₆) δ: 11.33-11.30 (1H, br m), 9.43 (1H, s), 8.28 (1H, br s), 4.57 (1H, s), 4.49 (1H, s), 3.74 (1H, d, J = 7.6 Hz), 3.63 (1H, dd, J = 7.6, 1.6 Hz), 3.49 (1H, d, J = 8.3 Hz), 3.28 (1H, br s), 2.86 (4H, t, J = 7.3 Hz), 2.77 (4H, t, J = 7.3 Hz), 2.07-2.00 (4H, m), 1.73 (1H, d, J = 9.5 Hz), 1.64 (1H, d, J = 9.2 Hz). | 479 |
| 57 | ¹H-NMR (DMSO-D₆) δ: 11.57 (1H, br s), 9.40 (1H, s), 8.26 (1H, br s), 4.40 (2H, d, J = 6.9 Hz), 4.32 (2H, d, J = 7.2 Hz), 3.61-3.60 (2H, m), 3.41 (2H, br s), 3.17 (2H, br s), 2.86 (4H, t, J = 7.4 Hz), 2.77 (4H, t, J = 7.4 Hz), 2.07-1.97 (4H, m). | 509 |
| 58 | ¹H-NMR (DMSO-D₆) δ: 11.36 (1H, br s), 9.31 (1H, s), 8.25 (1H, br s), 3.82-3.79 (2H, m), 3.22-3.19 (1H, m), 2.91 (3H, s), 2.89-2.83 (6H, m), 2.72 (4H, t, J = 7.3 Hz), 2.08-1.99 (6H, m), 1.59-1.56 (2H, m). | 527 |
| 59 | ¹H-NMR (DMSO-D₆) δ: 11.32 (1H, br s), 9.34 (1H, s), 8.29 (1H, s), 4.34 (2H, br s), 3.26 (2H, d, J = 11.3 Hz), 3.06 (2H, dd, J = 11.9, 2.0 Hz), 2.85 (4H, t, J = 7.3 Hz), 2.72 (4H, t, J = 7.3 Hz), 2.06-2.00 (4H, m), 1.78-1.72 (4H, m). | 477 |
| 60 | ¹H-NMR (DMSO-D₆) δ: 11.23 (1H, br s), 9.33 (1H, s), 8.29 (1H, s), 3.68 (2H, dd, J = 8.9, 6.6 Hz), 3.58 (2H, dd, J = 10.3, 7.7 Hz), 3.47 (2H, dd, J = 9.0, 3.2 Hz), 3.14 (2H, dd, J = 10.3, 3.8 Hz), 2.87-2.85 (6H, m), 2.72 (4H, t, J = 7.3 Hz), 2.06-2.00 (4H, m). | 477 |
| 61 | ¹H-NMR (DMSO-D₆) δ: 11.25 (1H, br s), 9.41 (1H, s), 8.29 (1H, s), 3.68 (2H, dd, J = 8.9, 6.6 Hz), 3.57 (2H, dd, J = 10.1, 7.5 Hz), 3.47 (2H, dd, J = 9.0, 3.5 Hz), 3.13 (2H, dd, J = 10.4, 3.7 Hz), 2.87-2.85 (6H, m), 2.77 (4H, t, J = 7.4 Hz), 2.07-1.97 (4H, m). | 493 |
| 62 | ¹H-NMR (DMSO-D₆) δ: 11.25 (1H, br s), 9.23 (1H, br s), 8.18 (1H, br s), 7.81 (1H, d, J = 7.6 Hz), 3.58-3.55 (2H, m), 2.85-2.84 (5H, m), 2.72 (4H, t, J = 7.3 Hz), 2.06-1.99 (4H, m), 1.76-1.72 (4H, m), 1.35-1.26 (5H, m). | 506 |
| 63 | ¹H-NMR (DMSO-D₆+TFA) δ: 11.22 (1H, s), 9.33 (1H, s), 3.68 (2H, d, J = 12.7 Hz), 2.98-2.70 (19H, m), 2.04-2.00 (4H, m), 1.68-1.65 (1H, m), 1.52-1.49 (1H, m). (-NH) | 520 |
| 64 | ¹H-NMR (DMSO-D₆+TFA) δ: 9.35 (1H, s), 3.87 (2H, s), 3.54 (2H, t, J = 5.4 Hz), 3.32 (2H, t, J = 5.5 Hz), 2.85 (4H, t, J = 6.5 Hz), 2.80 (3H, s), 2.72 (4H, t, J = 7.3 Hz), 2.06-1.99 (4H, m).(-2NH) | 478 |
| 65 | ¹H-NMR (CDCl₃) δ: 8.93 (1H, br s), 7.83 (1H, s), 6.15 (1H, s), 3.94 (2H, t, J = 7.6 Hz), 3.90 (2H, s), 2.98 (4H, t, J = 7.5 Hz), 2.83 (4H, t, J = 7.4 Hz), 2.63 (2H, t, J = 7.7 Hz), 2.16-2.09 (4H, m). | 465 |
| 66 | ¹H-NMR (CDCl₃+TFA) δ: 9.64 (1H, br s), 7.93 (1H, s), 3.96 (2H, s), 3.02-2.83 (16H, m), 2.10-1.99 (9H, m). | 556 |
| 67 | ¹H-NMR (DMSO-D₆) δ: 11.52 (1H, br s), 9.28 (1H, br s), 8.24 (1H, br s), 4.64 (2H, br s), 4.57 (2H, br s), 4.25 (2H, q, J = 7.1 Hz), 4.04 (3H, s), 2.83 (4H, t, J = 7.4 Hz), 2.70 (4H, t, J = 7.6 Hz), 2.05-1.99 (4H, m), 1.27 (3H, t, J = 7.1 Hz). | 559 |
| 68 | ¹H-NMR (DMSO-D₆) δ: 11.36 (1H, br s), 9.54 (1H, br s), 8.28 (1H, s), 7.42 (1H, d, J = 8.1 Hz), 7.31 (1H, d, J = 8.6 Hz), 2.77 (6H, br s), 2.34 (3H, s) (-NH). | 393 |
| 69 | ¹H-NMR (DMSO-D₆) δ: 11.19 (1H, br s), 9.22 (1H, s), 8.24 (1H, s), 7.01 (2H, s), 2.84 (2H, t, J = 7.5 Hz), 2.80 (6H, s), 2.68 (2H, t, J = 7.5 Hz), 2.14 (3H, s), 1.99-1.92 (2H, m). | 365 |
| 70 | ¹H-NMR (DMSO-D₆) δ: 11.54 (1H, br s), 9.67 (1H, s), 8.45 (1H, s), 8.21 (1H, s), 7.43 (1H, d, J = 8.3 Hz), 7.34 (1H, d, J = 7.9 Hz), 3.63-3.61 (4H, m), 3.25-3.24 (4H, m), 2.34 (3H, s). | 435 |
| 71 | ¹H-NMR (DMSO-D₆) δ: 11.49 (1H, br s), 9.35 (1H, s), 8.30 (1H, s), 3.19 (8H, br s), 2.85 (4H, t, J = 7.3 Hz), 2.71 (4H, t, J = 7.3 Hz), 2.06-1.99 (4H, m), 1.23-1.22 (1H, m), 0.77 (4H, br s). | 490 |
| 72 | ¹H-NMR (DMSO-D₆) δ: 11.45 (1H, br s), 9.45 (1H, s), 8.30 (1H, s), 4.51 (1H, d, J = 3.2 Hz), 4.13 (1H, d, J = 12.0 Hz), 3.80-3.79 (1H, m), 3.65 (3H, s), 3.56 (2H, dd, J = 11.8, 3.7 Hz), 3.44-3.39 (1H, m), 3.30 (1H, td, J = 11.6, 2.9 Hz), 2.86 (4H, t, J = 7.4 Hz), 2.77 (4H, t, J = 7.4 Hz), 2.04-2.02 (4H, m). | 525 |
| 73 | ¹H-NMR (DMSO-D₆) δ: 11.51 (1H, br s), 9.45 (1H, s), 8.31 (1H, s), 4.15-4.12 (1H, br m), 3.75 (1H, d, J = 8.1 Hz), 3.64 (1H, d, J = 11.6 Hz), 3.57 (3H, s), 3.54 (1H, d, J = 9.5 Hz), 3.45-3.42 (1H, m), 3.31-3.26 (2H, m), 2.91-2.86 (5H, m), 2.77 (4H, t, J = 7.5 Hz), 2.60 (1H, dd, J = 15.3, 5.5 Hz), 2.05-1.98 (4H, m). | 539 |
| 74 | ¹H-NMR (DMSO-D₆) δ: 11.48 (1H, br s), 10.76 (1H, br s), 9.36 (1H, s), 8.32 (1H, s), 3.91 (1H, dd, J = 9.9, 7.9 Hz), 3.83-3.81 (1H, m), 3.60-3.49 (2H, m), 3.37 (1H, dd, J = 17.1, 9.2 Hz), 2.85 (4H, t, J = 7.3 Hz), 2.73-2.71 (10H, m), 2.31-2.27 (1H, m), 2.18-2.10 (1H, m), 2.06-2.01 (4H, m). | 478 |
| 75 | ¹H-NMR (DMSO-D₆) δ: 11.54 (1H, br s), 9.67 (1H, s), 8.45 (1H, s), 8.22 (1H, s), 7.43-7.42 (1H, m), 7.36-7.33 (1H, m), 3.79 (2H, d, J = 12.7 Hz), 3.43-3.40 (2H, m), 2.96 (2H, dd, J = 12.6, 10.3 Hz), 2.80 (6H, s), 2.34 (3H, s), 2.01-1.99 (1H, m), 1.60 (2H, ddd, J = 25.0, 12.6, 4.3 Hz). | 540 |
| 76 | ¹H-NMR (DMSO-D₆) δ: 11.33 (1H, s), 9.66 (1H, s), 8.45 (1H, s), 8.21 (1H, s), 7.43 (1H, d, J = 8.1 Hz), 7.34 (1H, d, J = 7.6 Hz), 3.39-3.38 (4H, m), 2.33 (3H, s), 1.82-1.78 (4H, m). | 419 |
| 77 | ¹H-NMR (DMSO-D₆) δ: 11.49 (1H, br s), 9.66 (1H, s), 8.43 (1H, s), 8.21 (1H, s), 7.43 (1H, d, J = 7.9 Hz), 7.34 (1H, d, J = 7.9 Hz), 4.36 (2H, br s), 3.30-3.27 (2H, m), 3.11-3.10 (2H, m), 2.33 (3H, s), 1.81-1.74 (4H, m). | 461 |

**[Table 5]**

| Example | NMR | MS (M+H) |
|---|---|---|
| 2-001 | 1H-NMR (DMSO-D6) δ: 11.43 (1H, s), 9.31 (1H, s), 8.39 (1H, s), 7.42 (1H, d, J = 1.7 Hz), 7.07 (1H, d, J = 7.8 Hz), 6.72 (1H, dd, J = 7.8, 2.0 Hz), 3.66-3.59 (4H, m), 3.29-3.21 (4H, m), 2.18 (3H, s), 1.93-1.84 (1H, m), 0.95-0.88 (2H, m), 0.65-0.59 (2H, m). | 407 |
| 2-002 | 1H-NMR (DMSO-D6) δ: 11.20 (1H, s), 9.29 (1H, s), 8.36 (1H, s), 7.43 (1H, d, J = 1.7 Hz), 7.07 (1H, d, J = 8.1 Hz), 6.71 (1H, dd, J = 7.8, 1.7 Hz), 4.31-4.25 (1H, m), 3.58-3.47 (3H, m), 3.21-3.15 (1H, m), 2.18 (3H, s), 1.92-1.83 (2H, m), 1.80-1.70 (1H, m), 0.95-0.88 (2H, m), 0.65-0.60 (2H, m). | 407 |
| 2-003 | 1H-NMR (DMSO-D6) δ: 11.43 (1H, s), 9.67 (1H, s), 8.44 (1H, s), 8.21 (1H, s), 7.44 (1H, d, J = 7.9 Hz), 7.35 (1H, d, J = 7.9 Hz), 4.13-4.03 (1H, m), 2.80 (3H, s), 2.35 (3H, s), 1.08 (6H, d, J = 6.7 Hz). | 421 |
| 2-004 | 1H-NMR (DMSO-D6) δ: 11.51 (1H, br s), 9.68 (1H, s), 8.45 (1H, s), 8.23 (1H, s), 7.45 (1H, d, J = 8.0 Hz), 7.35 (1H, d, J = 8.0 Hz), 3.71-3.65 (4H, m), 3.55-3.49 (4H, m), 2.35 (3H, s), 1.88-1.80 (2H, m) . | 449 |
| 2-005 | 1H-NMR (CDCl3) δ: 8.99 (1H, br s), 8.17 (1H, s), 7.95 (1H, s), 7.34 (2H, s), 6.68 (1H, s), 4.08-4.01 (1H, m), 3.91-3.82 (1H, m), 3.78-3.50 (5H, m), 1.40 (3H, d, J = 9.7 Hz). | 449 |
| 2-006 | 1H-NMR (CDCl3) δ: 8.88 (1H, br s), 8.16 (1H, s), 7.95 (1H, s), 7.35 (2H, s), 6.68 (1H, s), 4.00-3.85 (1H, m), 3.80-3.58 (3H, m), 3.50-3.32 (1H, m), 3.21-3.09 (1H, m), 2.88-2.76 (1H, m), 2.38 (3H, s), 1.60-1.42 (2H, m), 0.97 (3H, t, J = 6.6 Hz). | 463 |
| 2-007 | 1H-NMR (DMSO-D6) δ: 11.52 (1H, s), 9.68 (1H, s), 8.45 (1H, s), 8.25-8.22 (1H, br m), 7.45 (1H, d, J = 7.9 Hz), 7.35 (1H, d, J = 7.9 Hz), 7.09-7.04 (1H, m), 3.87-3.78 (2H, m), 3.46-3.35 (1H, m), 3.03-2.93 (2H, m), 2.62-2.53 (1H, m), 2.08-2.00 (2H, m), 1.67-1.50 (2H, m), 1.10 (6H, d, J = 6.7 Hz). | 554 |
| 2-008 | 1H-NMR (DMSO-D6) δ: 11.17 (1H, s), 9.34 (1H, s), 8.31 (1H, s), 3.59-3.49 (2H, m), 3.35 (1H, dd, J = 16.2, 9.0 Hz), 2.86 (5H, t, J = 7.5 Hz), 2.73 (4H, t, J = 7.3 Hz), 2.19 (1H, dd, J = 14.9, 7.3 Hz), 2.04 (4H, t, J = 7.3 Hz), 1.95-1.92 (1H, m), 1.46-1.42 (1H, m), 0.96 (3H, d, J = 6.7 Hz). | 448 |
| 2-009 | 1H-NMR (DMSO-D6) δ: 11.18 (1H, s), 9.34 (1H, s), 8.30 (1H, s), 3.53 (2H, t, J = 7.1 Hz), 3.08 (2H, s), 2.86 (4H, t, J = 7.4 Hz), 2.73 (4H, t, J = 7.3 Hz), 2.08-2.00 (4H, m), 1.62 (2H, t, J = 7.1 Hz), 1.01 (6H, s). | 462 |
| 2-010 | 1H-NMR (DMSO-D6) δ: 11.33 (1H, s), 9.54 (1H, s), 8.43 (1H, s), 8.00 (1H, s), 7.34 (1H, d, J = 7.6 Hz), 7.20 (1H, d, J = 8.1 Hz), 6.98 (1H, t, J = 55.9 Hz), 2.85 (6H, s), 2.30 (3H, s). | 375 |
| 2-011 | 1H-NMR (DMSO-D6) δ: 10.74 (s, 1H), 9.53 (s, 1H), 7.69 (s, 1H), 7.01 (s, 1H), 2.85 (s, 6H), 2.84 (t, J = 7.30 Hz, 4H), 2.71 (t, J = 7.28 Hz, 4H), 1.98 (dt, J = 15.10, 7.11 Hz, 4H). | 407 |
| 2-012 | 1H-NMR (DMSO-D6) δ: 10.95 (br s, 1H), 9.53 (s, 1H), 7.70 (s, 1H), 7.01 (s, 1H), 3.63-3.62 (m, 4H), 3.26-3.25 (m, 4H), 2.84 (t, J = 7.28 Hz, 4H), 2.71 (t, J = 7.28 Hz, 4H), 2.02-1.95 (m, 4H). | 449 |
| 2-013 | 1H-NMR (DMSO-D6) δ: 11.29 (1H, s), 9.51 (1H, s), 8.39 (1H, s), 7.92 (1H, s), 7.31 (1H, d, J = 8.3 Hz), 7.20 (1H, d, J = 8.3 Hz), 2.83 (6H, s), 2.27 (3H, s), 1.94 (3H, t, J = 18.8 Hz). | 389 |
| 2-014 | 1H-NMR (DMSO-D6) δ: 11.27 (1H, br s), 9.85 (1H, s), 8.35 (1H, s), 4.39-4.33 (1H, m), 2.82 (6H, s), 2.55 (2H, t, J = 7.2 Hz), 2.45-2.44 (2H, m), 2.30-2.23 (2H, m), 1.29 (6H, d, J = 6.5 Hz). | 383 |
| 2-015 | 1H-NMR (DMSO-D6) δ: 11.47 (1H, s), 9.56 (1H, s), 8.44 (1H, br s), 7.93 (1H, s), 7.33 (1H, d, J = 8.0 Hz), 7.22 (1H, d, J = 8.0 Hz), 3.66-3.60 (4H, m), 3.28-3.22 (4H, m), 2.29 (3H, s), 1.96 (3H, t, J = 19.0 Hz). | 431 |
| 2-016 | 1H-NMR (DMSO-D6) δ: 11.38 (1H, s), 9.67 (1H, s), 8.44 (1H, s), 8.24 (1H, s), 7.44 (1H, d, J = 7.9 Hz), 7.35 (1H, d, J = 7.9 Hz), 3.25 (4H, t, J = 5.1 Hz), 2.35 (3H, s), 1.55-1.49 (6H, m). | 432 |
| 2-017 | 1H-NMR (DMSO-D6) δ: 11.71 (1H, s), 9.70 (1H, s), 8.48 (1H, s), 8.23 (1H, s), 7.45 (1H, d, J = 7.9 Hz), 7.36 (1H, d, J = 6.7 Hz), 4.44 (2H, d, J = 6.9 Hz), 4.36 (2H, d, J = 7.2 Hz), 3.65 (2H, t, J = 4.9 Hz), 3.49 (2H, s), 3.24 (2H, t, J = 4.7 Hz), 2.35 (2H, s). | 476 |
| 2-018 | 1H-NMR (DMSO-D6) δ: 11.57 (1H, s), 9.63 (1H, s), 8.37 (1H, s), 8.26 (1H, s), 7.44 (1H, d, J = 7.9 Hz), 7.34 (1H, d, J = 8.1 Hz), 3.83 (2H, d, J = 12.0 Hz), 3.23 (1H, t, J = 11.8 Hz), 2.93-2.90 (5H, m), 2.35 (3H, s), 2.09 (2H, d, J = 11.3 Hz), 1.61 (2H, ddd, J = 24.9, 12.7, 4.0 Hz). | 510 |
| 2-019 | 1H-NMR (DMSO-D6) δ: 11.35 (1H, s), 9.66 (1H, s), 8.45 (1H, s), 8.22 (1H, s), 7.45 (1H, d, J = 7.9 Hz), 7.35 (1H, d, J = 7.9 Hz), 3.62-3.51 (2H, m), 3.38 (1H, td, J = 9.1, 7.2 Hz), 2.88 (1H, t, J = 8.8 Hz), 2.35 (3H, s), 2.24-2.19 (1H, m), 1.98-1.95 (1H, m), 1.45 (1H, ddd, J = 19.0, 10.5, 6.8 Hz), 0.98 (3H, d, J = 6.7 Hz). | 432 |
| 2-020 | 1H-NMR (DMSO-D6) δ: 11.36 (1H, s), 9.66 (1H, s), 8.43 (1H, s), 8.22 (1H, s), 7.44 (1H, d, J = 7.9 Hz), 7.35 (1H, d, J = 7.9 Hz), 3.54 (2H, t, J = 7.1 Hz), 3.10 (2H, s), 2.35 (3H, s), 1.64 (2H, t, J = 7.1 Hz), 1.03 (6H, s). | 446 |
| 2-021 | 1H-NMR (DMSO-D6) δ: 11.71 (1H, s), 9.68 (1H, s), 8.47 (1H, s), 8.21 (1H, s), 7.45 (1H, d, J = 7.9 Hz), 7.36 (1H, t, J = 4.6 Hz), 3.86 (2H, t, J = 13.1 Hz), 3.66 (2H, t, J = 7.4 Hz), 2.43 (2H, td, J = 14.3, 7.1 Hz), 2.35 (3H, s). | 454 |
| 2-022 | 1H-NMR (DMSO-D6) δ: 11.43 (1H, s), 9.67 (1H, s), 8.44 (1H, s), 8.24 (1H, s), 7.44 (1H, d, J = 7.9 Hz), 7.35 (1H, d, J = 8.1 Hz), 3.67 (2H, d, J = 12.5 Hz), 3.11 (2H, d, J = 6.5 Hz), 2.96-2.93 (5H, m), 2.35 (3H, s), 2.07 (1H, s), 1.92 (2H, d, J = 10.9 Hz), 1.34 (2H, dd, J = 20.9, 11.9 Hz). | 524 |
| 2-023 | 1H-NMR (DMSO-D6) δ: 11.67 (1H, s), 9.69 (1H, s), 8.46 (1H, s), 8.22 (1H, s), 7.45 (1H, d, J = 8.1 Hz), 7.36 (1H, d, J = 8.1 Hz), 3.44 (4H, t, J = 5.8 Hz), 2.35 (3H, s), 2.12-2.02 (4H, m). | 468 |
| 2-024 | 1H-NMR (DMSO-D6) δ: 11.55 (1H, s), 9.63 (1H, s), 8.38 (1H, s), 8.26 (1H, s), 7.44 (1H, d, J = 7.9 Hz), 7.34 (1H, d, J = 8.1 Hz), 3.79 (2H, d, J = 12.7 Hz), 2.91 (2H, t, J = 11.9 Hz), 2.35 (3H, s), 1.88 (2H, d, J = 11.3 Hz), 1.45 (2H, ddd, J = 25.3, 12.7, 4.3 Hz). | 500 |
| 2-025 | 1H-NMR (DMSO-D6) δ: 11.32 (1H, s), 9.68 (1H, s), 8.40 (1H, s), 8.06 (1H, s), 7.54 (1H, d, J = 8.8 Hz), 7.46 (1H, d, J = 7.6 Hz), 3.36-3.34 (1H, m), 2.84 (6H, s), 1.17 (6H, d, J = 9.7 Hz). | 420 |
| 2-026 | 1H-NMR (DMSO-D6) δ: 11.48 (1H, s), 9.69 (1H, s), 8.41 (1H, s), 8.06 (1H, s), 7.54 (1H, d, J = 8.1 Hz), 7.46 (1H, d, J = 8.3 Hz), 3.61 (4H, t, J = 4.7 Hz), 3.35-3.33 (1H, m), 3.23 (4H, t, J = 4.6 Hz), 1.17 (6H, d, J = 6.7 Hz). | 462 |
| 2-027 | 1H-NMR (DMSO-D6) δ: 11.14 (1H, s), 9.44 (1H, s), 8.36 (1H, s), 7.74 (1H, s), 7.24 (1H, s), 3.25-3.22 (1H, m), 2.83 (6H, s), 2.18 (3H, s), 1.18 (6H, d, J = 6.9 Hz). | 400 |
| 2-028 | 1H-NMR (DMSO-D6) δ: 11.32 (1H, s), 9.45 (1H, s), 8.37 (1H, s), 7.74 (1H, s), 7.24 (1H, s), 3.61 (4H, t, J = 4.7 Hz), 3.24-3.21 (5H, m), 2.18 (3H, s), 1.18 (6H, d, J = 6.9 Hz). | 442 |
| 2-029 | 1H-NMR (DMSO-D6) δ: 11.73 (1H, br s), 9.71 (1H, s), 9.01 (2H, br s), 8.48 (1H, s), 8.23 (1H, s), 7.45 (1H, d, J = 7.9 Hz), 7.36 (1H, d, J = 7.9 Hz), 3.78-3.71 (2H, m), 3.51-3.43 (2H, m), 3.26-3.13 (4H, m), 2.35 (3H, s), 2.08-1.98 (2H, m). | 448 |
| 2-030 | 1H-NMR (DMSO-D6) δ: 11.60 (1H, br s), 9.68 (1H, s), 8.45 (1H, s), 8.23 (1H, s), 7.45 (1H, d, J = 8.0 Hz), 7.35 (1H, d, J = 8.0 Hz), 3.54 (2H, t, J = 5.9 Hz), 3.49 (2H, t, J = 5.9 Hz), 3.41 (2H, t, J = 5.9 Hz), 3.36 (2H, t, J = 5.9 Hz), 2.93 (3H, s), 2.35 (3H, s), 1.86-1.78 (2H, m). | 526 |
| 2-031 | 1H-NMR (DMSO-D6) δ: 11.42 (1H, s), 9.54 (1H, s), 8.41 (1H, s), 7.93 (1H, s), 7.33 (1H, d, J = 7.9 Hz), 7.22 (1H, d, J = 7.9 Hz), 3.71-3.64 (4H, m), 3.55-3.48 (4H, m), 2.29 (3H, s), 1.96 (3H, t, J = 18.7 Hz), 1.83 (2H, tt, J = 5.7, 5.7 Hz). | 445 |
| 2-032 | 1H-NMR (DMSO-D6) δ: 11.57 (1H, br s), 9.67 (1H, s), 8.44 (1H, s), 8.24 (1H, s), 7.44 (1H, d, J = 8.1 Hz), 7.35 (1H, d, J = 8.1 Hz), 3.62-3.37 (8H, m), 2.35 (3H, s), 2.01 (1.5H, s), 1.99 (1.5H, s), 1.81 (1H, tt, J = 5.6, 5.6 Hz), 1.70 (1H, tt, J = 5.6, 5.6 Hz). | 490 |
| 2-033 | 1H-NMR (DMSO-D6) δ: 11.45 (1H, br s), 9.68 (1H, s), 8.45 (1H, s), 8.23 (1H, s), 7.87-7.82 (1H, m), 7.45 (1H, d, J = 8.0 Hz), 7.36 (1H, d, J = 8.0 Hz), 3.72-3.60 (3H, m), 3.09-2.97 (2H, m), 2.35 (3H, s), 1.84-1.73 (2H, m), 1.78 (3H, s), 1.46-1.32 (2H, m). | 490 |
| 2-034 | 1H-NMR (DMSO-D6) δ: 11.30 (1H, s), 9.66 (1H, br s), 8.47 (1H, s), 8.29 (1H, br s), 7.49 (1H, dd, J = 8.3,2.1 Hz), 7.42 (1H, d, J = 7.9 Hz), 2.77 (6H, s), 2.32 (3H, s). | 451 |
| 2-035 | 1H-NMR (DMSO-D6) δ: 11.27 (1H, s), 9.30 (1H, s), 8.38 (1H, s), 7.43 (1H, d, J = 1.8 Hz), 7.07 (1H, d, J = 8.1 Hz), 6.71 (1H, dd, J = 7.8, 1.8 Hz), 2.86 (6H, s), 2.18 (3H, s), 1.92-1.85 (1H, m), 0.95-0.89 (2H, m), 0.65-0.59 (2H, m). | 365 |
| 2-036 | 1H-NMR (DMSO-D6) δ: 11.22 (1H, s), 9.33 (1H, s), 8.37 (1H, s), 7.49 (1H, s), 7.13 (1H, d, J = 7.8 Hz), 6.94 (1H, d, J = 7.5 Hz), 3.52-3.44 (1H, m), 2.85 (6H, s), 2.32-2.24 (2H, m), 2.20 (3H, s), 2.11-1.91 (3H, m), 1.83-1.76 (1H, m). | 379 |
| 2-037 | 1H-NMR (DMSO-D6) δ: 11.34 (1H, s), 9.68 (1H, s), 8.43 (1H, s), 8.24 (1H, s), 8.03 (1H, d, J = 7.8 Hz), 7.44 (1H, d, J = 7.8 Hz), 7.35 (1H, d, J = 7.8 Hz), 3.86-3.73 (3H, m), 3.46-3.19 (2H, m), 2.35 (3H, s), 1.76-1.64 (2H, m), 1.56-1.40 (2H, m). | 449 |
| 2-038 | 1H-NMR (DMSO-D6) δ: 11.40 (1H, s), 9.62 (1H, s), 8.39 (1H, s), 8.21 (1H, s), 7.42 (1H, d, J = 7.9 Hz), 7.33 (1H, d, J = 6.9 Hz), 4.18 (1H, s), 3.41-3.37 (2H, m), 3.27-3.24 (5H, br m), 2.33 (3H, s), 1.86-1.74 (4H, m). | 462 |
| 2-039 | 1H-NMR (DMSO-D6) δ: 11.20 (1H, br s), 9.57 (1H, s), 8.29 (1H, s), 7.50 (1H, s), 4.32-4.29 (1H, m), 2.82 (6H, s), 1.84 (3H, s), 1.35 (6H, d, J = 6.7 Hz). | 357 |
| 2-040 | 1H-NMR (DMSO-D6) δ: 11.21 (1H, s), 9.28 (1H, s), 8.32 (1H, s), 7.48 (1H, d, J = 9.5 Hz), 7.10 (1H, d, J = 7.6 Hz), 6.91 (1H, d, J = 7.9 Hz), 2.88-2.81 (7H, m), 2.17 (3H, s), 1.17 (6H, d, J = 6.9 Hz). | 366 |
| 2-041 | 1H-NMR (DMSO-D6) δ: 11.45 (1H, s), 10.01 (1H, s), 8.58 (1H, s), 8.48 (1H, s), 7.70 (1H, s), 2.84 (6H, s), 2.43 (3H, s). | 460 |
| 2-042 | 1H-NMR (DMSO-D6) δ: 11.39 (1H, s), 9.30 (1H, s), 8.37 (1H, s), 7.43 (1H, d, J = 1.8 Hz), 7.07 (1H, d, J = 7.8 Hz), 6.71 (1H, dd, J = 7.8, 1.8 Hz), 3.70 - 3.64 (4H, m), 3.54-3.48 (4H, m), 2.18 (3H, s), 1.92-1.80 (3H, m), 0.95-0.89 (2H, m), 0.65-0.59 (2H, m). | 421 |
| 2-043 | 1H-NMR (DMSO-D6) δ: 11.47 (1H, s), 9.56 (1H, s), 8.43 (1H, s), 8.01 (1H, s), 7.36 (1H, d, J = 7.8 Hz), 7.22 (1H, d, J = 7.8 Hz), 7.00 (1H, t, J = 55.9 Hz), 3.69-3.64 (4H, m), 3.52 (4H, dd, J = 10.8, 5.5 Hz), 2.31 (3H, s), 1.86-1.81 (2H, m). | 431 |
| 2-044 | 1H-NMR (DMSO-D6) δ: 11.20 (1H, s), 9.34 (1H, s), 8.37 (1H, s), 7.48 (1H, d, J = 2.0 Hz), 7.10 (1H, d, J = 8.0 Hz), 6.89 (1H, dd, J = 7.9, 1.9 Hz), 2.85 (6H, s), 2.18 (3H, s), 1.36 (3H, s), 0.81 - 0.71 (4H, m). | 379 |
| 2-045 | 1H-NMR (DMSO-D6) δ: 11.58 (1H, s), 9.70 (1H, s), 8.46 (1H, s), 8.23 (1H, s), 7.45 (1H, d, J = 8.0 Hz), 7.36 (1H, d, J = 7.8 Hz), 5.08 (1H, s), 3.91 (1H, d, J = 11.5 Hz), 3.79-3.64 (3H, m), 3.59-3.48 (2H, m), 3.40-3.24 (3H, m), 2.35 (3H, s). | 465 |
| 2-046 | 1H-NMR (DMSO-D6) δ: 11.29 (1H, s), 9.68 (1H, s), 8.44 (1H, s), 8.25 (1H, d, J = 1.3 Hz), 7.80 (1H, d, J = 7.4 Hz), 7.44 (1H, d, J = 7.9 Hz), 7.35 (1H, dd, J = 7.9, 1.3 Hz), 3.53-3.44 (1H, m), 2.35 (3H, s), 1.08 (6H, d, J = 6.7 Hz). | 407 |
| 2 - 047 | 1H-NMR (DMSO-D6) δ: 11.34 (1H, br s), 9.32 (1H, s), 8.36 (1H, s), 7.49 (1H, d, J = 1.6 Hz), 7.12 (1H, d, J = 7.6 Hz), 6.93 (1H, dd, J = 7.6, 1.6 Hz), 3.70-3.63 (4H, m), 3.55-3.47 (4H, m), 2.89-2.81 (1H, m), 2.19 (3H, s), 1.87-1.79 (2H, m), 1.19 (6H, d, J = 6.9 Hz). | 423 |
| 2-048 | 1H-NMR (DMSO-D6) δ: 11.42 (1H, s), 9.68 (1H, s), 8.44 (1H, s), 8.21 (1H, s), 7.44 (1H, d, J = 7.9 Hz), 7.35 (1H, d, J = 7.9 Hz), 4.07 - 3.98 (1H, m), 3.49-3.42 (4H, m), 3.24 (3H, s), 2.35 (3H, s), 1.10 (6H, d, J = 6.8 Hz). | 465 |
| 2 - 049 | 1H-NMR (CDCl3) δ: 8.87 (1H, s), 8.16 (1H, s), 7.95 (1H, s), 7.35 (2H, s), 6.68 (1H, s), 4.04-3.96 (1H, m), 3.83-3.64 (4H, m), 3.49-3.43 (2H, m), 3.37 (3H, s), 3.25-3.15 (1H, m), 3.06-2.97 (1H, m), 2.38 (3H, s). | 479 |
| 2-050 | 1H-NMR (DMSO-D6) δ: 11.61 (1H, br s), 9.68 (1H, s), 8.45 (1H, s), 8.22 (1H, s), 7.70 (1H, t, J = 5.3 Hz), 7.44 (1H, d, J = 8.0 Hz), 7.35 (1H, d, J = 8.0 Hz), 3.47-3.41 (4H, m), 3.26-3.20 (2H, m), 2.57-2.52 (2H, m), 2.35 (3H, s). | 462 |
| 2-051 | 1H-NMR (DMSO-D6) δ: 11.08 (1H, s), 9.68 (1H, s), 8.42 (1H, s), 8.23 (1H, s), 7.44 (1H, d, J = 8.1 Hz), 7.34 (1H, d, J = 9.0 Hz), 3.98-3.92 (2H, m), 2.35 (3H, s), 1.23 (12H, d, J = 6.9 Hz). | 448 |
| 2-052 | 1H-NMR (DMSO-D6) δ: 11.43 (1H, s), 9.65 (1H, s), 8.42 (1H, s), 8.23 (1H, s), 7.44 (1H, d, J = 8.1 Hz), 7.35 (1H, d, J = 7.9 Hz), 4.23-4.17 (1H, m), 3.44-3.38 (2H, m), 3.32-3.27 (5H, m), 2.35 (3H, s), 1.87-1.77 (4H, m). | 462 |
| 2-053 | 1H-NMR (DMSO-D6) δ: 11.34 (1H, s), 9.67 (1H, s), 8.43 (1H, s), 8.20 (1H, s), 7.45 (1H, d, J = 8.1 Hz), 7.35 (1H, d, J = 7.9 Hz), 4.14 (2H, t, J = 6.2 Hz), 2.35 (3H, s), 1.72-1.68 (1H, m), 1.53-1.47 (4H, m), 1.37 (1H, dt, J = 13.3, 3.7 Hz), 1.28 (6H, d, J = 7.2 Hz). | 460 |
| 2-054 | 1H-NMR (DMSO-D6) δ: 10.59 (br s, 1H), 9.54 (br s, 1H), 7.76 (s, 1H), 7.67 (d, J = 1.62 Hz, 1H), 7.15 (d, J = 7.86 Hz, 1H), 6.94 (dd, J = 7.63, 1.85 Hz, 1H), 2.87 (s, 6H), 2.85 (sept, J = 6.94 Hz, 1H), 2.21 (s, 3H), 1.19 (d, J = 7.05 Hz, 8H). | 383 |
| 2-055 | 1H-NMR (DMSO-D6) δ: 10.81 (s, 1H), 9.54 (s, 1H), 7.77 (s, 1H), 7.67 (d, J = 1.85 Hz, 1H), 7.15 (d, J = 8.09 Hz, 1H), 6.94 (dd, J = 7.63, 1.62 Hz, 1H), 3.67-3.61 (m, 4H), 3.30-3.24 (m, 4H), 2.85 (sept, J = 6.47, 1H), 2.22 (s, 3H), 1.19 (d, J = 6.47 Hz, 6H). | 425 |
| 2-056 | 1H-NMR (DMSO-D6) δ: 10.75 (s, 1H), 9.51 (s, 1H), 7.73 (s, 1H), 7.65 (d, J = 1.62 Hz, 1H), 7.13 (d, J = 7.86 Hz, 1H), 6.92 (dd, J = 7.74, 1.73 Hz, 1H), 3.66-3.65 (m, 4H), 3.54-3.50 (m, 4H), 2.84 (sept, J = 6.94 Hz, 1H), 2.19 (s, 3H), 1.85-1.79 (m, 2H), 1.18 (d, J = 6.94 Hz, 6H). | 439 |
| 2-057 | 1H-NMR (DMSO-D6) δ: 11.53 (1H, br s), 9.55 (1H, br s), 8.34-8.21 (2H, m), 7.48-7.40 (1H, m), 7.38-7.29 (1H, m), 4.14-3.97 (3H, m), 3.89-3.75 (2H, m), 3.16 (3H, s), 2.35 (3H, s). | 435 |
| 2-058 | 1H-NMR (CDCl3) δ: 9.05 (1H, s), 8.17 (1H, s), 7.94 (1H, s), 7.37-7.31 (2H, m), 6.66 (1H, s), 4.37-4.28 (1H, m), 4.14-4.06 (1H, m), 3.98-3.82 (2H, m), 3.69-3.58 (2H, m), 3.42-3.33 (1H, m), 2.38 (3H, s), 2.28-2.17 (1H, m), 1.80-1.68 (1H, m), 1.23 (3H, d, J = 6.5 Hz). | 463 |
| 2-059 | 1H-NMR (DMSO-D6) δ: 11.48 (1H, br s), 9.66 (1H, s), 8.40 (1H, s), 8.19 (1H, s), 7.45 (1H, d, J = 7.9 Hz), 7.39 (1H, d, J = 7.6 Hz), 3.67-3.64 (4H, m), 3.50-3.48 (4H, m), 2.74 (2H, q, J = 7.5 Hz), 1.84-1.79 (2H, m), 1.14 (3H, t, J = 7.5 Hz). | 463 |
| 2-060 | 1H-NMR (DMSO-D6) δ: 11.38 (1H, br s), 9.62 (1H, br s), 8.36 (1H, br s), 8.22 (1H, s), 7.44 (1H, d, J = 8.1 Hz), 7.38 (1H, d, J = 8.1 Hz), 2.81 (6H, s), 2.74 (2H, q, J = 7.5 Hz), 1.14 (3H, t, J = 7.5 Hz). | 407 |
| 2-061 | 1H-NMR (DMSO-D6) δ: 11.22 (1H, s), 9.30 (1H, s), 8.27 (1H, s), 7.59 (1H, s), 7.18 (1H, d, J = 7.8 Hz), 6.96 (1H, d, J = 8.0 Hz), 4.27 (1H, q, J = 6.3 Hz), 3.12 (3H, s), 2.80 (6H, s), 2.22 (3H, s), 1.32 (3H, d, J = 6.5 Hz). | 383 |
| 2-062 | 1H-NMR (DMSO-D6) δ: 10.86 (s, 1H), 9.74 (s, 1H), 8.51 (d, J = 1.39 Hz, 1H), 7.90 (s, 1H), 7.43 (d, J = 7.86 Hz, 1H), 7.31 (dd, J = 7.86, 1.39 Hz, 1H), 2.86 (s, 6H), 2.35 (s, 3H). | 409 |
| 2-063 | 1H-NMR (DMSO-D6) δ: 11.29 (1H, s), 9.40 (1H, s), 8.34 (1H, s), 7.67 (1H, s), 7.20 (1H, d, J = 7.9 Hz), 7.01 (1H, d, J = 8.1 Hz), 3.64-3.58 (6H, m), 3.50-3.47 (4H, m), 2.22 (3H, s), 1.84-1.78 (2H, m). | 463 |
| 2-064 | 1H-NMR (CDCl3) δ: 8.97 (1H, br s), 8.16 (1H, s), 7.94 (1H, s), 7.38-7.30 (2H, m), 6.68 (1H, br s), 3.74-3.64 (4H, m), 3.57-3.47 (4H, m), 3.03 (2H, q, J = 7.4 Hz), 2.38 (3H, s), 2.05 (2H, tt, J = 6.0, 6.0 Hz), 1.36 (3H, t, J = 7.4 Hz). | 540 |
| 2-065 | 1H-NMR (DMSO-D6) δ: 11.06 (s, 1H), 9.74 (s, 1H), 8.52 (d, J = 1.39 Hz, 1H), 7.91 (s, 1H), 7.43 (d, J = 7.86 Hz, 1H), 7.31 (dd, J = 7.86, 1.39 Hz, 1H), 3.63-3.61 (m, 4H), 3.26-3.25 (m, 4H), 2.35 (s, 3H). | 451 |
| 2-066 | 1H-NMR (DMSO-D6) δ: 11.02 (s, 1H), 9.73 (s, 1H), 8.51 (s, 1H), 7.89 (s, 1H), 7.43 (d, J = 7.86 Hz, 1H), 7.31 (d, J = 7.86 Hz, 1H), 3.66-3.65 (m, 4H), 3.53-3.50 (m, 4H), 2.34 (s, 3H), 1.85-1.79 (m, 2H). | 465 |
| 2-067 | 1H-NMR (DMSO-D6) δ: 11.39 (1H, s), 9.75 (1H, s), 8.45 (1H, s), 8.23 (1H, s), 7.33 (1H, d, J = 8.3 Hz), 7.19 (1H, d, J = 8.1 Hz), 2.85 (6H, s), 2.14-2.10 (1H, m), 1.01-0.98 (2H, m), 0.68-0.67 (2H, m). | 418 |
| 2-068 | 1H-NMR (DMSO-D6) δ: 11.31 (1H, s), 9.27 (1H, s), 8.35 (1H, s), 7.49 (1H, s), 7.07 (1H, d, J = 7.6 Hz), 6.83 (1H, d, J = 6.5 Hz), 2.84 (6H, s), 2.26 (3H, s), 2.18 (3H, s). | 338 |
| 2-069 | 1H-NMR (DMSO-D6) δ: 11.12 (1H, s), 9.33 (1H, s), 8.34 (1H, s), 7.61 (1H, s), 7.12 (1H, d, J = 7.9 Hz), 7.06 (1H, d, J = 7.9 Hz), 2.83 (6H, s), 2.17 (3H, s), 1.25 (9H, s). | 381 |
| 2-070 | 1H-NMR (DMSO-D6) δ: 11.17 (1H, s), 9.30 (1H, s), 8.31 (1H, s), 7.31 (1H, s), 7.23 (1H, d, J = 7.9 Hz), 7.05 (1H, d, J = 7.9 Hz), 2.85-2.82 (7H, m), 1.17 (6H, d, J = 6.9 Hz), 1.11 (6H, d, J = 6.7 Hz). | 394 |
| 2-071 | 1H-NMR (DMSO-D6) δ: 11.28 (1H, s), 9.30 (1H, s), 8.30 (1H, s), 7.31 (1H, d, J = 1.8 Hz), 7.23 (1H, d, J = 8.1 Hz), 7.05 (1H, dd, J = 8.1, 1.8 Hz), 3.65 (4H, dd, J = 9.1, 3.6 Hz), 3.48 (4H, dd, J = 10.6, 5.1 Hz), 3.18-3.11 (1H, m), 2.87-2.80 (1H, m), 1.83-1.78 (2H, m), 1.17 (6H, d, J = 6.9 Hz), 1.11 (6H, d, J = 6.9 Hz). | 450 |
| 2-072 | 1H-NMR (DMSO-D6) δ: 11.45 (1H, s), 9.69 (1H, s), 8.40 (1H, s), 8.05 (1H, s), 7.54 (1H, d, J = 8.1 Hz), 7.46 (1H, d, J = 8.3 Hz), 3.66-3.64 (4H, m), 3.50-3.48 (4H, m), 3.35-3.34 (1H, m), 1.84-1.78 (2H, m), 1.17 (6H, d, J = 6.7 Hz). | 476 |
| 2-073 | 1H-NMR (DMSO-D6) δ: 11.41 (1H, s), 9.28 (1H, s), 8.35 (1H, s), 7.49 (1H, s), 7.07 (1H, d, J = 7.9 Hz), 6.84 (1H, d, J = 7.4 Hz), 3.67-3.65 (4H, m), 3.51-3.50 (4H, m), 2.26 (3H, s), 2.18 (3H, s), 1.84-1.79 (2H, m). | 395 |
| 2-074 | 1H-NMR (DMSO-D6) δ: 11.61 (1H, s), 10.15 (1H, s), 8.71 (1H, s), 8.43-8.40 (2H, m), 2.78 (6H, s), 2.34 (3H, s). | 394 |
| 2-075 | 1H-NMR (DMSO-D6) δ: 11.70 (1H, br s), 10.10 (1H, br s), 8.71 (1H, s), 8.42 (1H, s), 8.28 (1H, br s), 3.68-3.63 (4H, m), 3.42-3.41 (4H, m), 2.34 (3H, s), 1.83-1.77 (2H, m). | 450 |
| 2-076 | 1H-NMR (CDCl3) δ: 9.10 (1H, br s), 8.18 (1H, s), 7.95 (1H, s), 7.38 - 7.30 (2H, m), 6.71 (1H, br s), 3.98 - 3.83 (4H, m), 3.81-3.65 (3H, m), 3.61-3.52 (2H, m), 3.38 (3H, s), 2.38 (3H, s). | 479 |
| 2 - 077 | 1H-NMR (DMSO-D6) δ: 9.71 (1H, s), 8.49 (1H, s), 8.22 (1H, s), 7.45 (1H, d, J = 7.6 Hz), 7.36 (1H, d, J = 9.0 Hz), 4.57 (4H, t, J = 12.7 Hz), 2.36 (3H, s). | 440 |
| 2-078 | 1H-NMR (DMSO-D6) δ: 11.39 (1H, br s), 9.63 (1H, br s), 8.39 (1H, br s), 8.29 (1H, s), 7.44 (1H, d, J = 8.1 Hz), 7.34 (1H, d, J = 8.1 Hz), 4.50 (1H, br s), 4.04 - 4.02 (1H, m), 3.65-3.61 (1H, m), 2.36 (3H, s), 2.17 (1H, d, J = 6.9 Hz), 1.83 (1H, t, J = 9.4 Hz), 1.31 (3H, d, J = 6.2 Hz). | 418 |
| 2-079 | 1H-NMR (DMSO-D6) δ: 11.41 (1H, s), 9.64 (1H, s), 8.40 (1H, s), 8.25 (1H, s), 7.44 (1H, d, J = 7.9 Hz), 7.34 (1H, d, J = 7.6 Hz), 5.10 (1H, dd, J = 8.4, 3.6 Hz), 3.42 (2H, dd, J = 15.0, 7.9 Hz), 3.04 (3H, s), 2.82 (3H, s), 2.35 (3H, s), 2.14-2.12 (1H, m), 1.93-1.78 (3H, m). | 489 |
| 2-080 | 1H-NMR (DMSO-D6) δ: 11.41 (1H, br s), 9.62 (1H, br s), 8.37 (1H, br s), 8.26 (1H, s), 7.44 (1H, d, J = 7.6 Hz), 7.34 (1H, d, J = 8.3 Hz), 5.10 (1H, dd, J = 8.4, 3.6 Hz), 3.43 (2H, t, J = 7.9 Hz), 3.04 (3H, s), 2.82 (3H, s), 2.35 (3H, s), 2.14-2.12 (1H, br m), 1.91-1.78 (3H, m). | 489 |
| 2-081 | 1H-NMR (DMSO-D6) δ: 11.40 (br s, 1H), 9.32 (s, 1H), 8.35 (s, 1H), 7.46 (d, J = 1.62 Hz, 1H), 7.11 (d, J = 7.86 Hz, 1H), 6.92 (dd, J = 7.86, 1.62 Hz, 1H), 3.83-3.81 (m, 1H), 3.76-3.74 (m, 1H), 3.56-3.53 (m, 1H), 3.49-3.45 (m, 2H), 3.34-3.32 (m, 2H), 2.84 (sept, J = 6.82 Hz, 1H), 2.17 (s, 3H), 1.22 (d, J = 6.70 Hz, 3H), 1.17 (d, J = 6.82 Hz, 6H). | 423 |
| 2-082 | 1H-NMR (DMSO-D6) δ: 11.42 (br s, 1H), 9.35 (s, 1H), 8.37 (s, 1H), 7.47 (d, J = 1.79 Hz, 1H), 7.13 (d, J = 7.77 Hz, 1H), 6.94 (dd, J = 7.77, 1.79 Hz, 1H), 3.74-3.60 (m, 3H), 3.56-3.54 (m, 1H), 3.45-3.42 (m, 1H), 3.30-3.27 (m, 2H), 2.85 (sept, J = 6.88 Hz, 1H), 2.19 (s, 3H), 1.77-1.64 (m, 2H), 1.19 (d, J = 6.88 Hz, 6H), 0.87 (t, J = 7.32 Hz, 3H). | 437 |
| 2-083 | 1H-NMR (DMSO-D6) δ: 10.76 (br s, 1H), 9.44 (br s, 1H), 7.64 (s, 1H), 7.07 (s, 2H), 2.86 (t, J = 7.40 Hz, 2H), 2.84 (s, 6H), 2.72 (t, J = 7.40 Hz, 2H), 2.17 (s, 3H), 1.98-1.95 (m, 2H). | 381 |
| 2-084 | 1H-NMR (DMSO-D6) δ: 10.96 (br s, 1H), 9.44 (s, 1H), 7.65 (s, 1H), 7.08 (s, 2H), 3.62-3.60 (m, 4H), 3.25-3.24 (m, 4H), 2.86 (t, J = 7.40 Hz, 2H), 2.72 (t, J = 7.40 Hz, 2H), 2.17 (s, 3H), 2.00-1.93 (m, 2H). | 423 |
| 2-085 | 1H-NMR (DMSO-D6) δ: 10.90 (br s, 1H), 9.42 (s, 1H), 7.63 (s, 1H), 7.07 (s, 2H), 3.66-3.64 (m, 4H), 3.51-3.49 (m, 4H), 2.86 (t, J = 7.40 Hz, 2H), 2.72 (t, J = 7.40 Hz, 2H), 2.17 (s, 3H), 1.98-1.95 (m, 2H), 1.84-1.78 (m, 2H). | 437 |
| 2-086 | 1H-NMR (DMSO-D6) δ: 11.45 (1H, s), 10.15 (1H, s), 8.54 (1H, s), 8.08 (1H, s), 2.85 (6H, s), 2.40 (3H, s), 2.34 (3H, s). | 340 |
| 2-087 | 1H-NMR (DMSO-D6) δ: 11.44 (1H, s), 10.26 (1H, s), 8.56 (1H, s), 7.85 (1H, d, J = 7.6 Hz), 7.47 (1H, d, J = 7.6 Hz), 2.85 (6H, s), 2.30 (3H, s). | 394 |
| 2-088 | 1H-NMR (DMSO-D6) δ: 11.55 (1H, s), 10.23 (1H, s), 8.53 (1H, s), 7.85 (1H, d, J = 7.6 Hz), 7.47 (1H, d, J = 7.6 Hz), 3.67-3.65 (4H, m), 3.51-3.48 (4H, m), 2.30 (3H, s), 1.85-1.79 (2H, m). | 450 |
| 2-089 | 1H-NMR (DMSO-D6) δ: 11.20 (1H, br s), 9.20 (1H, s), 8.28 (1H, s), 7.89 (1H, s), 4.39-4.32 (1H, m), 3.00 - 2.94 (1H, m), 2.83 (6H, s), 1.37 (6H, d, J = 6.7 Hz), 1.14 (6H, d, J = 6.9 Hz). | 385 |
| 2-090 | 1H-NMR (DMSO-D6) δ: 11.31 (1H, s), 9.20 (1H, s), 8.27 (1H, s), 7.90 (1H, s), 4.39-4.32 (1H, m), 3.67-3.65 (4H, m), 3.51 - 3.48 (4H, m), 3.00 - 2.94 (1H, m), 1.84-1.79 (2H, m), 1.37 (6H, d, J = 6.5 Hz), 1.14 (6H, d, J = 6.9 Hz). | 441 |
| 2-091 | 1H-NMR (CDCl3) δ: 8.98 (1H, br s), 8.16 (1H, s), 7.93 (1H, s), 7.37-7.31 (2H, m), 6.69 (1H, s), 4.19-4.10 (1H, m), 4.04-3.97 (1H, m), 3.91-3.75 (2H, m), 3.74-3.66 (1H, m), 3.53-3.38 (3H, m), 3.37 (3H, s), 3.31-3.22 (1H, m), 2.38 (3H, s), 2.07-1.98 (2H, m). | 493 |
| 2-092 | 1H-NMR (CDCl3) δ: 8.98 (1H, br s), 8.17 (1H, s), 7.94 (1H, s), 7.38-7.30 (2H, m), 6.66 (1H, br s), 3.91-3.69 (5H, m), 3.64- 3.54 (1H, m), 3.50-3.42 (1H, m), 3.26-3.18 (1H, m), 2.38 (3H, s), 2.29-2.20 (1H, m), 0.95 (3H, d, J = 6.9 Hz). | 463 |
| 2-093 | 1H-NMR (DMSO-D6) δ: 11.68 (1H, br s), 9.68 (1H, s), 8.45 (1H, s), 8.24 (1H, s), 7.45 (1H, d, J = 8.1 Hz), 7.35 (1H, d, J = 8.1 Hz), 4.10 (2H, s), 3.51-3.42 (4H, m), 2.83 (3H, s), 2.35 (3H, s), 1.89-1.80 (2H, m). | 476 |
| 2-094 | 1H-NMR (DMSO-D6) δ: 11.21 (1H, s), 9.42 (1H, s), 8.29 (1H, s), 7.70 (1H, d, J = 1.8 Hz), 7.22 (1H, d, J = 8.0 Hz), 6.99 (1H, dd, J = 8.0, 2.0 Hz), 2.80 (6H, s), 2.23 (3H, s), 1.75-1.70 (2H, m), 1.48-1.43 (2H, m). | 390 |
| 2-095 | 1H-NMR (DMSO-D6) δ: 11.46 (1H, s), 9.66 (1H, s), 8.43 (1H, br s), 8.24 (1H, s), 7.44 (1H, d, J = 8.1 Hz), 7.35 (1H, d, J = 7.9 Hz), 3.65 (2H, d, J = 13.2 Hz), 3.26 (2H, t, J = 6.9 Hz), 3.02 (2H, t, J = 11.2 Hz), 2.71 (3H, s), 2.35 (3H, s), 1.87 (2H, t, J = 6.9 Hz), 1.68 (2H, td, J = 17.8, 6.4 Hz), 1.43 (2H, d, J = 13.2 Hz). | 515 |
| 2-096 | 1H-NMR (DMSO-D6) δ: 11.13 (1H, s), 9.38 (1H, s), 8.36 (1H, s), 7.61 (1H, d, J = 1.8 Hz), 7.16 (1H, d, J = 7.9 Hz), 6.99 (1H, dd, J = 7.7, 1.7 Hz), 3.11-3.08 (1H, m), 2.83 (6H, s), 2.78 (2H, d, J = 6.9 Hz), 2.20 (3H, s), 1.28 (3H, d, J = 6.9 Hz). | 391 |
| 2-097 | 1H-NMR (DMSO-D6) δ: 11.32 (br s, 1H), 9.25 (s, 1H), 8.27 (s, 1H), 7.02 (s, 2H), 3.66-3.62 (m, 4H), 3.48-3.46 (m, 4H), 2.84 (t, J = 7.40 Hz, 2H), 2.68 (t, J = 7.40 Hz, 2H), 2.14 (s, 3H), 2.01-1.92 (m, 2H), 1.83-1.77 (m, 2H). | 421 |
| 2-098 | 1H-NMR (DMSO-D6) δ: 11.50 (br s, 1H), 9.28 (br s, 1H), 8.31 (br s, 1H), 7.50 (d, J = 1.39 Hz, 1H), 7.10 (d, J = 7.86 Hz, 1H), 6.91 (dd, J = 7.86, 1.39 Hz, 1H), 3.62- 3.61 (m, 2H), 3.50-3.49 (m, 2H), 3.22 (s, 2H), 2.83 (sept, J = 6.94 Hz, 1H), 2.17 (s, 3H), 1.31 (s, 6H), 1.17 (d, J = 6.94 Hz, 6H). | 437 |
| 2-099 | 1H-NMR (CDCl3) δ: 8.96 (1H, br s), 8.16 (1H, s), 7.94 (1H, s), 7.38-7.31 (2H, m), 6.70 (1H, s), 3.72-3.63 (4H, m), 3.55-3.48 (4H, m), 2.79 (6H, s), 2.38 (3H, s), 2.08 - 1.99 (2H, m). | 555 |
| 2-100 | 1H-NMR (CDCl3) δ: 8.96 (1H, s), 8.17 (1H, s), 7.94 (1H, s), 7.37-7.31 (2H, m), 6.67 (1H, s), 3.72-3.65 (4H, m), 3.54-3.47 (4H, m), 3.00-2.92 (2H, m), 2.38 (3H, s), 2.10-1.99 (2H, m), 1.88-1.77 (2H, m), 1.06 (3H, t, J = 7.4 Hz). | 554 |
| 2-101 | 1H-NMR (CDCl3) δ: 8.94 (1H, br s), 8.16 (1H, s), 7.93 (1H, s), 7.37-7.31 (2H, m), 6.67 (1H, s), 3.71 (3H, s), 3.68-3.49 (8H, m), 2.38 (3H, s), 2.03-1.93 (2H, m). | 506 |
| 2-102 | 1H-NMR (CDCl3) δ: 8.94 (1H, br s), 8.15 (1H, s), 7.93 (1H, d, J = 2.3 Hz), 7.37-7.31 (2H, m), 6.70 (1H, s), 3.78-3.53 (7H, m), 3.52-3.46 (1H, m), 2.40-2.31 (2H, m), 2.38 (3H, s), 2.04-1.95 (2H, m), 1.16 (3H, t, J = 7.4 Hz). | 504 |
| 2-103 | 1H-NMR (CDCl3) δ: 8.96 (1H, br s), 8.17 (1H, s), 7.94 (1H, s), 7.37-7.31 (2H, m), 6.67 (1H, s), 3.73-3.65 (4H, m), 3.60-3.52 (4H, m), 2.40-2.32 (1H, m), 2.38 (3H, s), 2.04 (2H, tt, J = 6.0, 6.0 Hz), 1.22-1.14 (2H, m), 1.04-0.98 (2H, m). | 552 |
| 2-104 | 1H-NMR (DMSO-D6) δ: 11.66 (1H, br s), 9.98 (1H, s), 8.73 (1H, s), 8.50 (1H, s), 8.45 (1H, br s), 3.67-3.65 (4H, m), 3.51-3.48 (4H, m), 2.59 (3H, s), 1.85-1.79 (2H, m). | 450 |
| 2-105 | 1H-NMR (DMSO-D6) δ: 11.30 (1H, s), 9.58 (1H, s), 8.40 (1H, s), 7.83 (1H, s), 7.48 (1H, d, J = 8.3 Hz), 7.35 (1H, d, J = 8.3 Hz), 7.00 (1H, t, J = 56.0 Hz), 3.37-3.26 (1H, m), 2.85 (6H, s), 1.17 (6H, d, J = 6.8 Hz). | 403 |
| 2-106 | 1H-NMR (DMSO-D6) δ: 11.42 (1H, s), 9.56 (1H, s), 8.35 (1H, s), 7.83 (1H, s), 7.48 (1H, d, J = 8.3 Hz), 7.34 (1H, d, J = 8.0 Hz), 7.00 (1H, t, J = 55.9 Hz), 3.70-3.62 (4H, m), 3.53-3.46 (4H, m), 3.36-3.26 (1H, m), 1.85-1.79 (2H, m), 1.17 (6H, d, J = 6.8 Hz). | 459 |
| 2-107 | 1H-NMR (DMSO-D6) δ: 11.32 (1H, s), 9.48 (1H, s), 8.37 (1H, s), 7.69 (1H, d, J = 1.8 Hz), 7.22 (1H, d, J = 7.8 Hz), 6.99 (1H, dd, J = 7.8, 1.8 Hz), 3.70-3.63 (4H, m), 3.54-3.46 (4H, m), 2.23 (3H, s), 1.86-1.79 (2H, m), 1.76-1.71 (2H, m), 1.48-1.43 (2H, m). | 446 |
| 2-108 | 1H-NMR (DMSO-D6) δ: 11.95 (1H, br s), 9.68 (1H, s), 8.44 (1H, s), 8.23 (1H, s), 7.45 (1H, d, J = 7.9 Hz), 7.35 (1H, d, J = 6.5 Hz), 4.49 (2H, s), 2.95 (3H, s), 2.35 (3H, s). | 417 |
| 2-109 | 1H-NMR (DMSO-D6) δ: 11.57 (1H, s), 9.69 (1H, s), 8.47 (1H, s), 8.23 (1H, s), 7.45 (1H, d, J = 8.1 Hz), 7.35 (1H, d, J = 6.5 Hz), 3.47 (2H, ddd, J = 12.9, 6.3, 3.9 Hz), 3.24-3.17 (2H, m), 3.08 - 3.02 (1H, m), 2.35 (3H, s), 1.98-1.93 (2H, m), 1.78-1.74 (2H, m). | 457 |
| 2-110 | 1H-NMR (DMSO-D6) δ: 11.65 (1H, s), 9.69 (1H, s), 8.43 (1H, s), 8.31 (1H, s), 7.44 (1H, d, J = 7.9 Hz), 7.34 (1H, d, J = 8.1 Hz), 4.26 (2H, s), 2.94 (3H, s), 2.90 (3H, s), 2.80 (3H, s), 2.36 (3H, s). | 463 |
| 2-111 | 1H-NMR (DMSO-D6) δ: 11.50 (1H, s), 9.69 (1H, s), 8.39 (1H, s), 8.06 (1H, s), 7.54 (1H, d, J = 8.1 Hz), 7.46 (1H, d, J = 9.2 Hz), 3.80-3.67 (4H, m), 3.58-3.55 (3H, m), 3.35-3.29 (2H, m), 3.14-3.08 (1H, m), 1.17 (6H, d, J = 6.9 Hz). | 492 |
| 2-112 | 1H-NMR (DMSO-D6) δ: 11.25 (1H, s), 9.33 (1H, s), 8.32 (1H, s), 7.60 (1H, d, J = 2.1 Hz), 7.12 (1H, d, J = 7.9 Hz), 7.06 (1H, dd, J = 8.0, 2.0 Hz), 3.66-3.64 (4H, m), 3.50-3.47 (4H, m), 2.17 (3H, s), 1.84-1.78 (2H, m), 1.25 (9H, s). | 436 |
| 2-113 | 1H-NMR (DMSO-D6) δ: 11.26 (1H, s), 9.04 (1H, s), 8.24 (1H, s), 7.33 (1H, d, J = 8.3 Hz), 7.25 (1H, dd, J = 8.3, 2.3 Hz), 7.14 (1H, d, J = 2.3 Hz), 3.66-3.62 (4H, m), 3.48-3.45 (4H, m), 1.83-1.77 (2H, m), 1.29 (9H, s), 1.23 (9H, s). | 478 |
| 2-114 | 1H-NMR (DMSO-D6) δ: 11.29 (1H, s), 9.14 (1H, s), 8.24 (1H, s), 7.00 (1H, d, J = 7.6 Hz), 6.98 (1H, d, J = 7.9 Hz), 3.65 - 3.62 (4H, m), 3.47-3.45 (4H, m), 2.21 (3H, s), 2.11 (3H, s), 2.05 (3H, s), 1.82-1.76 (2H, m). | 408 |
| 2-115 | 1H-NMR (DMSO-D6) δ: 11.31 (1H, s), 9.15 (1H, s), 8.24 (1H, s), 7.11-7.08 (3H, m), 3.65-3.62 (4H, m), 3.47-3.44 (4H, m), 2.15 (6H, s), 1.82-1.76 (2H, m). | 394 |
| 2-116 | 1H-NMR (DMSO-D6) δ: 11.31 (1H, br s), 9.39 (1H, br s), 8.22 (1H, br s), 7.51 (1H, s), 4.36-4.30 (1H, m), 3.66-3.63 (4H, m), 3.46-3.45 (4H, m), 2.72-2.65 (1H, m), 1.83-1.77 (2H, m), 1.36 (6H, d, J = 6.7 Hz), 1.08 (6H, d, J = 6.7 Hz). | 441 |
| 2-117 | 1H-NMR (DMSO-D6) δ: 11.20 (1H, br s), 9.42 (1H, s), 8.27 (1H, s), 7.52 (1H, s), 4.36-4.30 (1H, m), 2.82 (6H, s), 2.70-2.66 (1H, m), 1.36 (6H, d, J = 6.7 Hz), 1.08 (6H, d, J = 6.9 Hz). | 385 |
| 2-118 | 1H-NMR (DMSO-D6) δ: 11.20 (br s, 1H), 9.22 (s, 1H), 8.27 (s, 1H), 7.13-7.10 (m, 2H), 3.12 (sept, J = 6.70 Hz, 1H), 2.85 (t, J = 7.40 Hz, 2H), 2.81 (s, 6H), 2.65 (t, J = 7.40 Hz, 2H), 1.99-1.91 (m, 2H), 1.10 (d, J = 6.70 Hz, 6H). | 393 |
| 2-119 | 1H-NMR (DMSO-D6) δ: 11.31 (br s, 1H), 9.22 (s, 1H), 8.26 (s, 1H), 7.13-7.07 (m, 2H), 3.64-3.61 (m, 4H), 3.48-3.45 (m, 4H), 3.12 (sept, J = 6.94 Hz, 1H), 2.85 (t, J = 7.40 Hz, 2H), 2.64 (t, J = 7.40 Hz, 2H), 1.97-1.93 (m, 2H), 1.83-1.77 (m, 2H), 1.10 (d, J = 6.94 Hz, 6H). | 449 |
| 2-120 | 1H-NMR (DMSO-D6) δ: 11.40 (br s, 1H), 9.24 (s, 1H), 8.27 (s, 1H), 7.13-7.10 (m, 2H), 3.87-3.84 (m, 1H), 3.57-3.28 (m, 6H), 3.23 (s, 3H), 3.15-3.09 (m, 1H), 2.95-2.89 (m, 1H), 2.85 (t, J = 7.40 Hz, 2H), 2.75-2.72 (m, 1H), 2.65 (t, J = 7.40 Hz, 2H), 1.99-1.92 (m, 2H), 1.10 (d, J = 6.94 Hz, 6H). | 479 |
| 2-121 | 1H-NMR (CDCl3) δ: 9.06 (1H, s), 8.10 (1H, s), 7.93 (1H, s), 7.48-7.40 (2H, m), 6.70 (1H, s), 3.98-3.64 (7H, m), 3.60-3.50 (2H, m), 3.37 (3H, s), 3.13-3.05 (1H, m), 1.32 (6H, d, J = 6.8 Hz). | 507 |
| 2-122 | 1H-NMR (CDCl3) δ: 8.95 (1H, s), 8.16 (1H, s), 7.93 (1H, s), 7.38-7.32 (2H, m), 6.66 (1H, s), 3.88-3.80 (1H, m), 3.79-3.64 (2H, m), 3.36-3.26 (1H, m), 3.12-3.03 (1H, m), 3.00 (2H, q, J = 7.5 Hz), 2.58-2.43 (2H, m), 2.38 (3H, s), 2.18-2.09 (1H, m), 2.08 - 1.95 (1H, m), 1.87-1.72 (2H, m), 1.41 (3H, t, J = 7.5 Hz). | 539 |
| 2-123 | 1H-NMR (DMSO-D6) δ: 9.44 (1H, s), 7.98 (1H, s), 7.88 (1H, s), 7.56 (1H, d, J = 8.1 Hz), 7.48 (1H, d, J = 8.6 Hz), 3.50 (2H, t, J = 5.8 Hz), 3.19 (4H, t, J = 5.9 Hz), 3.04 (2H, t, J = 4.9 Hz), 2.40 (3H, s), 2.00-1.97 (2H, m), 1.15 (6H, d, J = 6.9 Hz). | 489 |
| 2-124 | 1H-NMR (DMSO-D6) δ: 11.31 (1H, s), 9.67 (1H, s), 8.38 (1H, s), 8.05 (1H, s), 7.54 (1H, d, J = 8.1 Hz), 7.46 (1H, d, J = 8.3 Hz), 4.79 (1H, s), 4.07-4.04 (1H, m), 3.49-3.46 (1H, m), 3.35-3.28 (4H, m), 1.89-1.80 (2H, m), 1.76-1.73 (2H, m), 1.17 (6H, d, J = 6.7 Hz). | 476 |
| 2-125 | 1H-NMR (DMSO-D6) δ: 11.55 (1H, br s), 10.21 (1H, s), 8.54 (1H, s), 7.95 (1H, d, J = 7.9 Hz), 7.54 (1H, d, J = 7.9 Hz), 3.67-3.65 (4H, m), 3.62-3.56 (1H, m), 3.52-3.49 (4H, m), 1.85-1.79 (2H, m), 1.19 (6H, d, J = 6.7 Hz). | 478 |
| 2-126 | 1H-NMR (CDCl3) δ: 8.96 (1H, br s), 8.17 (1H, s), 7.94 (1H, s), 7.37-7.31 (2H, m), 6.67 (1H, s), 3.72-3.64 (4H, m), 3.55-3.48 (4H, m), 3.02-2.95 (2H, m), 2.38 (3H, s), 2.09-2.01 (2H, m), 1.82-1.72 (2H, m), 1.51-1.40 (2H, m), 0.95 (3H, t, J = 7.4 Hz). | 568 |
| 2-127 | 1H-NMR (DMSO-D6) δ: 11.76 (1H, s), 9.70 (1H, s), 8.47 (1H, s), 8.24 (1H, s), 7.45 (1H, d, J = 8.0 Hz), 7.35 (1H, d, J = 8.0 Hz), 3.97-3.81 (4H, m), 3.79-3.66 (2H, m), 3.60-3.40 (3H, m), 2.35 (3H, s). | 474 |
| 2-128 | 1H-NMR (CDCl3) δ: 8.96 (1H, br s), 8.17 (1H, s), 7.94 (1H, s), 7.37-7.31 (2H, m), 6.67 (1H, s), 3.72-3.65 (4H, m), 3.52-3.46 (4H, m), 2.84 (2H, d, J = 6.5 Hz), 2.38 (3H, s), 2.30-2.19 (1H, m), 2.09-2.01 (2H, m), 1.10 (6H, d, J = 6.0 Hz). | 568 |
| 2-129 | 1H-NMR (CDCl3) δ: 8.90 (1H, br s), 8.16 (1H, s), 7.94 (1H, s), 7.38-7.31 (2H, m), 6.68 (1H, s), 3.73-3.63 (4H, m), 3.53-3.45 (4H, m), 3.24-3.16 (2H, m), 2.76 (3H, s), 2.38 (3H, s), 2.08-1.98 (2H, m), 1.18 (3H, t, J = 7.2 Hz). | 569 |
| 2-130 | 1H-NMR (CDCl3) δ: 8.94 (1H, br s), 8.16 (1H, s), 7.94 (1H, s), 7.38-7.31 (2H, m), 6.67 (1H, s), 3.74-3.65 (4H, m), 3.57-3.51 (4H, m), 3.22-3.15 (2H, m), 2.69-2.55 (2H, m), 2.38 (3H, s), 2.11-2.02 (2H, m). | 608 |
| 2-131 | 1H-NMR (DMSO-D6) δ: 11.37 (1H, s), 9.65 (1H, s), 8.41 (1H, s), 8.20 (1H, s), 7.43 (1H, d, J = 8.1 Hz), 7.34 (1H, d, J = 8.1 Hz), 4.21-4.13 (1H, m), 2.81 (3H, s), 2.33 (3H, s), 1.71-1.70 (2H, m), 1.58-1.43 (6H, m). | 446 |
| 2-132 | 1H-NMR (DMSO-D6) δ: 9.01 (1H, s), 7.88 (1H, s), 7.52 (1H, d, J = 1.8 Hz), 7.11 (1H, d, J = 8.1 Hz), 7.06 (1H, dd, J = 8.0, 2.0 Hz), 3.42-3.39 (2H, m), 3.14-3.13 (2H, m), 2.65 (3H, s), 2.63 (6H, s), 2.14 (3H, s), 1.25 (9H, s). | 437 |
| 2-133 | 1H-NMR (DMSO-D6) δ: 9.77 (1H, s), 9.63 (1H, s), 8.49 (1H, s), 8.41 (1H, d, J = 4.4 Hz), 8.19 (1H, s), 7.95 (1H, t, J = 7.5 Hz), 7.49 (2H, dd, J = 7.7, 5.0 Hz), 7.39 (1H, d, J = 7.9 Hz), 7.26 (1H, t, J = 6.1 Hz), 4.68 (2H, s), 2.88 (3H, s), 2.37 (3H, s). | 469 |
| 2-134 | 1H-NMR (DMSO-D6) δ: 11.83 (1H, s), 9.74 (1H, s), 8.76 (2H, s), 8.52 (1H, s), 8.25 (2H, d, J = 8.8 Hz), 7.84 (1H, dd, J = 8.0, 5.4 Hz), 7.46 (1H, d, J = 7.6 Hz), 7.36 (1H, d, J = 8.1 Hz), 4.65 (2H, s), 2.85 (3H, s), 2.37 (3H, s). | 469 |
| 2-135 | 1H-NMR (DMSO-D6) δ: 11.81 (1H, s), 10.23 (1H, s), 8.07 (1H, s), 7.95 (1H, s), 7.56 (1H, d, J = 8.3 Hz), 7.49 (1H, d, J = 8.3 Hz), 3.66 (4H, t, J = 5.3 Hz), 3.49 (4H, dd, J = 10.6, 5.1 Hz), 3.36-3.34 (1H, m), 1.85-1.80 (2H, m), 1.16 (6H, d, J = 6.9 Hz). | 476 |
| 2-136 | 1H-NMR (DMSO-D6) δ: 11.70 (1H, s), 11.06 (1H, br s), 8.01 (1H, s), 7.92 (1H, d, J = 8.3 Hz), 7.86 (1H, dd, J = 8.8, 2.1 Hz), 7.72 (1H, br s), 3.68-3.65 (4H, m), 3.48-3.46 (4H, m), 1.85-1.79 (2H, m). | 469 |
| 2-137 | 1H-NMR (DMSO-D6) δ: 11.58 (1H, s), 9.79 (1H, s), 8.53 (1H, d, J = 2.1 Hz), 8.43 (1H, s), 7.37 (1H, dd, J = 8.6, 1.6 Hz), 7.21 (1H, d, J = 8.6 Hz), 3.92 (3H, s), 3.67-3.66 (4H, m), 3.52-3.50 (4H, m), 1.86-1.80 (2H, m). | 465 |
| 2-138 | 1H-NMR (CDCl3) δ: 9.07 (1H, br s), 7.83 (1H, s), 7.22 (1H, s), 6.55 (1H, s), 4.46-4.36 (1H, m), 4.28-4.19 (1H, m), 2.92 (3H, s), 2.84-2.73 (1H, m), 1.51 (6H, d, J = 6.7 Hz), 1.21-1.14 (12H, m). | 413 |
| 2-139 | 1H-NMR (CDCl3) δ: 9.03 (1H, br s), 7.82 (1H, s), 7.22 (1H, s), 6.56 (1H, s), 4.46-4.34 (1H, m), 3.58-3.48 (4H, m), 2.84-2.72 (1H, m), 1.96-1.85 (4H, m), 1.52-1.46 (6H, m), 1.21-1.15 (6H, m). | 411 |
| 2-140 | 1H-NMR (DMSO-D6) δ: 11.56 (1H, s), 9.51 (1H, s), 8.41 (1H, s), 8.09 (1H, d, J = 1.6 Hz), 7.18-7.13 (2H, m), 3.66 (4H, dd, J = 7.4, 3.7 Hz), 3.51 (4H, dd, J = 10.9, 5.5 Hz), 2.22 (3H, s), 1.82 (2H, t, J = 5.8 Hz). | 459 461 (M+3) |
| 2-141 | 1H-NMR (DMSO-D6) δ: 11.53 (1H, s), 9.68 (1H, s), 8.44 (1H, s), 8.23 (1H, s), 7.44 (1H, d, J = 8.1 Hz), 7.35 (1H, d, J = 7.9 Hz), 3.51-3.47 (2H, m), 3.39 (2H, t, J = 5.8 Hz), 3.11-3.08 (1H, m), 2.35 (3H, s), 2.05-2.01 (1H, m), 1.94-1.74 (5H, m). | 471 |
| 2-142 | 1H-NMR (DMSO-D6) δ: 11.67 (1H, s), 10.23 (1H, s), 8.77 (1H, d, J = 8.5 Hz), 8.57 (1H, s), 8.01 (1H, s), 3.72-3.65 (4H, m), 3.57-3.50 (4H, m), 2.41 (3H, s), 1.89-1.80 (2H, m). | 474 |
| 2-143 | 1H-NMR (DMSO-D6) δ: 11.62 (1H, s), 9.69 (1H, s), 8.46 (1H, s), 8.24 (1H, s), 7.45 (1H, d, J = 8.0 Hz), 7.35 (1H, d, J = 7.8 Hz), 3.89-3.59 (7H, m), 3.50-3.37 (1H, m), 3.34-3.25 (1H, m), 3.02 (3H, s), 2.80 (3H, s), 2.35 (3H, s). | 520 |
| 2-144 | 1H-NMR (DMSO-D6) δ: 11.75 (1H, br s), 10.73 (1H, br s), 8.73 (1H, br s), 8.66 (1H, br s), 3.67-3.66 (4H, m), 3.53-3.51 (4H, br m), 3.25-3.22 (1H, m), 1.82-1.81 (2H, m), 1.25 (6H, d, J = 6.7 Hz). | 479 |
| 2-145 | 1H-NMR (DMSO-D6) δ: 11.37 (1H, s), 9.65 (1H, s), 8.42 (1H, s), 8.23 (1H, s), 7.43 (1H, d, J = 7.9 Hz), 7.33 (1H, d, J = 7.9 Hz), 3.37 (4H, t, J = 5.9 Hz), 2.33 (3H, s), 1.65-1.63 (4H, m), 1.54-1.52 (4H, m). | 446 |
| 2-146 | 1H-NMR (DMSO-D6) δ: 11.72 (1H, s), 9.71-9.66 (1H, m), 8.48-8.44 (1H, m), 8.26-8.21 (1H, m), 7.44 (1H, d, J = 8.0 Hz), 7.35 (1H, d, J = 8.0 Hz), 4.82-4.72 (1H, m), 4.00-3.69 (6H, m), 3.58-3.46 (1H, m), 3.37-3.14 (2H, m), 2.94 (2H, s), 2.35 (3H, s), 2.27 (2H, q, J = 7.3 Hz), 1.01-0.93 (3H, m). | 534 |
| 2-147 | 1H-NMR (DMSO-D6) δ: 11.32 (1H, br s), 9.55 (1H, s), 8.26 (1H, s), 7.50 (1H, s), 4.35-4.28 (1H, m), 3.66-3.63 (4H, m), 3.49-3.46 (4H, m), 1.84-1.77 (5H, m), 1.35 (6H, d, J = 6.7 Hz). | 413 |
| 2-148 | 1H-NMR (CDCl3) δ: 8.94 (1H, s), 8.16 (1H, s), 7.92 (1H, s), 7.38-7.29 (2H, m), 6.66 (1H, s), 3.72-3.34 (5H, m), 3.31 (3H, s), 2.38 (3H, s), 2.05-1.62 (6H, m). | 477 |
| 2-149 | 1H-NMR (DMSO-D6) δ: 11.61 (1H, s), 9.68 (1H, s), 8.44 (1H, s), 8.24 (1H, s), 7.45 (1H, d, J = 7.9 Hz), 7.35 (1H, d, J = 9.2 Hz), 3.68 (2H, t, J = 5.8 Hz), 3.49 (2H, t, J = 5.3 Hz), 2.61-2.57 (4H, m), 2.35 (3H, s), 1.74 (2H, t, J = 4.7 Hz). | 460 |
| 2-150 | 1H-NMR (DMSO-D6) δ: 11.46 (1H, s), 9.66 (1H, s), 8.42 (1H, s), 8.24 (1H, s), 7.44 (1H, d, J = 7.6 Hz), 7.35 (1H, d, J = 7.9 Hz), 3.60 (1H, td, J = 9.5, 4.7 Hz), 3.46 (1H, td, J = 9.0, 4.3 Hz), 3.33-3.26 (2H, m), 2.52-2.50 (2H, m), 2.35 (3H, s), 1.91-1.76 (4H, m), 1.59 (1H, d, J = 11.1 Hz), 1.44-1.37 (2H, m). | 485 |
| 2-151 | 1H-NMR (DMSO-D6) δ: 11.59 (1H, s), 9.68 (1H, s), 8.45 (1H, s), 8.24 (1H, s), 7.45 (1H, d, J = 8.1 Hz), 7.35 (1H, d, J = 7.9 Hz), 3.76-3.39 (8H, m), 3.27 (4H, dt, J = 13.2, 5.5 Hz), 3.22 (3H, s), 2.35 (4H, s), 2.21-2.15 (1H, m). | 492 |
| 2-152 | 1H-NMR (DMSO-D6) δ: 11.38 (1H, s), 9.81 (1H, s), 8.33 (1H, s), 7.59 (1H, s), 4.99 (2H, q, J = 9.2 Hz), 3.66 (4H, td, J = 5.1, 3.0 Hz), 3.50 (4H, dd, J = 10.8, 5.4 Hz), 1.90 (3H, s), 1.85-1.79 (2H, m). | 452 |
| 2-153 | 1H-NMR (DMSO-D6) δ: 11.33 (1H, s), 9.58 (1H, s), 8.29 (1H, s), 7.52 (1H, s), 4.56-4.49 (1H, m), 3.66 (4H, dd, J = 9.2, 3.5 Hz), 3.50 (4H, dd, J = 10.6, 5.3 Hz), 2.06-1.98 (3H, m), 1.91-1.70 (10H, m), 1.61 (2H, tt, J = 11.3, 4.4 Hz). | 438 |
| 2-154 | 1H-NMR (CDCl3) δ: 8.17 (1H, br s), 7.93 (1H, s), 7.37-7.28 (2H, m), 6.67 (1H, br s), 3.71-3.66 (2H, m), 3.65-3.59 (2H, m), 2.92-2.87 (2H, m), 2.87-2.80 (2H, m), 2.56-2.49 (2H, m), 2.37 (3H, s), 2.09-1.99 (2H, m), 1.52-1.41 (2H, m), 0.79 (3H, t, J = 7.4 Hz).(-NH) | 490 |
| 2-155 | 1H-NMR (DMSO-D6) δ: 11.68 (1H, s), 9.68 (1H, s), 8.45 (1H, s), 8.24 (1H, s), 7.45 (1H, d, J = 8.0 Hz), 7.35 (1H, d, J = 8.0 Hz), 4.10 (2H, s), 3.52-3.43 (4H, m), 3.20 (2H, t, J = 7.3 Hz), 2.35 (3H, s), 1.87-1.77 (2H, m), 1.49-1.37 (2H, m), 0.79 (3H, t, J = 7.3 Hz). | 504 |
| 2-156 | 1H-NMR (CDCl3) δ: 8.99 (1H, br s), 8.15 (1H, s), 7.93 (1H, s), 7.37-7.30 (2H, m), 6.65 (1H, s), 4.06-3.96 (1H, m), 3.72-3.47 (4H, m), 2.38 (3H, s), 2.12-1.63 (6H, m), 1.44 (1H, br s). | 463 |
| 2-157 | 1H-NMR (DMSO-D6) δ: 11.47 (1H, s), 9.64 (1H, s), 8.39 (1H, s), 8.26 (1H, s), 7.44 (1H, d, J = 7.8 Hz), 7.34 (1H, d, J = 7.8 Hz), 3.63-3.50 (2H, m), 3.41-3.20 (2H, m), 2.99 (3H, s), 2.84-2.74 (1H, m), 2.79 (3H, s), 2.35 (3H, s), 1.89-1.42 (6H, m). | 518 |
| 2-158 | 1H-NMR (DMSO-D6) δ: 11.46 (1H, s), 9.69 (1H, s), 8.46 (1H, s), 8.23 (1H, s), 7.45 (1H, d, J = 8.0 Hz), 7.35 (1H, d, J = 8.0 Hz), 3.49-3.41 (2H, m), 3.37-3.30 (1H, m), 3.22 (3H, s), 3.20-3.12 (2H, m), 2.35 (3H, s), 1.88-1.79 (2H, m), 1.58-1.47 (2H, m). | 463 |
| 2-159 | 1H-NMR (DMSO-D6) δ: 11.41 (1H, s), 9.69 (1H, s), 8.46 (1H, s), 8.22 (1H, s), 7.45 (1H, d, J = 7.8 Hz), 7.36 (1H, d, J = 8.0 Hz), 3.52-3.46 (1H, m), 3.36-3.25 (2H, m), 3.24 (3H, s), 3.21-3.09 (2H, m), 2.35 (3H, s), 1.81-1.69 (2H, m), 1.50-1.39 (2H, m). | 463 |
| 2-160 | 1H-NMR (DMSO-D6) δ: 11.41 (1H, br s), 9.08 (1H, br s), 8.09 (1H, br s), 7.87 (1H, s), 4.40-4.33 (1H, m), 3.48-3.23 (8H, m), 3.08-2.93 (3H, m), 1.82-1.73 (2H, m), 1.71-1.60 (2H, m), 1.38 (6H, d, J = 6.8 Hz), 1.15 (6H, d, J = 7.0 Hz), 0.96 (3H, t, J = 7.4 Hz). | 546 |
| 2-161 | 1H-NMR (DMSO-D6) δ: 11.41 (1H, br s), 8.99 (1H, br s), 7.98 (1H, br s), 7.85 (1H, s), 4.40-4.33 (1H, m), 3.47-3.21 (8H, m), 3.11-2.92 (3H, m), 1.82-1.70 (2H, m), 1.67-1.54 (2H, m), 1.41-1.34 (8H, m), 1.15 (6H, d, J = 7.0 Hz), 0.88 (3H, t, J = 7.4 Hz). | 559 |
| 2-162 | 1H-NMR (DMSO-D6) δ: 11.47 (1H, s), 9.66 (1H, s), 8.44 (1H, d, J = 1.6 Hz), 8.23 (1H, s), 7.93 (1H, t, J = 5.5 Hz), 7.43 (1H, d, J = 7.9 Hz), 7.33 (1H, d, J = 8.8 Hz), 3.26 (2H, t, J = 6.4 Hz), 3.20 (2H, t, J = 5.5 Hz), 2.86 (3H, s), 2.34 (3H, s), 1.78 (3H, s). | 463 |
| 2-163 | 1H-NMR (DMSO-D6) δ: 11.63 (1H, s), 9.68 (1H, s), 8.47 (1H, s), 8.25 (1H, s), 7.45-7.29 (7H, m), 4.42 (2H, s), 2.76 (3H, s), 2.35 (3H, s). | 468 |
| 2-164 | 1H-NMR (DMSO-D6) δ: 11.30 (1H, s), 9.66 (1H, s), 8.42 (1H, s), 8.20 (1H, s), 7.43 (1H, d, J = 7.9 Hz), 7.34 (1H, d, J = 7.6 Hz), 3.48-3.45 (8H, m), 3.19 (6H, s), 2.33 (3H, s). | 480 |
| 2-165 | 1H-NMR (DMSO-D6) δ: 11.57 (1H, s), 9.67 (1H, s), 8.47 (1H, s), 8.24 (1H, s), 7.43 (1H, d, J = 7.9 Hz), 7.34 (1H, d, J = 7.9 Hz), 2.90 (3H, s), 2.56-2.48 (1H, m), 2.34 (3H, s), 0.73-0.69 (4H, m). | 418 |
| 2-166 | 1H-NMR (DMSO-D6) δ: 11.50 (1H, s), 9.65 (1H, s), 8.40 (1H, s), 8.22 (1H, s), 7.42 (1H, d, J = 7.9 Hz), 7.33 (1H, d, J = 7.9 Hz), 3.46 (2H, d, J = 4.9 Hz), 2.33 (3H, s), 1.66-1.59 (4H, m), 1.44-1.42 (2H, m), 1.03-1.01 (2H, m), 0.60-0.60 (2H, m). | 458 |
| 2-167 | 1H-NMR (DMSO-D6) δ: 9.51 (1H, br s), 8.81 (1H, s), 8.46 (1H, d, J = 4.6 Hz), 7.76 (1H, td, J = 7.6, 1.8 Hz), 7.72 (1H, s), 7.63 (1H, s), 7.49 (1H, d, J = 7.9 Hz), 7.23 (1H, dd, J = 6.6, 5.0 Hz), 7.08 (1H, d, J = 7.9 Hz), 6.85 (1H, dd, J = 7.7, 1.7 Hz), 4.27 (2H, s), 2.87-2.80 (1H, m), 2.55 (3H, s), 2.19 (3H, s), 1.19 (6H, d, J = 6.9 Hz). | 443 |
| 2-168 | 1H-NMR (DMSO-D6) δ: 11.33 (1H, s), 9.61 (1H, s), 8.29 (1H, s), 7.49 (1H, s), 3.79 (1H, dt, J = 15.9, 5.5 Hz), 3.66 (4H, td, J = 5.1, 2.9 Hz), 3.50 (4H, dd, J = 10.9, 5.3 Hz), 1.87 (3H, s), 1.85-1.79 (2H, m), 1.74-1.71 (4H, m), 0.70 (6H, t, J = 7.3 Hz). | 440 |
| 2-169 | 1H-NMR (DMSO-D6) δ: 11.36 (1H, br s), 9.67 (1H, br s), 8.38 (1H, s), 8.33 (1H, br s), 4.60-4.54 (1H, m), 3.67-3.64 (4H, m), 3.51-3.48 (4H, m), 1.85-1.79 (2H, m), 1.43 (6H, d, J = 6.5 Hz). | 467 |
| 2-170 | 1H-NMR (DMSO-D6+TFA) δ: 11.42 (1H, s), 9.25 (1H, s), 8.30 (1H, s), 7.92 (1H, s), 4.41-4.35 (1H, m), 3.52-3.33 (8H, m), 3.03-2.94 (2H, m), 1.85-1.77 (3H, m), 1.39 (6H, d, J = 6.8 Hz), 1.16 (6H, d, J = 6.8 Hz), 1.08 (9H, s). | 574 |
| 2-171 | 1H-NMR (DMSO-D6) δ: 11.32 (1H, s), 9.23 (1H, s), 8.14 (1H, s), 7.93 (1H, s), 4.40-4.34 (1H, m), 3.70-3.63 (4H, m), 3.51-3.42 (4H, m), 2.53-2.44 (2H, m), 1.85-1.79 (2H, m), 1.60-1.48 (2H, m), 1.38 (6H, d, J = 6.5 Hz), 0.88 (3H, t, J = 7.4 Hz). | 441 |
| 2-172 | 1H-NMR (DMSO-D6) δ: 11.38 (1H, s), 9.65 (1H, s), 8.41 (1H, s), 8.20 (1H, s), 7.43 (1H, d, J = 7.9 Hz), 7.34 (1H, d, J = 7.9 Hz), 4.13-4.11 (1H, m), 3.68-3.65 (1H, m), 3.12-3.08 (1H, m), 2.33 (3H, s), 1.15 (3H, d, J = 6.9 Hz). | 446 |
| 2-173 | 1H-NMR (DMSO-D6) δ: 9.32 (1H, s), 8.38 (1H, s), 7.92 (1H, s), 7.39 (1H, d, J = 7.6 Hz), 7.26 (1H, d, J = 7.4 Hz), 3.51-3.50 (1H, m), 3.30-3.24 (2H, m), 3.17-3.14 (2H, m), 2.63 (6H, s), 2.33 (3H, s), 1.94-1.91 (2H, m), 1.79-1.77 (2H, m), 1.67-1.65 (1H, m), 1.56-1.53 (1H, m). | 489 |
| 2-174 | 1H-NMR (DMSO-D6) δ: 11.33 (1H, s), 9.66. (1H, s), 8.44 (1H, s), 8.20 (1H, s), 7.43 (1H, d, J = 7.9 Hz), 7.34 (1H, d, J = 9.2 Hz), 4.12-4.08 (1H, m), 3.51-3.45 (1H, m), 3.36-3.32 (1H, m), 2.33 (3H, s), 1.96-1.81 (2H, m), 1.76-1.69 (1H, m), 1.55-1.49 (1H, m), 1.18 (3H, d, J = 6.5 Hz). | 432 |
| 2-175 | 1H-NMR (DMSO-D6) δ: 11.44 (1H, s), 10.09 (1H, s), 8.42 (1H, s), 8.06 (1H, d, J = 2.1 Hz), 7.28 (1H, dd, J = 8.4, 1.5 Hz), 7.02 (1H, dd, J = 8.4, 2.2 Hz), 3.67-3.65 (4H, m), 3.52-3.49 (4H, m), 2.96-2.89 (1H, m), 1.85-1.80 (2H, m), 1.21 (6H, d, J = 6.9 Hz). | 493 |
| 2-176 | 1H-NMR (DMSO-D6) δ: 10.78 (1H, br s), 7.85 (1H, br s), 7.57 (1H, d, J = 8.3 Hz), 7.32 (1H, s), 7.12-7.10 (2H, m), 3.69-3.67 (4H, m), 3.56-3.55 (4H, m), 3.02-2.95 (1H, m), 1.87-1.81 (2H, m), 1.22 (6H, d, J = 6.9 Hz). | 477 |
| 2-177 | 1H-NMR (CDC13) δ: 9.11 (1H, br s), 7.83 (1H, s), 7.22 (1H, s), 6.48 (1H, br s), 4.46-4.35 (1H, m), 3.69-3.61 (4H, m), 3.53-3.45 (4H, m), 3.01-2.94 (2H, m), 2.83-2.73 (1H, m), 2.08-1.98 (2H, m), 1.82-1.71 (2H, m), 1.51 (6H, d, J = 6.7 Hz), 1.49-1.40 (2H, m), 1.19 (6H, d, J = 6.9 Hz), 0.95 (3H, t, J = 7.4 Hz). | 560 |
| 2-178 | 1H-NMR (DMSO-D6) δ: 11.38 (1H, s), 9.82 (1H, s), 8.33 (1H, s), 7.67 (1H, s), 5.27-5.20 (1H, m), 3.68-3.65 (4H, m), 3.50 (4H, dd, J = 10.8, 5.2 Hz), 1.89 (3H, s), 1.85-1.79 (2H, m), 1.62 (3H, d, J = 7.2 Hz). | 466 |
| 2-179 | 1H-NMR (DMSO-D6) δ: 11.31 (1H, s), 9.38 (1H, s), 8.02 (1H, s), 7.51 (1H, s), 3.66-3.61 (4H, m), 3.47 (2H, s), 3.39 (4H, br s), 3.18 (4H, s), 1.85 (3H, s), 1.81-1.76 (3H, m), 1.42 (6H, s). | 456 |
| 2-180 | 1H-NMR (DMSO-D6) δ: 11.32 (1H, s), 9.35 (1H, s), 8.30 (1H, s), 7.98 (1H, s), 4.41-4.35 (1H, m), 3.71-3.65 (4H, m), 3.55-3.48 (4H, m), 2.41 (2H, d, J = 7.0 Hz), 1.90-1.78 (3H, m), 1.38 (6H, d, J = 6.8 Hz), 0.85 (6H, d, J = 6.5 Hz). | 455 |
| 2-181 | 1H-NMR (DMSO-D6) δ: 11.42 (1H, s), 9.58 (1H, s), 8.29 (1H, s), 7.51 (1H, s), 4.36-4.30 (1H, m), 3.54-3.32 (8H, m), 3.11-3.03 (2H, m), 1.86 (3H, s), 1.84-1.74 (2H, m), 1.66-1.55 (2H, m), 1.43-1.32 (8H, m), 0.88 (3H, t, J = 7.3 Hz). | 532 |
| 2-182 | 1H-NMR (CDCl3) δ: 8.95 (1H, br s), 8.15 (1H, s), 7.95-7.91 (1H, m), 7.38-7.31 (2H, m), 6.68 (1H, s), 3.78-3.45 (8H, m), 2.38 (3H, s), 2.36-2.27 (2H, m), 2.05-1.94 (2H, m), 1.68-1.58 (2H, m), 1.41-1.31 (2H, m), 0.93 (3H, t, J = 7.3 Hz). | 532 |
| 2-183 | 1H-NMR (CDCl3) δ: 8.93 (1H, s), 8.29 (2H, d, J = 4.7 Hz), 8.14 (1H, s), 7.90 (1H, s), 7.37-7.31 (2H, m), 6.66 (1H, s), 6.49 (1H, t, J = 4.7 Hz), 3.99 (2H, t, J = 5.2 Hz), 3.90 (2H, t, J = 6.2 Hz), 3.68 (2H, t, J = 5.2 Hz), 3.50 (2H, t, J = 6.2 Hz), 2.37 (3H, s), 2.13-2.05 (2H, m). | 526 |
| 2-184 | 1H-NMR (CDCl3) δ: 9.17 (1H, br s), 7.83 (1H, s), 7.22 (1H, s), 6.49 (1H, s), 4.45-4.36 (1H, m), 4.32-4.22 (1H, m), 4.11-4.03 (1H, m), 3.95-3.87 (1H, m), 3.87-3.80 (1H, m), 3.66-3.57 (2H, m), 3.40-3.29 (1H, m), 2.83-2.73 (1H, m), 2.27-2.16 (1H, m), 1.79-1.65 (1H, m), 1.51 (6H, d, J = 6.7 Hz), 1.22-1.15 (9H, m). | 455 |
| 2-185 | 1H-NMR (CDCl3) δ: 9.06 (1H, s), 8.00 (1H, s), 7.93 (1H, s), 7.29 (1H, d, J = 7.9 Hz), 7.22 (1H, d, J = 7.9 Hz), 6.61 (1H, s), 3.98-3.65 (7H, m), 3.59-3.49 (2H, m), 3.38 (3H, s), 2.34 (3H, s), 1.95 (3H, t, J = 18.1 Hz). | 475 |
| 2-186 | 1H-NMR (CDCl3) δ: 8.92 (1H, br s), 8.20-8.12 (2H, m), 7.90 (1H, s), 7.38-7.30 (2H, m), 6.65 (1H, s), 6.38 (1H, d, J = 5.1 Hz), 4.03-3.96 (2H, m), 3.95-3.88 (2H, m), 3.70-3.64 (2H, m), 3.52-3.46 (2H, m), 2.82-2.73 (1H, m), 2.37 (3H, s), 2.13-2.05 (2H, m), 1.22 (6H, d, J = 6.9 Hz). | 568 |
| 2-187 | 1H-NMR (DMSO-D6) δ: 11.65 (1H, s), 9.68 (1H, s), 8.44 (1H, s), 8.18 (1H, s), 7.43 (1H, d, J = 8.1 Hz), 7.34 (1H, d, J = 8.1 Hz), 6.31 (1H, td, J = 55.4, 4.9 Hz), 4.20-4.17 (1H, m), 3.76-3.72 (1H, m), 3.15-3.12 (1H, m), 2.33 (3H, s), 1.76-1.73 (2H, m), 1.56-1.48 (3H, m), 1.26-1.23 (1H, m). | 482 |
| 2-188 | 1H-NMR (DMSO-D6) δ: 11.34 (1H, s), 9.62 (1H, s), 8.30 (1H, s), 7.56 (1H, s), 4.70-4.62 (1H, m), 3.66 (4H, t, J = 5.5 Hz), 3.50 (4H, dd, J = 10.8, 5.2 Hz), 2.37 (4H, dd, J = 20.7, 11.2 Hz), 1.84-1.74 (7H, m). | 424 |
| 2-189 | 1H-NMR (CDCl3) δ: 9.00 (1H, s), 7.87 (1H, s), 7.21 (1H, s), 3.85-3.79 (6H, m), 3.65 (4H, dd, J = 10.9, 5.5 Hz), 2.19-2.12 (1H, m), 2.01-1.98 (5H, m), 0.94 (6H, d, J = 6.7 Hz). | 426 |
| 2-190 | 1H-NMR (DMSO-D6) δ: 11.87 (1H, s), 9.70 (1H, s), 8.47 (1H, s), 8.23 (1H, s), 7.45 (1H, d, J = 7.9 Hz), 7.36 (1H, d, J = 7.4 Hz), 3.96 (4H, t, J = 13.2 Hz), 3.86 (2H, t, J = 5.0 Hz), 3.54 (2H, t, J = 4.9 Hz), 2.35 (3H, s). | 484 |
| 2-191 | 1H-NMR (DMSO-D6) δ: 11.33 (1H, s), 9.22 (1H, s), 8.29 (1H, s), 7.91 (1H, s), 4.40-4.34 (1H, m), 3.47-3.39 (4H, m), 3.11-3.07 (1H, m), 3.02-2.95 (1H, m), 2.06-2.00 (1H, m), 1.89-1.79 (5H, m), 1.38 (6H, d, J = 6.7 Hz), 1.16 (6H, d, J = 6.7 Hz). | 463 |
| 2-192 | 1H-NMR (DMSO-D6) δ: 11.34 (1H, s), 9.57 (1H, s), 8.28 (1H, s), 7.51 (1H, s), 4.36-4.29 (1H, m), 3.47 (2H, t, J = 6.8 Hz), 3.37 (2H, t, J = 5.5 Hz), 3.10-3.06 (1H, m), 2.03-1.99 (1H, m), 1.92-1.74 (8H, m), 1.36 (6H, d, J = 6.7 Hz). | 435 |
| 2-193 | 1H-NMR (DMSO-D6) δ: 11.32 (1H, s), 9.33 (1H, s), 8.30 (1H, s), 7.95 (1H, s), 4.41-4.34 (1H, m), 3.69-3.66 (4H, m), 3.54-3.49 (4H, m), 2.56-2.48 (2H, m), 1.86-1.81 (2H, m), 1.38 (6H, d, J = 6.8 Hz), 1.11 (3H, t, J = 7.6 Hz). | 427 |
| 2-194 | 1H-NMR (DMSO-D6) δ: 11.19 (1H, br s), 8.61 (1H, br s), 8.04 (1H, br s), 7.77 (1H, s), 4.39-4.28 (1H, m), 3.69-3.61 (4H, m), 3.48-3.38 (4H, m), 1.84-1.75 (2H, m), 1.38 (6H, d, J = 6.8 Hz), 1.23 (9H, s). | 455 |
| 2-195 | 1H-NMR (DMSO-D6) δ: 11.33 (1H, s), 9.32 (1H, s), 8.16 (1H, s), 7.90 (1H, s), 4.36-4.29 (1H, m), 3.71-3.63 (4H, m), 3.51-3.42 (4H, m), 1.91-1.79 (3H, m), 1.35 (6H, d, J = 6.8 Hz), 0.80-0.68 (4H, m). | 439 |
| 2-196 | 1H-NMR (DMSO-D6) δ: 11.31 (1H, s), 9.23 (1H, s), 8.29 (1H, s), 7.92 (1H, s), 4.43-4.36 (1H, m), 3.70-3.64 (4H, m), 3.53-3.47 (5H, m), 2.24-2.13 (4H, m), 1.95-1.75 (4H, m), 1.40 (6H, d, J = 6.8 Hz). | 453 |
| 2-197 | 1H-NMR (DMSO-D6) δ: 11.75 (1H, s), 9.69 (1H, s), 8.46 (1H, s), 8.24 (1H, s), 7.45 (1H, d, J = 8.0 Hz), 7.35 (1H, d, J = 7.5 Hz), 4.97-4.76 (1H, m), 3.92-3.77 (1H, m), 3.75-3.48 (5H, m), 3.47-3.22 (2H, m), 3.20-3.08 (2H, m), 2.35 (3H, s), 1.67-1.59 (2H, m), 1.44-1.31 (2H, m), 0.89 (3H, t, J = 7.4 Hz). | 586 |
| 2-198 | 1H-NMR (DMSO-D6) δ: 11.75 (1H, s), 9.69 (1H, s), 8.46 (1H, s), 8.24 (1H, s), 7.45 (1H, d, J = 7.8 Hz), 7.36 (1H, d, J = 8.3 Hz), 4.96-4.78 (1H, m), 4.61-4.43 (2H, m), 3.90-3.77 (1H, m), 3.75-3.54 (5H, m), 3.48-3.28 (2H, m), 3.28-3.20 (2H, m), 2.35 (3H, s), 2.11-1.98 (2H, m). | 590 |
| 2-199 | 1H-NMR (DMSO-D6) δ: 11.35 (1H, s), 9.67 (1H, s), 8.44 (1H, s), 8.22 (1H, s), 7.44 (1H, d, J = 7.9 Hz), 7.35 (1H, d, J = 8.3 Hz), 3.98-3.95 (1H, m), 3.51-3.44 (4H, m), 3.19 (3H, s), 2.35 (3H, s), 1.92-1.89 (2H, m) | 448 |
| 2-200 | 1H-NMR (DMSO-D6) δ: 11.26 (1H, br s), 9.57 (1H, s), 8.27 (1H, s), 7.50 (1H, s), 4.07 (1H, dd, J = 14.0, 6.6 Hz), 3.66 (4H, dd, J = 9.2, 3.9 Hz), 3.49 (4H, dd, J = 10.8, 5.2 Hz), 1.86-1.79 (5H, m), 1.72-1.67 (2H, m), 1.35 (3H, d, J = 6.7 Hz), 0.73 (3H, t, J = 7.4 Hz). | 426 |
| 2-201 | 1H-NMR (DMSO-D6) δ: 11.47 (1H, s), 9.67 (1H, s), 8.47 (1H, s), 8.22 (1H, s), 7.45 (1H, d, J = 7.6 Hz), 7.36 (1H, d, J = 7.6 Hz), 3.78 (2H, t, J = 6.6 Hz), 3.71 (2H, t, J = 7.2 Hz), 3.35-3.32 (2H, m), 3.17 (2H, dd, J = 11.7, 7.3 Hz), 2.41-2.37 (5H, m). | 460 |
| 2-202 | 1H-NMR (CDC13) δ: 8.96 (1H, s), 7.88 (1H, s), 6.30 (1H, s), 6.03 (1H, s), 4.42-4.35 (1H, m), 3.83 (2H, t, J = 5.3 Hz), 3.80 (2H, t, J = 4.2 Hz), 3.67-3.65 (4H, m), 3.01-2.94 (1H, m), 2.03-1.97 (2H, m), 1.47 (6H, d, J = 6.7 Hz), 1.27 (6H, d, J = 6.9 Hz). | 440 |
| 2-203 | 1H-NMR (DMSO-D6) δ: 11.50 (1H, s), 9.29 (1H, s), 8.37 (1H, s), 7.50 (1H, s), 7.08 (1H, d, J = 7.8 Hz), 6.85 (1H, d, J = 7.5 Hz), 3.56-3.31 (8H, m), 3.12-3.03 (2H, m), 2.27 (3H, s), 2.19 (3H, s), 1.85-1.76 (2H, m), 1.65-1.57 (2H, m), 1.45-1.31 (2H, m), 0.89 (3H, t, J = 7.4 Hz). | 514 |
| 2-204 | 1H-NMR (DMSO-D6) δ: 11.62 (1H, s), 9.69 (1H, s), 8.46 (1H, s), 8.23 (1H, s), 7.45 (1H, d, J = 8.0 Hz), 7.36 (1H, d, J = 8.0 Hz), 3.67-3.60 (1H, m), 3.56-3.42 (6H, m), 3.39-3.31 (2H, m), 3.29 (3H, s), 3.18-3.09 (2H, m), 2.35 (3H, s), 1.67-1.57 (2H, m), 1.44-1.32 (2H, m), 0.89 (3H, t, J = 7.3 Hz). | 598 |
| 2-205 | 1H-NMR (DMSO-D6) δ: 11.63 (1H, s), 9.69 (1H, s), 8.46 (1H, s), 8.23 (1H, s), 7.45 (1H, d, J = 8.0 Hz), 7.36 (1H, d, J = 8.0 Hz), 4.62-4.43 (2H, m), 3.65-3.39 (9H, m), 3.31-3.20 (3H, m), 3.29 (2H, s), 2.35 (3H, s), 2.13-1.95 (2H, m). | 602 |
| 2-206 | 1H-NMR (DMSO-D6) δ: 11.39 (1H, s), 9.66 (1H, s), 8.43 (1H, s), 8.22 (1H, s), 7.43 (1H, d, J = 7.9 Hz), 7.34 (1H, d, J = 8.1 Hz), 3.03 (2H, d, J = 7.2 Hz), 2.82 (3H, s), 2.34 (3H, s), 1.66-1.63 (6H, m), 1.19-1.17 (3H, m), 0.87-0.85 (2H, m). | 474 |
| 2-207 | 1H-NMR (DMSO-D6) δ: 11.59 (1H, s), 9.68 (1H, s), 8.44 (1H, s), 8.22 (1H, s), 7.44 (1H, d, J = 7.9 Hz), 7.38-7.30 (6H, m), 7.23-7.22 (1H, m), 4.60 (2H, s), 4.14-4.07 (1H, m), 2.34 (3H, s), 1.00 (6H, d, J = 6.7 Hz). | 496 |
| 2-208 | 1H-NMR (DMSO-D6) δ: 11.40 (1H, s), 9.66 (1H, s), 8.43 (1H, s), 8.23 (1H, s), 7.43 (1H, d, J = 8.1 Hz), 7.34 (1H, d, J = 8.1 Hz), 3.09 (2H, d, J = 7.9 Hz), 2.85 (3H, s), 2.34 (3H, s), 2.19-2.12 (1H, m), 1.63-1.52 (6H, m), 1.23-1.16 (2H, m). | 460 |
| 2-209 | 1H-NMR (DMSO-D6) δ: 11.44 (1H, s), 10.13 (1H, s), 8.38 (1H, s), 7.94 (1H, s), 7.62 (1H, t, J = 59.3 Hz), 3.68-3.64 (4H, m), 3.50-3.48 (4H, m), 1.93 (3H, s), 1.84-1.78 (2H, m). | 421 |
| 2-210 | 1H-NMR (CDCl3+TFA) δ: 8.17-7.66 (3H, m), 7.47-7.36 (2H, m), 6.37-6.29 (1H, m), 4.13-3.42 (11H, m), 2.36 (3H, s), 2.08-1.94 (2H, m). (-2NH) | 556 |
| 2-211 | 1H-NMR (CDCl3+TFA) δ: 8.36-7.46 (3H, m), 7.45-7.35 (2H, m), 6.79-6.73 (1H, m), 4.18-3.84 (4H, m), 3.79-3.64 (2H, m), 3.59-3.46 (2H, m), 2.58 (3H, s), 2.37 (3H, s), 2.10-1.96 (2H, m).(-2NH) | 540 |
| 2-212 | 1H-NMR (DMSO-D6) δ: 11.70 (1H, s), 9.60 (1H, s), 8.31 (1H, s), 7.51 (1H, s), 4.36-4.30 (1H, m), 3.94 (4H, td, J = 13.1, 8.4 Hz), 3.84 (2H, t, J = 4.9 Hz), 3.52 (2H, t, J = 4.9 Hz), 1.86 (3H, s), 1.36 (6H, d, J = 6.7 Hz). | 448 |
| 2-213 | 1H-NMR (DMSO-D6) δ: 11.48 (1H, br s), 9.67 (1H, s), 8.41 (1H, s), 8.16 (1H, d, J = 6.7 Hz), 7.42 (1H, d, J = 11.1 Hz), 3.67-3.65 (4H, m), 3.51-3.48 (4H, m), 2.33 (3H, s), 1.84-1.79 (2H, m) . | 467 |
| 2-214 | 1H-NMR (CDC13+TFA) δ: 8.76-8.70 (1H, m), 8.36-8.28 (1H, m), 7.99-7.93 (2H, m), 7.44-7.36 (2H, m), 6.81-6.74 (1H, m), 4.27-4.22 (1H, m), 4.19-4.13 (1H, m), 3.93-3.84 (3H, m), 3.80-3.75 (1H, m), 3.66-3.61 (1H, m), 3.59-3.54 (1H, m), 2.39 (3H, s), 2.19-2.05 (2H, m).(-2NH) | 526 |
| 2-215 | 1H-NMR (CDC13) δ: 8.94 (1H, br s), 8.15 (1H, s), 7.95-7.91 (1H, m), 7.38-7.31 (2H, m), 6.68 (1H, s), 3.78-3.45 (8H, m), 2.38 (3H, s), 2.34-2.27 (2H, m), 2.07-1.94 (2H, m), 1.75-1.62 (2H, m), 0.97 (3H, t, J = 7.4 Hz). | 518 |
| 2-216 | 1H-NMR (CDC13) δ: 8.96 (1H, br s), 8.15 (1H, s), 7.93 (1H, s), 7.39-7.30 (2H, m), 6.68 (1H, s), 4.09-3.42 (12H, m), 3.29-3.17 (1H, m), 2.38 (3H, s), 2.27-1.92 (4H, m) . | 546 |
| 2-217 | 1H-NMR (DMSO-D6) δ: 11.98 (1H, br s), 9.54 (1H, br s), 8.34 (1H, s), 8.27-8.19 (1H, m), 8.23 (1H, s), 7.49-7.41 (1H, m), 7.38-7.28 (1H, m), 7.28 (1H, s), 4.28-4.19 (2H, m), 3.63-3.56 (2H, m), 3.50-3.42 (2H, m) , 3.15-3.07 (2H, m), 2.34 (3H, s), 1.91 (3H, s) . | 486 |
| 2-218 | 1H-NMR (CDC13) δ: 9.08 (1H, s), 8.18 (1H, s), 7.95 (1H, s), 7.37-7.30 (2H, m), 6.67 (1H, s), 3.98-3.83 (4H, m), 3.81-3.66 (3H, m), 3.61-3.52 (2H, m), 3.38 (3H, s) , 2.38 (3H, s). | 479 |
| 2-219 | 1H-NMR (CDC13) δ: 9.08 (1H, s), 8.18 (1H, s), 7.95 (1H, s), 7.38-7.30 (2H, m), 6.67 (1H, s), 3.98-3.83 (4H, m), 3.81-3.65 (3H, m), 3.61-3.52 (2H, m), 3.38 (3H, s), 2.38 (3H, s) . | 479 |
| 2-220 | 1H-NMR (CDC13) δ: 8.95 (1H, s), 8.17 (1H, s), 7. 94 (1H, d, J = 0.7 Hz), 7.37-7.32 (2H, m), 6.66 (1H, s), 4.12-3.93 (3H, m), 3.89-3.75 (2H, m), 3.73-3.64 (4H, m), 3.58-3.51 (4H, m), 2.38 (3H, s), 2.36-2.21 (2H, m), 2.10-2.01 (2H, m). | 582 |
| 2-221 | 1H-NMR (DMSO-D6) δ: 11.61 (1H, s), 9.67 (1H, s), 8.45 (1H, s), 8.20 (1H, s), 7.43 (1H, d, J = 8.1 Hz), 7.34 (1H, d, J = 7.6 Hz), 3.76 (1H, dd, J = 9.7, 7.9 Hz), 3.61-3.40 (4H, m), 2.34 (3H, s), 2.26-2.23 (1H, m), 2.13-2.10 (1H, m). | 443 |
| 2-222 | 1H-NMR (DMSO-D6) δ: 11.74 (1H, s), 9.70 (1H, s), 8.47 (1H, s), 8.23 (1H, s), 7.45 (1H, d, J = 8.0 Hz), 7.36 (1H, d, J = 7.3 Hz), 4.96-4.75 (1H, m), 3.92-3.76 (1H, m), 3.76-3.28 (8H, m), 3.16-3.06 (2H, m), 2.35 (3H, s), 1.70-1.63 (2H, m), 0.97 (3H, t, J = 7.4 Hz). | 572 |
| 2-223 | 1H-NMR (DMSO-D6) δ: 11.51-11.36 (1H, br m), 9.58 (1H, s), 8.38 (1H, s), 7.83 (1H, s), 7.48 (1H, d, J = 8.0 Hz), 7.35 (1H, d, J = 8.0 Hz), 7.00 (1H, t, J = 55.9 Hz), 3.58-3.17 (9H, m), 2.26 (2H, q, J = 7.3 Hz), 1.83-1.65 (2H, m), 1.56-1.44 (2H, m), 1.17 (6H, d, J = 6.8 Hz), 0.87 (3H, t, J = 7.4 Hz). | 528 |
| 2-224 | 1H-NMR (DMSO-D6) δ: 11.49 (1H, s), 9.59 (1H, s), 8.39 (1H, s), 7.83 (1H, s), 7.48 (1H, d, J = 8.0 Hz), 7.35 (1H, d, J = 7.8 Hz), 7.00 (1H, t, J = 55.9 Hz), 3.53-3.26 (9H, m), 3.09-3.03 (2H, m), 1.84-1.77 (2H, m), 1.72-1.61 (2H, m) , 1.17 (6H, d, J = 6.8 Hz), 0.96 (3H, t, J = 7.4 Hz). | 564 |
| 2-225 | 1H-NMR (DMSO-D6) δ: 11.49 (1H, s), 9.59 (1H, s), 8.39 (1H, s), 7.83 (1H, s), 7.48 (1H, d, J = 8.3 Hz), 7.35 (1H, d, J = 8.3 Hz), 7.00 (1H, t, J = 55.9 Hz), 3.54-3.26 (9H, m), 3.12-3.03 (2H, m), 1.84-1.76 (2H, m), 1.66-1.55 (2H, m), 1.43-1.32 (2H, m), 1.17 (6H, d, J = 6.8 Hz), 0.88 (3H, t, J = 7.3 Hz). | 578 |
| 2-226 | 1H-NMR (DMSO-D6) δ: 11.50 (1H, s), 9.59 (1H, s), 8.39 (1H, s), 7.83 (1H, s), 7.48 (1H, d, J = 8.0 Hz), 7.35 (1H, d, J = 7.5 Hz), 7.00 (1H, t, J = 55.9 Hz), 4.12-4.03 (1H, m), 3.96-3.78 (4H, m), 3.71-3.62 (1H, m), 3.55-3.37 (7H, m), 3.35-3.24 (1H, m), 2.25-2.14 (1H, m), 2.13-2.03 (1H, m), 1.85-1.77 (2H, m), 1.17 (6H, d, J = 6.8 Hz) . | 592 |
| 2-227 | 1H-NMR (DMSO-D6+TFA) δ: 11.35 (1H, br s), 8.82 (1H, s), 8.25 (1H, s), 4.47-4.40 (1H, m), 3.70-3.63 (4H, m), 3.54-3.46 (4H, m), 2.86-2.79 (1H, m), 2.08 (3H, s), 1.85-1.80 (2H, m), 1.36 (6H, d, J = 6.5 Hz), 1.13 (6H, d, J = 7.0 Hz). | 455 |
| 2-228 | 1H-NMR (DMSO-D6) δ: 11.49 (1H, s), 9.29 (1H, s), 8.37 (1H, s), 7.50 (1H, s), 7.08 (1H, d, J = 7.8 Hz), 6.85 (1H, d, J = 8.0 Hz), 3.54-3.35 (6H, m), 3.09-3.03 (2H, m), 2.28 (3H, s), 2.19 (3H, s), 1.85-1.76 (2H, m), 1.72-1.61 (2H, m), 0.97 (3H, t, J = 7.4 Hz). | 500 |
| 2-229 | 1H-NMR (DMSO-D6) δ: 11.50 (1H, s), 9.29 (1H, s), 8.37 (1H, s), 7.50 (1H, s), 7.08 (1H, d, J = 7.5 Hz), 6.85 (1H, d, J = 7.8 Hz), 3.57-3.38 (8H, m), 2.69-2.60 (1H, m), 2.27 (3H, s), 2.19 (3H, s), 1.87-1.79 (2H, m), 0.99-0.92 (4H, m) . | 498 |
| 2-230 | 1H-NMR (DMSO-D6) δ: 11.51 (1H, s), 9.68 (1H, s), 8.45 (1H, s), 8.23 (1H, s), 7.45 (1H, d, J = 8.0 Hz), 7.35 (1H, d, J = 8.0 Hz), 3.94-3.86 (1H, m), 3.76-3.59 (3H, m), 3.56-3.48 (1H, m), 3.37-3.27 (2H, m), 3.07-2.97 (1H, m), 2.35 (3H, s), 1.89-1.80 (2H, m), 1.07 (3H, d, J = 6.3 Hz). | 463 |
| 2-231 | 1H-NMR (DMSO-D6) δ: 11.45 (1H, s), 9.67 (1H, s), 8.44 (1H, s), 8.21 (1H, s), 7.44 (1H, d, J = 8.0 Hz), 7.35 (1H, d, J = 8.0 Hz), 4.23-4.14 (1H, m), 3.86-3.67 (3H, m), 3.60-3.51 (1H, m), 3.46-3.32 (2H, m), 2.35 (3H, s), 1.92-1.79 (1H, m), 1.76-1.65 (1H, m), 1.05 (3H, d, J = 6.5 Hz). | 463 |
| 2-232 | 1H-NMR (DMSO-D6) δ: 11.53-11.45 (1H, m), 9.67 (1H, s), 8.45 (1H, d, J = 2.0 Hz), 8.24 (1H, s), 7.44 (1H, d, J = 8.0 Hz), 7.35 (1H, d, J = 8.3 Hz), 4.48-3.72 (1H, m), 3.64-3.45 (2H, m), 3.43-3.24 (2H, m), 2.80-2.61 (3H, m), 2.35 (3H, s), 2.02-1.93 (3H, m), 1.92-1.51 (6H, m). | 518 |
| 2-233 | 1H-NMR (DMSO-D6) δ: 11.37 (1H, s), 9.66 (1H, s), 8.43 (1H, s), 8.23 (1H, s), 7.44 (1H, d, J = 7.9 Hz), 7.35 (1H, d, J = 9.0 Hz), 3.51 (2H, dtd, J = 35.3, 9.5, 4.2 Hz), 3.35-3.21 (2H, m), 2.35 (3H, s), 1.86-1.78 (2H, m), 1.71-1.65 (1H, m), 1.56-1.43 (2H, m), 1.33-1.14 (6H, m), 0.85 (3H, dd, J = 8.9, 5.2 Hz). | 488 |
| 2-234 | 1H-NMR (DMSO-D6) δ: 11.57 (1H, s), 9.66 (1H, s), 8.44 (1H, s), 8.23 (1H, s), 7.44 (1H, d, J = 7.6 Hz), 7.35 (1H, d, J = 7.9 Hz), 3.52-3.49 (2H, m), 3.44 (2H, t, J = 6.1 Hz), 2.35 (3H, s), 2.26-2.04 (4H, m), 1.83-1.77 (2H, m). | 482 |
| 2-235 | 1H-NMR (DMSO-D6) δ: 11.43 (1H, s), 9.66 (1H, s), 8.44 (1H, s), 8.22 (1H, s), 7.43 (1H, d, J = 7.9 Hz), 7.34 (1H, d, J = 7.9 Hz), 3.34-3.24 (4H, m), 3.20 (3H, s), 2.85 (3H, s), 2.34 (3H, s), 1.74-1.73 (2H, m). | 450 |
| 2-236 | 1H-NMR (DMSO-D6) δ: 11.76 (1H, s), 9.70 (1H, s), 8.65 (1H, d, J = 1.4 Hz), 8.52-8.49 (2H, m), 8.46 (1H, s), 8.20 (1H, s), 7.45 (1H, d, J = 7.9 Hz), 7.36 (1H, d, J = 8.1 Hz), 4.66 (2H, s), 2.90 (3H, s), 2.35 (3H, s). | 470 |
| 2-237 | 1H-NMR (CDCl3) δ: 11.50 (1H, s), 9.67 (1H, s), 8.44 (1H, s), 8.24 (1H, s), 7.66 (1H, s), 7.45 (1H, d, J = 8.1 Hz), 7.35 (2H, d, J = 8.3 Hz), 4.09-4.07 (2H, m), 3.77 (3H, s), 2.78 (3H, s), 2.36 (3H, s). | 472 |
| 2-238 | 1H-NMR (DMSO-D6) δ: 11.06 (1H, s), 9.27 (1H, s), 8.33 (1H, s), 7.41 (1H, s), 6.94 (1H, s), 3.33-3.31 (2H, m), 3.23 (3H, s), 3.15 (3H, s), 3.07 (1H, t, J = 6.9 Hz), 2.83 (6H, s), 2.57 (2H, t, J = 7.9 Hz), 2.13 (3H, s), 1.72-1.68 (2H, m), 1.15 (6H, d, J = 6.9 Hz). | 438 |
| 2-239 | 1H-NMR (DMSO-D6) δ: 11.73 (1H, s), 9.68 (1H, s), 8.45 (1H, s), 8.23 (1H, s), 7.45 (1H, d, J = 8.1 Hz), 7.36 (1H, d, J = 7.9 Hz), 3.73 (2H, t, J = 5.5 Hz), 3.53 (2H, t, J = 6.2 Hz), 3.42 (2H, t, J = 5.3 Hz), 3.29 (2H, dd, J = 12.6, 6.4 Hz), 2.35 (3H, s), 2.06-2.00 (2H, m). | 496 |
| 2-240 | 1H-NMR (DMSO-D6) δ: 11.51 (1H, s), 9.65 (1H, s), 8.43 (1H, s), 8.23 (1H, s), 7.44 (1H, d, J = 8.1 Hz), 7.35 (1H, d, J = 9.5 Hz), 3.76 (1H, dq, J = 14.8, 2.8 Hz), 3.47-3.26 (3H, m), 2.35 (3H, s), 2.07 (1H, d, J = 10.9 Hz), 1.97-1.92 (2H, m), 1.79-1.53 (5H, m), 0.98 (3H, t, J = 7.4 Hz). | 499 |
| 2-241 | 1H-NMR (DMSO-D6) δ: 11.54 (1H, s), 9.67 (1H, s), 8.44 (1H, s), 8.22 (1H, s), 7.44 (1H, d, J = 8.0 Hz), 7.35 (1H, d, J = 7.8 Hz), 4.18-4.08 (1H, m), 4.02-3.92 (1H, m), 3.67-3.39 (4H, m), 3.30-3.05 (5H, m), 2.35 (3H, s), 1.19 (3H, t, J = 7.4 Hz), 1.03 (3H, d, J = 6.5 Hz). | 554 |
| 2-242 | 1H-NMR (DMSO-D6) δ: 11.60 (1H, s), 9.68 (1H, s), 8.45 (1H, s), 8.24 (1H, s), 7.45 (1H, d, J = 8.3 Hz), 7.35 (1H, d, J = 7.5 Hz), 4.02-3.92 (1H, m), 3.89-3.81 (1H, m), 3.69-3.58 (2H, m), 3.31-2.95 (5H, m), 2.35 (3H, s), 1.84-1.66 (2H, m), 1.22 (3H, t, J = 7.4 Hz), 1.10 (3H, d, J = 6.5 Hz). | 554 |
| 2-243 | 1H-NMR (DMSO-D6) δ: 11.28 (1H, br s), 8.81 (1H, s), 8.24 (1H, s), 4.44-4.38 (1H, m), 3.69-3.61 (4H, m), 3.53-3.44 (4H, m), 2.09 (3H, s), 2.00-1.98 (3H, m), 1.85-1.79 (2H, m), 1.34 (6H, d, J = 6.8 Hz). | 427 |
| 2-244 | 1H-NMR (CDC13) δ: 9.03 (1H, s), 8.05 (1H, d, J = 6.5 Hz), 7.92 (1H, s), 7.11 (1H, d, J = 10.2 Hz), 6.51 (1H, s), 3.97-3.82 (4H, m), 3.80-3.65 (3H, m), 3.59-3.50 (2H, m), 3.37 (3H, s) , 2.37 (3H, s). | 497 |
| 2-245 | 1H-NMR (DMSO-D6) δ: 11.50 (1H, s), 9.61 (1H, s), 8.33 (1H, s), 8.24 (1H, s), 8.19 (1H, s), 8.04-8.00 (1H, m), 7.76 (1H, d, J = 2.5 Hz), 7.43 (1H, d, J = 7.5 Hz), 7.34 (1H, d, J = 7.5 Hz), 3.84-3.79 (2H, m), 3.77-3.72 (2H, m), 3.59-3.52 (2H, m) , 3.40-3.35 (2H, m), 2.34 (3H, s), 1.90-1.81 (2H, m). | 526 |
| 2-246 | 1H-NMR (CDC13) δ: 8.93 (1H, br s), 8.15 (1H, s), 7.93 (1H, s), 7.37-7.30 (2H, m), 6.73 (1H, br s), 4.00-3.91 (1H, m), 3.78-3.68 (1H, m), 3.60-3.50 (1H, m), 3.45-3.33 (2H, m), 3.26-3.15 (1H, m), 3.02 (1.5H, s), 2.91 (1.5H, s), 2.86-2.78 (1H, m), 2.38 (3H, s), 2.15-2.02 (1H, m), 2.01-1.86 (3H, m), 1.80-1.66 (2H, m), 1.20 (1.5H, t, J = 7.2 Hz), 1.09 (1.5H, t, J = 7.2 Hz). | 532 |
| 2-247 | 1H-NMR (DMSO-D6) δ: 11.65 (1H, s), 9.68 (1H, s), 8.46 (1H, s), 8.22 (1H, s), 7.45 (1H, d, J = 7.9 Hz), 7.35 (1H, d, J = 7.9 Hz), 3.36 (4H, dd, J = 6.1, 3.4 Hz), 3.27 (4H, dd, J = 6.0, 3.2 Hz), 3.06 (2H, t, J = 7.7 Hz), 2.35 (3H, s), 1.62 (2H, dt, J = 16.3, 6.8 Hz), 1.37 (2H, td, J = 14.8, 7.4 Hz), 0.87 (3H, t, J = 7.4 Hz). | 553 |
| 2-248 | 1H-NMR (DMSO-D6) δ: 11.28 (1H, s), 8.56 (1H, s), 8.22 (1H, s), 4.33-4.28 (1H, m), 4.22-4.17 (2H, m), 3.68-3.64 (4H, m), 3.49 (4H, dd, J = 10.8, 5.0 Hz), 2.79-2.72 (1H, m), 2.30-2.23 (1H, m), 1.93-1.79 (3H, m), 1.44 (3H, d, J = 6.5 Hz), 1.11 (6H, d, J = 6.9 Hz). | 468 |
| 2-249 | 1H-NMR (DMSO-D6) δ: 11.50 (1H, s), 9.66 (1H, s), 8.44 (1H, s), 8.21 (1H, s), 7.43 (1H, d, J = 7.9 Hz), 7.34 (1H, d, J = 8.1 Hz), 3.74 (1H, dq, J = 14.9, 2.8 Hz), 3.46-3.26 (3H, m), 2.33 (3H, s), 2.06 (1H, dd, J = 14.7, 5.0 Hz), 1.92 (2H, dd, J = 15.0, 9.9 Hz), 1.81-1.65 (2H, m), 1.56 (3H, dd, J = 14.2, 9.6 Hz), 1.32 (4H, ddd, J = 25.4, 13.6, 6.5 Hz), 0.87 (3H, dd, J = 11.6, 4.6Hz) . | 527 |
| 2-250 | 1H-NMR (DMSO-D6) δ: 11.51 (1H, s), 9.67 (1H, s), 8.45 (1H, s), 8.22 (1H, s), 7.44 (1H, d, J = 8.1 Hz), 7.35 (1H, d, J = 8.3 Hz), 3. 76 (1H, dt, J = 12.3, 2.9 Hz), 3.51-3.30 (5H, m), 3.24 (3H, s), 2.35 (3H, s), 2.11 (1H, dd, J = 14.8, 5.3 Hz), 2.03-1.73 (6H, m), 1.63 (1H, t, J = 11.8 Hz). | 529 |
| 2-251 | 1H-NMR (DMSO-D6) δ: 11.51 (1H, s), 9.29 (1H, s), 8.37 (1H, s), 7.50 (1H, s), 7.08 (1H, d, J = 7.8 Hz), 6.85 (1H, d, J = 8.0 Hz), 3.66-3.59 (1H, m), 3.55-3.41 (7H, m), 3.38-3.31 (1H, m), 3.28 (3H, s), 3.15-3.07 (2H, m), 2.28 (3H, s), 2.19 (3H, s), 1.72-1.60 (2H, m), 0.97 (3H, t, J = 7.5 Hz). | 530 |
| 2-252 | 1H-NMR (DMSO-D6) δ: 11.51 (1H, s), 9.29 (1H, s), 8.37 (1H, s), 7.50 (1H, s), 7.08 (1H, d, J = 7.8 Hz), 6.85 (1H, d, J = 7.5 Hz), 3.66-3.59 (1H, m), 3.56-3.41 (7H, m), 3.39-3.29 (1H, m), 3.28 (3H, s), 3.14 (2H, q, J = 7.4 Hz), 2.27 (3H, s), 2.19 (3H, s), 1.19 (3H, t, J = 7.3 Hz). | 516 |
| 2-253 | 1H-NMR (CDC13) δ: 8.93 (1H, s), 8.16 (1H, s), 7.92 (1H, d, J = 1.2 Hz), 7.37-7.30 (2H, m), 6.69 (1H, s), 4.00-3.91 (1H, m) , 3.78-3.69 (1H, m), 3.60-3.50 (1H, m), 3.36-3.15 (3H, m) , 3.02 (1.5H, s), 2.91 (1.5H, s), 2.88-2.78 (1H, m), 2.38 (3H, s), 2.17-2.02 (1H, m), 2.02-1.86 (3H, m), 1.80-1.67 (2H, m), 1.66-1.49 (2H, m), 0.94 (1.5H, t, J = 7.5 Hz), 0.88 (1.5H, t, J = 7.5 Hz). | 546 |
| 2-254 | 1H-NMR (DMSO-D6) δ: 9.21 (1H, s), 8.41 (1H, s), 7.77 (1H, s), 7.38 (1H, d, J = 8.3 Hz), 7.24 (1H, d, J = 9.0 Hz), 7.17 (1H, d, J = 1.6 Hz), 6.07 (1H, d, J = 1.6 Hz), 4.34 (2H, s), 4.27-4.25 (2H, m), 3.38-3.37 (2H, m), 2.33 (3H, s), 1.83-1.80 (2H, m). (-NH) | 485 |
| 2-255 | 1H-NMR (DMSO-D6) δ: 11.49 (1H, s), 9.59 (1H, s), 8.29-8.27 (2H, br m), 7.43 (1H, d, J = 8.1 Hz), 7.33 (1H, d, J = 7.6 Hz), 4.34 (1H, br s), 3.82-3.54 (2H, br m), 3.47 (1H, br s), 3.31 (1H, br s), 2.82 (3H, s), 2.32 (5H, dd, J = 16.0, 8.3 Hz), 1.68 (4H, d, J = 77.7 Hz), 0.98 (3H, t, J = 7.4 Hz). | 517 |
| 2-256 | 1H-NMR (DMSO-D6) δ: 11.44 (1H, s), 9.30 (1H, s), 8.36 (1H, s), 7.49 (1H, s), 7.11 (1H, d, J = 7.8 Hz), 6.89 (1H, d, J = 7.8 Hz), 3.54-3.33 (8H, m), 3.09-3.02 (2H, m), 2.57 (2H, q, J = 7.6 Hz), 2.19 (3H, s), 1.85-1.76 (2H, m), 1.71-1.61 (2H, m), 1.17 (3H, t, J = 7.6 Hz), 0.97 (3H, t, J = 7.4 Hz). | 514 |
| 2-257 | 1H-NMR (DMSO-D6) δ: 11.47 (1H, s), 9.29 (1H, s), 8.35 (1H, s), 7.44 (1H, s), 7.11 (1H, d, J = 7.8 Hz), 6.91 (1H, d, J = 8.8 Hz), 3.52-3.32 (8H, m), 3.08-3.04 (2H, m), 2.59 (2H, q, J = 7.5 Hz), 2.28 (3H, s), 1.84-1.77 (2H, m), 1.71-1.61 (2H, m), 1.09 (3H, t, J = 7.5 Hz), 0.97 (3H, t, J = 7.4 Hz). | 514 |
| 2-258 | 1H-NMR (DMSO-D6) δ: 11.43 (1H, s), 9.30 (1H, s), 8.35 (1H, s), 7.44 (1H, d, J = 1.5 Hz), 7.14 (1H, d, J = 7.8 Hz), 6.96 (1H, dd, J = 7.8, 1.5 Hz), 3.52-3.36 (8H, m), 3.08-3.04 (2H, m), 2.62-2.55 (4H, m), 1.85-1.75 (2H, m), 1.72-1.60 (2H, m), 1.17 (3H, t, J = 7.5 Hz), 1.10 (3H, t, J = 7.5 Hz), 0.96 (3H, t, J = 7.4 Hz). | 528 |
| 2-259 | 1H-NMR (DMSO-D6) δ: 11.38 (1H, s), 9.67 (1H, s), 8.43 (1H, s), 8.23 (1H, s), 7.44 (1H, d, J = 7.8 Hz), 7.35 (1H, d, J = 7.8 Hz), 3.60-3.41 (2H, m), 3.27-3.14 (2H, m), 2.94 (6H, s), 2.35 (3H, s), 2.31-2.08 (1H, m), 1.85-1.71 (1H, m), 1.68-1.41 (3H, m), 1.31-1.15 (4H, m). | 532 |
| 2-260 | 1H-NMR (DMSO-D6) δ: 11.54 (1H, s), 9.66 (1H, s), 8.43 (1H, s), 8.23 (1H, s), 7.44 (1H, d, J = 7.9 Hz), 7.35 (1H, d, J = 8.1 Hz), 3.74 (1H, dt, J = 15.1, 4.5 Hz), 3.48 (2H, dt, J = 19.8, 8.1 Hz), 3.36 (1H, dt, J = 13.7, 4.6 Hz), 3.19 (3H, s), 2.91 (3H, s), 2.35 (3H, s), 2.27 (1H, d, J = 16.4 Hz), 2.18 (2H, dt, J = 15.6, 5.5 Hz), 2.03-1.82 (3H, m). | 542 |
| 2-261 | 1H-NMR (DMSO-D6) δ: 11.54 (1H, s), 9.64 (1H, s), 8.40 (1H, s), 8.24 (1H, s), 7.44 (1H, d, J = 7.9 Hz), 7.34 (1H, d, J = 9.0 Hz), 3.75 (1H, ddd, J = 15.0, 5.3, 3.4 Hz), 3.68 (2H, t, J = 6.5 Hz), 3.42 (5H, dt, J = 36.2, 10.7 Hz), 2.35 (3H, s), 2.20 (4H, tt, J = 17.3, 7.1 Hz), 2.04-1.76 (6H, m). | 568 |
| 2-262 | 1H-NMR (CDC13) δ: 8.97 (1H, br s), 8.16 (1H, s), 7. 94 (1H, s), 7.37-7.31 (2H, m), 6.67 (1H, br s), 4.05-3.97 (1H, m), 3.91-3.82 (1H, m), 3.82-3.55 (4H, m), 3.53-3.44 (1H, m), 2.38 (3H, s), 2.09-1.99 (1H, m), 1.83-1.72 (1H, m), 1.22 (3H, d, J = 6.2 Hz). | 463 |
| 2-263 | 1H-NMR (CDC13) δ: 8.96 (1H, br s), 8.15 (1H, s), 7. 94 (1H, s), 7.37-7.31 (2H, m), 6.68 (1H, br s), 4.49-4.43 (1H, m), 4.16-4.09 (1H, m), 3.99-3.91 (1H, m), 3.89-3.81 (1H, m), 3.75-3.68 (1H, m), 3.65-3.56 (1H, m), 3.54-3.46 (1H, m), 3.06 (3H, s), 2.96 (3H, s), 2.38 (3H, s), 2.36-2.21 (2H, m). | 520 |
| 2-264 | 1H-NMR (CDC13) δ: 8.98 (1H, br s), 8.15 (1H, s), 7.95 (1H, s), 7.38-7.32 (2H, m), 6.69 (1H, br s), 4.72-4.66 (1H, m), 4.14-4.06 (1H, m), 3.91-3.50 (5H, m), 2.50-2.24 (2H, m), 2.38 (3H, s). | 474 |
| 2-265 | 1H-NMR (DMSO-D6) δ: 11.57 (1H, s), 9.68 (1H, s), 8.45 (1H, s), 8.38 (2H, d, J = 4.6 Hz), 8.20 (1H, s), 7.44 (1H, d, J = 7.9 Hz), 7.35 (1H, d, J = 7.4 Hz), 6.68 (1H, t, J = 4.7 Hz), 3.84 (4H, t, J = 5.1 Hz), 3.36 (4H, t, J = 5.1 Hz), 2.34 (3H, s). | 511 |
| 2-266 | 1H-NMR (DMSO-D6) δ: 11.63 (1H, s), 9.56 (1H, s), 8.29 (2H, s), 7.43 (1H, d, J = 7.6 Hz), 7.32 (1H, d, J = 7.9 Hz), 3.33-3.24 (8H, m), 2.76 (6H, s), 2.35 (3H, s). | 540 |
| 2-267 | 1H-NMR (DMSO-D6) δ: 11.63 (1H, s), 9.65 (1H, s), 8.42 (1H, s), 8.23 (1H, s), 7.44 (1H, d, J = 8.1 Hz), 7.35 (1H, d, J = 9.0 Hz), 3.35-3.33 (9H, m), 2.35 (3H, s), 1.20 (6H, d, J = 6.7 Hz). | 539 |
| 2-268 | 1H-NMR (DMSO-D6) δ: 11.50 (1H, s), 9.67 (1H, s), 8.44 (1H, s), 8.22 (1H, s), 7.44 (1H, d, J = 7.9 Hz), 7.35-7.34 (5H, m), 7.30-7.26 (1H, m), 4.56 (2H, s), 3.39 (2H, t, J = 5.8 Hz), 3.31 (2H, t, J = 5.2 Hz), 3.06 (3H, s), 2.34 (3H, s). | 512 |
| 2-269 | 1H-NMR (DMSO-D6) δ: 11.50 (1H, s), 9.35 (1H, s), 8.38 (1H, s), 7.84 (1H, d, J = 8.0 Hz), 7.24-7.18 (2H, m), 7.05-6.99 (1H, m), 3.53-3.36 (8H, m), 3.08-3.04 (2H, m), 2.26 (3H, s), 1.85-1.77 (2H, m), 1.71-1.61 (2H, m), 0.97 (3H, t, J = 7.5 Hz). | 486 |
| 2-270 | 1H-NMR (DMSO-D6) δ: 11.52 (1H, s), 9.56 (1H, s), 8.43 (1H, s), 8.01 (1H, s), 7.36 (1H, d, J = 8.0 Hz), 7.22 (1H, d, J = 7.8 Hz), 7.00 (1H, t, J = 55.9 Hz), 3.53-3.36 (8H, m), 3.08-3.04 (2H, m), 2.31 (3H, s), 1.86-1.76 (2H, m), 1.72-1.61 (2H, m), 0.97 (3H, t, J = 7.4 Hz). | 536 |
| 2-271 | 1H-NMR (DMSO-D6) δ: 12.07 (1H, s), 10.20 (1H, br s), 8.90 (1H, s), 8.71 (1H, s), 7.84 (1H, d, J = 7.5 Hz), 7.29 (1H, t, J = 8.0 Hz), 6.80 (1H, d, J = 7.5 Hz), 3.63-'3.35 (8H, m), 3.11-3.04 (2H, m), 1.88-1.78 (2H, m), 1.73-1.60 (2H, m), 0.97 (3H, t, J = 7.4 Hz). | 513 |
| 2-272 | 1H-NMR (CDC13) δ: 9.01 (1H, br s), 8.15 (1H, s), 7. 94 (1H, s), 7.37-7.30 (2H, m), 6.68 (1H, br s), 4.16-4.09 (1H, m), 3.96-3.87 (1H, m), 3.85-3.42 (7H, m), 2.38 (3H, s), 2.04-1.96 (1H, m), 1.84-1.71 (1H, m) . (-OH) | 479 |
| 2-273 | 1H-NMR (CDC13) δ: 8.96 (1H, br s), 8.15 (1H, s), 7. 94 (1H, s), 7.50 (1H, d, J = 1.8 Hz), 7.42 (1H, d, J = 2.3 Hz), 7.37-7.31 (2H, m), 6.68 (1H, br s), 6.26-6.23 (1H, m), 4.46-4.37 (1H, m), 3.91-3.82 (1H, m), 3.80-3.73 (1H, m), 3.62-3.45 (2H, m), 2.41-1.99 (5H, m), 2.38 (3H, s), 1.89-1.76 (1H, m) . | 513 |
| 2-274 | 1H-NMR (DMSO-D6) δ: 11.58 (1H, s), 9.68 (1H, s), 8.47 (1H, s), 8.22 (1H, s), 7.44 (1H, d, J = 8.1 Hz), 7.35 (1H, dd, J = 8.0, 1.3 Hz), 7.22 (2H, dd, J = 8.7, 7.3 Hz), 6.96 (2H, d, J = 7.9 Hz), 6.81 (1H, t, J = 7.3 Hz), 3.42 (4H, dd, J = 6.2, 3.7 Hz), 3.22 (4H, t, J = 5.0 Hz), 2.35 (3H, s). | 509 |
| 2-275 | 1H-NMR (DMSO-D6) δ: 11.37 (1H, s), 9.20 (1H, s), 8.35-8.32 (2H, m), 8.24 (1H, br s), 7.51 (1H, s), 7.07 (1H, d, J = 7.5 Hz), 6.83 (1H, d, J = 7.5 Hz), 6.60 (1H, t, J = 4.8 Hz), 3.89-3.77 (4H, m), 3.56-3.50 (2H, m), 3.39-3.33 (2H, m), 2.27 (3H, s), 2.19 (3H, s), 1.87-1.79 (2H, m) . | 472 |
| 2-276 | 1H-NMR (DMSO-D6) δ: 11.54 (1H, s), 9.68 (1H, s), 8.45 (1H, s), 8.23 (1H, s), 7.44 (1H, d, J = 7.8 Hz), 7.35 (1H, d, J = 8.0 Hz), 3.73-3.65 (1H, m), 3.51-3.27 (3H, m), 2.82 (6H, s), 2.35 (3H, s), 2.24-2.11 (2H, m), 2.02-1.92 (1H, m), 1.83-1.70 (1H, m), 1.68-1.56 (2H, m), 1.27-1.21 (1H, m). | 554 |
| 2-277 | 1H-NMR (DMSO-D6) δ: 11.50 (1H, s), 9.67 (1H, s), 8.45 (1H, s) , 8.23 (1H, s), 7.44 (1H, d, J = 7.5 Hz), 7.35 (1H, d, J = 7.3 Hz), 3.77-3.67 (1H, m), 3.63-3.24 (4H, m), 3.04 (2H, q, J = 7.3 Hz), 2.68 (3H, s), 2.35 (3H, s), 1.92-1.56 (6H, m), 1.17 (3H, t, J = 7.3 Hz). | 568 |
| 2-278 | 1H-NMR (CDCl3) δ: 8.15 (1H, s), 7.93 (1H, s), 7.37-7.31 (2H, m), 6.68 (1H, br s), 4.14-4.07 (1H, m), 3.94-3.78 (3H, m), 3.72-3.64 (1H, m), 3.59-3.50 (1H, m), 3.48-3.33 (3H, m), 3.38 (3H, s), 2.38 (3H, s), 2.12-2.02 (1H, m), 1.87-1.77 (1H, m) . (-NH) | 493 |
| 2-279 | 1H-NMR (DMSO-D6) δ: 11.52 (1H, s), 9.66 (1H, s), 8.43 (1H, s), 8.21 (1H, s), 7.70 (1H, d, J = 1.8 Hz), 7.44-7.42 (2H, m), 7.33 (1H, d, J = 6.7 Hz), 6.22 (1H, t, J = 2.1 Hz), 4.29 (2H, t, J = 6.2 Hz), 3.65 (2H, t, J = 6.1 Hz), 2.67 (3H, s), 2.33 (3H, s). | 472 |
| 2-280 | 1H-NMR (DMSO-D6) δ: 11.64 (1H, s), 9.67 (1H, s), 8.46 (1H, s), 8.21 (1H, s), 7.43 (1H, d, J = 7.9 Hz), 7.34 (1H, d, J = 7.9 Hz), 3.69 (2H, t, J = 7.2 Hz), 3.44 (2H, t, J = 7.2 Hz), 2.88 (3H, s), 2.34 (3H, s). | 484 |
| 2-281 | 1H-NMR (DMSO-D6) δ: 11.44 (1H, br s), 9.64 (1H, s), 8.42 (1H, s), 8.21 (1H, s), 7.42 (1H, d, J = 7.9 Hz), 7.34-7.17 (6H, m), 3.45-3.43 (2H, m), 2.86 (3H, s), 2.84-2.82 (2H, m) , 2.33 (3H, s). | 483 |
| 2-282 | 1H-NMR (DMSO-D6) δ: 11.46 (1H, s), 9.54 (1H, s), 8.30 (2H, d, J = 22.9 Hz), 7.43 (1H, d, J = 8.1 Hz), 7.34-7.17 (6H, m), 3.76 (2H, d, J = 11.8 Hz), 2.91 (2H, t, J = 11.6 Hz), 2.61 (1H, t, J = 12.1 Hz), 2.36 (3H, s), 1.82 (2H, d, J = 11.3 Hz) , 1.65 (2H, ddd, J = 25.2, 12.6, 3.8 Hz). | 508 |
| 2-283 | 1H-NMR (DMSO-D6) δ: 12.41 (1H, s), 9.65 (1H, s), 8.42 (1H, s), 8.17 (1H, s), 7.43 (1H, d, J = 7.9 Hz), 7.35 (1H, d, J = 7.6 Hz), 7.28 (1H, d, J = 2.1 Hz), 6.07 (1H, d, J = 2.1 Hz), 4.06 (2H, t, J = 6.0 Hz), 3.97 (2H, t, J = 5.5 Hz), 2.33 (3H, s), 2.16-2.11 (2H, m). | 470 |
| 2-284 | 1H-NMR (DMSO-D6) δ: 11.44 (1H, s), 9.33 (1H, s), 8.38 (1H, s), 7.97 (1H, s), 7.40 (1H, d, J = 7.6 Hz), 7.29 (5H, dt, J = 13.6, 5.7 Hz), 7.23-7.19 (1H, m), 3.59 (2H, d, J = 9.7 Hz), 2.73-2.67 (3H, m), 2.34 (3H, s), 1.79 (2H, dd, J = 28.8, 11.9 Hz), 1.55 (2H, dd, J = 26.6, 13.4 Hz). | 508 |
| 2-285 | 1H-NMR (DMSO-D6) δ: 11.47 (1H, s), 9.67 (1H, s), 8.44 (1H, s), 8.19 (1H, s), 7.44 (1H, d, J = 8.1 Hz), 7.35 (1H, d, J = 8.1 Hz), 3.57 (2H, dt, J = 13.1, 4.5 Hz), 3.10-3.03 (2H, m), 2.34 (3H, s), 2.31 (3H, s), 2.20 (2H, d, J = 14.3 Hz), 1.81-1.74 (2H, m), 1.33 (3H, s). | 528 |
| 2-286 | 1H-NMR (DMSO-D6) δ: 11.45 (1H, s), 9.34 (1H, s), 8.30 (1H, s), 7.92 (1H, s), 7.39 (1H, d, J = 8.1 Hz), 7.27 (1H, d, J = 8.1 Hz), 3.32-3.28 (2H, m), 2.90 (2H, t, J = 10.3 Hz), 2.44 (3H, s), 2.33 (3H, s), 2.11-2.07 (2H, m), 1.70 (2H, t, J = 9.4 Hz), 1.27 (3H, s). | 528 |
| 2-287 | 1H-NMR (DMSO-D6) δ: 11.48 (1H, s), 9.64 (1H, s), 8.47-8.47 (1H, m), 8.42 (1H, s), 8.22-8.19 (1H, m), 7.69 (1H, td, J = 7.7, 1.9 Hz), 7.42 (1H, d, J = 8.1 Hz), 7.33 (1H, d, J = 7.6 Hz), 7.29 (1H, d, J = 7.9 Hz), 7.22-7.19 (1H, m), 3.59 (2H, t, J = 7.5 Hz), 2.99 (2H, t, J = 7.6 Hz), 2.86 (3H, s), 2.33 (3H, s). | 483 |
| 2-288 | 1H-NMR (DMSO-D6) δ: 11.44 (1H, s), 9.29 (1H, s), 8.33 (1H, s), 7.20 (1H, s), 6.81 (1H, s), 3.58-3.34 (8H, m), 3.08-3.04 (2H, m), 2.23 (3H, s), 2.22 (3H, s), 2.07 (3H, s), 1.84-1.76 (2H, m), 1.71-1.60 (2H, m), 0.96 (3H, t, J = 7.4 Hz). | 514 |
| 2-289 | 1H-NMR (DMSO-D6) δ: 11.42 (1H, s), 9.21 (1H, s), 8.33 (1H, s), 7.36 (1H, s), 6.97 (1H, s), 3.52-3.35 (8H, m), 3.08-3.04 (2H, m), 2.18 (3H, s), 2.16 (3H, s), 2.15 (3H, s), 1.84-1.76 (2H, m), 1.71-1.60 (2H, m), 0.97 (3H, t, J = 7.4 Hz). | 514 |
| 2-290 | 1H-NMR (DMSO-D6) δ: 11.37 (1H, s), 9.16 (1H, s), 8.26 (1H, s), 7.02 (1H, d, J = 7.8 Hz), 6.99 (1H, d, J = 8.0 Hz), 3.49-3.34 (8H, m), 3.08-3.02 (2H, m), 2.22 (3H, s), 2.12 (3H, s), 2.07 (3H, s), 1.83-1.74 (2H, m), 1.71-1.60 (2H, m), 0.92 (3H, q, J = 16.6 Hz). | 514 |
| 2-291 | 1H-NMR (DMSO-D6) δ: 11.65 (1H, s), 9.53 (1H, s), 8.44 (1H, s), 8.02 (1H, d, J = 2.0 Hz), 7.22 (1H, d, J = 8.3 Hz), 7.05 (1H, dd, J = 8.3, 2.3 Hz), 3.54-3.36 (8H, m), 3.08-3.05 (2H, m), 2.25 (3H, s), 1.86-1.77 (2H, m), 1.72-1.61 (2H, m), 0.97 (3H, t, J = 7.4 Hz). | 520 |
| 2-292 | 1H-NMR (DMSO-D6) δ: 11.64 (1H, s), 9.53 (1H, s), 8.43 (1H, s), 8.11-8.10 (1H, m), 7.20-7.15 (2H, m), 3.54-3.35 (8H, m), 3.09-3.03 (2H, m), 2.23 (3H, s), 1.86-1.77 (2H, m), 1.72-1.61 (2H, m), 0.97 (3H, t, J = 7.4 Hz). | 564 566 (M+3) |
| 2-293 | 1H-NMR (DMSO-D6) δ: 11.43 (1H, s), 9.28 (1H, s), 8.36 (1H, s), 7.50 (1H, s), 7.08 (1H, d, J = 7.8 Hz), 6.85 (1H, d, J = 7.0 Hz), 3.72-3.63 (1H, m), 3.51-3.28 (3H, m), 2.82 (6H, s), 2.27 (3H, s), 2.23-2.11 (3H, m), 2.19 (3H, s), 2.03-1.90 (1H, m), 1.83-1.70 (1H, m), 1.69-1.55 (2H, m). | 500 |
| 2-294 | 1H-NMR (CDC13) δ: 9.08 (1H, br s), 7.89 (1H, s), 7.65 (1H, d, J = 1.4 Hz), 7.15 (1H, d, J = 7.9 Hz), 6.98 (1H, dd, J = 7.9, 1.4 Hz), 6.51 (1H, br s), 3.99-3.65 (7H, m), 3.56-3.48 (2H, m), 3.38 (3H, s), 2.98-2.88 (1H, m), 2.27 (3H, s), 1.27 (6H, d, J = 6.9 Hz). | 453 |
| 2-295 | 1H-NMR (CDCl3) δ: 8.99 (1H, br s), 7.88 (1H, s), 7.65 (1H, d, J = 1.6 Hz), 7.15 (1H, d, J = 7.9 Hz), 6.98 (1H, dd, J = 7.9, 1.6 Hz), 6.51 (1H, s), 4.12-4.07 (1H, m), 3.93-3.77 (3H, m), 3.72-3.65 (1H, m), 3.57-3.50 (1H, m), 3.47-3.33 (3H, m), 3.38 (3H, s), 2.99-2.90 (1H, m), 2.27 (3H, s), 2.11-2.02 (1H, m), 1.86-1.77 (1H, m), 1.27 (6H, d, J = 6.9 Hz). | 467 |
| 2-296 | 1H-NMR (DMSO-D6) δ: 11.44 (1H, s), 9.67 (1H, s), 8.45 (1H, s), 8.23 (1H, s), 7.44 (1H, d, J = 7.9 Hz), 7.35 (1H, d, J = 7.6 Hz), 3.27-3.08 (7H, m), 2.85 (3H, s), 2.35 (3H, s), 2.01-1.97 (2H, m), 0.88 (3H, d, J = 6.7 Hz). | 464 |
| 2-297 | 1H-NMR (CDCl3) δ: 8.16 (1H, s), 7.94 (1H, s), 7.37-7.31 (2H, m), 6.67 (1H, br s), 5.75 (1H, s), 4.28-4.17 (1H, m), 3.98-3.91 (1H, m), 3.79-3.64 (2H, m), 3.36-3.27 (1H, m), 2.41-1.72 (6H, m), 2.38 (3H, s), 2.23 (3H, s), 2.20 (3H, s).(-NH) | 541 |
| 2-298 | 1H-NMR (CDCl3) δ: 8.97 (1H, br s), 8.15 (1H, s), 7.94 (1H, s), 7.38-7.31 (2H, m), 7.28 (1H, s), 7.19 (1H, s), 6.71 (1H, br s), 4.36-4.27 (1H, m), 3.88-3.80 (1H, m), 3.78-3.71 (1H, m), 3.58-3.45 (2H, m), 2.42-1.74 (6H, m), 2.38 (3H, s), 2.06 (3H, s). | 527 |
| 2-299 | 1H-NMR (CDCl3) δ: 9.03 (1H, br s), 7.89 (1H, s), 7.59 (1H, s), 7.11 (1H, d, J = 7.6 Hz), 6.90 (1H, d, J = 7.6 Hz), 6.51 (1H, s), 4.13-4.07 (1H, m), 3.94-3.77 (3H, m), 3.72-3.64 (1H, m), 3.58-3.49 (1H, m), 3.48-3.33 (3H, m), 3.38 (3H, s), 2.38 (3H, s), 2.27 (3H, s), 2.10-2.02 (1H, m), 1.87-1.76 (1H, m). | 439 |
| 2-300 | 1H-NMR (DMSO-D6) δ: 11.41 (1H, s), 9.28 (1H, s), 8.36 (1H, s), 7.50 (1H, s), 7.08 (1H, d, J = 7.6 Hz), 6.85 (1H, d, J = 7.6 Hz), 3.50-3.47 (2H, m), 3.39 (2H, t, J = 5.5 Hz), 3.10-3.07 (1H, m), 2.27 (3H, s), 2.19 (3H, s), 2.04-2.01 (1H, m), 1.94-1.74 (5H, m). | 417 |
| 2-301 | 1H-NMR (CDCl3) δ: 8.97 (1H, br s), 8.15 (1H, s), 7.93 (1H, s), 7.37-7.31 (2H, m), 6.68 (1H, s), 4.13-4.06 (1H, m), 3.93-3.76 (3H, m), 3.72-3.65 (1H, m), 3.60-3.37 (6H, m), 2.38 (3H, s), 2.13-2.04 (1H, m), 1.88-1.78 (1H, m), 1.20 (3H, t, J = 6.7 Hz). | 507 |
| 2-302 | 1H-NMR (DMSO-D6) δ: 11.35 (1H, s), 9.55 (1H, s), 8.26 (1H, s), 7.51 (1H, s), 4.36-4.29 (1H, m), 3.65 (1H, d, J = 14.8 Hz), 3.50 (1H, d, J = 13.6 Hz), 3.41-3.35 (1H, m), 3.33-3.27 (1H, m), 3.09 (3H, dt, J = 15.5, 8.1 Hz), 2.30-2.25 (2H, m), 1.99-1.96 (1H, m), 1.86 (3H, d, J = 0.5 Hz), 1.77-1.56 (5H, m), 1.36 (6H, d, J = 6.5 Hz), 0.99 (3H, t, J = 7.4 Hz). | 516 |
| 2-303 | 1H-NMR (DMSO-D6) δ: 11.32 (1H, s), 9.28 (1H, s), 8.38 (1H, s), 7.48 (1H, s), 7.08 (1H, d, J = 7.6 Hz), 6.85 (1H, d, J = 7.6 Hz), 4.23 (1H, dt, J = 10.8, 3.6 Hz), 3.45-3.22 (7H, m), 2.27 (3H, s), 2.19 (3H, s), 1.91-1.74 (4H, m). | 408 |
| 2-304 | 1H-NMR (CDC13) δ: 9.04 (1H, s), 8.14 (1H, s), 7.93 (1H, s), 7.54 (2H, t, J = 5.9 Hz), 7.34 (2H, s), 6.66 (1H, s), 6.26 (1H, t, J = 2.1 Hz), 4.86-4.85 (1H, m), 4.32-4.27 (2H, m), 4.15-4.01 (3H, m), 3.83-3.77 (1H, m), 3.71 (1H, dd, J = 14.9, 9.1 Hz), 3.52 (1H, dd, J = 17.7, 7.3 Hz), 2.37 (3H, s). | 514 |
| 2-305 | 1H-NMR (CDCl3) δ: 9.03 (1H, br s), 7.89 (1H, s), 7.59 (1H, s), 7.11 (1H, d, J = 7.6 Hz), 6.90 (1H, d, J = 7.6 Hz), 6.51 (1H, br s), 4.12-4.06 (1H, m), 3.93-3.75 (3H, m), 3.72-3.64 (1H, m), 3.59-3.37 (6H, m), 2.38 (3H, s), 2.26 (3H, s), 2.13-2.03 (1H, m), 1.87-1.77 (1H, m), 1.20 (3H, t, J = 6.9 Hz). | 453 |
| 2-306 | 1H-NMR (DMSO-D6) δ: 11.54 (1H, s), 9.67 (1H, s), 8.45 (1H, s), 8.23 (1H, s), 7.44 (1H, d, J = 8.1 Hz), 7.35 (1H, d, J = 9.0 Hz), 3.69-3.66 (1H, m), 3.53-3.50 (1H, m), 3.45-3.38 (1H, m), 3.32 (1H, s), 3.14 (1H, s), 3.09-3.05 (2H, m), 2.33-2.29 (5H, m), 2.02-1.99 (1H, m), 1.81-1.57 (5H, m), 0.99 (3H, t, J = 7.4 Hz). | 552 |
| 2-307 | 1H-NMR (DMSO-D6) δ: 11.38 (1H, s), 9.67 (1H, s), 8.47 (1H, s), 8.22 (1H, s), 7.45 (1H, d, J = 8.1 Hz), 7.35 (1H, d, J = 7.9 Hz), 3.57-3.48 (2H, m), 3.44-3.21 (6H, m), 3.11 (1H, dd, J = 9.7, 7.2 Hz), 2.43 (1H, dd, J = 14.0, 7.3 Hz), 2.35 (3H, s), 1.97-1.91 (1H, m), 1.62-1.58 (1H, m). | 462 |
| 2-308 | 1H-NMR (DMSO-D6) δ: 11.33 (1H, br s), 9.27 (1H, s), 8.29 (1H, s), 7.31-7.28 (1H, m), 7.18 (1H, d, J = 8.1 Hz), 6.98-6.96 (1H, m), 3.67-3.63 (4H, m), 3.49-3.47 (4H, m), 3.21-3.14 (1H, m), 2.25 (3H, s), 1.84-1.78 (2H, m), 1.11 (6H, d, J = 6.7 Hz). | 423 |
| 2-309 | 1H-NMR (CDCl3) δ: 9.02 (1H, br s), 7.87 (1H, s), 7.49 (1H, s), 7.22 (1H, d, J = 7.9 Hz), 7.02 (1H, d, J = 7.9 Hz), 6.52 (1H, s), 4.12-4.06 (1H, m), 3.93-3.77 (3H, m), 3.72-3.64 (1H, m), 3.57-3.49 (1H, m), 3.47-3.33 (3H, m), 3.38 (3H, s), 3.07-2.98 (1H, m), 2.37 (3H, s), 2.11-2.02 (1H, m), 1.86-1.76 (1H, m), 1.26 (6H, d, J = 6.7 Hz). | 467 |
| 2-310 | 1H-NMR (CDCl3) δ: 9.13 (1H, br s), 7.88 (1H, s), 7.49 (1H, s), 7.22 (1H, d, J = 7.9 Hz), 7.02 (1H, d, J = 7.9 Hz), 6.55 (1H, s), 3.99-3.65 (7H, m), 3.57-3.47 (2H, m), 3.38 (3H, s), 3.07-2.98 (1H, m), 2.37 (3H, s), 1.26 (6H, d, J = 6.9 Hz). | 453 |
| 2-311 | 1H-NMR (DMSO-D6) δ: 11.38 (1H, s), 9.27 (1H, s), 8.28 (1H, s), 7.32 (1H, s), 7.20 (1H, d, J = 7.9 Hz), 6.98 (1H, d, J = 7.9 Hz), 3.64 (1H, d, J = 14.1 Hz), 3.50 (1H, d, J = 13.4 Hz), 3.42-3.26 (2H, m), 3.17-3.10 (3H, m), 2.26-2.21 (4H, m), 1.99-1.96 (2H, m), 1.77-1.56 (6H, m), 1.12 (6H, d, J = 6.7 Hz), 0.99 (3H, dd, J = 9.1, 5.7 Hz). | 526 |
| 2-312 | 1H-NMR (DMSO-D6) δ: 11.44 (1H, s), 9.66 (1H, s), 8.40 (1H, s), 8.31 (2H, d, J = 4.4 Hz), 8.23 (1H, s), 7.45 (1H, d, J = 7.4 Hz), 7.35 (1H, d, J = 7.9 Hz), 6.56 (1H, t, J = 4.3 Hz), 4.81-4.75 (1H, m), 4.27 (1H, d, J = 14.6 Hz), 4.03 (1H, dd, J = 14.0, 6.8 Hz), 3.75 (1H, d, J = 12.3 Hz), 3.37-3.28 (1H, m), 3.17 (1H, t, J = 13.8 Hz), 3.01 (1H, s), 2.35 (3H, s), 1.70 (2H, s), 1.05 (3H, d, J = 6.2 Hz). | 539 |
| 2-313 | 1H-NMR (DMSO-D6) δ: 11.44 (1H, s), 8.31 (3H, d, J = 4.9 Hz), 8.26 (1H, s), 7.44 (1H, d, J = 8.1 Hz), 7.34 (1H, d, J = 7.6 Hz), 6.56 (1H, t, J = 4.7 Hz), 4.27 (1H, d, J = 14.6 Hz), 4.03 (2H, q, J = 7.1 Hz), 3.73 (1H, d, J = 12.0 Hz), 3.33-3.25 (2H, m), 3.10-2.97 (2H, m), 2.35 (3H, s), 1.69 (2H, s), 1.04 (3H, d, J = 6.2 Hz). | 539 |
| 2-314 | 1H-NMR (DMSO-D6) δ: 11.31 (1H, s), 9.28 (1H, s), 8.38 (1H, s), 7.48 (1H, s), 7.08 (1H, d, J = 7.6 Hz), 6.85 (1H, d, J = 7.6 Hz), 4.21 (1H, dd, J = 8.1, 3.9 Hz), 3.45-3.42 (4H, m), 3.33-3.30 (2H, m), 2.27 (3H, s), 2.19 (3H, s), 1.87-1.80 (4H, m), 1.10 (3H, t, J = 6.9 Hz). | 422 |
| 2-315 | 1H-NMR (DMSO-D6) δ: 11.57 (1H, s), 10.22 (1H, s), 8.55 (1H, s), 7.96 (1H, d, J = 7.9 Hz), 7.55 (1H, d, J = 7.9 Hz), 3.68 (1H, td, J = 9.7, 4.6 Hz), 3.53 (1H, td, J = 9.2, 4.5 Hz), 3.43-3.40 (1H, m), 3.32-3.21 (2H, m), 3.12-3.08 (3H, m), 2.27 (2H, t, J = 18.8 Hz), 2.03-1.97 (1H, m), 1.82-1.57 (5H, m), 1.21 (6H, d, J = 6.7 Hz), 0.99 (3H, t, J = 7.4 Hz). | 581 |
| 2-316 | 1H-NMR (DMSO-D6) δ: 11.48 (1H, s), 9.69 (1H, s), 8.40 (1H, s), 8.07 (1H, s), 7.56 (1H, d, J = 8.3 Hz), 7.47 (1H, d, J = 8.6 Hz), 3.66 (1H, d, J = 14.3 Hz), 3.51 (1H, d, J = 13.9 Hz), 3.38-3.31 (3H, m), 3.13 (1H, t, J = 10.6 Hz), 3.07 (2H, t, J = 7.9 Hz), 2.33-2.26 (2H, m), 1.99-1.97 (1H, m), 1.78-1.57 (5H, m), 1.18 (6H, d, J = 6.7 Hz), 0.99 (3H, t, J = 7.4 Hz). | 580 |
| 2-317 | 1H-NMR (DMSO-D6) δ: 11.55 (1H, s), 9.64 (1H, s), 8.40 (1H, s), 8.25 (1H, s), 7.44 (1H, d, J = 8.1 Hz), 7.34 (1H, d, J = 7.4 Hz), 3.66 (1H, d, J = 14.6 Hz), 3.53-3.44 (3H, m), 3.35-3.23 (2H, m), 2.35 (3H, s), 2.24 (2H, s), 1.99 (1H, s), 1.78 (1H, s), 1.65-1.57 (2H, m), 1.21 (6H, dd, J = 6.9, 1.8 Hz). | 552 |
| 2-318 | 1H-NMR (CDCl3) δ: 9.05 (1H, br s), 8.17 (1H, s), 7.94 (1H, s), 7.38-7.31 (2H, m), 6.68 (1H, s), 3.99-3.69 (7H, m), 3.60-3.45 (4H, m), 2.38 (3H, s), 1.17 (3H, t, J = 7.0 Hz). | 493 |
| 2-319 | 1H-NMR (CDCl3) δ: 9.06 (1H, br s), 7.89 (1H, s), 7.65 (1H, s), 7.16 (1H, d, J = 7.9 Hz), 6.98 (1H, d, J = 7.9 Hz), 6.53 (1H, s), 4.01-3.67 (7H, m), 3.60-3.40 (4H, m), 2.99-2.90 (1H, m), 2.27 (3H, s), 1.27 (6H, d, J = 6.9 Hz), 1.18 (3H, t, J = 7.1 Hz). | 467 |
| 2-320 | 1H-NMR (CDCl3) δ: 8.94 (1H, br s), 8.15 (1H, s), 7.94 (1H, s), 7.38-7.31 (2H, m), 6.74 (1H, s), 3.73-3.67 (2H, m), 3.64-3.58 (2H, m), 3.58-3.52 (2H, m), 3.38-3.32 (2H, m), 2.81-2.75 (2H, m), 2.38 (3H, s), 1.60-1.48 (2H, m), 0.90 (3H, t, J = 7.3 Hz). | 504 |
| 2-321 | 1H-NMR (DMSO-D6) δ: 10.84 (1H, s), 8.97 (1H, s), 7.89 (1H, s), 7.46 (1H, s), 7.06 (1H, d, J = 7.4 Hz), 6.81 (1H, d, J = 7.4 Hz), 4.22 (1H, br s), 3.45-3.34 (5H, m), 3.08-2.97 (3H, m), 2.26 (3H, s), 2.18 (3H, s), 2.02-1.82 (6H, m), 1.63 (1H, br s), 1.49 (1H, br s). | 447 |
| 2-322 | 1H-NMR (DMSO-D6) δ: 11.25 (1H, s), 9.30 (1H, s), 8.34 (1H, s), 7.30 (1H, s), 7.20 (1H, d, J = 7.9 Hz), 6.99 (1H, d, J = 7.9 Hz), 4.22-4.21 (1H, m), 3.41 (1H, td, J = 9.2, 5.5 Hz), 3.30-3.28 (5H, m), 3.22-3.15 (1H, m), 2.27 (3H, s), 1.87-1.77 (5H, m), 1.12 (6H, d, J = 6.7 Hz). | 436 |
| 2-323 | 1H-NMR (DMSO-D6) δ: 11.40 (1H, s), 9.25 (1H, s), 8.32 (1H, s), 7.50 (1H, s), 7.08 (1H, d, J = 7.9 Hz), 6.84 (1H, d, J = 6.9 Hz), 3.92 (1H, dt, J = 12.6, 3.4 Hz), 3.66-3.62 (2H, m), 3.53-3.50 (3H, m), 3.39-3.25 (5H, m), 2.27 (3H, s), 2.19 (3H, s), 1.93-1.89 (1H, m), 1.62-1.59 (1H, m), 1.48 (2H, td, J = 14.0, 7.3 Hz), 0.84 (3H, t, J = 7.4 Hz). | 466 |
| 2-324 | 1H-NMR (CDCl3+TFA) δ: 7.97 (1H, br s), 7.83 (1H, br s), 7.46-7.37 (2H, m), 6.23 (1H, t, J = 75.0 Hz), 4.14-4.06 (1H, m), 3.94-3.35 (8H, m), 2.39 (3H, s), 2.10-2.01 (1H, m), 1.88-1.78 (1H, m).(-2NH) | 529 |
| 2-325 | 1H-NMR (CDCl3) δ: 9.00 (1H, br s), 7.88 (1H, s), 7.65 (1H, s), 7.15 (1H, d, J = 7.8 Hz), 6.97 (1H, d, J = 7.8 Hz), 6.51 (1H, s), 4.13-4.05 (1H, m), 3.93-3.62 (4H, m), 3.60-3.35 (6H, m), 2.98-2.90 (1H, m), 2.27 (3H, s), 2.13-2.03 (1H, m), 1.87-1.77 (1H, m), 1.27 (6H, d, J = 6.9 Hz), 1.20 (3H, t, J = 6.9 Hz). | 481 |
| 2-326 | 1H-NMR (CDCl3+TFA) δ: 8.81 (1H, br s), 7.91 (1H, s), 7.21 (1H, d, J = 7.9 Hz), 7.11 (1H, d, J = 7.9 Hz), 6.24 (1H, t, J = 74.2 Hz), 4.14-4.06 (1H, m), 3.95-3.38 (8H, m), 2.95-2.86 (1H, m), 2.27 (3H, s), 2.12-2.01 (1H, m), 1.89-1.78 (1H, m), 1.25 (6H, d, J = 6.7 Hz). (-2NH) | 503 |
| 2-327 | 1H-NMR (CDCl3) δ: 9.01 (1H, s), 8.01 (1H, s), 7.92 (1H, s), 7.32 (1H, d, J = 7.9 Hz), 7.23 (1H, d, J = 7.9 Hz), 6.68 (1H, t, J = 57.0 Hz), 6.66 (1H, s), 4.14-4.07 (1H, m), 3.94-3.77 (3H, m), 3.72-3.64 (1H, m), 3.60-3.33 (4H, m), 3.38 (3H, s), 2.36 (3H, s), 2.11-2.02 (1H, m), 1.87-1.77 (1H, m). | 475 |
| 2-328 | 1H-NMR (CDCl3) δ: 9.03 (1H, br s), 8.02 (1H, s), 7.93 (1H, s), 7.32 (1H, d, J = 7.9 Hz), 7.23 (1H, d, J = 7.9 Hz), 6.69 (1H, t, J = 57.0 Hz), 6.65 (1H, s), 6.25 (1H, t, J = 74.4 Hz), 4.14-4.07 (1H, m), 3.96-3.79 (5H, m), 3.74-3.65 (1H, m), 3.58-3.39 (2H, m), 2.36 (3H, s), 2.16-2.06 (1H, m), 1.88-1.77 (1H, m). | 511 |
| 2-329 | 1H-NMR (DMSO-D6) δ: 8.74 (1H, s), 7.23 (1H, d, J = 12.3 Hz), 7.14 (1H, d, J = 8.1 Hz), 4.25 (2H, q, J = 7.1 Hz), 3.79 (3H, s), 2.11 (3H, s), 1.40 (9H, s), 1.26 (3H, t, J = 7.1 Hz). | 438 |
| 2-330 | 1H-NMR (DMSO-D6) δ: 11.42 (1H, s), 7.42-7.33 (5H, m), 5.14 (2H, s), 4.26 (1H, s), 3.90 (1H, dt, J = 8.6, 5.2 Hz), 3.66 (1H, td, J = 9.2, 5.5 Hz), 3.56-3.37 (5H, m), 3.32-3.28 (2H, m), 3.16 (2H, s), 1.94-1.87 (1H, m), 1.64-1.54 (1H, m), 1.06 (6H, s). | 428 |
| 2-331 | 1H-NMR (CDCl3) δ: 9.15 (1H, br s), 8.04 (1H, s), 7.94 (1H, s), 7.32 (1H, d, J = 7.6 Hz), 7.23 (1H, d, J = 7.6 Hz), 6.68 (1H, t, J = 57.0 Hz), 6.66 (1H, br s), 3.99-3.82 (4H, m), 3.80-3.66 (3H, m), 3.61-3.52 (2H, m), 3.38 (3H, s), 2.36 (3H, s). | 461 |
| 2-332 | 1H-NMR (CDCl3) δ: 9.00 (1H, br s), 8.01 (1H, s), 7.92 (1H, s), 7.32 (1H, d, J = 7.9 Hz), 7.23 (1H, d, J = 7.9 Hz), 6.69 (1H, t, J = 57.2 Hz), 6.68 (1H, br s), 4.13-4.06 (1H, m), 3.94-3.75 (3H, m), 3.72-3.64 (1H, m), 3.60-3.36 (6H, m), 2.36 (3H, s), 2.13-2.04 (1H, m), 1.88-1.77 (1H, m), 1.20 (3H, t, J = 7.1 Hz). | 489 |
| 2-333 | 1H-NMR (DMSO-D6) δ: 11.32 (1H, br s), 9.58 (1H, s), 8.30 (1H, s), 7.51 (1H, s), 4.38-4.29 (1H, m), 3.96-3.88 (1H, m), 3.69-3.24 (10H, m), 1.95-1.83 (1H, m), 1.86 (3H, s), 1.65-1.54 (1H, m), 1.36 (6H, d, J = 6.7 Hz), 1.08 (3H, t, J = 6.9 Hz). (-HCl) | 471 |
| 2-334 | 1H-NMR (DMSO-D6) δ: 11.33 (1H, s), 9.30 (1H, s), 8.31 (1H, s), 7.36 (1H, d, J = 7.4 Hz), 7.07 (1H, d, J = 11.1 Hz), 3.67-3.65 (4H, m), 3.51-3.48 (4H, m), 3.19-3.12 (1H, m), 2.19 (3H, d, J = 1.2 Hz), 1.85-1.79 (2H, m), 1.12 (6H, d, J = 6.9 Hz). | 440 |
| 2-335 | 1H-NMR (DMSO-D6) δ: 11.34 (1H, s), 9.28 (1H, s), 8.29 (1H, s), 7.36 (1H, d, J = 7.6 Hz), 7.07 (1H, d, J = 11.3 Hz), 3.92 (1H, dt, J = 12.6, 3.4 Hz), 3.68-3.60 (2H, m), 3.55-3.24 (8H, m), 3.19-3.13 (1H, m), 2.19 (3H, d, J = 1.4 Hz), 1.93-1.88 (1H, m), 1.61-1.57 (1H, m), 1.13-1.07 (9H, m). | 498 |
| 2-336 | 1H-NMR (DMSO-D6) δ: 11.29 (1H, s), 9.34 (1H, s), 8.34 (1H, s), 7.49 (1H, d, J = 7.4 Hz), 7.02 (1H, d, J = 11.1 Hz), 3.67-3.66 (4H, m), 3.51-3.49 (4H, m), 3.14-3.07 (1H, m), 2.18 (3H, s), 1.85-1.80 (2H, m), 1.20 (6H, d, J = 6.9 Hz). | 440 |
| 2-337 | 1H-NMR (DMSO-D6) δ: 11.29 (1H, s), 9.34 (1H, s), 8.33 (1H, s), 7.48 (1H, d, J = 7.4 Hz), 7.02 (1H, d, J = 11.1 Hz), 3.92 (1H, dt, J = 12.8, 3.4 Hz), 3.67-3.62 (2H, m), 3.56-3.24 (8H, m), 3.14-3.07 (1H, m), 2.18 (3H, s), 1.92-1.88 (1H, m), 1.61-1.58 (1H, m), 1.20 (6H, d, J = 6.9 Hz), 1.08 (3H, t, J = 6.9 Hz). | 498 |
| 2-338 | 1H-NMR (DMSO-D6) δ: 11.45 (1H, s), 9.65 (1H, s), 8.43 (1H, s), 8.22 (1H, s), 7.43 (1H, d, J = 7.9 Hz), 7.34 (1H, d, J = 7.6 Hz), 3.45 (2H, t, J = 7.4 Hz), 2.93 (3H, s), 2.88 (3H, s), 2.78 (3H, s), 2.60 (2H, t, J = 7.5 Hz), 2.34 (3H, s). | 447 |
| 2-339 | 1H-NMR (DMSO-D6) δ: 11.38 (1H, s), 9.30 (1H, s), 8.35 (1H, s), 7.51 (1H, d, J = 7.4 Hz), 7.02 (1H, d, J = 10.2 Hz), 3.68-3.66 (4H, m), 3.52-3.49 (4H, m), 2.20 (6H, s), 1.86-1.80 (2H, m). | 412 |
| 2-340 | 1H-NMR (DMSO-D6) δ: 11.39 (1H, s), 9.30 (1H, s), 8.35 (1H, s), 7.51 (1H, d, J = 7.6 Hz), 7.02 (1H, d, J = 10.2 Hz), 3.93 (1H, dt, J = 12.8, 3.5 Hz), 3.69-3.61 (2H, m), 3.57-3.25 (8H, m), 2.19 (6H, s), 1.94-1.90 (1H, m), 1.62-1.58 (1H, m), 1.08 (3H, t, J = 6.9 Hz). | 470 |
| 2-341 | 1H-NMR (DMSO-D6) δ: 9.11 (1H, s), 8.08 (1H, s), 7.53 (1H, d, J = 1.4 Hz), 7.11 (1H, d, J = 7.9 Hz), 6.90 (1H, dd, J = 7.7, 1.7 Hz), 4.09-4.07 (1H, m), 3.95-3.94 (2H, m), 3.63-3.60 (2H, m), 3.53-3.50 (1H, m), 3.39-3.38 (1H, m), 3.17 (3H, s), 3.09-2.92 (1H, m), 2.88-2.81 (1H, m), 2.65 (6H, s), 2.18 (3H, s), 1.93-1.88 (1H, m), 1.58-1.55 (1H, m), 1.19 (6H, d, J = 6.9 Hz). | 479 |
| 2-342 | 1H-NMR (DMSO-D6) δ: 11.40 (1H, s), 9.28 (1H, s), 8.36 (1H, s), 7.50 (1H, s), 7.08 (1H, d, J = 7.9 Hz), 6.85 (1H, d, J = 8.1 Hz), 3.93 (1H, dd, J = 9.4, 3.6 Hz), 3.70-3.62 (2H, m), 3.53-3.25 (8H, m), 2.45 (1H, t, J = 7.6 Hz), 2.27 (3H, s), 2.19 (3H, s), 1.99-1.77 (5H, m), 1.67-1.63 (3H, m). | 492 |
| 2-343 | 1H-NMR (DMSO-D6) δ: 11.33 (1H, s), 9.32 (1H, s), 8.30 (1H, s), 7.48 (1H, d, J = 7.4 Hz), 7.02 (1H, d, J = 11.1 Hz), 3.76-3.72 (4H, m), 3.61-3.51 (3H, m), 3.36-3.32 (2H, m), 3.24 (3H, s), 3.14-3.07 (1H, m), 2.18 (3H, s), 1.20 (6H, d, J = 6.9 Hz). | 470 |
| 2-344 | 1H-NMR (DMSO-D6) δ: 11.32 (1H, br s), 9.44 (1H, s), 8.29 (1H, s), 7.53 (1H, s), 4.39-4.30 (1H, m), 3.96-3.88 (1H, m), 3.70-3.23 (10H, m), 2.75-2.65 (1H, m), 1.95-1.86 (1H, m), 1.65-1.54 (1H, m), 1.37 (6H, d, J = 6.7 Hz), 1.13-1.05 (9H, m).(-HCl) | 499 |
| 2-345 | 1H-NMR (CDCl3) δ: 9.03 (1H, br s), 7.89 (1H, s), 7.59 (1H, s), 7.11 (1H, d, J = 7.9 Hz), 6.90 (1H, d, J = 7.9 Hz), 6.50 (1H, s), 4.12-4.05 (1H, m), 3.92-3.41 (12H, m), 3.38 (3H, s), 2.38 (3H, s), 2.27 (3H, s), 2.14-2.04 (1H, m), 1.86-1.75 (1H, m). | 483 |
| 2-346 | 1H-NMR (DMSO-D6) δ: 11.69 (1H, br s), 9.69 (1H, s), 8.44 (1H, s), 8.21 (1H, s), 7.52-7.48 (2H, m), 7.47-7.44 (1H, m), 7.39-7.34 (3H, m), 7.30-7.25 (1H, m), 4.98-4.94 (1H, m), 4.32-4.25 (1H, m), 3.85-3.76 (2H, m), 3.69-3.61 (1H, m), 3.50-3.40 (1H, m), 3.37-3.26 (1H, m), 2.36 (3H, s). | 511 |
| 2-347 | 1H-NMR (DMSO-D6) δ: 11.39 (1H, s), 9.30 (1H, s), 8.34 (1H, s), 7.51 (1H, d, J = 7.6 Hz), 7.02 (1H, d, J = 10.2 Hz), 3.93 (1H, dt, J = 12.9, 3.4 Hz), 3.68-3.64 (2H, m), 3.57-3.29 (5H, m), 3.25-3.22 (4H, m), 2.19 (6H, s), 1.91-1.89 (1H, m), 1.61-1.58 (1H, m). | 456 |
| 2-348 | 1H-NMR (DMSO-D6) δ: 11.28 (1H, s), 9.35 (1H, s), 8.34 (1H, s), 7.48 (1H, d, J = 7.2 Hz), 7.02 (1H, d, J = 11.1 Hz), 4.65 (1H, s), 3.93 (1H, dt, J = 12.9, 3.3 Hz), 3.64 (1H, d, J = 13.9 Hz), 3.51-3.45 (4H, m), 3.37-3.27 (3H, m), 3.14-3.07 (1H, m), 2.18 (3H, s), 1.94-1.91 (1H, m), 1.58-1.55 (1H, m), 1.20 (6H, d, J = 7.2 Hz). | 470 |
| 2-349 | 1H-NMR (DMSO-D6) δ: 11.46 (1H, s), 9.28 (1H, s), 8.36 (1H, s), 7.50 (1H, s), 7.08 (1H, d, J = 7.5 Hz), 6.85 (1H, d, J = 7.5 Hz), 3.99-3.97 (1H, m), 3.86-3.82 (1H, m), 3.66-3.56 (2H, m), 3.52 (2H, t, J = 6.0 Hz), 3.45-3.40 (1H, m), 2.77-2.70 (2H, m), 2.28 (3H, s), 2.19 (3H, s), 2.02-1.95 (1H, m), 1.71-1.68 (1H, m). | 433 |
| 2-350 | 1H-NMR (DMSO-D6) δ: 11.34 (1H, s), 9.35 (1H, s), 8.35 (1H, s), 7.49 (1H, d, J = 7.4 Hz), 7.02 (1H, d, J = 11.6 Hz), 3.80-3.72 (4H, m), 3.64-3.53 (3H, m), 3.33-3.29 (2H, m), 3.14-3.08 (2H, m), 2.18 (3H, s), 1.20 (6H, d, J = 6.9 Hz). | 456 |
| 2-351 | 1H-NMR (DMSO-D6) δ: 11.34 (1H, s), 9.34 (1H, s), 8.34 (1H, s), 7.49 (1H, d, J = 7.4 Hz), 7.02 (1H, d, J = 11.3 Hz), 4.79 (1H, s), 3.76 (1H, dt, J = 12.3, 4.6 Hz), 3.66-3.48 (4H, m), 3.39 (1H, d, J = 12.5 Hz), 3.29-3.26 (1H, m), 3.19 (1H, d, J = 14.3 Hz), 3.14-3.07 (1H, m), 2.18 (3H, s), 1.20 (6H, d, J = 6.9 Hz), 1.10 (3H, s). | 470 |
| 2-352 | 1H-NMR (DMSO-D6) δ: 11.37 (1H, s), 9.40 (1H, s), 8.37 (1H, s), 7.50 (1H, d, J = 8.3 Hz), 7.09 (1H, d, J = 12.3 Hz), 3.93 (1H, dt, J = 12.8, 3.4 Hz), 3.81 (3H, s), 3.67-3.63 (2H, m), 3.57-3.25 (8H, m), 2.16 (3H, s), 1.93-1.90 (1H, m), 1.62-1.58 (1H, m), 1.08 (3H, t, J = 6.9 Hz). | 486 |
| 2-353 | 1H-NMR (DMSO-D6) δ: 11.39 (1H, s), 9.30 (1H, s), 8.33 (1H, d, J = 9.5 Hz), 7.51 (1H, d, J = 7.4 Hz), 7.02 (1H, d, J = 10.2 Hz), 4.26 (1H, s), 3.93 (1H, dt, J = 12.6, 3.5 Hz), 3.72-3.61 (2H, m), 3.55-3.31 (6H, m), 3.15 (2H, s), 2.19 (6H, s), 1.96-1.92 (1H, m), 1.66-1.57 (1H, m), 1.05 (6H, s). | 514 |

**[Table 6]**

| Example | NMR | MS (M+H) |
|---|---|---|
| 3-001 | ¹H-NMR (DMSO-D₆) δ: 11.40 (1H, s), 9.29 (1H, s), 8.33 (1H, s), 7.51 (1H, d, J = 7.6 Hz), 7.02 (1H, d, J = 10.4 Hz), 3.92 (1H, dt, J = 12.9, 3.5 Hz), 3.71-3.45 (5H, m), 3.32-3.30 (3H, m), 3.23 (3H, s), 2.19 (6H, s), 1.94-1.86 (1H, m), 1.64-1.54 (1H, m). | 456 |
| 3-002 | ¹H-NMR (DMSO-D₆) δ: 11.38 (1H, s), 9.30 (1H, s), 8.34 (1H, s), 7.51 (1H, d, J = 7.4 Hz), 7.02 (1H, d, J = 10.2 Hz), 4.31 (1H, s), 3.97 (1H, dt, J = 12.5, 3.4 Hz), 3.66 (1H, dt, J = 14.1, 3.0 Hz), 3.55-3.30 (4H, m), 3.22 (1H, dd, J = 9.7, 3.2 Hz), 2.19 (6H, s), 2.09-1.98 (1H, m), 1.69-1.62 (1H, m), 1.08 (3H, s), 0.99 (3H, s). | 470 |
| 3-003 | ¹H-NMR (DMSO-D₆) δ: 11.39 (1H, s), 9.30 (1H, s), 8.33 (1H, s), 7.51 (1H, d, J = 7.5 Hz), 7.02 (1H, d, J = 10.3 Hz), 3.96-3.89 (1H, m), 3.65-3.58 (2H, m), 3.56-3.45 (4H, m), 3.40-3.28 (2H, m), 3.27-3.22 (1H, m), 2.20 (3H, s), 2.19 (3H, s), 1.97-1.87 (1H, m), 1.66-1.55 (1H, m), 1.06 (6H, d, J = 6.3 Hz). | 485 |
| 3-004 | ¹H-NMR (DMSO-D₆) δ: 11.43 (1H, br s), 9.30 (1H, s), 8.34 (1H, s), 7.51 (1H, d, J = 7.5 Hz), 7.02 (1H, d, J = 10.3 Hz), 4.10-4.01 (1H, m), 3.98-3.91 (1H, m), 3.65-3.36 (6H, m), 3.27-3.19 (1H, m), 3.18-3.08 (1H, m), 2.20 (3H, s), 2.19 (3H, s), 2.06-1.96 (1H, m), 1.80-1.69 (1H, m), 1.23 (3H, d, J = 7.0 Hz), 1.21 (3H, d, J = 7.0 Hz). | 533 |
| 3-005 | ¹H-NMR (CDCl₃) δ: 8.97 (1H, br s), 7.86 (1H, s), 7.47 (1H, d, J = 7.2 Hz), 6.89 (1H, d, J = 9.7 Hz), 6.35 (1H, s), 4.11-4.04 (1H, m), 3.92-3.61 (6H, m), 3.59-3.40 (6H, m), 3.37 (3H, s), 2.29 (3H, s), 2.25 (3H, s), 2.14-2.03 (1H, m), 1.85-1.75 (1H, m). | 501 |
| 3-006 | ¹H-NMR (DMSO-D₆) δ: 11.37 (1H, s), 9.31 (1H, s), 8.34 (1H, s), 7.50 (1H, d, J = 7.2 Hz), 7.02 (1H, d, J = 10.2 Hz), 4.00 (2H, d, J = 11.8 Hz), 3.60-3.55 (2H, m), 3.45-3.36 (5H, m), 3.24-3.22 (5H, m), 3.15-3.08 (1H, m), 2.20 (6H, s), 1.09 (3H, t, J = 6.9 Hz). | 500 |
| 3-007 | ¹H-NMR (DMSO-D₆) δ: 11.36 (1H, s), 9.30 (1H, s), 8.34 (1H, s), 7.51 (1H, d, J = 7.6 Hz), 7.02 (1H, d, J = 10.4 Hz), 4.00 (2H, d, J = 11.1 Hz), 3.61-3.50 (2H, m), 3.42-3.39 (5H, m), 3.24-3.21 (5H, m), 3.12-3.09 (1H, m), 2.20 (6H, s), 1.09 (3H, t, J = 6.9 Hz). | 500 |

### Test example 1: Evaluation of NLRP3 inflammasome inhibitory activity

The NLRP3 inflammasome inhibitory activity of test compounds were evaluated on the basis of the inhibitory activity of the IL-1β production in THP1-Null cells (Product Number: thp-null, InvivoGen). Cells were maintained for culture in RPMI-1640 media containing 10% (v/v) fetal bovine serum, 25 mmol/L HEPES, 100 U/mL penicillin, 100 µg/mL streptomycin, 100 µg/mL normocin, and 200 µg/mL hygromycin B (set at 37°C, 5% CO₂/95% air).

Cells were suspended with media for assay containing 0.5 µmol/L PMA (RPMI-1640 media containing 10% (v/v) fetal bovine serum, 100 U/mL penicillin, and 100 µg/mL streptomycin), and the suspended cells were seeded on Corning (registered trademark) 384-well Flat Clear Bottom Black Polystyrene TC-treated Microplates (25,000 cells/25 µL/well), followed by incubation (set at 37°C, 5% CO₂/95% air) overnight. The supernatant of the culture was removed, and thereto was added media for assay (25 µL/well) containing 1 µg/mL Lipopolysaccharides (Product Number: L2654, Sigma-Aldrich (registered trademark)). Then, the culture was further incubated for 3 hours (set at 37°C, 5% CO₂/95% air). The supernatant of the culture was removed. Then, a vehicle solution prepared from Opti-MEM (trademark) medium (Product Number: 31985-070, Invitrogen) was added to blank-setting wells and control-setting wells (20 µL/well), followed by incubation for 15 minutes (set at 37°C, 5% CO₂/95% air). A solution containing a test compound (20 µL/well) was added to test compound-setting wells. Further, Opti-MEM (trademark) medium containing Nigericin (Product Number: N7143, Sigma-Aldrich (registered trademark)) was added to the control-setting wells and test compound-setting wells (5 µL/well), followed by incubation for 1.5 hours (set at 37°C, 5% CO₂/95% air). The final concentration of Nigericin was adjusted to be 7.5 µmol/L. 5 µL/well of Opti-MEM (trademark) medium was added to the blank-setting wells. The supernatant of the culture was cryonically stored (set at -20°C) until measurement of IL-1β.

The amount of IL-1β in the culture supernatant was quantitated with AlphaLISA IL1 beta kit (Product Number: AL220C, Perkin Elmer). Fluorescence intensity was measured with a microplate reader EnSpier (Model number: 2300-00J, Perkin Elmer) according to procedure manuals attached thereto. Inhibition rates of the test compound-setting wells were calculated on the basis of 100% for the blank-setting wells and 0% for the control-setting wells. IC₅₀ values (i.e., 50% inhibitory concentrations) of the test compounds were calculated by logistic regression analysis. The result of each Example compound is shown in the following tables.

**[Table 7]**

| Example | IC₅₀ (µM) | Example | IC₅₀ (µM) |
|---|---|---|---|
| 1 | 0.050 | 21 | 0.043 |
| 2 | < 0.030 | 22 | < 0.030 |
| 3 | 0.091 | 23 | < 0.030 |
| 4 | 0.081 | 24 | 0.16 |
| 5 | < 0.030 | 25 | 0.051 |
| 6 | < 0.030 | 26 | 0.13 |
| 7 | 0.20 | 27 | 0.058 |
| 8 | 0.44 | 28 | 0.12 |
| 9 | 0.21 | 29 | 0.12 |
| 10 | 0.21 | 30 | 0.19 |
| 11 | 0.072 | 31 | 0.31 |
| 12 | 4.0 | 32 | 0.15 |
| 13 | 0.048 | 33 | 0.099 |
| 14 | 0.097 | 34 | 0.048 |
| 15 | < 0.030 | 35 | < 0.030 |
| 16 | < 0.030 | 36 | 0.092 |
| 17 | 0.83 | 37 | < 0.030 |
| 18 | 5.4 | 38 | 0.073 |
| 19 | 5.7 | 39 | 0.22 |
| 20 | 48% Inhibition at 30 µM | 40 | 0.93 |

**[Table 8]**

| | | | |
|---|---|---|---|
| 41 | 0.077 | 61 | 0.17 |
| 42 | 0.97 | 62 | 0.27 |
| 43 | < 0.030 | 63 | 0.90 |
| 44 | 0.23 | 64 | 5.7 |
| 45 | 0.20 | 65 | 0.069 |
| 46 | 0.35 | 66 | < 0.030 |
| 47 | 0.42 | 67 | 0.84 |
| 48 | 23% Inhibition at 30 µM | 68 | 0.053 |
| 49 | 0.27 | 69 | 0.15 |
| 50 | 0.72 | 70 | < 0.030 |
| 51 | < 0.030 | 71 | 0.19 |
| 52 | 0.032 | 72 | 0.31 |
| 53 | < 0.030 | 73 | 0.16 |
| 54 | 0.17 | 74 | 0.18 |
| 55 | 0.16 | 75 | 0.032 |
| 56 | 0.046 | 76 | 0.092 |
| 57 | 0.074 | 77 | 0.056 |
| 58 | 0.039 | | |
| 59 | 0.044 | | |
| 60 | 0.13 | | |

**[Table 9]**

| Example | IC₅₀ (µM) | Example | IC₅₀ (µM) |
|---|---|---|---|
| 2-001 | 0.063 | 2-178 | 0.88 |
| 2-002 | 0.16 | 2-179 | 11 |
| 2-003 | < 0.030 | 2-180 | -3% inhibition at 3µM |
| 2-004 | 0.021 | 2-181 | 0.031 |
| 2-005 | < 0.030 | 2-182 | 0.010 |
| 2-006 | 0.031 | 2-183 | 0.015 |
| 2-007 | < 0.030 | 2-184 | 0.14 |
| 2-008 | 0.18 | 2-185 | 0.038 |
| 2-009 | 0.38 | 2-186 | 0.041 |
| 2-010 | 0.12 | 2-187 | 0.11 |
| 2-011 | 0.06 | 2-188 | 0.79 |
| 2-012 | < 0.030 | 2-189 | 16% inhibition at 3µM |
| 2-013 | 0.064 | 2-190 | 0.017 |
| 2-014 | 6.0 | 2-191 | 0.35 |
| 2-015 | 0.035 | 2-192 | 0.16 |
| 2-016 | 0.042 | 2-193 | 46% inhibition at 3µM |
| 2-017 | 0.083 | 2-194 | 1.5 |
| 2-018 | 0.050 | 2-195 | 1.4 |
| 2-019 | 0.070 | 2-196 | 2.7 |
| 2-020 | 0.10 | 2-197 | 0.017 |
| 2-021 | 0.090 | 2-198 | 0.029 |
| 2-022 | 0.24 | 2-199 | 0.16 |
| 2-023 | 0.13 | 2-200 | 1.1 |
| 2-024 | 0.031 | 2-201 | 0.32 |
| 2-025 | < 0.030 | 2-202 | 2.2 |
| 2-026 | < 0.030 | 2-203 | 0.018 |
| 2-027 | 0.034 | 2-204 | 0.029 |
| 2-028 | < 0.030 | 2-205 | 0.036 |
| 2-029 | 0.18 | 2-206 | 0.15 |
| 2-030 | 0.036 | 2-207 | 0.18 |
| 2-031 | 0.036 | 2-208 | 0.089 |
| 2-032 | 0.089 | 2-209 | 33% inhibition at 3µM |
| 2-033 | 0.15 | 2-210 | 0.048 |
| 2-034 | 0.10 | 2-211 | 0.016 |
| 2-035 | 0.22 | 2-212 | 0.28 |
| 2-036 | 0.31 | 2-213 | 0.039 |
| 2-037 | 2.7 | 2-214 | 0.089 |
| 2-038 | < 0.030 | 2-215 | 0.061 |
| 2-039 | 0.50 | 2-216 | 0.18 |
| 2-040 | 0.13 | 2-217 | < 3.0 |
| 2-041 | 0.34 | 2-218 | 0.072 |
| 2-042 | 0.056 | 2-219 | 0.029 |
| 2-043 | 0.049 | 2-220 | 0.067 |
| 2-044 | 0.06 | 2-221 | 0.21 |
| 2-045 | 0.18 | 2-222 | 0.030 |
| 2-046 | 0.32 | 2-223 | 0.071 |
| 2-047 | < 0.030 | 2-224 | 0.027 |
| 2-048 | < 0.030 | 2-225 | 0.036 |
| 2-049 | 0.046 | 2-226 | 0.097 |
| 2-050 | 0.60 | 2-227 | 1.5 |
| 2-051 | 0.044 | 2-228 | 0.028 |
| 2-052 | 0.13 | 2-229 | 0.083 |
| 2-053 | 0.089 | 2-230 | 0.089 |
| 2-054 | 0.052 | 2-231 | 0.089 |
| 2-055 | < 0.030 | 2-232 | 0.030 |
| 2-056 | < 0.030 | 2-233 | 0.48 |
| 2-057 | 0.57 | 2-234 | 0.050 |
| 2-058 | 0.054 | 2-235 | 0.094 |
| 2-059 | < 0.030 | 2-236 | 0.10 |
| 2-060 | 0.046 | 2-237 | 0.20 |
| 2-061 | 0.57 | 2-238 | 11% inhibition at 3µM |
| 2-062 | 0.048 | 2-239 | 0.17 |
| 2-063 | 0.067 | 2-240 | 0.031 |
| 2-064 | 0.026 | 2-241 | 0.037 |
| 2-065 | 0.032 | 2-242 | 0.045 |
| 2-066 | < 0.030 | 2-243 | 2.7 |
| 2-067 | 0.092 | 2-244 | 0.051 |
| 2-068 | 0.51 | 2-245 | 0.073 |
| 2-069 | 0.066 | 2-246 | 0.067 |
| 2-070 | 0.12 | 2-247 | 0.022 |
| 2-071 | 0.043 | 2-248 | 44% inhibition at 3µM |
| 2-072 | 0.027 | 2-249 | 0.030 |
| 2-073 | 0.28 | 2-250 | 0.030 |
| 2-074 | 11 | 2-251 | 0.032 |
| 2-075 | 4.5 | 2-252 | 0.19 |
| 2-076 | 0.025 | 2-253 | 0.042 |
| 2-077 | 0.53 | 2-254 | 0.064 |
| 2-078 | 0.10 | 2-255 | 0.21 |
| 2-079 | 0.28 | 2-256 | 0.015 |
| 2-080 | 0.93 | 2-257 | 0.022 |
| 2-081 | 0.094 | 2-258 | 0.018 |
| 2-082 | 0.16 | 2-259 | 0.087 |
| 2-083 | 0.49 | 2-260 | 0.041 |
| 2-084 | 0.14 | 2-261 | 0.028 |
| 2-085 | 0.12 | 2-262 | 0.018 |
| 2-086 | 17% inhibition at 30µM | 2-263 | 0.079 |
| 2-087 | 0.55 | 2-264 | 0.038 |
| 2-088 | 0.17 | 2-265 | 1.1 |
| 2-089 | 1.2 | 2-266 | 0.05 |
| 2-090 | 0.57 | 2-267 | 0.088 |
| 2-091 | 0.038 | 2-268 | 0.055 |
| 2-092 | 0.022 | 2-269 | 0.35 |
| 2-093 | 0.059 | 2-270 | 0.0098 |
| 2-094 | 0.27 | 2-271 | 0.94 |
| 2-095 | 0.63 | 2-272 | 0.027 |
| 2-096 | 1.8 | 2-273 | 0.024 |
| 2-097 | 0.15 | 2-274 | 0.18 |
| 2-098 | 0.25 | 2-275 | 0.16 |
| 2-099 | 0.021 | 2-276 | 0.0086 |
| 2-100 | 0.010 | 2-277 | 0.033 |
| 2-101 | 0.039 | 2-278 | 0.017 |
| 2-102 | 0.050 | 2-279 | 0.074 |
| 2-103 | 0.020 | 2-280 | 0.42 |
| 2-104 | 1.8 | 2-281 | 0.17 |
| 2-105 | 0.086 | 2-282 | 2.5 |
| 2-106 | 0.044 | 2-283 | 1.1 |
| 2-107 | 0.15 | 2-284 | 1.4 |
| 2-108 | 0.13 | 2-285 | 0.17 |
| 2-109 | 0.038 | 2-286 | 0.72 |
| 2-110 | 0.34 | 2-287 | 0.064 |
| 2-111 | 0.065 | 2-288 | 0.12 |
| 2-112 | 0.065 | 2-289 | 0.046 |
| 2-113 | 0.65 | 2-290 | 0.089 |
| 2-114 | 0.34 | 2-291 | 0.073 |
| 2-115 | 1.3 | 2-292 | 0.034 |
| 2-116 | 0.085 | 2-293 | 0.042 |
| 2-117 | 0.11 | 2-294 | 0.087 |
| 2-118 | 0.26 | 2-295 | 0.030 |
| 2-119 | 0.096 | 2-296 | 0.081 |
| 2-120 | 0.16 | 2-297 | 0.087 |
| 2-121 | 0.035 | 2-298 | 0.067 |
| 2-122 | 0.026 | 2-299 | 0.19 |
| 2-123 | 0.21 | 2-300 | 0.19 |
| 2-124 | 0.047 | 2-301 | 0.011 |
| 2-125 | 0.25 | 2-302 | 0.036 |
| 2-126 | 0.010 | 2-303 | 0.14 |
| 2-127 | 0.13 | 2-304 | 0.059 |
| 2-128 | 0.020 | 2-305 | 0.049 |
| 2-129 | 0.020 | 2-306 | 0.0098 |
| 2-130 | 0.020 | 2-307 | 0.069 |
| 2-131 | 0.38 | 2-308 | 0.082 |
| 2-132 | 1.9 | 2-309 | 0.039 |
| 2-133 | 0.049 | 2-310 | 0.098 |
| 2-134 | 0.079 | 2-311 | 0.018 |
| 2-135 | 0.044 | 2-312 | 0.14 |
| 2-136 | -1.6% inhibition at 30µM | 2-313 | 0.022 |
| 2-137 | 0.13 | 2-314 | 0.21 |
| 2-138 | 0.092 | 2-315 | 0.085 |
| 2-139 | 0.27 | 2-316 | 0.022 |
| 2-140 | 0.074 | 2-317 | 0.027 |
| 2-141 | 0.0097 | 2-318 | 0.077 |
| 2-142 | 6.3 | 2-319 | 0.11 |
| 2-143 | 0.086 | 2-320 | 0.60 |
| 2-144 | 23% inhibition at 30µM | 2-321 | -11% inhibition at 3µM |
| 2-145 | 0.042 | 2-322 | 0.27 |
| 2-146 | 0.11 | 2-323 | 0.035 |
| 2-147 | 0.42 | 2-324 | 0.017 |
| 2-148 | 0.040 | 2-325 | 0.025 |
| 2-149 | 0.032 | 2-326 | 0.026 |
| 2-150 | 0.025 | 2-327 | 0.029 |
| 2-151 | 0.064 | 2-328 | 0.030 |
| 2-152 | 6.1 | 2-329 | 0.40 |
| 2-153 | 2.3 | 2-330 | 0.086 |
| 2-154 | 0.23 | 2-331 | 0.059 |
| 2-155 | 0.062 | 2-332 | 0.022 |
| 2-156 | 0.039 | 2-333 | 0.078 |
| 2-157 | 0.038 | 2-334 | 0.026 |
| 2-158 | 0.10 | 2-335 | 0.018 |
| 2-159 | 0.061 | 2-336 | 0.010 |
| 2-160 | 0.17 | 2-337 | 0.013 |
| 2-161 | 0.070 | 2-338 | 0.077 |
| 2-162 | 0.30 | 2-339 | 0.030 |
| 2-163 | 0.049 | 2-340 | 0.012 |
| 2-164 | 0.14 | 2-341 | 0.088 |
| 2-165 | 0.10 | 2-342 | 0.010 |
| 2-166 | 0.26 | 2-343 | 0.016 |
| 2-167 | 0.048 | 2-344 | 0.065 |
| 2-168 | 9.8 | 2-345 | 0.071 |
| 2-169 | 1.7 | 2-346 | 0.29 |
| 2-170 | 0.6 | 2-347 | 0.055 |
| 2-171 | 2.7 | 2-348 | 0.028 |
| 2-172 | 0.076 | 2-349 | 1.2 |
| 2-173 | 0.12 | 2-350 | 0.015 |
| 2-174 | 0.055 | 2-351 | 0.022 |
| 2-175 | 0.40 | 2-352 | 0.026 |
| 2-176 | 18 | 2-353 | 0.016 |
| 2-177 | 0.047 | | |

**[Table 10]**

| Example | IC₅₀ (µM) | Example | IC₅₀ (µM) |
|---|---|---|---|
| 3-001 | 0.072 | 3-005 | 0.040 |
| 3-002 | 0.049 | 3-006 | 0.030 |
| 3-003 | 0.021 | 3-007 | 0.82 |
| 3-004 | 0.026 | | |

### Formulation Examples

Formulation examples of a compound of Formula [I] or Formula[Ia] include, for example, the following formulations, but are not intended to be limited thereto.

### Formulation Example 1: Preparation of a capsule

| | |
|---|---|
| (1) A compound of Example 1 | 30 mg |
| (2) Microcrystalline cellulose | 10 mg |
| (3) Lactose | 19 mg |
| (4) Magnesium stearate | 1 mg |

Ingredients (1), (2), (3), and (4) are mixed to be filled in a gelatin capsule.

### Formulation Example 2: Preparation of a tablet

| | |
|---|---|
| (1) A compound of Example 1 | 10 g |
| (2) Lactose | 50 g |
| (3) Cornstarch | 15 g |
| (4) Carmellose calcium | 44 g |
| (5) Magnesium stearate | 1 g |

The total amounts of Ingredients (1), (2), and (3) and 30 g of Ingredient (4) are combined with water, dried in vacuo, and then granulated. The resulted granules are mixed with 14 g of Ingredient (4) and 1 g of Ingredient (5), and tableted with a tabletting machine. In this manner, 1,000 tablets of which each tablet comprises 10 mg of Example 1 are obtained.

### INDUSTRIAL APPLICABILITY

A compound of Formula [I] or a compound of Formula [Ia], or a pharmaceutically acceptable salt thereof, has an NLRP3 inflammasome inhibitory activity, and thus is expected to be useful for treating or preventing a disease selected from the group consisting of multiple sclerosis, chronic kidney disease, inflammatory bowel disease (for example, ulcerative colitis, Crohn's disease), arteriosclerosis, Cryopyrin-associated periodic syndrome (CAPS), nonalcoholic steato-hepatitis (NASH), gout, gouty arthritis, rheumatoid arthritis, contact dermatitis, dry eye, ischemic heart disease and systemic lupus erythematosus (SLE).

## Claims

1. A compound of Formula [Ia]: 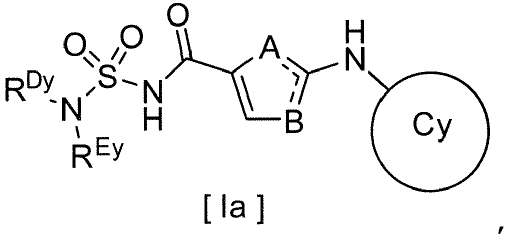 or a pharmaceutically acceptable salt thereof, wherein
a partial structure: 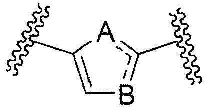 is: 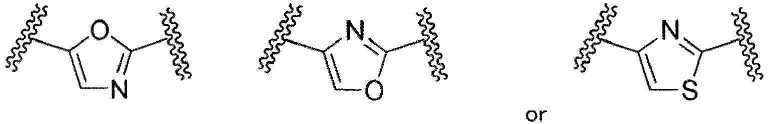
Ring Cy is 5- to 6-membered heteroaryl comprising 1 to 3 nitrogen atoms, or phenyl, wherein the heteroaryl or the phenyl is substituted with R^{F} at one of the atoms of α-position to a Cy ring-constituting atom attached to the NH group directly attached to the partial structure, and may be optionally substituted with the same or different 1 to 4 R^{G}s;
R^{Dy} and R^{Ey} are each independently
(1) hydrogen,
(2) C₁₋₆ alkyl, wherein the alkyl may be optionally substituted with the same or different 1 to 3 R^{d1}s,
(3) C₁₋₄ haloalkyl,
(4) C₃₋₆ cycloalkyl, wherein the cycloalkyl may be optionally substituted with the same or different 1 to 3 halogen atoms,
(5) 4- to 7-membered heterocycloalkyl comprising 1 to 3 heteroatoms independently selected from the group consisting of nitrogen and oxygen atoms, wherein the heterocycloalkyl may be optionally substituted with the same or different 1 to 3 R^{d2}s, or
(6) 5- to 6-membered heteroaryl comprising 1 to 3 heteroatoms independently selected from the group consisting of nitrogen and oxygen atoms, wherein the heteroaryl may be optionally substituted with C₁₋₆ alkyl or C₁₋₄ haloalkyl, or alternatively,
R^{Dy} and R^{Ey} may combine together with the nitrogen atom to which they attach to form
(a) 4- to 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s1y}s and / or may be fused with a 5- to 6-membered heteroaromatic ring comprising 1 to 3 nitrogen atoms wherein the heteroaromatic ring may be optionally substituted with the same or different 1 or 2 R^{d3}s,
(b) 7- to 11-membered spiro heterocycloalkyl comprising 1 to 3 heteroatoms independently selected from the group consisting of nitrogen and oxygen atoms, wherein the spiro heterocycloalkyl may be optionally substituted with the same or different 1 to 3 R^{d4}s and/or may be optionally fused with a benzene ring,
(c) 6- to 10-membered fused heterocycloalkyl comprising 1 or 2 heteroatoms independently selected from the group consisting of nitrogen and oxygen atoms, wherein the fused heterocycloalkyl may be optionally substituted with the same or different 1 to 3 R^{d5}s, or
(d) 5- to 9-membered bridged heterocycloalkyl comprising 1 or 2 heteroatoms independently selected from the group consisting of nitrogen and oxygen atoms, wherein the bridged heterocycloalkyl may be optionally substituted with the same or different 1 to 3 R^{d6}s;
R^{F} is
(1) halogen,
(2) C₁₋₄ alkyl,
(3) C₁₋₄ haloalkyl,
(4) C₁₋₆ alkoxy, wherein the alkoxy may be optionally substituted with the same or different 1 to 3 halogen atoms, or
(5) C₃₋₅ cycloalkyl,
R^{G} is each independently a substituent selected from the group consisting of:
(1) halogen,
(2) C₁₋₆ alkyl, wherein the alkyl may be optionally substituted with:
(a) C₁₋₄ alkoxy,
(b) cyano,
(c) phenyl, or
(d) 5- to 6-membered heteroaryl comprising 1 or 2 heteroatoms independently selected from the group consisting of nitrogen, oxygen, and sulfur atoms,
(3) C₁₋₄ haloalkyl,
(4) C₁₋₆ alkoxy, wherein the alkoxy may be optionally substituted with the same or different 1 to 3 halogen atoms,
(5) cyano,
(6) CONR^{G1}R^{G2}, wherein R^{G1} and R^{G2} are each independently
(a) hydrogen,
(b) C₁₋₆ alkyl, or
(c) C₁₋₄ haloalkyl,
(7) trialkylsilyl,
(8) pentafluorosulfanyl,
(9) C₃₋₇ cycloalkyl, wherein the cycloalkyl may be optionally substituted with the same or different 1 to 3 R^{s2}s,
(10) C₅₋₁₃ spiro cycloalkyl, wherein the spiro cycloalkyl may be optionally substituted with the same or different 1 to 3 R^{s3}s,
(11) 4- to 7-membered heterocycloalkyl comprising 1 or 2 heteroatoms independently selected from the group consisting of nitrogen and oxygen atoms, wherein the heterocycloalkyl may be optionally substituted with the same or different 1 to 3 R^{s4}s,
(12) 7- to 11-membered spiro heterocycloalkyl comprising 1 or 2 heteroatoms independently selected from the group consisting of nitrogen and oxygen atoms, wherein the spiro heterocycloalkyl may be optionally substituted with the same or different 1 to 3 R^{s5}s,
(13) phenyl, wherein the phenyl may be optionally substituted with the same or different 1 to 3 R^{s6}s, and
(14) 5- to 6-membered heteroaryl comprising 1 to 3 heteroatoms independently selected from the group consisting of nitrogen, oxygen, and sulfur atoms, wherein the heteroaryl may be optionally substituted with the same or different 1 to 3 R^{s7}s, or alternatively,
in the case where R^{F} and R^{G} or two R^{G}s are substituted on neighboring atoms, then the R^{F} and R^{G} and/or the two R^{G}s may combine together with the atoms to which they attach to form:
(a) a C₅₋₆ cycloalkene ring, wherein the cycloalkene ring may be optionally substituted with the same or different 1 to 3 R^{c1}s,
(b) a 5- to 7-membered heterocycloalkene ring comprising 1 or 2 heteroatoms independently selected from the group consisting of nitrogen and oxygen atoms, wherein the heterocycloalkene ring may be optionally subsituted with the same or different 1 to 3 R^{c2}s,
(c) a benzene ring, wherein the benzene ring may be optionally substituted with the same or different 1 to 3 R^{c3}s, or
(d) a 5- to 7-membered heteroaromatic ring comprising 1 or 2 heteroatoms independently selected from the group consisting of nitrogen and oxygen atoms, wherein the heteroaromatic ring may be optionally substituted with the same or different 1 to 3 R^{c4}s, so that Ring Cy may form a bi- or tri-cyclic fused ring group;
R^{c1}, R^{c2}, R^{c3}, and R^{c4} are each independently a substituent selected from the group consisting of:
(1) halogen,
(2) C₁₋₆ alkyl,
(3) C₁₋₄ haloalkyl, and
(4) oxo;
R^{d1} is each independently a substituent selected from the group consisting of:
(1) halogen,
(2) hydroxy,
(3) C₁₋₆ alkoxy, wherein the alkoxy may be optionally substituted with the same or different 1 to 3 halogen atoms,
(4) cyano,
(5) COOR^{e1}, wherein R^{e1} is hydrogen or C₁₋₆ alkyl,
(6) CONR^{e2}R^{e3}, wherein R^{e2} and R^{e3} are independently
(a) hydrogen,
(b) C₁₋₆ alkyl, or
(c) C₁₋₄ haloalkyl, or alternatively, R^{e2} and R^{e3} may combine together with the nitrogen atom to which they attach to form 5-to 6-membered heterocycloalkyl comprising 1 or 2 heteroatoms independently selected from the group consisting of nitrogen and oxygen atoms, wherein the heterocycloalkyl may be optionally substituted with the same or different 1 or 2 halogen atoms,
(7) COR^{e4}, wherein R^{e4} is C₁₋₆ alkyl,
(8) SO₂R^{e5}, wherein R^{e5} is C₁₋₆ alkyl,
(9) NR^{e6}R^{e7}, wherein R^{e6} is
(a) hydrogen,
(b) C₁₋₆ alkyl, or
(c) C₁₋₄ haloalkyl, and
R^{e7} is
(a) hydrogen,
(b) C₁₋₆ alkyl,
(c) C₁₋₄ haloalkyl,
(d) COR^{e8}, wherein R^{e8} is C₁₋₆ alkyl, and the alkyl may be optionally substituted with 1 to 3 substituents independently selected from the group consisting of:
(i) halogen,
(ii) hydroxy,
(iii) C₁₋₆ alkoxy, and
(iv) cyano, or
(e) SO₂R^{e9}, wherein R^{e9} is C₁₋₆ alkyl or C₁₋₄ haloalkyl,
(10) SO₂NR^{f1}R^{f2}, wherein R^{f1} and R^{f2} are independently
(a) hydrogen,
(b) C₁₋₆ alkyl, or
(c) C₁₋₄ haloalkyl,
(11) C₃₋₆ cycloalkyl, wherein the cycloalkyl may be optionally substituted with hydroxy or C₁₋₆ alkoxy,
(12) 4- to 6-membered heterocycloalkyl comprising 1 or 2 heteroatoms independently selected from the group consisting of nitrogen, oxygen, and sulfur atoms, wherein the heterocycloalkyl may be optionally substituted with 1 or 2 substituents independently selected from the group consisting of halogen and oxo,
(13) phenyl, and
(14) 5- to 6-membered heteroaryl comprising 1 or 2 heteroatoms independently selected from the group consisting of nitrogen, oxygen, and sulfur atoms, wherein the heteroaryl may be optionally substituted with C₁₋₆ alkyl or C₁₋₄ haloalkyl;
R^{d2} is each independently a substituent selected from the group consisting of:
(1) C₁₋₆ alkyl,
(2) C₁₋₄ haloalkyl,
(3) oxo, and
(4) COR^{g1}, wherein R^{g1} is C₁₋₆ alkyl, and the alkyl may be optionally substituted with:
(a) hydroxy,
(b) C₁₋₆ alkoxy, and
(c) cyano;
R^{d3} is each independently a substituent selected from the group consisting of:
(1) C₁₋₆ alkyl, and
(2) COOR^{g2}, wherein R^{g2} is hydrogen or C₁₋₆ alkyl;
R^{d4}, R^{d5}, and R^{d6} are each independently a substituent selected from the group consisting of:
(1) halogen,
(2) hydroxy,
(3) oxo,
(4) cyano,
(5) C₁₋₆ alkyl,
(6) C₁₋₄ haloalkyl,
(7) C₁₋₆ alkoxy, wherein the alkoxy may be optionally substituted with the same or different 1 to 3 halogen atoms,
(8) NR^{g3}R^{g4}, wherein R^{g3} and R^{g4} are independently
(a) hydrogen,
(b) C₁₋₆ alkyl, or
(c) C₁₋₄ haloalkyl, and
(9) 4- to 7-membered heterocycloalkyl comprising 1 or 2 heteroatoms independently selected from the group consisting of nitrogen and oxygen atoms, wherein the heterocycloalkyl may be optionally substituted with 1 or 2 substituents independently selected from the group consisting of:
(a) halogen,
(b) hydroxy,
(c) C₁₋₆ alkyl,
(d) C₁₋₆ alkoxy, wherein the alkoxy may be optionally substituted with the same or different 1 to 3 halogen atoms, and
(e) oxo;
R^{s1y}, R^{s2}, R^{s3}, R^{s4}, R^{s5}, R^{s6}, and R^{s7} are each independently a substituent selected from the group consisting of:
(1) halogen,
(2) hydroxy,
(3) C₁₋₆ alkyl, wherein the alkyl may be optionally substituted with the same or different 1 to 3 R^{11y}s,
(4) C₁₋₆ alkoxy, wherein the alkoxy may be optionally substituted with the same or different 1 to 3 halogen atoms,
(5) cyano,
(6) OR^{t1}, wherein R^{t1} is C₃₋₆ cycloalkyl,
(7) COR¹²,
(8) SO₂R¹³,
(9) NR^{t2}R^{t3}, wherein R^{t2} is
(a) hydrogen,
(b) C₁₋₆ alkyl, or
(c) C₁₋₄ haloalkyl, and
R^{t3} is
(a) hydrogen,
(b) C₁₋₆ alkyl,
(c) C₁₋₄ haloalkyl,
(d) COR¹⁴, or
(e) SO₂R¹⁵,
(10) CONR^{t4}R^{t5}, wherein R^{t4} and R^{t5} are independently
(a) hydrogen,
(b) C₁₋₆ alkyl, or
(c) C₁₋₄ haloalkyl, or alternatively, R^{t4} and R^{t5} may combine together with the nitrogen atom to which they attach to form 4-to 6-membered heterocycloalkyl comprising 1 or 2 heteroatoms independently selected from the group consisting of nitrogen and oxygen atoms, wherein the heterocycloalkyl may be optionally substituted with the same or different 1 or 2 halogen or hydroxy,
(11) SO₂NR^{t6}R^{t7}, wherein R^{t6} and R^{t7} are independently
(a) hydrogen,
(b) C₁₋₆ alkyl, or
(c) C₁₋₄ haloalkyl,
(12) COOR^{t8}, wherein R^{t8} is hydrogen or C₁₋₆ alkyl,
(13) oxo,
(14) C₃₋₆ cycloalkyl,
(15) phenyl,
(16) 4- to 7-membered heterocycloalkyl comprising 1 or 2 heteroatoms independently selected from the group consisting of nitrogen and oxygen atoms, wherein the heterocycloalkyl may be optionally substituted with the same or different 1 to 3 R¹⁶s, and
(17) 5- to 6-membered heteroaryl comprising 1 to 3 heteroatoms independently selected from the group consisting of nitrogen, oxygen, and sulfur atoms, wherein the heteroaryl may be optionally substituted with the same or different 1 to 3 R¹⁷s;
R^{11y} is each independently a substituent selected from the group consisting of:
(1) halogen,
(2) hydroxy,
(3) C₁₋₆ alkoxy, wherein the alkoxy may be optionally substituted with 1 to 3 substituents independently selected from the group consisting of:
(a) halogen,
(b) hydroxy,
(c) C₁₋₆ alkoxy, and
(d) C₃₋₆ cycloalkyl, wherein the cycloalkyl may be optionally substituted with the same or different 1 to 3 R^{1dy}s,
(4) cyano,
(5) NR²¹R²², wherein R²¹ is
(a) hydrogen,
(b) C₁₋₆ alkyl, or
(c) C₁₋₄ haloalkyl, and
R²² is
(a) hydrogen,
(b) C₁₋₆ alkyl,
(c) C₁₋₄ haloalkyl,
(d) COR^{1a}, or
(e) SO₂R^{1b},
(6) COR²³, wherein R²³ is C₁₋₆ alkyl or C₁₋₄ haloalkyl,
(7) SO₂R^{24y}, wherein R^{24y} is C₁₋₆ alkyl, wherein the alkyl may be optionally substituted with 1 to 3 substituents independently selected from the group consisting of:
(a) halogen, and
(b) C₃₋₆ cycloalkyl,
(8) COOR²⁵, wherein R²⁵ is hydrogen or C₁₋₆ alkyl,
(9) CONR²⁶R²⁷, wherein R²⁶ and R²⁷ are independently
(a) hydrogen,
(b) C₁₋₆ alkyl, or
(c) C₁₋₄ haloalkyl, or alternatively, R²⁶ and R²⁷ may combine together with the nitrogen atom to which they attach to form 5-to 6-membered heterocycloalkyl comprising 1 or 2 heteroatoms independently selected from the group consisting of nitrogen and oxygen atoms, wherein the heterocycloalkyl may be optionally substituted with the same or different 1 or 2 halogen atoms,
(10) SO₂NR²⁸R²⁹, wherein R²⁸ and R²⁹ are independently
(a) hydrogen,
(b) C₁₋₆ alkyl, or
(c) C₁₋₄ haloalkyl, or alternatively, R²⁸ and R²⁹ may combine together with the nitrogen atom to which they attach to form 4-to 7-membered heterocycloalkyl comprising 1 or 2 heteroatoms independently selected from the group consisting of nitrogen and oxygen atoms, wherein the heterocycloalkyl may be optionally substituted with the same or different 1 or 2 halogen atoms,
(11) C₃₋₆ cycloalkyl,
(12) 4- to 6-membered heterocycloalkyl comprising 1 or 2 heteroatoms independently selected from the group consisting of nitrogen and oxygen atoms, wherein the heterocycloalkyl may be optionally substituted with the same or different 1 to 3 R^{1c}s,
(13) phenyl,
(14) 5- to 6-membered heteroaryl comprising 1 or 2 heteroatoms independently selected from the group consisting of nitrogen and oxygen atoms, wherein the heteroaryl may be optionally substituted with the same or different 1 to 3 R^{1ey}s, and
(15) OR^{30y}, wherein R^{30y} is C₃₋₆ cycloalkyl which may be optionally substituted with the same or different 1 to 3 R^{1fy}s;
R¹⁷ and R^{1ey} are each independently a substituent selected from the group consisting of:
(1) halogen,
(2) C₁₋₆ alkyl,
(3) C₁₋₄ haloalkyl,
(4) C₁₋₆ alkoxy,
(5) cyano,
(6) C₃₋₆ cycloalkyl, and
(7) 4- to 6-membered heterocycloalkyl comprising an oxygen atom;
R¹², R¹³, R¹⁴, R¹⁵, R^{1a}, and R^{1b} are each independently a substituent selected from the group consisting of:
(1) C₁₋₆ alkyl,
(2) C₁₋₄ haloalkyl,
(3) C₃₋₆ cycloalkyl,
(4) 4- to 6-membered heterocycloalkyl comprising an oxygen atom,
(5) phenyl, and
(6) 5- to 6-membered heteroaryl comprising 1 or 2 heteroatoms independently selected from the group consisting of nitrogen and oxygen atoms;
R¹⁶, R^{1c}, R^{1dy}, and R^{1fy} are each independently a substituent selected from the group consisting of:
(1) halogen,
(2) hydroxy,
(3) C₁₋₆ alkyl,
(4) C₁₋₄ haloalkyl,
(5) C₁₋₆ alkoxy, wherein the alkoxy may be optionally substituted with the same or different 1 to 3 halogen atoms, and
(6) oxo.

2. The compound according to claim 1, or a pharmaceutically acceptable salt thereof, wherein
R^{Dy} and R^{Ey} are each independently
(1) hydrogen,
(2) C₁₋₆ alkyl, wherein the alkyl may be optionally substituted with the same or different 1 to 3 R^{d1}s,
(3) C₃₋₆ cycloalkyl,
(4) 4- to 7-membered heterocycloalkyl comprising 1 to 3 heteroatoms independently selected from the group consisting of nitrogen and oxygen atoms, or
(5) 5- to 6-membered heteroaryl comprising 1 to 3 heteroatoms independently selected from the group consisting of nitrogen and oxygen atoms, wherein the heteroaryl may be optionally substituted with C₁₋₄ alkyl,
or alternatively,
R^{Dy} and R^{Ey} may combine together with the nitrogen atom to which they attach to form
(a) 4- to 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s1y}s and / or may be fused with a 5- to 6-membered heteroaromatic ring comprising 1 to 3 nitrogen atoms wherein the heteroaromatic ring may be optionally substituted with the same or different 1 or 2 R^{d3}s,
(b) 7- to 11-membered spiro heterocycloalkyl comprising 1 to 3 heteroatoms independently selected from the group consisting of nitrogen and oxygen atoms, wherein the spiro heterocycloalkyl may be optionally substituted with the same or different 1 to 3 R^{d4}s,
(c) 6- to 10-membered fused heterocycloalkyl comprising 1 or 2 heteroatoms independently selected from the group consisting of nitrogen and oxygen atoms, or
(d) 5- to 9-membered bridged heterocycloalkyl comprising 1 or 2 heteroatoms independently selected from the group consisting of nitrogen and oxygen atoms,
R^{F} is
(1) halogen,
(2) C₁₋₄ alkyl,
(3) C₁₋₄ haloalkyl,
(4) C₁₋₄ alkoxy, wherein the alkoxy may be optionally substituted with the same or different 1 to 3 halogen atoms, or
(5) C₃₋₅ cycloalkyl,
R^{G} is each independently a substituent selected from the group consisting of:
(1) halogen,
(2) C₁₋₆ alkyl, wherein the alkyl may be optionally substituted with:
(a) C₁₋₄ alkoxy, or
(b) cyano,
(3) C₁₋₄ haloalkyl,
(4) C₁₋₄ alkoxy, wherein the alkoxy may be optionally substituted with the same or different 1 to 3 halogen atoms,
(5) cyano,
(6) pentafluorosulfanyl, and
(7) C₃₋₇ cycloalkyl, wherein the cycloalkyl may be optionally substituted with the same or different 1 or 2 R^{s2}s, or alternatively,
in the case where R^{F} and R^{G} or two R^{G}s are substituted on neighboring atoms, then the R^{F} and R^{G} and/or the two R^{G}s may combine together with the atoms to which they attach to form:
(a) a C₅₋₆ cycloalkene ring,
(b) a 5- to 7-membered heterocycloalkene ring comprising 1 or 2 heteroatoms independently selected from the group consisting of nitrogen and oxygen atoms, wherein the heterocycloalkene ring may be optionally subsituted with the same or different 1 or 2 R^{c2}s, or
(c) a 5- to 7-membered heteroaromatic ring comprising 1 or 2 heteroatoms independently selected from the group consisting of nitrogen and oxygen atoms,
so that Ring Cy may form a bi- or tri-cyclic fused ring group;,
R^{c2} is each independently C₁₋₄ alkyl,
R^{d1} is each independently a substituent selected from the group consisting of:
(1) halogen,
(2) C₁₋₄ alkoxy,
(3) cyano,
(4) CONR^{e2}R^{e3}, wherein R^{e2} and R^{e3} are independently
(a) hydrogen, or
(b) C₁₋₄ alkyl,
(5) SO₂R^{e5}, wherein R^{e5} is C₁₋₄ alkyl,
(6) NR^{e6}R^{e7}, wherein R^{e6} is
(a) hydrogen, or
(b) C₁₋₄ alkyl, and
R^{e7} is
(a) hydrogen,
(b) C₁₋₄ alkyl, or
(c) COR^{e8}, wherein R^{e8} is C₁₋₄ alkyl,
(7) C₃₋₆ cycloalkyl,
(8) phenyl, and
(9) 5- to 6-membered heteroaryl comprising 1 or 2 heteroatoms independently selected from the group consisting of nitrogen, oxygen, and sulfur atoms, wherein the heteroaryl may be optionally substituted with C₁₋₄ alkyl,
R^{d3} is each independently a substituent selected from the group consisting of:
(1) C₁₋₄ alkyl, and
(2) COOR^{g2}, wherein R^{g2} is hydrogen or C₁₋₄ alkyl;
R^{d4} is each independently a substituent selected from the group consisting of:
(1) oxo, and
(2) C₁₋₄ alkyl,
R^{s1y} and R^{s2} are each independently a substituent selected from the group consisting of:
(1) halogen,
(2) hydroxy,
(3) C₁₋₆ alkyl, wherein the alkyl may be optionally substituted with the same or different 1 to 3 R^{11y}s,
(4) C₁₋₆ alkoxy,
(5) cyano,
(6) COR¹²,
(7) SO₂R¹³,
(8) NR^{t2}R^{t3}, wherein R^{t2} is
(a) hydrogen, or
(b) C₁₋₆ alkyl, and
R^{t3} is
(a) hydrogen,
(b) C₁₋₆ alkyl,
(c) COR¹⁴, or
(d) SO₂R¹⁵,
(9) CONR^{t4}R^{t5}, wherein R^{t4} and R^{t5} are independently
(a) hydrogen, or
(b) C₁₋₆ alkyl, or alternatively, R^{t4} and R^{t5} may combine together with the nitrogen atom to which they attach to form 4-to 6-membered heterocycloalkyl comprising 1 or 2 heteroatoms independently selected from the group consisting of nitrogen and oxygen atoms,
(10) SO₂NR^{t6}R^{t7}, wherein R^{t6} and R^{t7} are independently
(a) hydrogen, or
(b) C₁₋₆ alkyl,
(11) COOR^{t8}, wherein R^{t8} is hydrogen or C₁₋₄ alkyl,
(12) oxo,
(13) phenyl, and
(14) 5- to 6-membered heteroaryl comprising 1 to 3 heteroatoms independently selected from the group consisting of nitrogen, oxygen, and sulfur atoms, wherein the heteroaryl may be optionally substituted with the same or different 1 to 3 R¹⁷s;
R^{11y} is each independently a substituent selected from the group consisting of:
(1) halogen,
(2) hydroxy,
(3) C₁₋₆ alkoxy, wherein the alkoxy may be optionally substituted with 1 to 3 substituents independently selected from the group consisting of:
(a) halogen,
(b) hydroxy,
(c) C₁₋₄ alkoxy, and
(d) C₃₋₆ cycloalkyl, wherein the cycloalkyl may be optionally substituted with the same or different 1 to 3 R^{1dy}s,
(4) cyano,
(5) NR²¹R²², wherein R²¹ and R²² are each independently
(a) hydrogen, or
(b) C₁₋₄ alkyl,
(6) SO₂R^{24y}, wherein R^{24y} is C₁₋₆ alkyl, wherein the alkyl may be optionally substituted with 1 to 3 substituents independently selected from the group consisting of:
(a) halogen, and
(b) C₃₋₆ cycloalkyl,
(7) 4- to 6-membered heterocycloalkyl comprising 1 or 2 heteroatoms independently selected from the group consisting of nitrogen and oxygen atoms, wherein the heterocycloalkyl may be optionally substituted with the same or different 1 to 3 R^{1c}s, and
(8) 5- to 6-membered heteroaryl comprising 1 or 2 heteroatoms independently selected from the group consisting of nitrogen and oxygen atoms, wherein the heteroaryl may be optionally substituted with the same or different 1 to 3 R^{1ey}s;
R¹⁷ and R^{1ey} are each independently a substituent selected from the group consisting of:
(1) C₁-₆ alkyl,
(2) C₁₋₄ haloalkyl, and
(3) C₁₋₆ alkoxy;
R¹², R¹³, R¹⁴, and R¹⁵ are each independently a substituent selected from the group consisting of:
(1) C₁₋₆ alkyl,
(2) C₁₋₄ haloalkyl,
(3) C₃₋₆ cycloalkyl, and
(4) 4- to 6-membered heterocycloalkyl comprising an oxygen atom;
R^{1dy} is each independently a substituent selected from the group consisting of:
(1) halogen,
(2) hydroxy, and
(3) C₁₋₄ alkyl.

3. The compound according to claim 1 or 2, or a pharmaceutically acceptable salt thereof, wherein
R^{F} is
(1) methyl, ethyl, isopropyl, or tert-butyl,
(2) C₁₋₄ haloalkyl,
(3) C₁₋₄ alkoxy, wherein the alkoxy may be optionally substituted with the same or different 1 to 3 halogen atoms, or
(4) C₃₋₅ cycloalkyl,
R^{G} is each independently a substituent selected from the group consisting of:
(1) halogen,
(2) C₁₋₆ alkyl, wherein the alkyl may be optionally substituted with:
(a) C₁₋₄ alkoxy, or
(b) cyano,
(3) C₁₋₄ haloalkyl,
(4) C₁-₄ alkoxy, wherein the alkoxy may be optionally substituted with the same or different 1 to 3 halogen atoms,
(5) cyano,
(6) pentafluorosulfanyl, and
(7) C₃-₇ cycloalkyl, wherein the cycloalkyl may be optionally substituted with the same or different 1 or 2 R^{s2}s, or alternatively,
in the case where R^{F} and R^{G} or two R^{G}s are substituted on neighboring atoms, then the R^{F} and R^{G} and/or the two R^{G}s may combine together with the atoms to which they attach to form:
(a) a C₅₋₆ cycloalkene ring,
(b) a 5- to 7-membered heterocycloalkene ring comprising 1 or 2 heteroatoms independently selected from the group consisting of nitrogen and oxygen atoms, wherein the heterocycloalkene ring may be optionally subsituted with the same or different 1 or 2 R^{c2}s, or
(c) a 5- to 7-membered heteroaromatic ring comprising 1 or 2 heteroatoms independently selected from the group consisting of nitrogen and oxygen atoms,
so that Ring Cy may form a bi- or tri-cyclic fused ring group;,
R^{s2} is each independently a substituent selected from the group consisting of:
(1) C₁₋₄ alkyl, and
(2) cyano;
R^{11y} is each independently a substituent selected from the group consisting of:
(1) halogen,
(2) hydroxy,
(3) C₁₋₆ alkoxy, wherein the alkoxy may be optionally substituted with 1 to 3 substituents independently selected from the group consisting of:
(a) halogen,
(b) hydroxy,
(c) C₁₋₄ alkoxy, and
(d) C₃₋₆ cycloalkyl, wherein the cycloalkyl may be optionally substituted with the same or different 1 to 3 R^{1dy}s,
(4) cyano,
(5) NR²¹R²², wherein R²¹ and R²² are each independently
(a) hydrogen, or
(b) C₁₋₄ alkyl,
(6) SO₂ R^{24y}, wherein R^{24y} is C₁₋₆ alkyl, wherein the alkyl may be optionally substituted with 1 to 3 substituents independently selected from the group consisting of:
(a) halogen, and
(b) C₃₋₆ cycloalkyl, and
(7) 5- to 6-membered heteroaryl comprising 1 or 2 heteroatoms independently selected from the group consisting of nitrogen and oxygen atoms, wherein the heteroaryl may be optionally substituted with the same or different 1 to 3 R^{1ey}s_{.}

4. The compound according to any one of claims 1 to 3, or a pharmaceutically acceptable salt thereof, wherein
a partial structure: 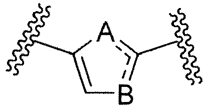 is a group of the following formula:

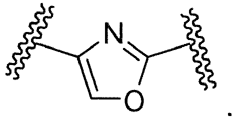

5. The compound according to any one of claims 1 to 4, or a pharmaceutically acceptable salt thereof, wherein
R^{Dy} and R^{Ey} combine together with the nitrogen atom to which they attach to form
(1) 4- to 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s1y} s and / or may be fused with a 5- to 6-membered heteroaromatic ring comprising 1 to 3 nitrogen atoms wherein the heteroaromatic ring may be optionally substituted with the same or different 1 or 2 R^{d3}s,
(2) 7- to 11-membered spiro heterocycloalkyl comprising 1 to 3 heteroatoms independently selected from the group consisting of nitrogen and oxygen atoms, wherein the spiro heterocycloalkyl may be optionally substituted with the same or different 1 to 3 R^{d4}s,
(3) 6- to 10-membered fused heterocycloalkyl comprising 1 or 2 heteroatoms independently selected from the group consisting of nitrogen and oxygen atoms, or
(4) 5- to 9-membered bridged heterocycloalkyl comprising 1 or 2 heteroatoms independently selected from the group consisting of nitrogen and oxygen atoms.

6. The compound according to any one of claims 1 to 5, or a pharmaceutically acceptable salt thereof, wherein
R^{Dy} and R^{Ey} combine together with the nitrogen atom to which they attach to form 4- to 7-membered heterocycloalkyl comprising 1 or 2 heteroatoms independently selected from the group consisting of nitrogen, oxygen, and sulfur atoms, wherein the heterocycloalkyl may be optionally substituted with the same or different 1 to 3 R^{s1y}s and / or may be fused with a 5- to 6-membered heteroaromatic ring comprising 1 to 3 nitrogen atoms wherein the heteroaromatic ring may be optionally substituted with the same or different 1 or 2 R^{d3}s.

7. The compound according to any one of claims 1 to 6, having a structure of the following formula [II]: 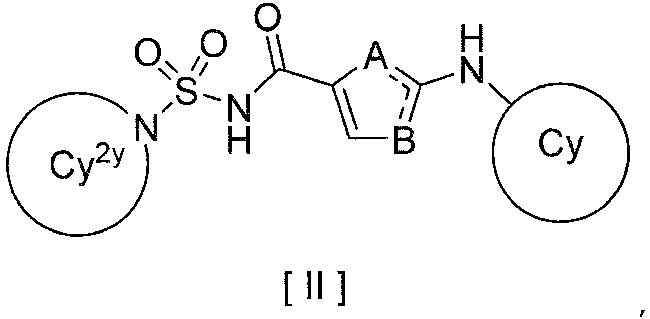 or a pharmaceutically acceptable salt thereof,
wherein
Ring Cy^{2y} is 4- to 7-membered heterocycloalkyl comprising 1 or 2 heteroatoms independently selected from the group consisting of nitrogen, oxygen, and sulfur atoms, wherein the heterocycloalkyl comprises at least one nitrogen atom and may be optionally substituted with the same or different 1 to 3 R^{s1y}s ,
R^{s1y}, Ring Cy, and the partial structure comprising A and B are those as defined in claim 1.

8. The compound according to any one of claims 1 to 7, having a structure of the following formula [III]: 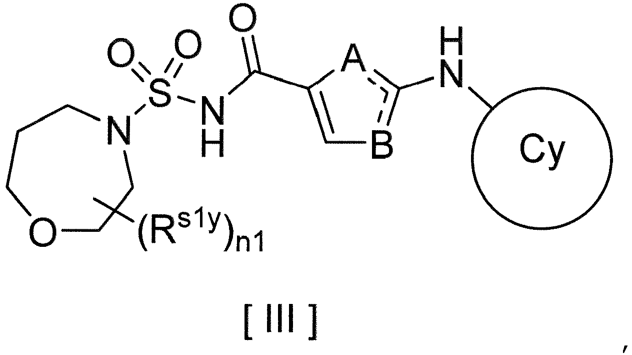 or a pharmaceutically acceptable salt thereof,
wherein
n1 is an integer from 0 to 3,
R^{s1y}, Ring Cy, and the partial structure comprising A and B are those as defined in claim 1.

9. The compound according to any one of claims 1 to 8, having a structure of the following formula [IV]: 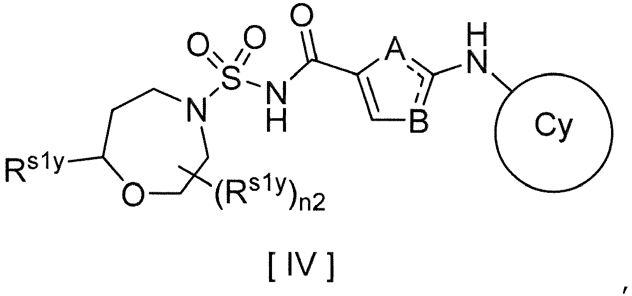 or a pharmaceutically acceptable salt thereof,
wherein
n2 is an integer from 0 to 2,
R^{s1y}, Ring Cy, and the partial structure comprising A and B are those as defined in claim 1.

10. The compound according to any one of claims 1 to 9, or a pharmaceutically acceptable salt thereof, wherein Ring Cy is the following formula: 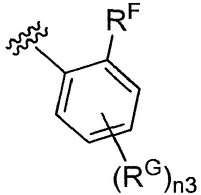 wherein
n3 is an integer from 0 to 4,
R^{F} and R^{G} are those as defined in claim 1.

11. The compound according to any one of claims 1 to 10, or a pharmaceutically acceptable salt thereof, wherein Ring Cy is the following formula: 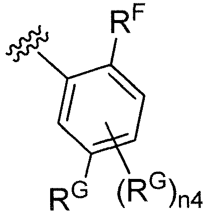 wherein
n4 is an integer from 0 to 3,
R^{F} and R^{G} are those as defined in claim 1.

12. The compound according to any one of claims 1 to 11, or a pharmaceutically acceptable salt thereof, wherein Ring Cy is the following formula: 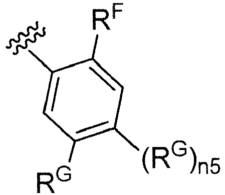 wherein
n5 is an integer of 0 or 1,
R^{F} and R^{G} are those as defined in claim 1.

13. The compound according to claim 1 selected from: 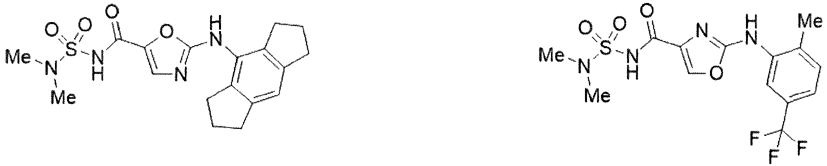 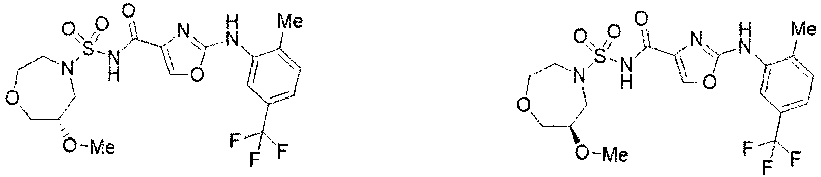 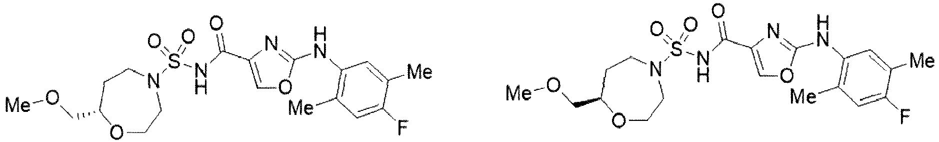 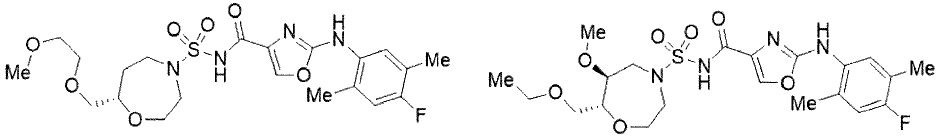 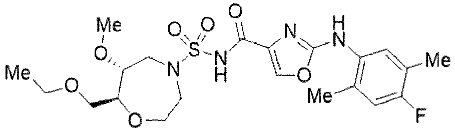 or , or a pharmaceutically acceptable salt thereof.

14. A pharmaceutical composition comprising a compound according to any one of claims 1 to 13, or a pharmaceutically acceptable salt thereof, and a pharmaceutically acceptable carrier.

15. An NLRP3 inflammasome inhibitor comprising a compound according to any one of claims 1 to 13, or a pharmaceutically acceptable salt thereof.

16. A medicament for treating or preventing a disease selected from the group consisting of multiple sclerosis, chronic kidney disease and inflammatory bowel disease, comprising a compound according to any one of claims 1 to 13, or a pharmaceutically acceptable salt thereof.

17. The medicament according to claim 16, wherein the inflammatory bowel disease is ulcerative colitis or Crohn's disease.

18. A method for inhibiting NLRP3 inflammasome, comprising administering a therapeutically effective amount of a compound according to any one of claims 1 to 13, or a pharmaceutically acceptable salt thereof, to a mammal.

19. A method for treating or preventing a disease selected from the group consisting of multiple sclerosis, chronic kidney disease and inflammatory bowel disease, comprising administering a therapeutically effective amount of a compound according to any one of claims 1 to 13, or a pharmaceutically acceptable salt thereof, to a mammal.

20. The method according to claim 19, wherein the inflammatory bowel disease is ulcerative colitis or Crohn's disease.

21. Use of a compound according to any one of claims 1 to 13, or a pharmaceutically acceptable salt thereof, in the manufacture of an NLRP3 inflammasome inhibitor.

22. Use of a compound according to any one of claims 1 to 13, or a pharmaceutically acceptable salt thereof, in the manufacture of a medicament for treating or preventing a disease selected from the group consisting of multiple sclerosis, chronic kidney disease and inflammatory bowel disease.

23. The use according to claim 22, wherein the inflammatory bowel disease is ulcerative colitis or Crohn's disease.

24. A compound according to any one of claims 1 to 13, or a pharmaceutically acceptable salt thereof, for use in inhibiting NLRP3 inflammasome.

25. A compound according to any one of claims 1 to 13, or a pharmaceutically acceptable salt thereof, for use in treating or preventing a disease selected from the group consisting of multiple sclerosis, chronic kidney disease and inflammatory bowel disease.

26. The compound according to claim 25, or a salt thereof, wherein the inflammatory bowel disease is ulcerative colitis or Crohn's disease.
